# EUROPÄISCHE PATENTSCHRIFT

(11) **EP 3 521 401 B1**
(45) Veröffentlichungstag und Bekanntmachung des Hinweises auf die Patenterteilung: **21.04.2021**
(21) Anmeldenummer: 19155191.0
(22) Anmeldetag: 04.02.2019
(51) Int. Cl.: C09K 19/02, C09K 19/04, C09K 19/12, C09K 19/30

(54) **VERBINDUNGEN ZUR HOMÖOTROPEN AUSRICHTUNG VON FLÜSSIGKRISTALLINEN MEDIEN**
COMPOUNDS FOR HOMEOTROPIC ALIGNMENT OF LIQUID CRYSTALINE MEDIA
COMPOSÉS D'ORIENTATION HOMÉOTROPE DES MILIEUX À BASE DE CRISTAUX LIQUIDES

(30) Priorität: 05.02.2018 DE 102018000894
(43) Veröffentlichungstag der Anmeldung: 07.08.2019
(73) Patentinhaber: Merck Patent GmbH, 64293 Darmstadt (DE)
(72) Erfinder: Klasen-Memmer, Melanie, 67259 HEUCHELHEIM (DE); Plummer, Edward, 60314 FRANKFURT AM MAIN (DE); Fortte, Rocco, 65933 FRANKFURT AM MAIN (DE); Haensel, Helmut, 64367 MUEHLTAL (DE); Uebel, Timo, 64291 DARMSTADT (DE); Lehmann, Tamara, 64853 OTZBERG (DE)

(56) Entgegenhaltungen:
- EP-A1- 3 263 673
- EP-A2- 2 918 658
- WO-A1-2017/041893
- WO-A1-2017/045740
- DE-A1-102011 108 708
- DE-A1-102015 008 172

## Beschreibung

Die vorliegende Erfindung betrifft flüssigkristalline Medien (FK-Medien) enthaltend neue selbst-ausrichtende Mesogene (Selbstorientierungsadditive), die eine homöotrope (vertikale) Ausrichtung der FK-Medien an einer Oberfläche oder den Zellwänden einer Flüssigkristallanzeige (FK-Anzeige) bewirken. Die neuen Selbstorientierungsadditive besitzen Fünfringsysteme. Die Erfindung umfasst auch FK-Anzeigen mit homöotroper Ausrichtung des flüssigkristallinen Mediums (FK-Mediums) ohne konventionelle Orientierungsschichten.

Das Prinzip der elektrisch kontrollierten Doppelbrechung, der ECB-Effekt (electrically controlled birefringence) oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten K₃/K₁, hohe Werte für die optische Anisotropie Δn und Werte für die dielektrische Anisotropie von Δε ≤ -0,5 aufweisen müssen, um für hochinformative Anzeigeelemente basierend auf dem ECB-Effekt eingesetzt werden zu können. Auf dem ECB-Effekt basierende elektrooptische Anzeigeelemente weisen eine homöotrope Randorientierung auf (VA-Technologie = Vertical Aligned).

Anzeigen, die den ECB-Effekt verwenden, haben sich als sogenannte VAN-(Vertically Aligned Nematic) Anzeigen beispielsweise in den Bauformen MVA-(Multi-Domain Vertical Alignment, z.B.: Yoshide, H. et al., Vortrag 3.1: "MVA LCD for Notebook or Mobile PCs ...", SID 2004 International Symposium, Digest of Technical Papers, XXXV, Buch I, S. 6 bis 9 und Liu, C.T. et al., Vortrag 15.1: "A 46-inch TFT-LCD HDTV Technnology ...", SID 2004 International Symposium, Digest of Technical Papers, XXXV, Buch II, S. 750 bis 753), PVA- (Patterned Vertical Alignment, z.B.: Kim, Sang Soo, Vortrag 15.4: "Super PVA Sets New State-of-the-Art for LCD-TV", SID 2004 International Symposium, Digest of Technical Papers, XXXV, Buch II, S. 760 bis 763), ASV- (Advanced Super View, z.B.: Shigeta, Mitzuhiro und Fukuoka, Hirofumi, Vortrag 15.2: "Development of High Quality LCDTV", SID 2004 International Symposium, Digest of Technical Papers, XXXV, Buch II, S. 754 bis 757) Anzeigen, neben IPS- (In Plane Switching) (z.B.: Yeo, S.D., Vortrag 15.3: "A LC Display for the TV Application", SID 2004 International Symposium, Digest of Technical Papers, XXXV, Buch II, S. 758 & 759) und den lange bekannten TN- (Twisted Nematic) Anzeigen, als eine der drei zur Zeit wichtigsten neueren Typen von Flüssigkristallanzeigen, insbesondere für Fernsehanwendungen, etabliert. In allgemeiner Form werden die Technologien z.B. in Souk, Jun, SIDSeminar 2004, Seminar M-6: "Recent Advances in LCD Technology", Seminar Lecture Notes, M-6/1 bis M-6/26 und Miller, lan, SIDSeminar 2004, Seminar M-7: "LCD-Television", Seminar Lecture Notes, M-7/1 bis M-7/32, verglichen. Obwohl die Schaltzeiten moderner ECB-Anzeigen durch Ansteuerungsmethoden mit Übersteuerung (overdrive) bereits deutlich verbessert wurden, z.B.: Kim, Hyeon Kyeong et al., Vortrag 9.1: "A 57-in. Wide UXGA TFT-LCD for HDTV Application", SID 2004 International Symposium, Digest of Technical Papers, XXXV, Buch I, S. 106 bis 109, ist die Erzielung von videotauglichen Schaltzeiten, insbesondere beim Schalten von Graustufen, immer noch ein noch nicht zufriedenstellend gelöstes Problem.

Mit der Erzeugung der VA-Displays mit zwei oder mehr Domänen unterschiedlicher Vorzugsrichtung ist ein beträchtlicher Aufwand verbunden. Ein Ziel dieser Erfindung ist es, die Herstellprozesse und die Anzeigevorrichtungen selbst zu vereinfachen, ohne die Vorteile der VA-Technik, wie relativ schnelle Schaltzeiten und gute Blickwinkelabhängigkeit, aufzugeben.

VA-Anzeigen die FK-Medien mit positiver dielektrischer Anisotropie enthalten, werden in S.H. Lee et al. Appl. Phys. Lett. (1997), 71, 2851-2853 beschrieben. Diese Anzeigen verwenden auf einer Substratoberfläche angeordnete Interdigitalelektroden (In-plane Ansteuerelektroden-Konfiguration kammförmiger Struktur), wie sie unter anderem bei den kommerziell erhältlichen lPS-(in-plane switching) Anzeigen zum Einsatz kommen (wie z.B. in DE 40 00 451 und EP 0 588 568 offenbart), und weisen eine homöotrope Anordnung des Flüssigkristallmediums auf, die zu einer planaren Anordnung beim Anlegen einer elektrischen Feldes wechselt.

Weiterentwicklungen der oben genannten Anzeige sind zum Beispiel in K.S. Hun et al. J. Appl. Phys. (2008), 104, 084515 (DSIPS: 'double-side in-plane switching' für Verbesserungen von Treiberspannung und Transmission), M. Jiao et al. App. Phys. Lett (2008), 92, 111101 (DFFS: 'dual fringe field switching' für verbesserte Schaltzeiten) und Y.T. Kim et al. Jap. J. App. Phys. (2009), 48, 110205 (VAS: 'viewing angle switchable' LCD) zu finden. Darüber hinaus sind VA-IPS-Anzeigen auch unter dem Namen HT-VA (High transmittance VA) bekannt.

Bei allen solchen Anzeigen (hier nachfolgend allgemein als VA-IPS-Anzeigen bezeichnet) ist auf beiden Substratoberflächen eine Orientierungsschicht zur homöotropen Ausrichtung des FK-Mediums aufgebracht, deren Erzeugung bisher mit einem beträchtlichen Aufwand verbunden ist.

Ein Ziel dieser Erfindung ist es, die Herstellprozesse selbst zu vereinfachen, ohne die Vorteile der VA- oder VA-IPS-Technik, wie relativ schnelle Schaltzeiten, gute Blickwinkelabhängigkeit und hohen Kontrast aufzugeben.

Für die technische Anwendung dieser Effekte in elektrooptischen Anzeigeelementen werden FK-Phasen benötigt, die einer Vielzahl von Anforderungen genügen müssen. Besonders wichtig sind hier die chemische Beständigkeit gegenüber Feuchtigkeit, Luft, den Materialien in den Substratoberflächen und physikalischen Einflüssen wie Wärme, Strahlung im infraroten, sichtbaren und ultravioletten Bereich und elektrische Gleich- und Wechselfelder.

Ferner wird von technisch verwendbaren FK-Phasen eine flüssigkristalline Mesophase in einem geeigneten Temperaturbereich und eine niedrige Viskosität gefordert.

VA- und VA-IPS-Anzeigen sollen im Allgemeinen einen sehr hohen spezifischen Widerstand bei gleichzeitig großem Arbeitstemperaturbereich, kurze Schaltzeiten und niedriger Schwellenspannung, mit deren Hilfe verschiedene Graustufen erzeugt werden können, besitzen.

In den herkömmlichen VA- und VA-IPS-Displays sorgt eine Polyimidschicht auf den Substratoberflächen für die homöotrope Orientierung des Flüssigkristalls. Die Herstellung einer geeigneten Orientierungsschicht im Display erfordert einen erheblichen Aufwand. Außerdem können Wechselwirkungen der Orientierungsschicht mit dem FK-Medium den elektrischen Widerstand der Anzeige verschlechtern. Wegen solcher möglichen Wechselwirkungen reduziert sich die Zahl der geeigneten Flüssigkristallkomponenten erheblich. Daher wäre es erstrebenswert die homöotrope Ausrichtung des FK-Mediums ohne Polyimid zu erreichen.

Der Nachteil der häufig verwendeten Aktivmatrix-TN-Anzeigen beruht in ihrem vergleichsweise niedrigen Kontrast, der relativ hohen Blickwinkel-abhängigkeit und der Schwierigkeit in diesen Anzeigen Graustufen zu erzeugen.

Wesentlich bessere Blickwinkelabhängigkeiten weisen VA-Displays auf und werden daher hauptsächlich für Fernseher und Monitore verwendet.

Eine Weiterentwicklung stellen die sogenannten PS-bzw. PSA-Anzeigen ("Polymer Stabilized" bzw. "Polymer Sustained Alignment") dar. Ohne nennenswerte Einbußen sonstiger Parameter, wie insbesondere der günstigen Blickwinkelabhängigkeit des Kontrastes, zeichnen sich die PSA-Anzeigen durch die Verkürzung der Schaltzeiten aus.

In diesen Anzeigen wird dem FK-Medium eine geringe Menge (zum Beispiel 0,3 Gew.-%, typischerweise <1 Gew.-%) einer oder mehrerer polymerisierbarer Verbindung(en) zugesetzt, welche nach Einfüllen in die FK-Zelle mit oder ohne angelegte elektrische Spannung zwischen den Elektroden *in situ* polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als reaktive Mesogene oder "RM"s bezeichnet, zur FK-Mischung erwiesen. Die PSA-Technik wird bisher hauptsächlich für FK-Medien mit negativer dielektrischer Anisotropie eingesetzt.

Mittlerweile wird das Prinzip der Polymerstabilisierung in diversen klassischen FK-Anzeigen angewendet. So sind beispielsweise PS-VA-, PS-OCB-, PS-IPS-, PS-FFS- und PS-TN-Anzeigen bekannt. Die Polymerisation der polymerisierbaren Verbindung(en) erfolgt bei PS-VA- und PS-OCB-Anzeigen vorzugsweise bei angelegter elektrischer Spannung, bei PS-IPS-Anzeigen mit oder ohne angelegte elektrische Spannung. Wie man in Testzellen nachweisen kann, führt das PS(A)-Verfahren zu einem 'pretilt' in der Zelle. Bei PS-OCB-Anzeigen beispielsweise kann man erreichen, dass die Bend-Struktur stabilisiert wird, so dass man ohne Offset-Spannung auskommt oder diese reduzieren kann. Im Falle von PS-VA-Anzeigen wirkt sich der 'pretilt' positiv auf die Schaltzeiten aus. Für PS-VA-Anzeigen kann ein Standard- Pixel- und Elektroden-Layout verwendet werden. Darüber hinaus kann man aber beispielsweise auch mit nur einer strukturierten Elektrodenseite und ohne 'Protrusions' auskommen, was die Herstellung wesentlich vereinfacht und gleichzeitig zu einem sehr guten Kontrast bei sehr guter Lichtdurchlässigkeit führt.

PS-VA-Anzeigen sind beispielsweise in JP 10-036847 A, EP 1 170 626 A2, US 6,861,107, US 7,169,449, US 2004/0191428 A1, US 2006/0066793 A1 und US 2006/0103804 A1 beschrieben. PS-OCB-Anzeigen sind beispielsweise in T.-J- Chen et al., Jpn. J. Appl. Phys. (2006), 45, 2702-2704 und S. H. Kim, L.-C- Chien, Jpn. J. Appl. Phys. (2004), 43, 7643-7647 beschrieben. PS-IPS-Anzeigen sind zum Beispiel in US 6,177,972 und Appl. Phys. Lett. (1999), 75(21), 3264 beschrieben. PS-TN-Anzeigen sind zum Beispiel in Optics Express (2004), 12(7), 1221 beschrieben. PS-VA-IPS Anzeigen sind zum Beispiel in WO 2010/089092 A1 offenbart.

PS-Anzeigen können ebenso wie die oben beschriebenen konventionellen FK-Anzeigen als Aktivmatrix- oder Passivmatrix-Anzeigen (PM) betrieben werden. Bei Aktivmatrix-Anzeigen erfolgt die Ansteuerung einzelner Bildpunkte üblicherweise durch integrierte, nicht-lineare aktive Elemente wie beispielsweise Transistoren (z.B. Dünnfilmtransistoren, engl. 'thin film transistor' bzw. "TFT"), bei Passivmatrix-Anzeigen üblicherweise nach dem Multiplex-Verfahren, wobei beide Verfahren aus dem Stand der Technik bekannt sind.

Insbesondere für Monitor- und vor allem TV-Anwendungen ist nach wie vor die Optimierung der Schaltzeiten, wie aber auch des Kontrastes und der Luminanz (also auch Transmission) der FK-Anzeige gefragt. Hier kann das PSA-Verfahren entscheidende Vorteile bringen. Insbesondere bei PS-VA-Anzeigen kann man ohne nennenswerte Einbußen sonstiger Parameter eine Verkürzung der Schaltzeiten erreichen, die mit einem in Testzellen messbaren 'pretilt' korrelieren.

Im Stand der Technik werden für PS-VA beispielsweise polymerisierbare Verbindungen der folgenden Formel verwendet 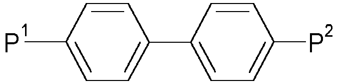 worin P^{1/2} eine polymerisierbare Gruppe, üblicherweise eine Acrylat- oder Methacrylatgruppe bedeutet, wie beispielsweise in US 7,169,449 beschrieben.

Der Aufwand für das Erzeugen einer Polyimidschicht, Behandlung der Schicht und Verbesserung mit Erhebungen oder Polymerschichten, ist relativ groß. Eine vereinfachende Technologie ist daher wünschenswert, die einerseits die Produktionskosten verringert und andererseits die Bildqualität (Blickwinkelabhängigkeit, Kontrast, Schaltzeiten) zu optimieren hilft.

Die Druckschriften EP 2918658 A2, EP 3263673 A1, DE 102011108708 A1, DE 102015008172 A1, WO 2017/045740 A1 und WO 2017/041893 A1 beschreiben selbstausrichtende, teils polymerisierbare Mesogene mit einer Ankergruppe (z. B. OH, SH) und flüssigkristalline Medien enthaltend solche Additive. Die dort offenbarten Additive besitzen eine andere bzw. allgemeinere Struktur wie die erfindungsgemäßen Verbindungen. Die vorliegenden Verbindungen besitzen fünf Ringsysteme gemäß Formel I-A in linearer Abfolge.

Die bestehenden Ansätze um zu VA-Displayanwendungen ohne Polyimidschicht zu gelangen sind jedoch noch nicht vollständig zufrieden stellend. Für kommerzielle Displays werden hohe Anforderungen an die Prozessierbarkeit gestellt. Dafür benötigt werden Eigenschaften wie z. B. gute Löslichkeit, schnelle Tiltwinkeleinstellung, gute Tiltstabilität und eine niedrige Tendenz zu Mura-Defekten.

Die vorliegende Erfindung betrifft Verbindungen der folgenden Formel I-A, sowie ein FK-Medium enthaltend eine niedermolekulare, nicht polymerisierbare, flüssigkristalline Komponente und eine polymerisierbare oder polymerisierte Komponente umfassend eine oder mehrere Verbindungen der Formel I-A, wobei die polymerisierte Komponente durch Polymerisieren der polymerisierbaren Komponente erhältlich ist, 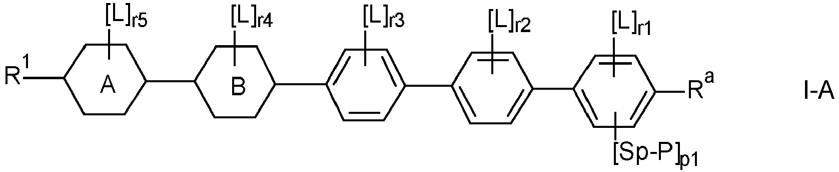 worin
- Ringe A, B: jeweils unabhängig einen Ring der Formeln der Formel 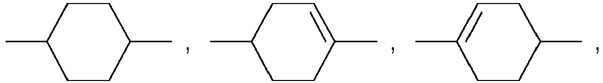 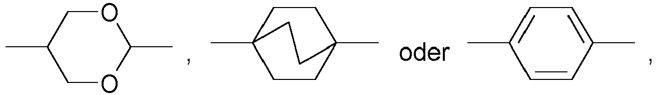
- L: jeweils unabhängig voneinander unverzweigtes oder verzweigtes Alkyl, Alkenyl, Alkoxy, Alkylcarbonyl, Alkoxycarbonyl, Alkylcarbonyloxy oder Alkoxycarbonyloxy mit 1 bis 25 C-Atomen, worin auch ein oder mehrere H-Atome durch F oder Cl ersetzt sein können, F, Cl, Br, I, -CN, -NO₂, -NCO, -NCS, -OCN, -SCN, -C(=O)N(R⁰)R⁰⁰ oder Cycloalkyl mit 3 bis 6 C-Atomen,
- r1: 0,
- r2: 1,
- r3: 1,
- r4: 0 oder 1,
- r5: 0 oder 1,
- P: eine polymerisierbare Gruppe,
- Sp: eine Abstandsgruppe (auch bezeichnet als Spacer oder Spacergruppe) oder eine Einfachbindung,
- p1: 1 oder 2,
- R¹: einen Alkylyrest mit 1 bis 25 C-Atomen, wobei in diesem Rest auch eine oder mehrere CH₂-Gruppen jeweils unabhängig voneinander durch -CH=CH-, -C≡C-, -CF₂O-, -OCF₂-, 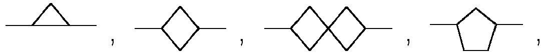 -O-, -S-, -CO-, -CO-O-, oder -O-CO- so ersetzt sein können, dass O/S-Atome nicht direkt miteinander verknüpft sind, und worin auch ein oder mehrere H-Atome durch F oder Cl ersetzt sein können, H,
oder eine Gruppe -Sp-P,
- R^{a}: eine Ankergruppe der Formel 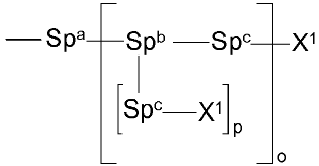 oder 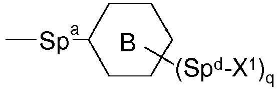
- p: 1 oder 2,
- q: 2 oder 3,
- B: ein substituiertes oder unsubstituiertes Ringsystem oder kondensiertes Ringsystem,
- o: 0 oder 1,
- X¹: unabhängig voneinander OH, SH, NH₂, NHR¹¹, NR¹¹₂, C(O)OH oder -CHO,
- R¹¹: Alkyl mit 1 bis 12 C-Atomen, welches linear oder verzweigt sein kann, und worin H durch Fluor oder Alkoxy mit 1 bis 8 C-Atomen substituiert sein kann, und
- Sp^{a}, Sp^{c}, Sp^{d}: jeweils unabhängig voneinander eine Abstandsgruppe oder eine Einfachbindung, und
- Sp^{b}: eine tri- oder tetravalente Gruppe
bedeuten.

Die polymerisierbare oder polymerisierte Komponente des FK-Medium enthält optional weitere polymerisierbare Verbindungen. Dabei kommen bevorzugt solche zum Einsatz, die für das PS-Prinzip geeignet sind.

Die erfindungsgemäßen Verbindungen der Formel I-A sind gemäß einer bevorzugten Ausführungsform polymerisierbar, in dem sie eine, zwei oder mehrere polymerisierbare Gruppen (P) umfassen. Eine bevorzugte Ausführungsform der Erfindung ist daher auch ein Polymer, das Monomere der Formel I umfasst, d.h. ein Polymer, das wenigstens teilweise aus entsprechendem Polymerisationsprodukt aufgebaut ist. Solch ein Polymer ist im Regelfall in einem flüssigkristallinen Medium homogen oder inhomogen verteilt oder ganz oder teilweise auf einem angrenzenden Substrat abgeschieden, wobei Mischformen dieser Zustände eingeschlossen sind.

Die Erfindung betrifft weiter eine FK-Anzeige enthaltend eine FK-Zelle mit zwei Substraten und mindestens zwei Elektroden, wobei mindestens ein Substrat lichtdurchlässig ist und mindestens ein Substrat eine oder zwei Elektroden aufweist, sowie einer zwischen den Substraten befindlichen Schicht eines erfindungsgemäßen FK-Mediums. Die FK-Anzeige ist vorzugsweise eine des PS-Typs.

Ein weiterer Gegenstand der Erfindung ist die Verwendung von Verbindungen der Formel I-A als Additiv für FK-Medien zur Herbeiführung einer homöotropen Orientierung gegenüber einer das FK-Medium begrenzenden Oberfläche.

Ein weiterer Aspekt der vorliegenden Erfindung ist ein Verfahren zur Herstellung eines erfindungsgemäßen FK-Medium, das dadurch gekennzeichnet ist, dass man eine oder mehrere optional polymerisierbare Selbstorientierungsadditive (Verbindungen der Formel I-A) mit einer niedermolekularen, flüssigkristallinen Komponente mischt und optional eine oder mehrere weitere polymerisierbare Verbindungen zugibt.

Ein weiterer Gegenstand der Erfindung ist ein Verfahren zu Herstellung einer FK-Anzeige enthaltend eine FK-Zelle mit zwei Substraten und mindestens zwei Elektroden, wobei mindestens ein Substrat lichtdurchlässig ist und mindestens ein Substrat eine oder zwei Elektroden aufweist, umfassend die Verfahrensschritte:
- Füllen der Zelle mit einem erfindungsgemäßen FK-Medium, wobei sich eine homöotrope (vertikale) Ausrichtung des FK-Mediums gegenüber den Substratoberflächen einstellt, und
- Polymerisieren der polymerisierbaren Komponente(n), optional unter Anlegen einer Spannung an die Zelle oder unter der Wirkung eines elektrischen Feldes, in einem oder mehreren Verfahrensschritten.

Die erfindungsgemäße Verwendung der Selbstorientierungsadditive als Additive von FK-Medien ist nicht an bestimmte FK-Medien gebunden. Das FK-Medium bzw. die darin enthaltene nicht-polymerisierbare Komponente kann positive oder negative dielektrische Anisotropie aufweisen. Das FK-Medium ist bevorzugt nematisch, da die meisten auf dem VA-Prinzip basierten Anzeigen nematische FK-Medien umfassen.

Das Selbstorientierungsadditiv wird im FK-Medium als Additiv eingebracht. Es bewirkt eine homöotrope Ausrichtung des Flüssigkristalls gegenüber den Substratoberflächen (wie z.B. eine Oberfläche aus Glas oder mit ITO oder mit Polyimid beschichtet). Es scheint in Anbetracht der Untersuchungen zu dieser Erfindung so, dass die polare Ankergruppe in Wechselwirkung mit der Substratoberfläche tritt. Dadurch richten sich die Selbstorientierungsadditive auf der Substratoberfläche aus und induzieren eine homöotrope Orientierung des angrenzenden FK-Mediums.

Im Speziellen werden Ankergruppen R^{a} vorgezogen, die nicht nur aus einer einfachen OH-Gruppe bestehen. Für die Ankergruppe R^{a} der Formel 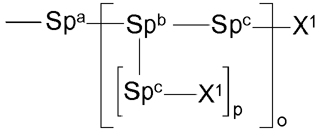 ist die Gruppe Sp^{a} für o = 0 daher bevorzugt eine Abstandsgruppe, und nicht nur eine Einfachbindung. Sp^{a} ist besonders bevorzugt eine unverzweigte oder verzweigte Alkylenkette mit 1 bis 8 C-Atomen, worin eine oder mehrere CH₂-Gruppen durch -O-, -NH-, -NR³-, -S- und -(CO)- ersetzt sein können, so dass O/S-Atome nicht direkt miteinander verknüpft sind, und worin auch ein oder mehrere H-Atome durch F, Cl oder -OH ersetzt sein können.

Die erfindungsgemäßen Selbstorientierungsadditive sind bei Raumtemperatur überwiegend kristalline Feststoffe, wodurch das Handling und die Lagerfähigkeit im Vergleich zu z.B. öligen Substanzen verbessert sind. Der Schmelzpunkt kann weiterhin über Variation der Seitenketten zu vorteilhaften Werten hin variiert werden.

Darüber hinaus verleihen die Verbindungen den LC-Medien vergleichsweise gute VHR-Werte unter Anwendungsbedingungen, d.h. nach dem UV-Bestrahlungsprozess der Displayherstellung. Dadurch lassen sich mit den erfindungsgemäßen Additiven nun auch Mischungskonzepte realisieren, die bisher zu Instabilitäten in Belastungstest geführt haben. Die anderen Parameter von VA-Anzeigen, wie z.B. die Schaltzeiten oder die Stabilität des Tiltwinkels in der Herstellung von PS-VA Anzeigen, werden durch die erfindungsgemäßen Additive nicht nachteilig beeinflusst. Die LC-Medien besitzen eine sehr gute Prozessierbarkeit in der Herstellung von VA-Anzeigen und vergleichsweise niedrige Mura-Defekte.

Die FK-Zelle der erfindungsgemäße FK-Anzeige weist bevorzugt keine Orientierungsschicht auf, insbesondere keine Polyimidschicht zur homöotropen Ausrichtung des FK-Mediums. Als Orientierungsschicht ist hier eine Schicht gemeint, die schon vor dem Füllen der Zelle vorhanden ist. Die polymerisierte Komponente des FK-Mediums wird in diesem Zusammenhang nicht als Orientierungsschicht angesehen. Eine FK-Zelle kann dennoch eine Orientierungsschicht oder eine vergleichbare Schicht aufweisen, aber vorzugsweise ist diese Schicht nicht alleine für die homöotrope Orientierung ursächlich, sondern unterstützt oder modifiziert den Effekt des Selbstorientierungsadditivs. Das Reiben von z. B. Polyimidschichten ist erfindungsgemäß nicht erforderlich, um eine homöotrope Orientierung des FK-Mediums gegenüber der Substratoberfläche zu erreichen. Die erfindungsgemäße FK-Anzeige ist vorzugsweise eine VA-Anzeige mit einem FK-Medium mit negativer dielektrischer Anisotropie und auf gegenüberliegenden Substraten angeordneten Elektroden. Alternativ handelt es sich um eine VA-IPS-Anzeige mit einem FK-Medium mit positiver dielektrischer Anisotropie und mindestens auf einem Substrat angeordneten Interdigital-Elektroden.

Das Selbstorientierungsadditiv der Formel I wird vorzugsweise in einer Konzentration von weniger als 10 Gew.-%, besonders bevorzugt ≤ 5 Gew.-% und ganz besonders ≤ 3 Gew.-% eingesetzt. Es wird bevorzugt in einer Konzentration von mindestens 0,05 Gew.-% eingesetzt, bevorzugt mindestens 0,2 Gew.-%. Der Einsatz von 0,1 bis 2,5 Gew.-% des Selbstorientierungsadditivs führt in der Regel schon zu vollständig homöotroper Orientierung der FK-Schicht bei den üblichen Zelldicken (3 bis 4 µm) mit den üblichen Substratmaterialien und unter den üblichen Bedingungen der Herstellungsprozesse einer FK-Anzeige. Durch die polymerisierbare Natur werden auch höhere Konzentrationen an Selbstorientierungsadditiven möglich, ohne das FK-Medium nachhaltig zu beeinflussen, da die polymerisierbare Substanz durch die Polymerisation wieder gebunden wird.

Nachfolgend werden weitere bevorzugte und beispielhafte Ausführungsformen der erfindungsgemäßen Selbstorientierungsadditive der Formel I-A und ihren Unterformeln offenbart.

Die Ankergruppe R^{a} enthält definitionsgemäß eine, zwei oder drei Gruppen X¹, die als Bindeglied zu einer Oberfläche dienen sollen.

Die Abstandsgruppen Sp^{a} bis Sp^{c} sollen eine flexible Bindung zwischen der mesogenen Gruppe mit Ringen und der Gruppe(n) X¹ herstellen. Die Struktur der Abstandsgruppen ist daher sehr variabel und im allgemeinsten Fall der Formel I nicht abschließend definiert. Der Fachmann wird erkennen, dass dabei eine Vielzahl von möglichen Variationen von Ketten in Frage kommt.

Eine Ankergruppe der Formel 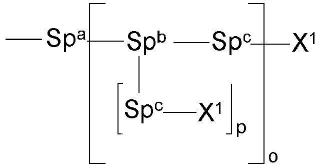 wie vor und nachstehend definiert,
steht bevorzugt für eine Ankergruppe ausgewählt aus den folgenden Formeln: 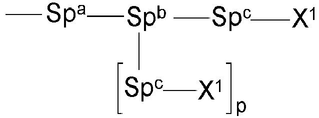 oder

-Sp^{a}-X¹,

worin jeweils unabhängig die Gruppen wie vor und nachstehend definiert sind, besonders bevorzugt für eine Gruppe der Formeln

-Sp^{a}-X¹

oder 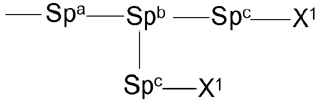 oder 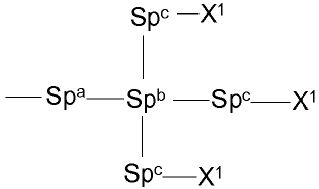 worin jeweils unabhängig die Gruppen wie vor- und nachstehend definiert sind.

Die Gruppe Sp^{b} bedeutet bevorzugt eine trivalente Gruppe der Formeln ausgewählt aus C(R³) oder N (für p = 1),
oder die tetravalente Gruppe C (Kohlenstoffatom, für p = 2).

Besonders bevorzugt bedeutet die trivalente Gruppe
- Sp^{b}: CR³ (für p = 1) oder C (für p = 2), insbesondere CR³, worin R³ H oder einen Alkylrest mit 1 bis 10 C-Atomen, der linear oder verzweigt ist, und worin H durch Fluor oder Alkoxy mit 1 bis 8 C-Atomen substituiert sein kann, bedeutet.

Die Gruppe R³ in Sp^{b} (als trivalente Gruppe) bedeutet bevorzugt H oder einen Alkylrest mit 1 bis 10 C-Atomen, der linear oder verzweigt ist. Durch die Wahl des Restes R³ kann der Schmelzpunkt der erfindungsgemäßen Additive der Formel (I) eingestellt werden. Der Rest R³ kann auch Einfluss auf die homogene Verteilung der Additive auf der Substratoberfläche haben. In einer bevorzugten Ausführungsform ist Sp^{b} eine Gruppe -C(R³), worin R³ H oder einen Rest mit 1 bis 8 C-Atomen bedeutet, z.B. bevorzugt C(CH₂CH₂CH₃), C(CH₂CH₂CH₂CH₃), C(CH₂CH(CH₃)CH₃), C(CH₂CH₂C(CH₃)₃). Bevorzugte Reste R³ sind auch in den expliziten Ankergruppen unten offenbart.

Die Gruppe Sp^{a} bedeutet für o=0 bevorzugt keine Einfachbindung. Bevorzugt ist eine unverzweigte oder verzweigte Alkylenkette mit 1 bis 8 C-Atomen, worin eine oder mehrere CH₂-Gruppen durch -O-, -NH-, -NR³-, -S- und -(CO)-ersetzt sein können, so dass O/S-Atome nicht direkt miteinander verknüpft sind, und worin auch ein oder mehrere H-Atome durch F, Cl oder -OH ersetzt sein können, oder für o = 1 zusätzlich auch eine Einfachbindung, besonders bevorzugt eine Gruppe ausgewählt aus den Formeln: -CH₂-, -CH₂CH₂-, -OCH₂CH₂-, -CH₂CH₂CH₂-, -OCH₂CH₂CH₂-, -CH₂CH₂CH₂CH₂-, -OCH₂CH₂CH₂CH₂-, -CH₂CH₂OCH₂CH₂- und -OCH₂CH₂OCH₂CH₂-, besonders bevorzugt -CH₂-, -OCH₂-, -CH₂CH₂-, -OCH₂CH₂-, -OCH₂CH₂CH₂-, -OCH₂CH₂CH₂CH₂- und -CH₂CH₂CH₂-.

Die Gruppe Sp^{c} bedeutet bevorzugt keine Einfachbindung, bevorzugt eine unverzweigte oder verzweigte Alkylenkette mit 1 bis 8 C-Atomen, worin eine oder mehrere CH₂-Gruppen durch -O- ersetzt sein können, und worin auch ein oder mehrere H-Atome durch F, Cl oder -OH ersetzt sein können, bevorzugt eine Gruppe ausgewählt aus den Formeln -CH₂-, -CH₂CH₂-, -CH₂CH₂CH₂-, -CH₂CH₂CH₂CH₂-, -CH₂CH₂OCH₂CH₂-, besonders bevorzugt -CH₂-.

Die Gruppe Sp^{d} bedeutet bevorzugt eine unverzweigte oder verzweigte Alkylenkette mit 1 bis 8 C-Atomen, worin eine oder mehrere CH₂-Gruppen durch -O-, -NH-, -NR³-, -S- und -(CO)- ersetzt sein können, so dass O/S-Atome nicht direkt miteinander verknüpft sind, und worin auch ein oder mehrere H-Atome durch F, Cl oder -OH ersetzt sein können.

In einer bevorzugten Ausführungsform bedeutet die Ankergruppe R^{a} einen Rest der Formel 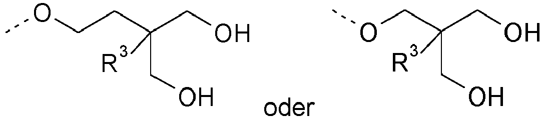 worin R³ H oder einen Alkylrest mit 1 bis 10 C-Atomen, der linear oder verzweigt ist, und worin H durch Fluor oder Alkoxy mit 1 bis 8 C-Atomen substituiert sein kann, bedeutet. Besonders bevorzugt bedeutet R³ eine geradkettige Alkylgruppe mit 1, 2, 3, 4, 5 oder 6 C-Atomen oder H.

Die Gruppe R¹ bedeutet bevorzugt einen unsubstituierten Alkylrest oder Alkoxyrest mit 1 bis 15 Kohlenstoffatomen oder einen Alkenyl-, Alkenyloxy- oder Alkinylrest mit 2 bis 15 C-Atomen, welche jeweils optional ein- oder mehrfach halogeniert sind. Besonders bevorzugt bedeutet R¹ einen Alkylrest mit 2 bis 8 Kohlenstoffatomen.

Die Ankergruppe R^{a} in den vorangehenden Formeln umfasst besonders bevorzugt eine, zwei oder drei OH-Gruppen, besonders bevorzugt eine oder zwei OH-Gruppen.

Besonders bevorzugte Ankergruppen der Formel R^{a} sind ausgewählt aus den folgenden Teilformeln, wobei die Gruppe R^{a} über die gestrichelte Bindung an die jeweilige Formel angebunden ist:

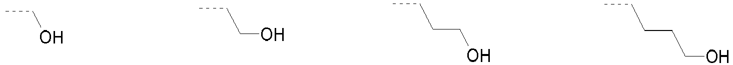

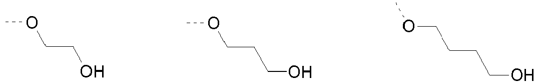

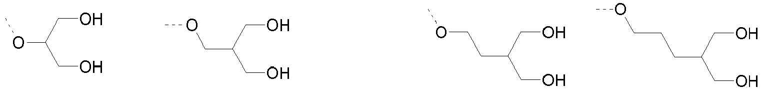

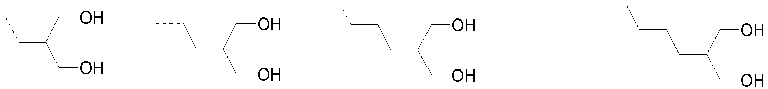

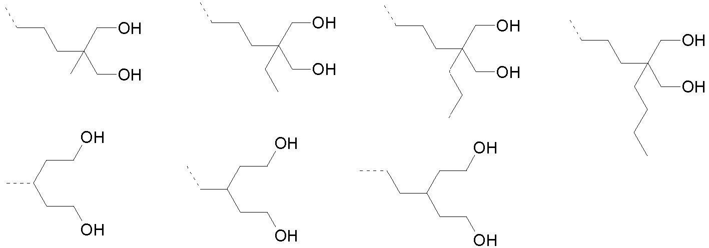

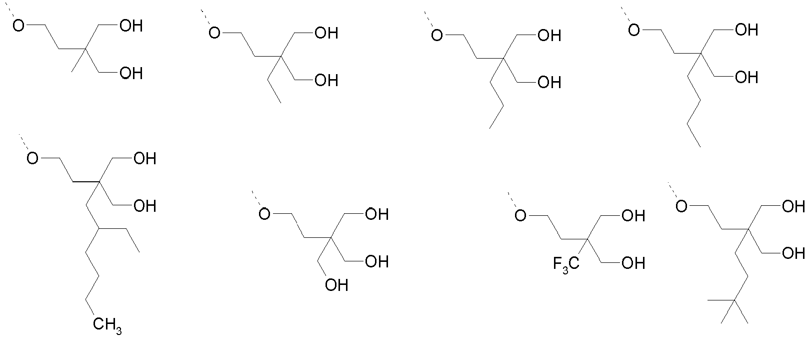

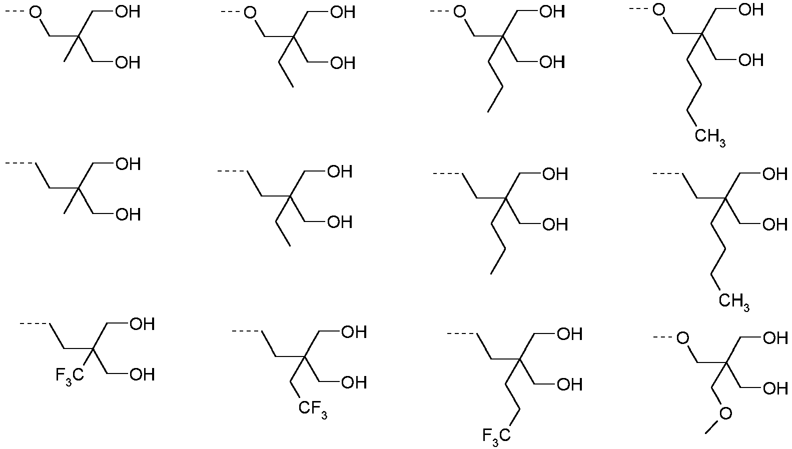

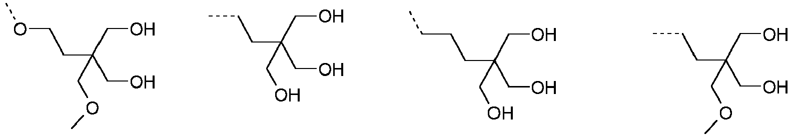

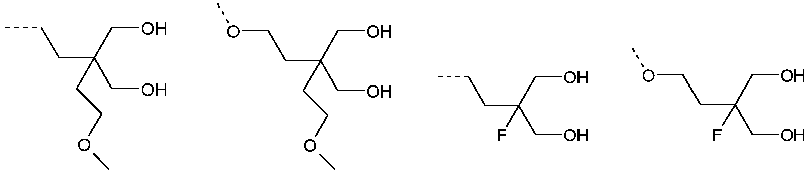

In der Formel I-A und ihren Unterformeln bedeuten die Variablen r3 und r4 bevorzugt beide 0. Die Variable r1 bedeutet bevorzugt 0 oder 1. Die Variable r2 bedeutet bevorzugt 0 oder 1.

Die Anzahl der polymerisierbaren Gruppen P in den erfindungsgemäßen Additiven der Formel I ist 1 oder 2, insbesondere 2.

Die polymerisierbare Gruppe ist besonders bevorzugt eine Methacrylatgruppe. Die Spacergruppe Sp ist bevozugt eine Gruppe der Formel -(CH₂)ₙ- mit n = 1, 2, 3, 4 oder 5, insbesondere -(CH₂)₃-.

Die Gruppe L bedeutet bevorzugt H, F, Cl, CH₃, Ethyl, Propyl, Cyclopropyl oder Isopropyl.

Ein bevorzugtes Selbstorientierungsadditiv der Formel I ist ein Additiv ausgewählt aus den Formeln I-1, I-2 und I-3: 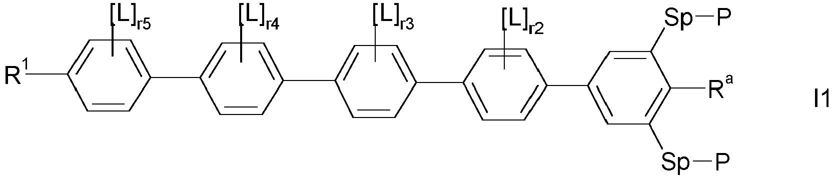 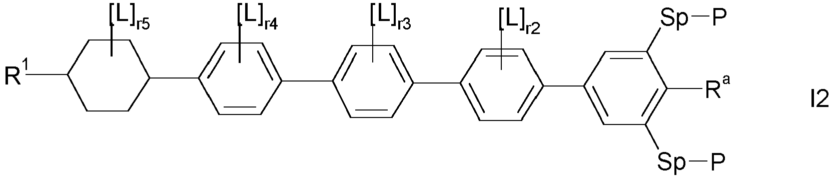 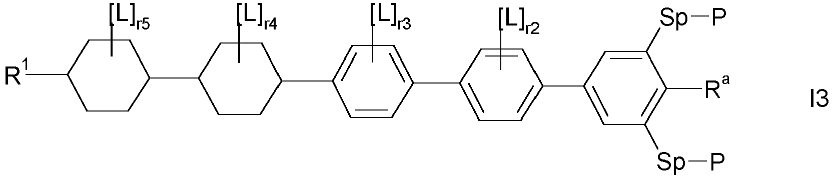 worin jeweils unabhängig
- R¹, Sp, P, L und R^{a}: wie für Formel I-A definiert sind, und
- r2, r3, r4, r5: jeweils unabhängig 0, 1 oder 2, bevorzugt 0 oder 1,
und im Einzelnen, jeweils unabhängig:
- r2: bevorzugt 1,
- r3: bevorzugt 1,
- r4: bevorzugt 0 oder 1, und
- r5: bevorzugt 0 der 1, besonders bevorzugt 0,
bedeuten.

Besonders bevorzugte erfindungsgemäße Selbstorientierungsadditive sind ausgewählt aus den folgenden Formeln: 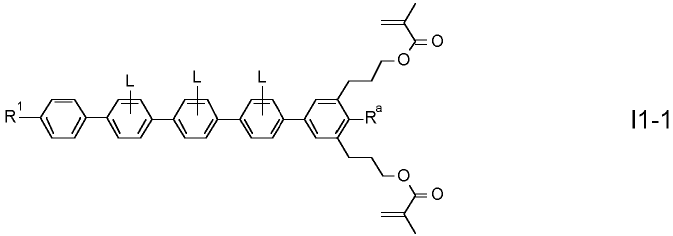 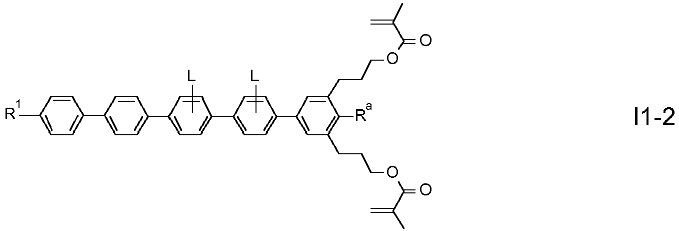 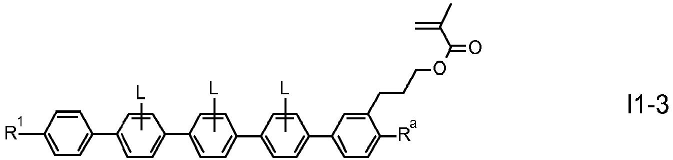 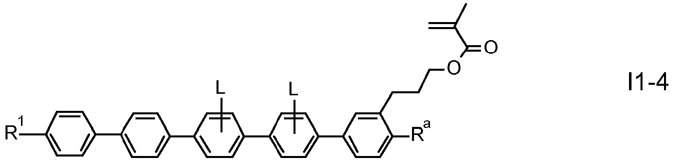 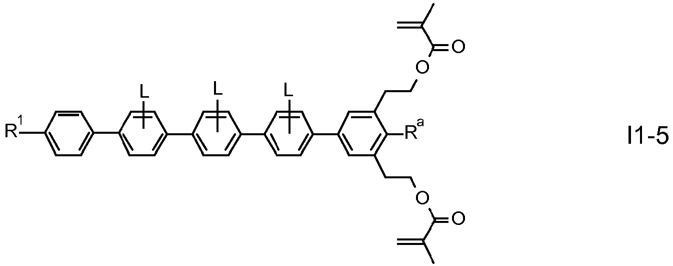 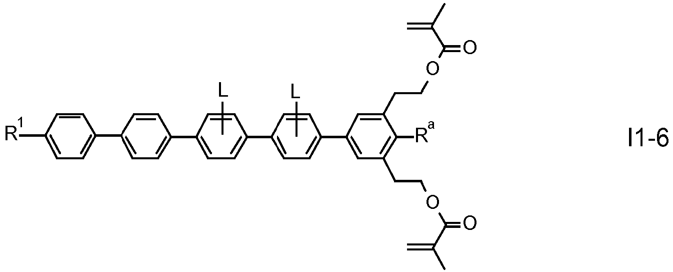 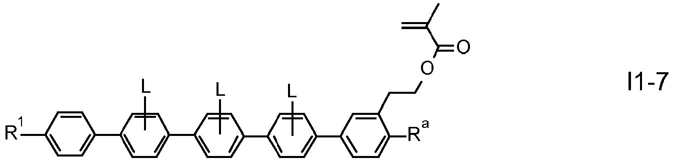 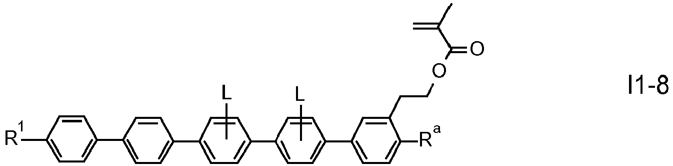 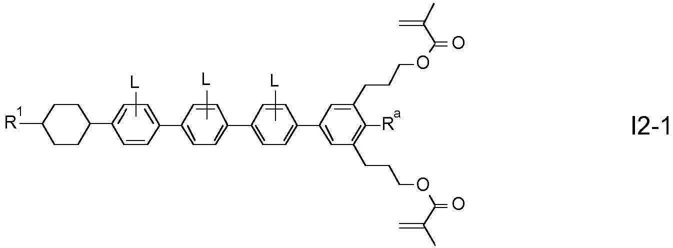 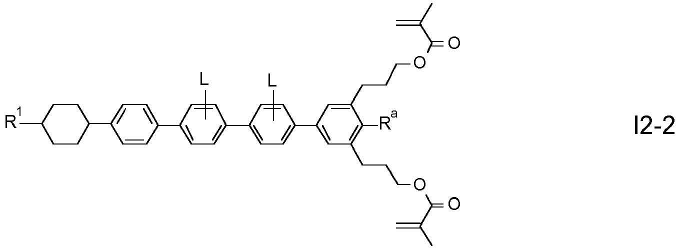 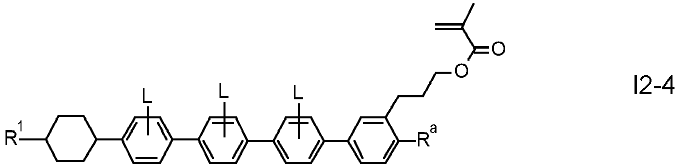 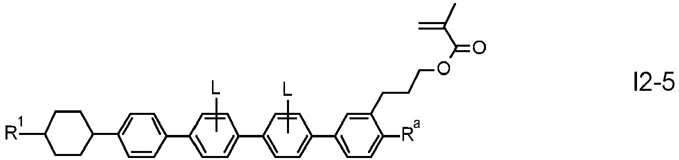 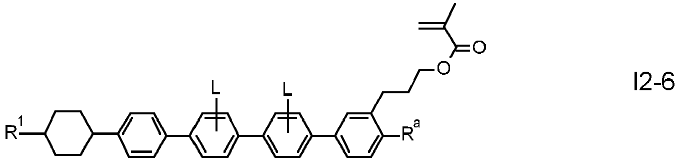 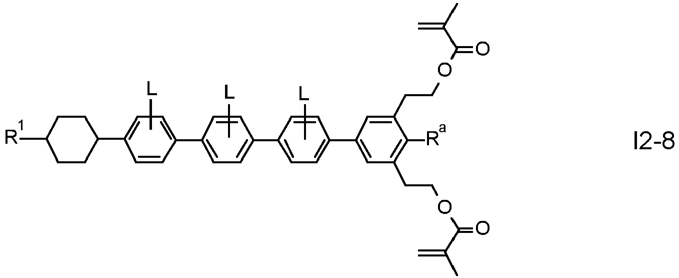 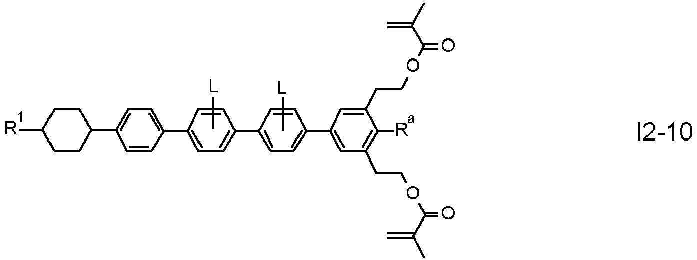 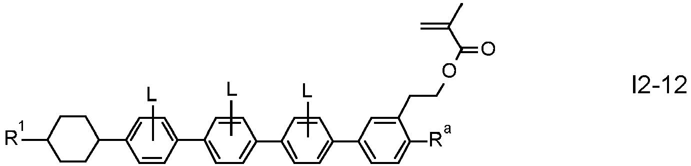 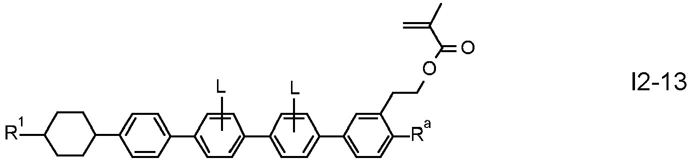 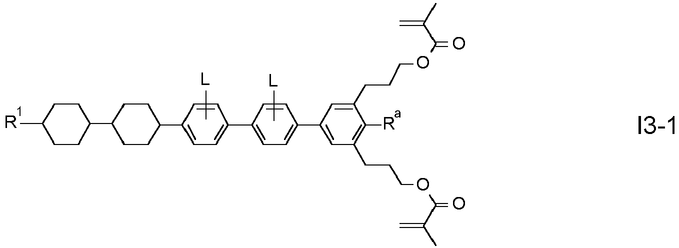 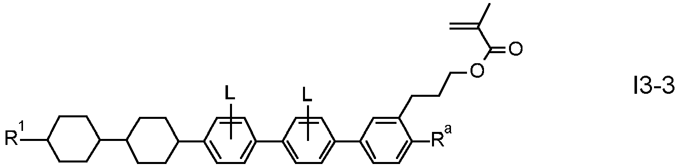 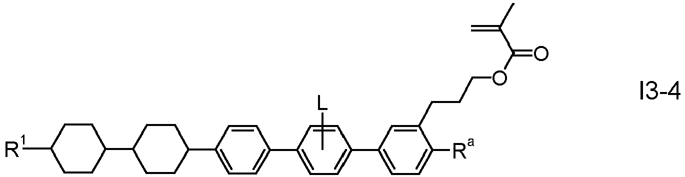 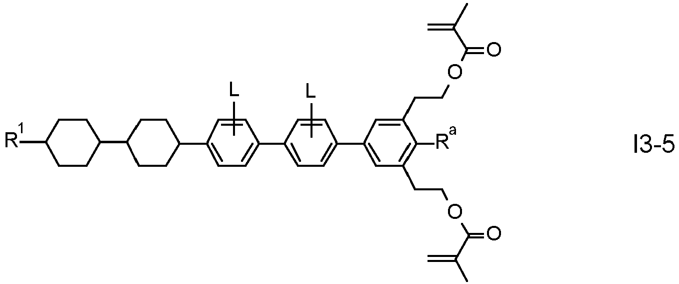 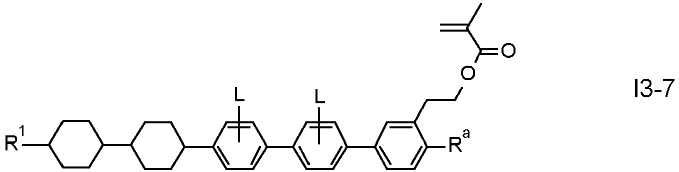 worin R¹, L und R^{a} jeweils unabhängig wie für Formel I und deren Unterformeln definiert sind. Die Substituenten L können daher bei mehrfachem Auftreten unterschiedliche Bedeutungen annehmen.

Beispielhaft für ganz besonders bevorzugte Selbstorientierungsadditive sind die folgenden Formeln: 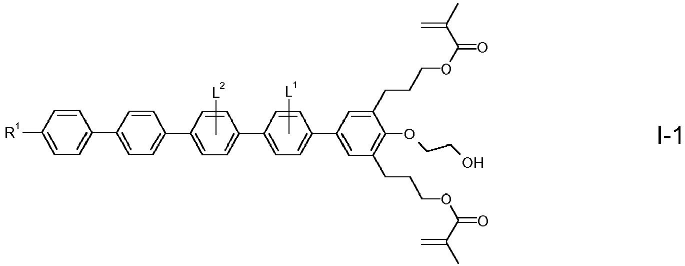 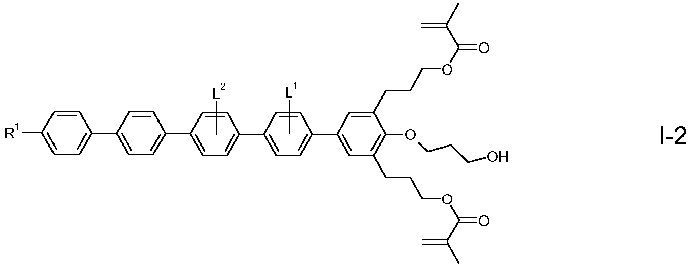 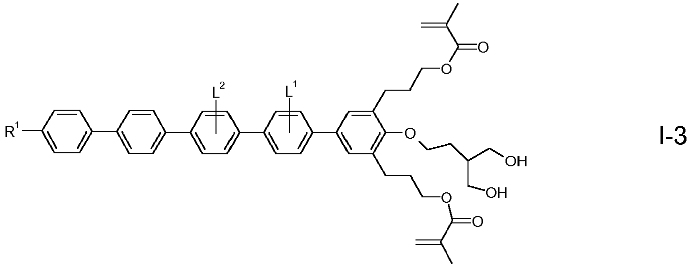 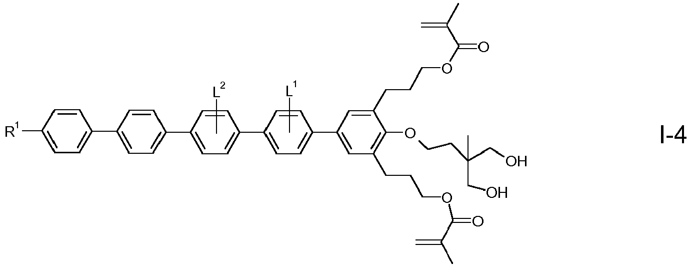 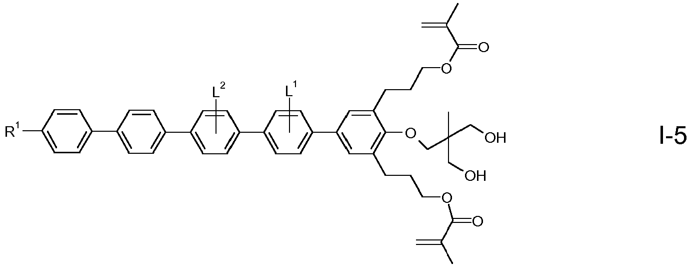 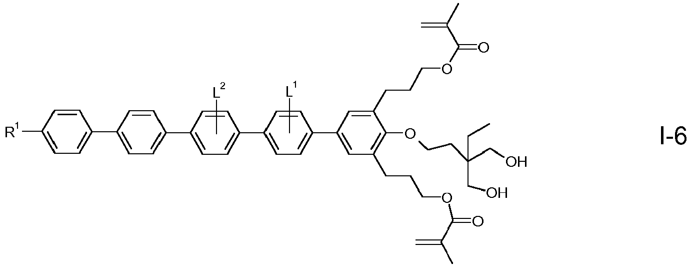 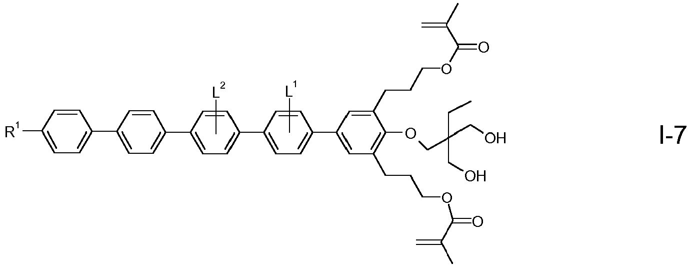 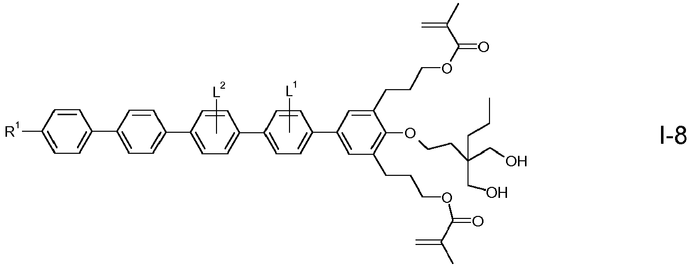 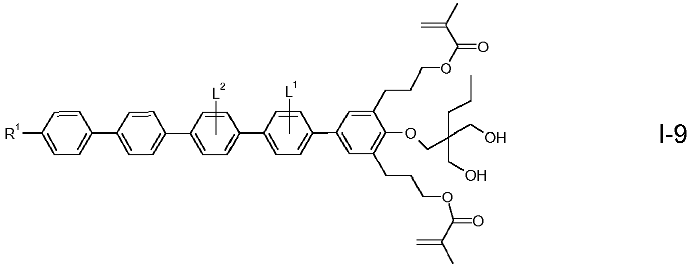 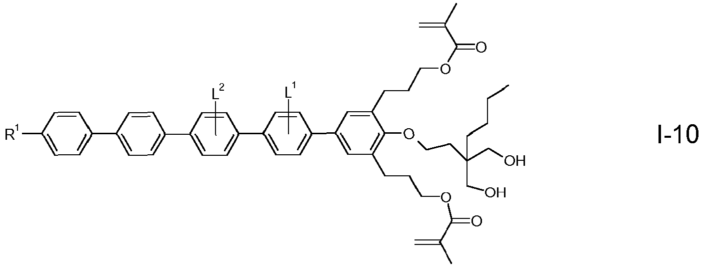 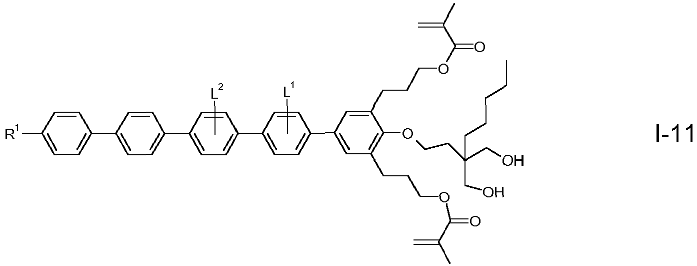 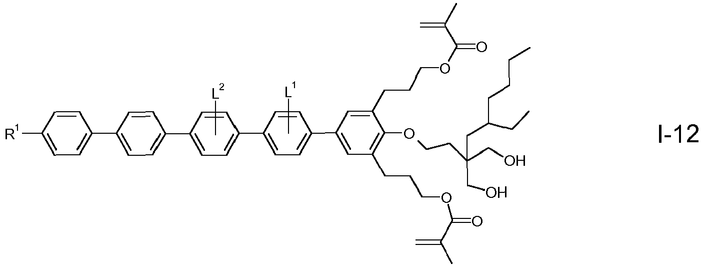 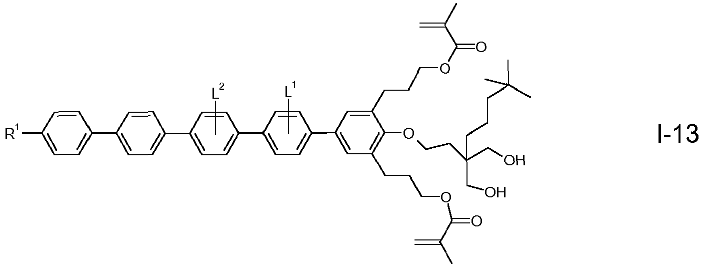 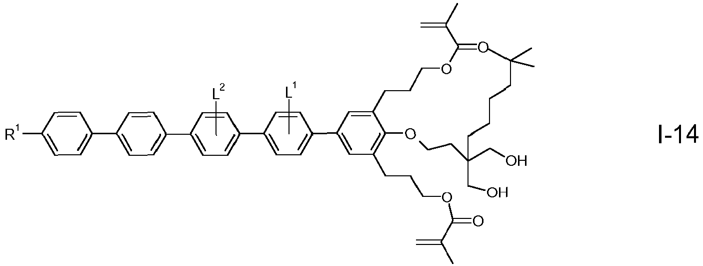 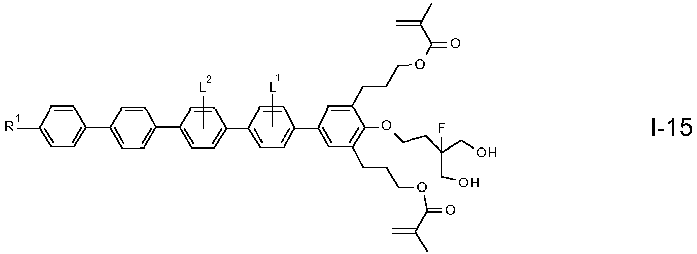 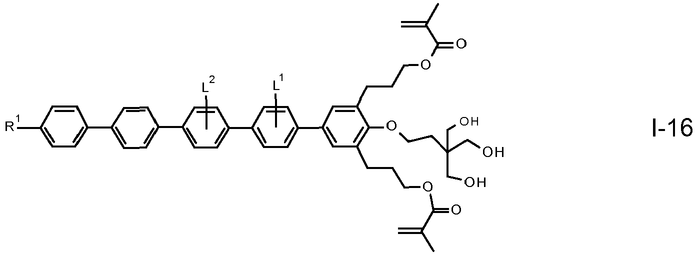 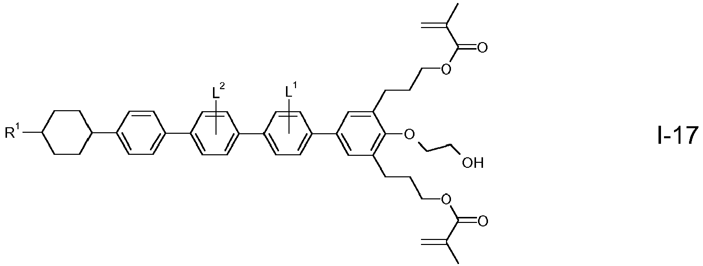 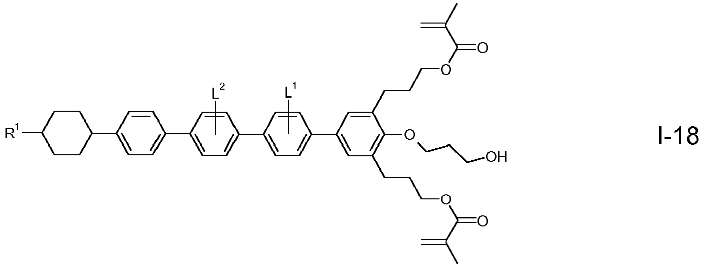 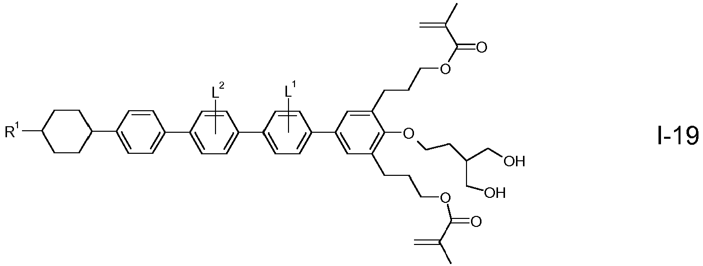 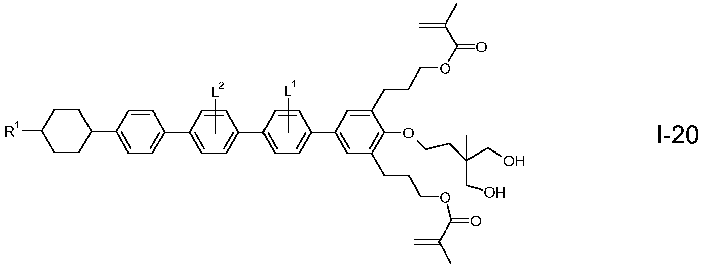 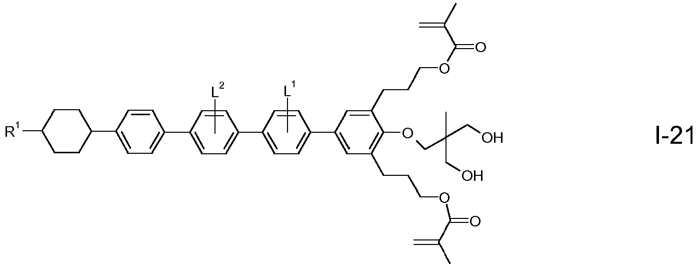 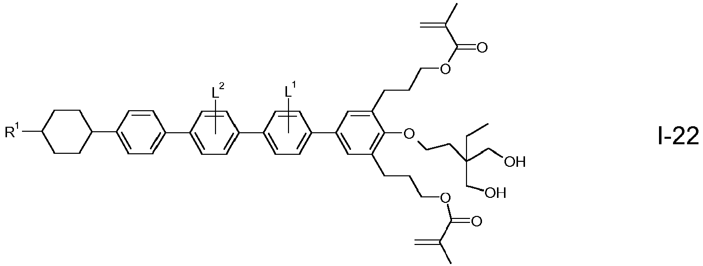 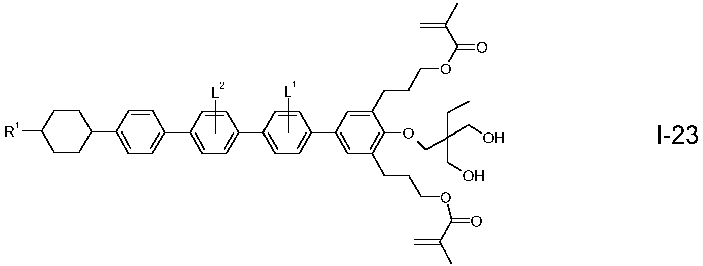 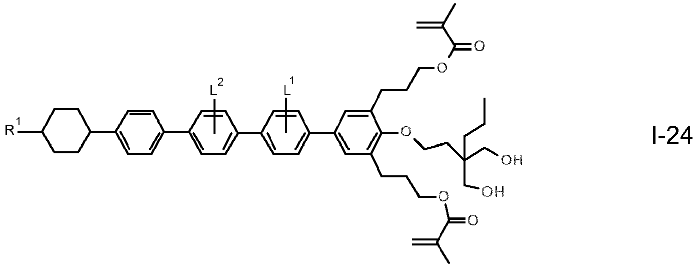 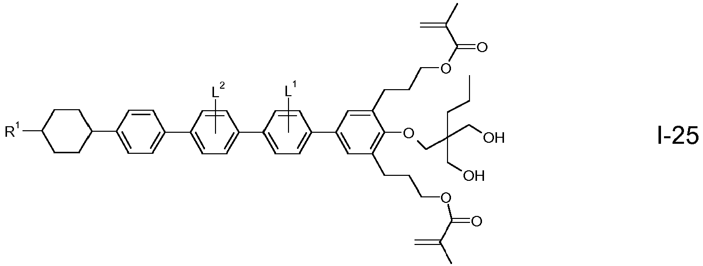 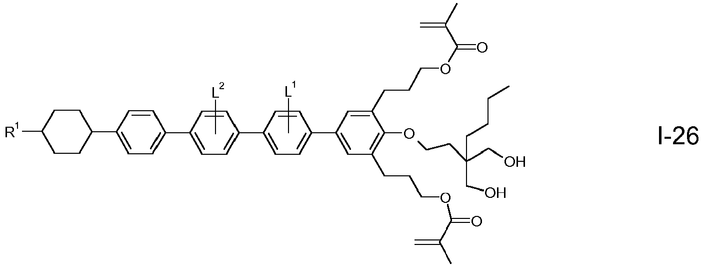 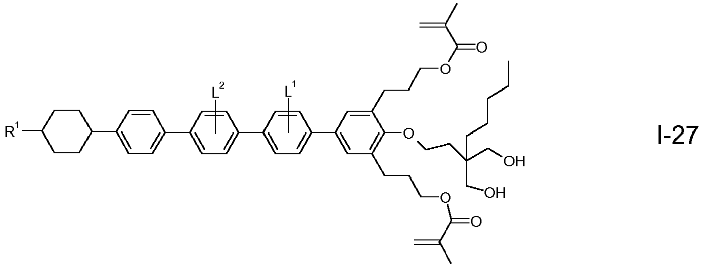 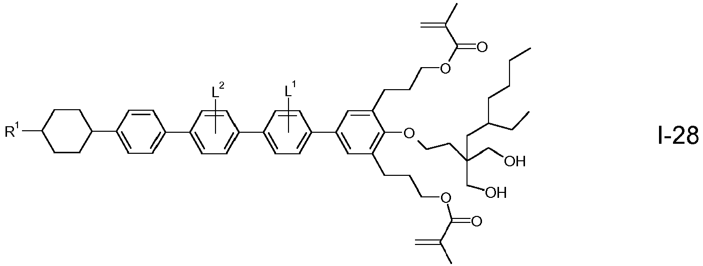 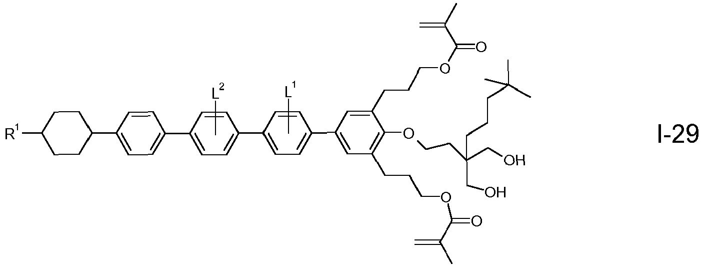 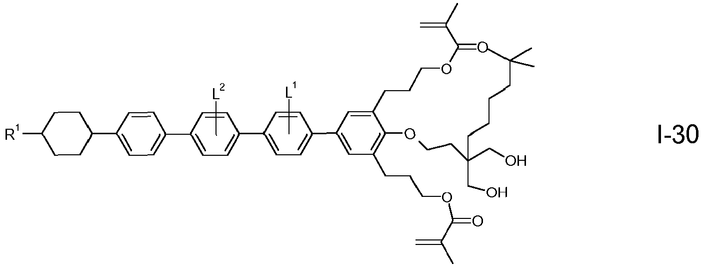 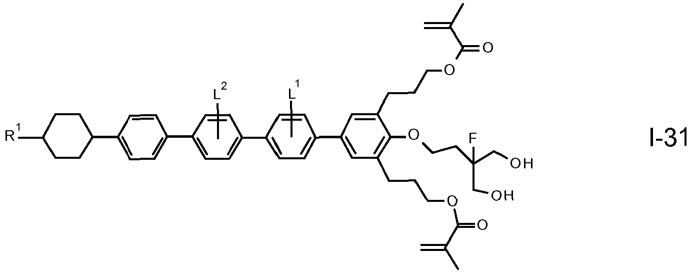 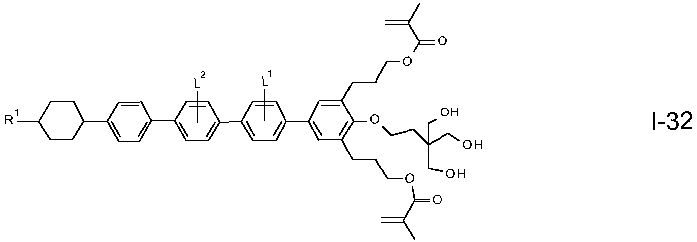 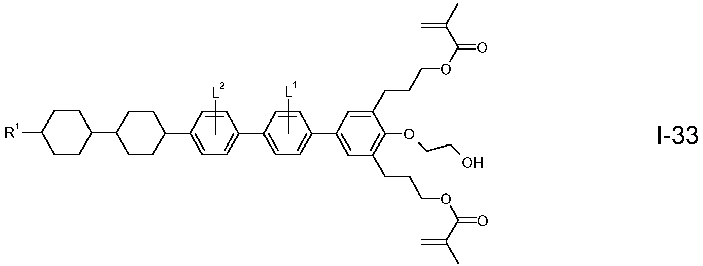 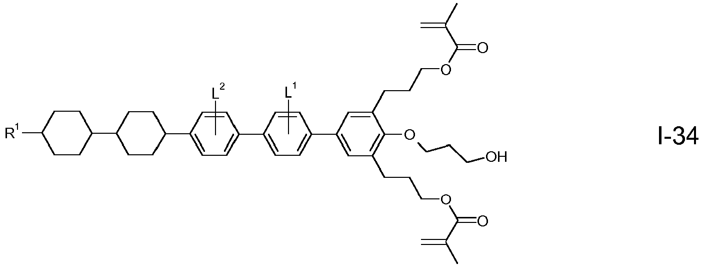 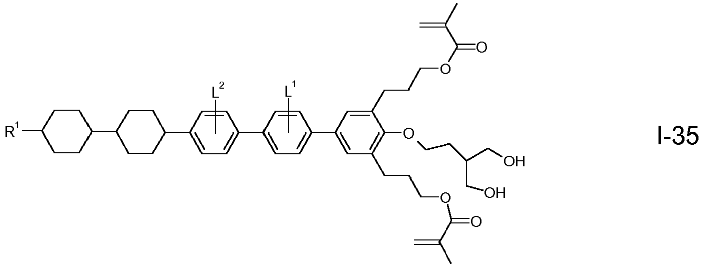 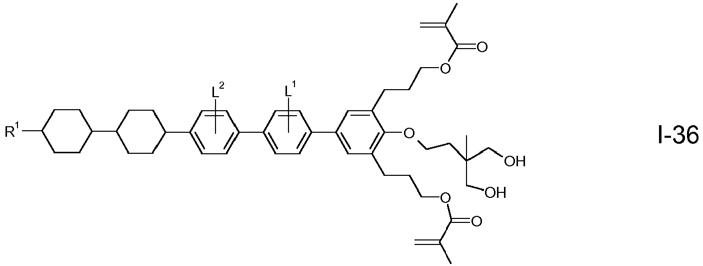 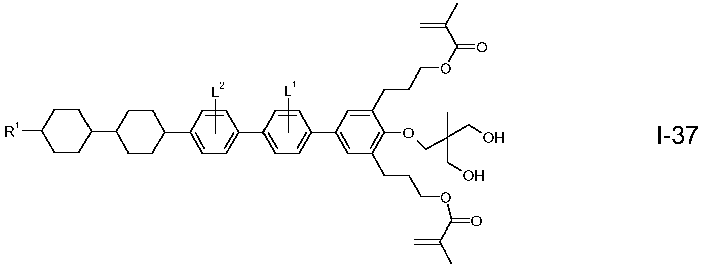 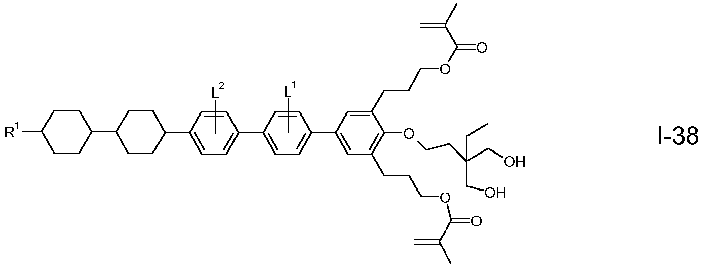 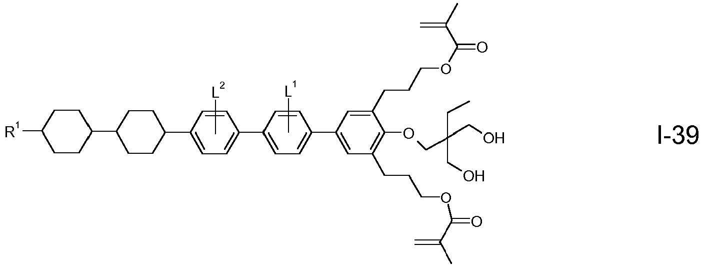 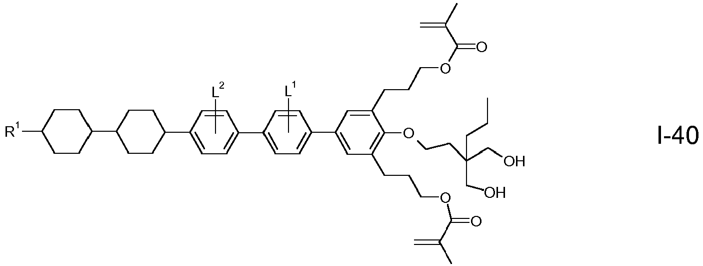 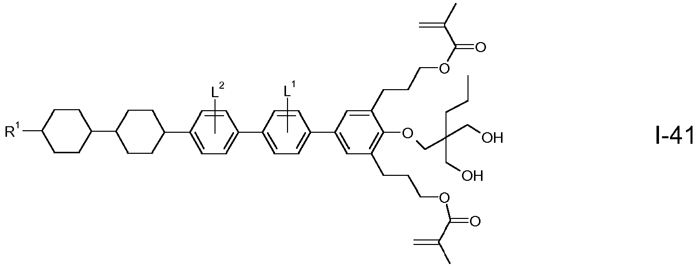 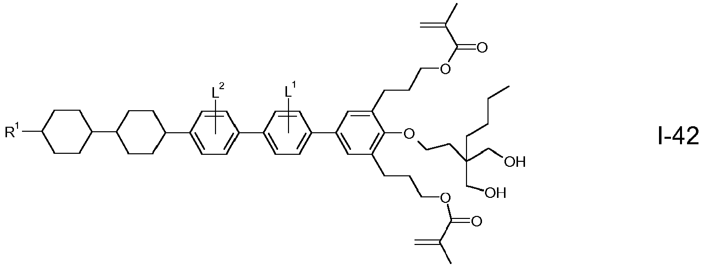 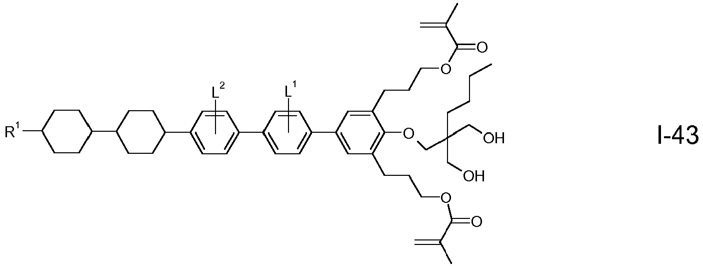 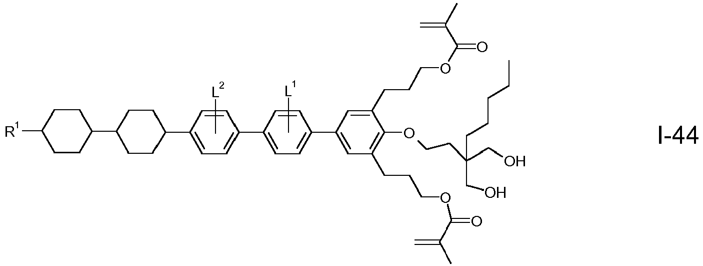 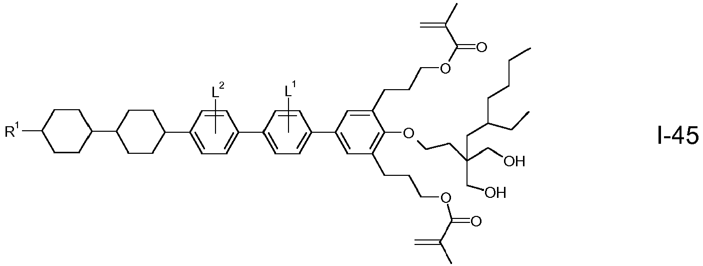 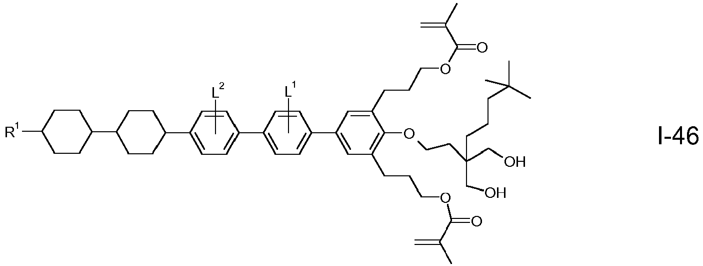 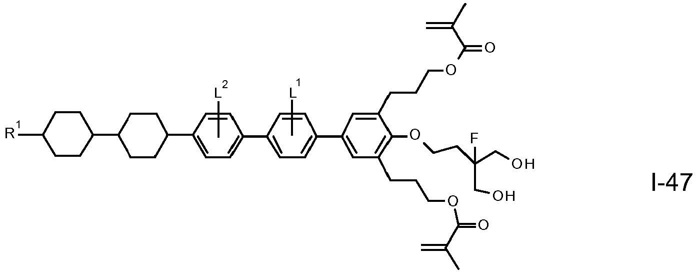 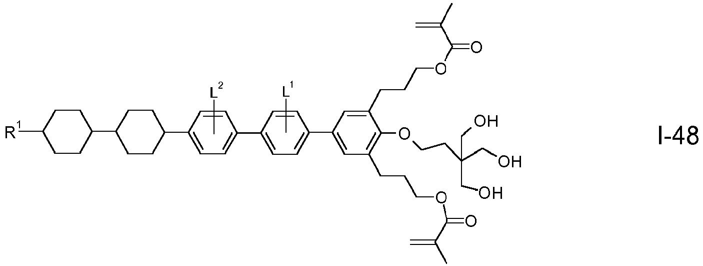 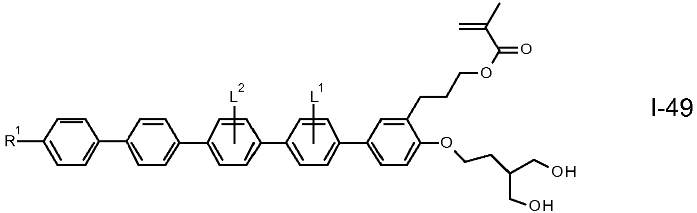 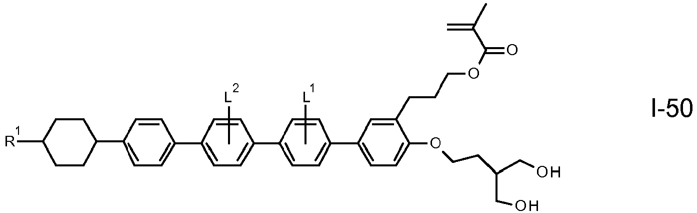 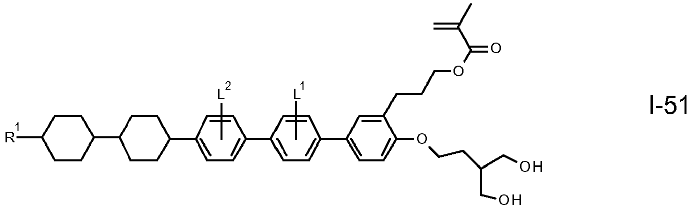 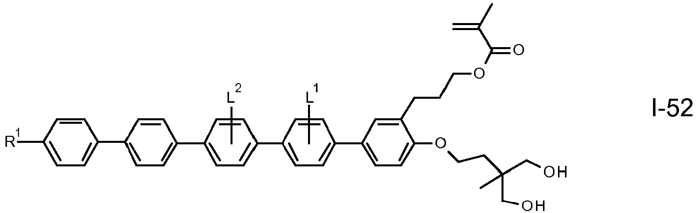 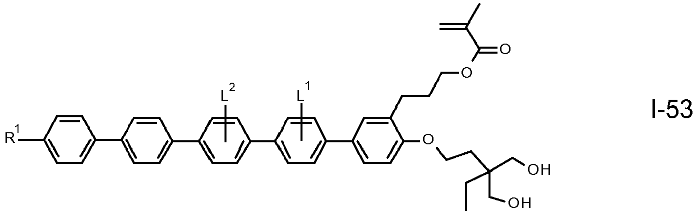 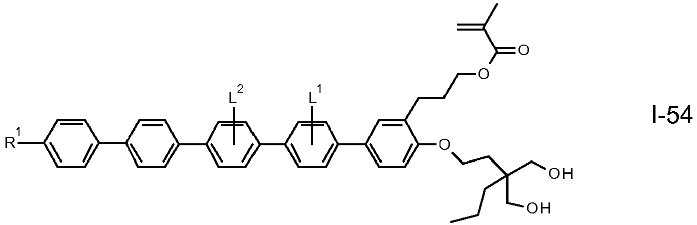 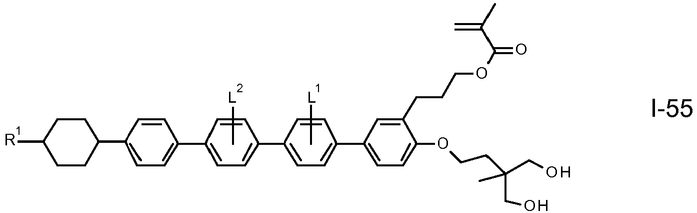 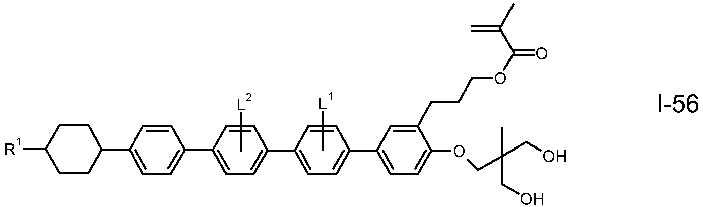 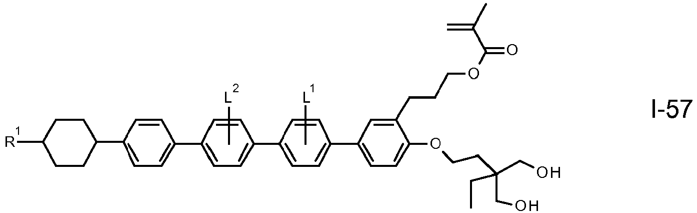 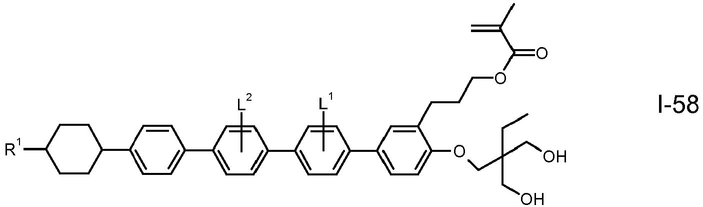 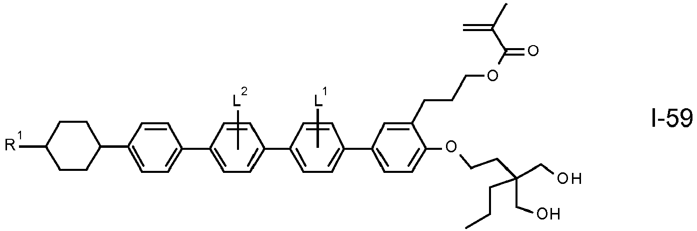 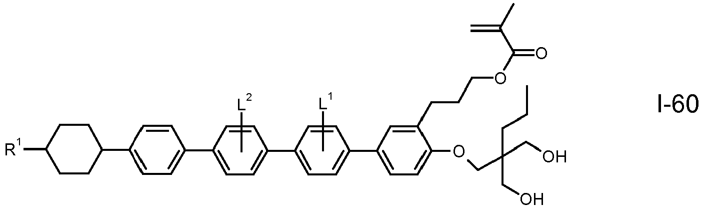 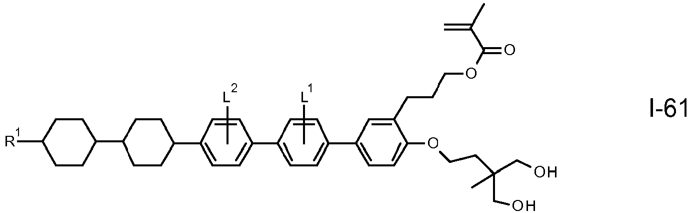 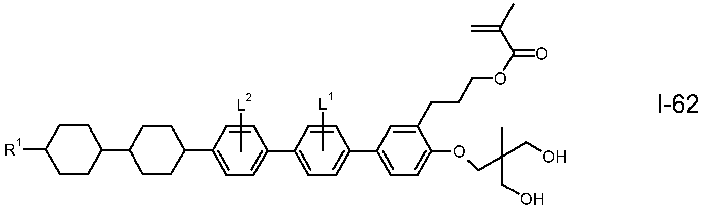 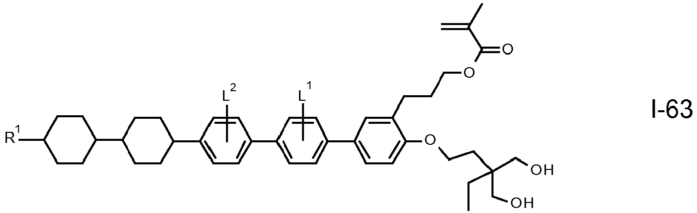 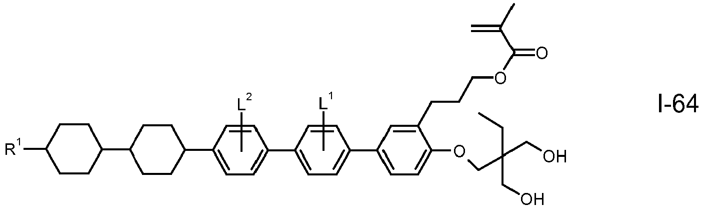 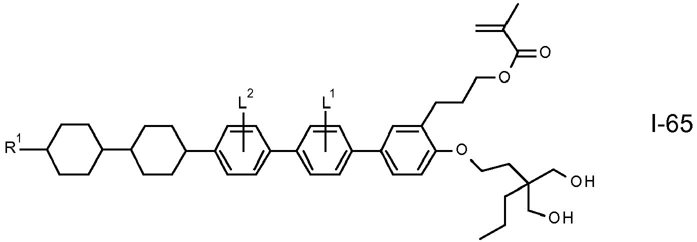 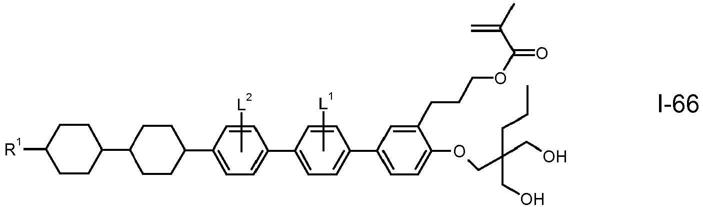 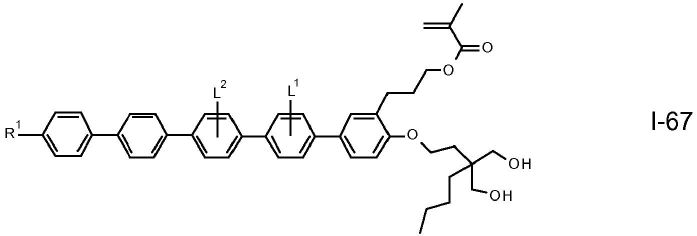 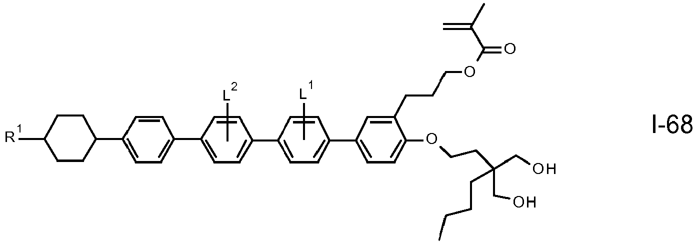 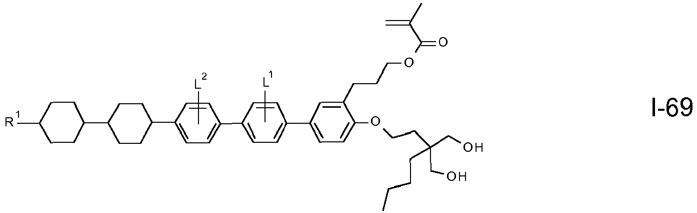 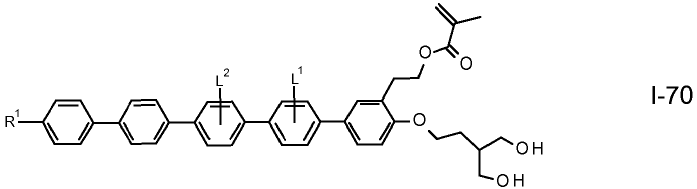 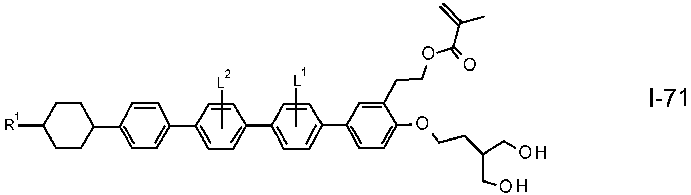 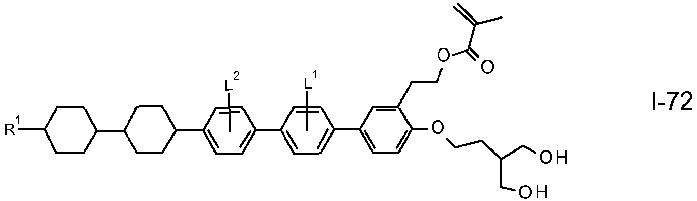 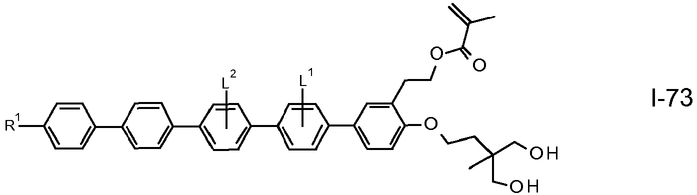 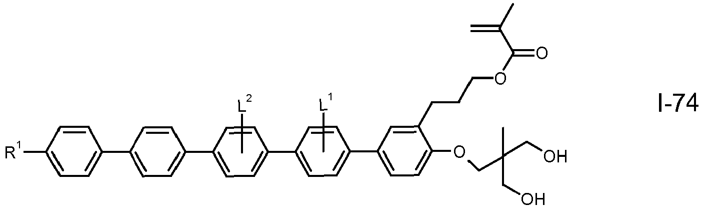 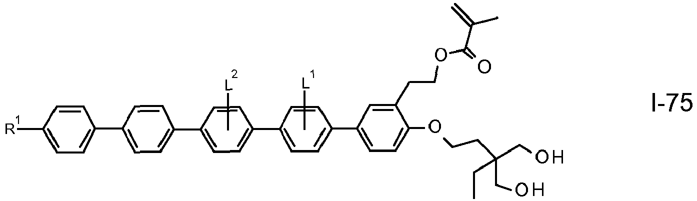 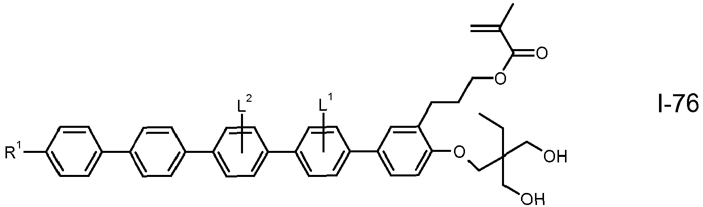 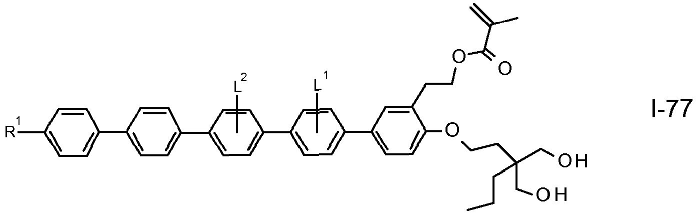 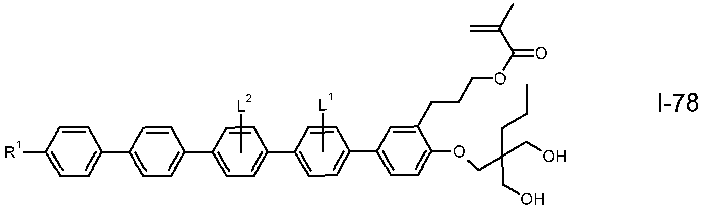 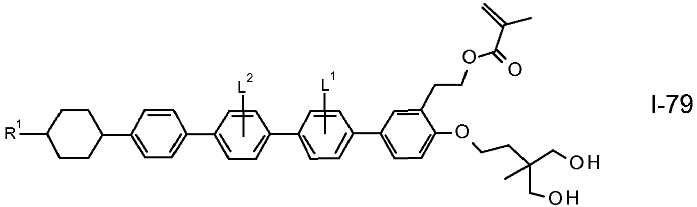 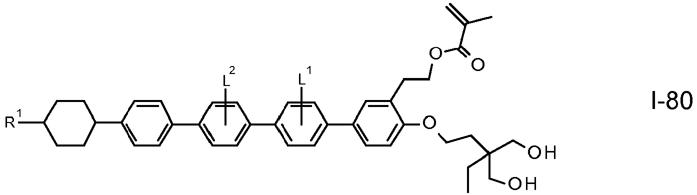 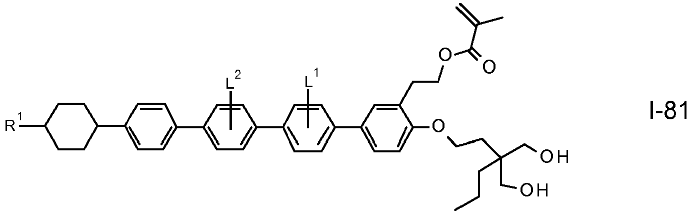 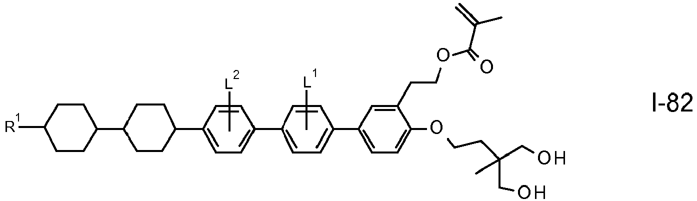 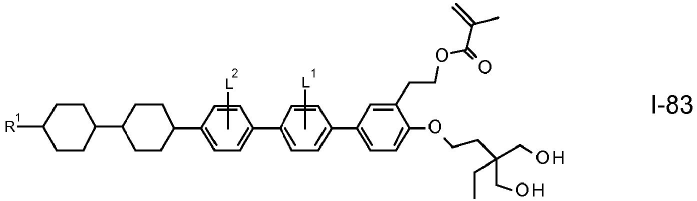 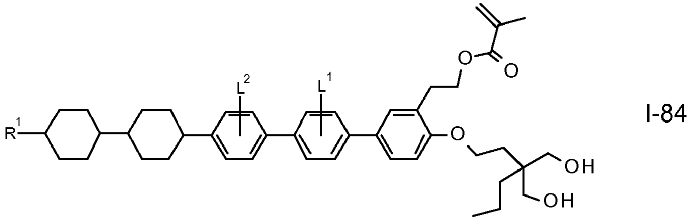 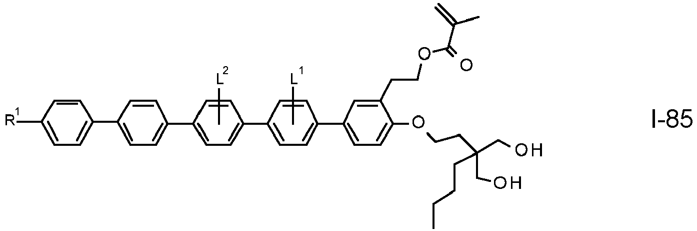 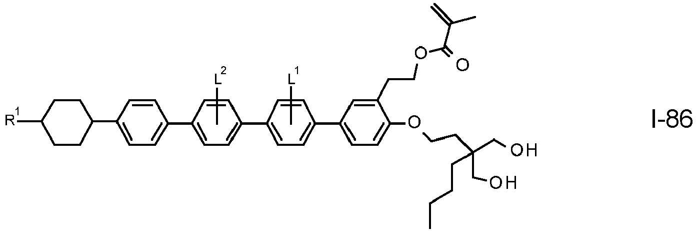 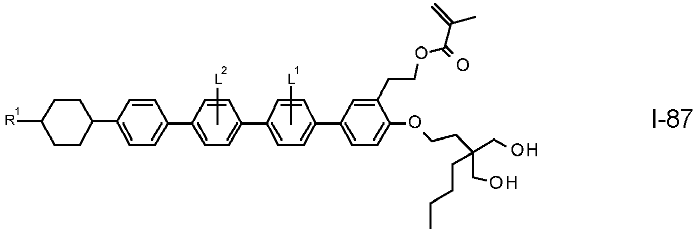 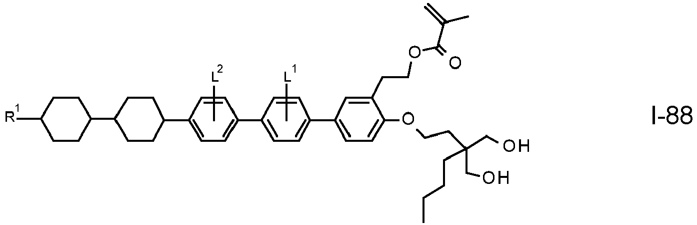 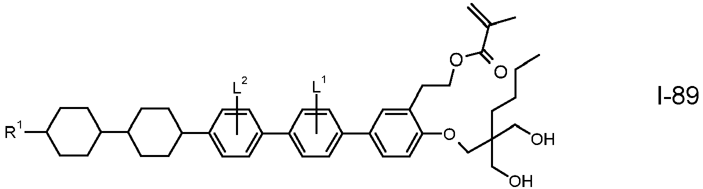 worin R¹ unabhängig wie in Formel I-A definiert ist, und L¹ und L² unabhängig H oder eine Bedeutung von L wie in Formel I-A annehmen, und bevorzugt unabhängig
- L¹: H, -CH₃, -CH₂CH₃, F oder Cl, und
- L²: H, -CH₃, -CH₂CH₃, F oder Cl bedeuten.

Besonders bevorzugt ist wenigstens eine Gruppe aus L¹ und L² nicht H.

Das erfindungsgemäße FK-Medium kann neben den Selbstorientierungsadditiven der Formel I-A auch weitere Selbstorientierungsadditive der Formel K enthalten, die z. B. weniger als fünf Ringe aufweisen. Die gesamte Konzentration der polymerisierbaren Selbstorientierungsadditive der Formel I-A und der weiteren (konventionellen) Selbstorientierungsadditive der Formel K zusammen beträgt bevorzugt die oben angegebenen Werte, also beispielsweise 0,1 bis 2,5 Gew.-%.

Die weiteren Selbstorientierungsadditive können eine Struktur der Formel K haben:

R¹-[A^{K2}-Z²]ₘ-A^{K1}-R^{a} K

worin die Gruppen R¹, Z² und R^{a} unabhängig wie für Formel I-A voranstehend definiert sind,
m 1, 2 oder 3 bedeutet,
und jeweils unabhängig,
A^{K1} eine aromatische, heteroaromatische, alicyclische oder heterocyclische Gruppe, welche auch anellierte Ringe enthalten kann, und welche auch durch eine Gruppe L oder -Sp-P ein- oder mehrfach substituiert sein kann, A^{K2} jeweils unabhängig voneinander, gleich oder verschieden, eine aromatische, heteroaromatische, alicyclische oder heterocyclische Gruppe, welche auch anellierte Ringe enthalten kann, und welche auch durch eine Gruppe L oder - Sp-P ein- oder mehrfach substituiert sein kann, bedeuten, jeweils auch bezüglich der bevorzugten Definitionen.

Die Formel K umfasst polymerisierbare und nicht polymerisierbare Verbindungen. Die bevorzugten Ausführungsformen zur Ankergruppe R^{a}, den Elementen R¹ und den Substituenten L und -Sp-P etc. sind auch auf die herkömmlichen Additive der Formel K anwendbar.

Die Herstellung der konventionellen Selbstorientierungsadditive ist z. B. der Druckschrift WO 2012/038026, EP 2918658 A2, US 2015/0252265 A1 oder WO 2016/015803 A1 zu entnehmen.

Der Begriff "Aryl" bedeutet eine aromatische Kohlenstoffgruppe oder eine davon abgeleitete Gruppe.

Arylgruppen können einkernig oder mehrkernig sein, d.h. sie können einen Ring (wie z.B. Phenyl) oder zwei oder mehr anellierte Ringe aufweisen. Wenigstens einer der Ringe besitzt dabei eine aromatische Konjugation. Heteroarylgruppen enthalten ein oder mehrere Heteroatome, vorzugsweise ausgewählt aus O, N, S und Se.

Besonders bevorzugt sind ein-, zwei- oder dreikernige Arylgruppen mit 6 bis 25 C-Atomen. Ferner bevorzugt sind 5-, 6- oder 7-gliedrige Arylgruppen, worin auch eine oder mehrere CH-Gruppen durch N, S oder O so ersetzt sein können, dass O-Atome und/oder S-Atome nicht direkt miteinander verknüpft sind.

Bevorzugte Arylgruppen sind beispielsweise Phenyl, Naphthyl, Anthracen, Phenanthren, Pyren, Dihydropyren, Chrysen, Perylen, Tetracen, Pentacen, Benzpyren, Fluoren, Inden, Indenofluoren, Spirobifluoren, etc.

Im Zusammenhang mit der vorliegenden Erfindung bedeutet der Ausdruck "Alkyl" einen unverzweigten oder verzweigten, gesättigten, aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen.

Der Begriff "cyclisches Alkyl" umfasst Alkylgruppen, die wenigstens einen carbocyclischen Teil aufweisen, also beispielsweise auch Cycloalkylalkyl, Alkylcycloalkyl und Alkylcycloalkylalkyl. Die carbocyclischen Gruppen umfassen darin z. B. Cyclopropyl, Cyclobutyl, Cyclopentyl, Bicyclo[1.1.1]pentyl, Cyclohexyl, Spiro[3.3]bicycloheptyl, Cycloheptyl, Cyclooctyl, usw.

Im Zusammenhang mit der vorliegenden Erfindung bedeutet der Ausdruck "Fluoralkyl" einen unverzweigten oder verzweigten, gesättigten oder ungesättigten, bevorzugt gesättigten, aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen, der durch ein oder mehrere Fluoratome substituiert ist. Bevorzugt ist der Rest perfluoriert.

"Halogen" steht im Zusammenhang der vorliegenden Erfindung für Fluor, Chlor, Brom beziehungsweise Iod, bevorzugt für Fluor oder Chlor.

Der Begriff "Abstandsgruppe" oder "Spacer", hierin in der Regel mit "Sp" (bzw. Sp^{a/c/d/1/2/3}) bezeichnet, ist dem Fachmann bekannt und in der Literatur beschrieben, z.B. in Pure Appl. Chem. 73(5), 888 (2001) und C. Tschierske, G. Pelzl, S. Diele, Angew. Chem. (2004), 116, 6340-6368. In der vorliegenden Offenbarung bezeichnet der Begriff "Abstandsgruppe" oder "Spacer" eine verbindende Gruppe, beispielsweise eine Alkylengruppe, welche eine mesogene Gruppe mit einer polymerisierbaren Gruppe verbindet. Während die mesogene Gruppe in der Regel Ringe umfasst, ist die Abstandsgruppe in der Regel ohne Ringsysteme, also kettenförmig, wobei die Kette auch verzweigt sein kann. Als Kette wird beispielsweise eine Alkylengruppe aufgefasst. Substitutionen an und in der Kette, z.B. durch -O- oder -COO-, sind im Allgemeinen mit umfasst.

Die obenstehenden bevorzugten Verbindungen der Formel I-A lassen sich prinzipiell nachfolgenden exemplarischen Syntheserouten herstellen (Schema 1-3): 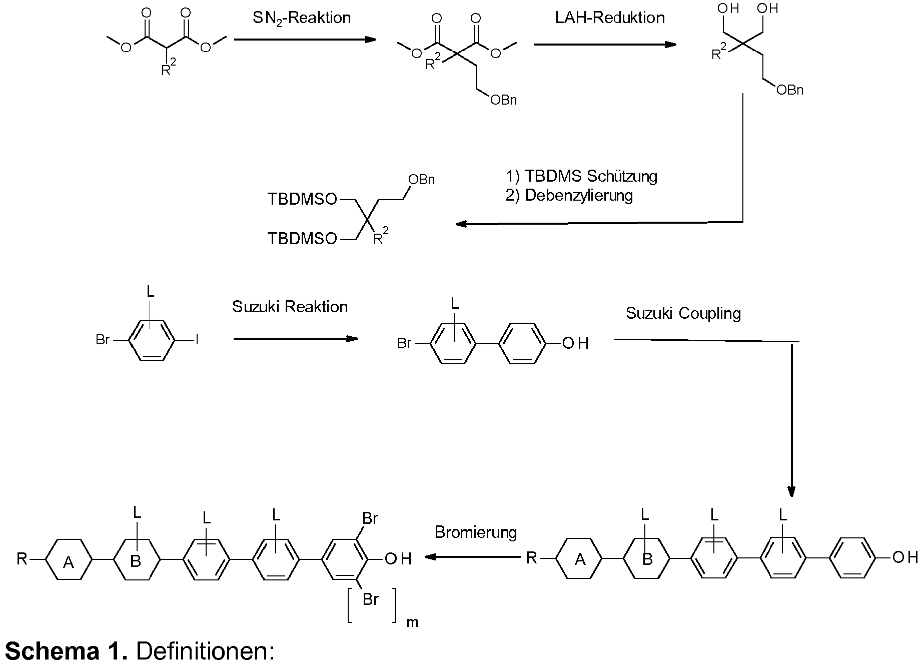
m = 0, 1;
Ring A, B = aromatisch oder nicht aromatischer Ring wie unter Formel I-A beschrieben;
L = unabhängig voneinander gleich oder verschieden und wie unter Formel I-A beschrieben.

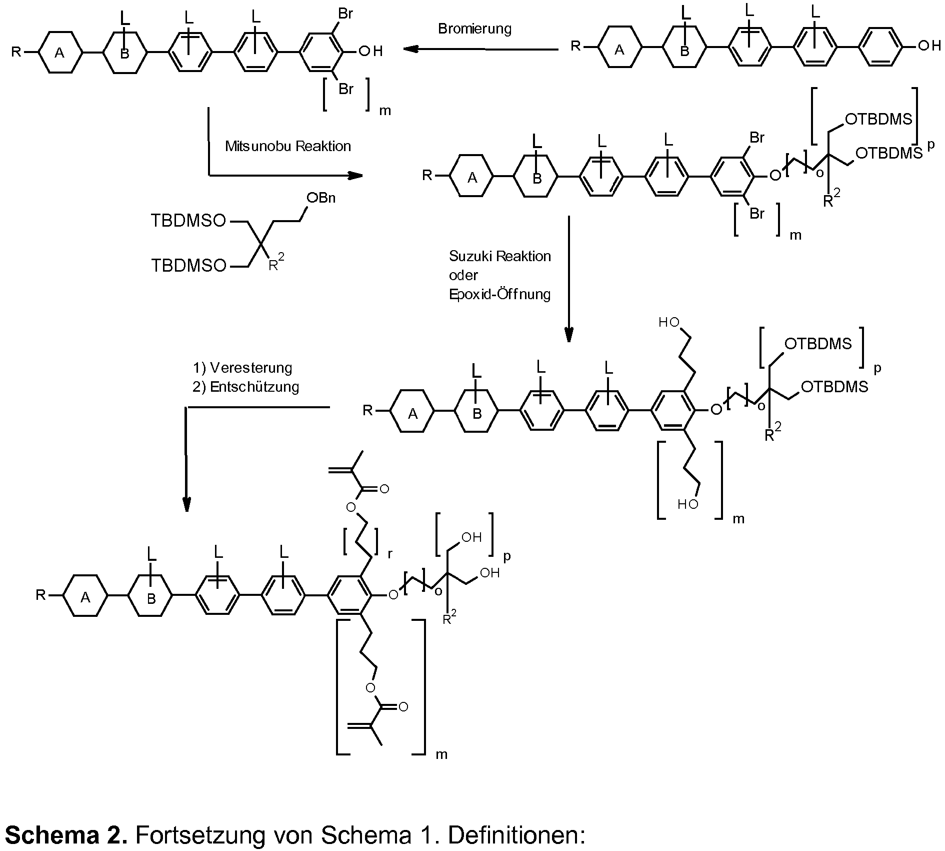

R² = wie unter Formel I beschrieben;
o = 0, 1, 2, 3;
p = 0, 1, 2 (bei p > 1 fällt R² weg);
r = 0,1;
m = 0, 1;
Ring A, B = aromatisch oder nicht aromatischer Ring wie unter Formel I-A beschrieben;
L = unabhängig voneinander gleich oder verschieden und wie unter Formel I-A beschrieben.

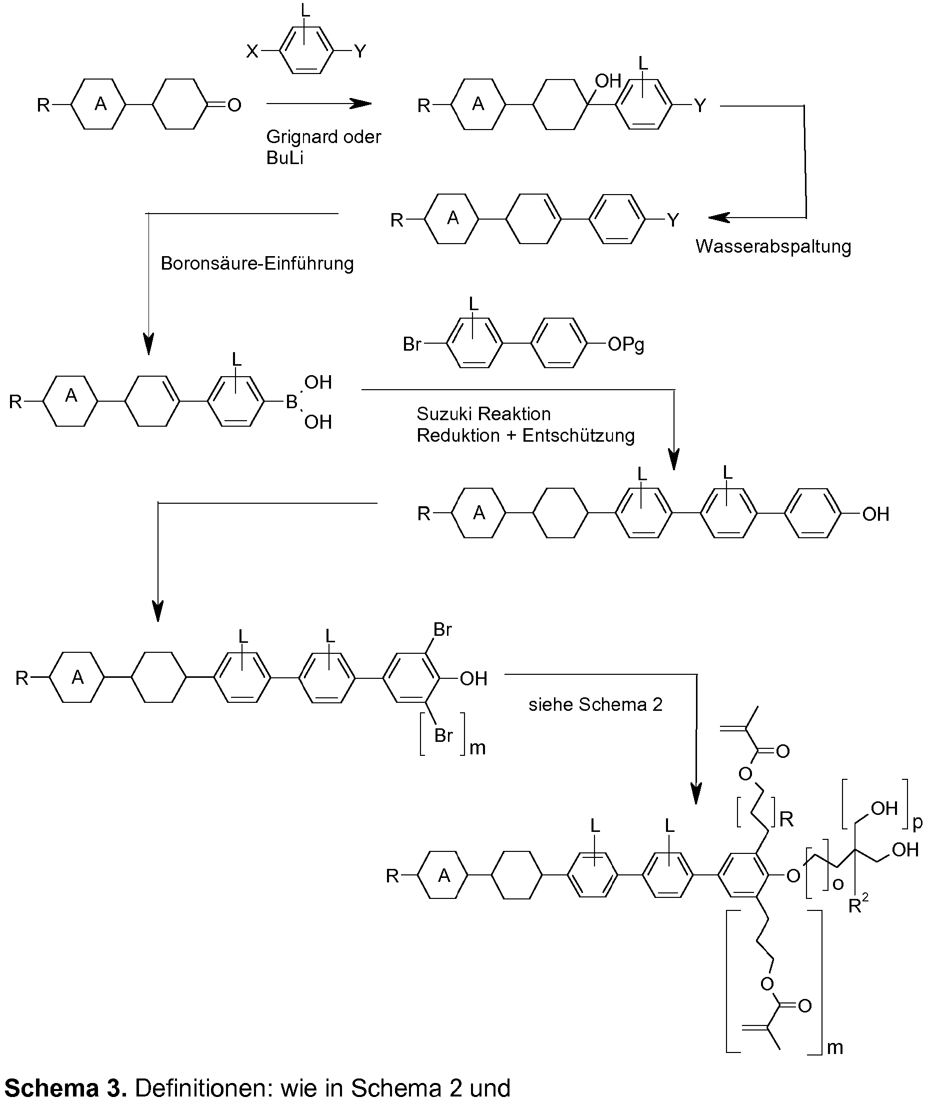

- Pg: = Schutzgruppe: z.B.: Benzyl.

Die polymerisierbare Komponente des erfindungsgemäßen FK-Mediums umfasst vorzugsweise neben den Verbindungen der Formel I weitere polymerisierbare oder (teil-)polymerisierte Verbindungen. Dabei handelt es sich vorzugsweise um herkömmliche polymerisierbare Verbindungen ohne Ankergruppe, vorzugsweise mesogene Verbindungen, insbesondere solche die geeignet für die PS-Technik sind. Dafür bevorzugte polymerisierbare Verbindungen sind die unten für Formel M und deren Unterformeln angegeben Strukturen. Das daraus gebildete Polymer kann die Ausrichtung des FK-Mediums stabilisieren, optional eine Passivierungsschicht ausbilden, und optional einen Pretilt erzeugen.

Die vorliegende Erfindung umfasst auch ein FK-Medium wie vor und nachstehend beschrieben, das
- 95 Gew.-% oder mehr einer niedermolekularen, nicht polymerisierbaren, flüssigkristallinen Komponente, und
- 5 Gew.-% oder weniger einer polymerisierbaren oder polymerisierten Komponente
enthält. Die polymerisierbare Komponente umfasst bevorzugt Verbindungen der Formel I oder der Formel M oder beides in veränderlichen Anteilen.

Die erfindungsgemäßen FK-Medien enthalten daher vorzugweise >0 bis <5 Gew.-%, besonders bevorzugt 0,05 bis 1 Gew.-% und ganz besonders bevorzugt 0,2 bis 1 Gew.-% an polymerisierbaren Verbindungen ohne Ankergruppe R^{a}, insbesondere Verbindungen der Formel M wie unten definiert und der darunter fallenden bevorzugten Formeln.

Die Polymerisation der polymerisierbaren Komponente(n) erfolgt zusammen oder in Teilschritten unter unterschiedlichen Polymerisationsbedingungen. Bevorzugt erfolgt die Polymerisation unter Einwirkung von UV-Licht. In der Regel wird die Polymerisation mit Hilfe eines Polymerisationsinitiators und UV-Licht eingeleitet. Bei den bevorzugten (Meth-)Acrylaten wird auf diese Weise eine praktisch vollständige Polymerisation erreicht. Bei der Polymerisation kann optional eine Spannung an die Elektroden der Zelle angelegt werden oder ein anderes elektrisches Feld angewendet werden, um die Ausrichtung des FK-Mediums zusätzlich zu beeinflussen.

Besonders bevorzugt sind erfindungsgemäße FK-Medien die neben den Verbindungen der Formel I weitere polymerisierbare oder (teil-) polymerisierte Verbindungen (ohne Ankergruppe) und optional weitere Selbstorientierungsadditive enthalten. Diese weiteren Selbstorientierungsadditive sind vorzugsweise solche der Formel K, wie oben definiert.

Die optional enthaltenen weiteren Monomere der polymerisierbaren Komponente des FK-Mediums werden vorzugsweise durch die folgende Formel M beschrieben:

P¹-Sp¹-A²-(Z¹-A¹)n-Sp²-P² M

worin die einzelnen Reste folgende Bedeutung besitzen:
- P¹, P²: jeweils unabhängig voneinander eine polymerisierbare Gruppe,
- Sp¹, Sp²: bei jedem Auftreten gleich oder verschieden eine Abstandsgruppe oder eine Einfachbindung,
- A¹, A²,: jeweils unabhängig voneinander einen Rest ausgewählt aus folgenden Gruppen
a) der Gruppe bestehend aus trans-1,4-Cyclohexylen, 1,4-Cyclohexenylen und 4,4'-Bicyclohexylen, worin auch eine oder mehrere nicht benachbarte CH₂-Gruppen durch -O- und/oder -S- ersetzt sein können und worin auch ein oder mehrere H-Atome durch eine Gruppe L ersetzt sein können, oder ein Rest der Formel 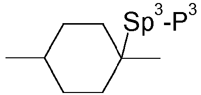
b) der Gruppe bestehend aus 1,4-Phenylen und 1,3-Phenylen, worin auch eine oder zwei CH-Gruppen durch N ersetzt sein können und worin auch ein oder mehrere H-Atome durch eine Gruppe L oder -Sp³-P ersetzt sein können,
c) der Gruppe bestehend aus Tetrahydropyran-2,5-diyl, 1,3-Dioxan-2,5-diyl, Tetrahydrofuran-2,5-diyl, Cyclobutan-1,3-diyl, Piperidin-1,4-diyl, Thiophen-2,5-diyl und Selenophen-2,5-diyl, welche auch ein oder mehrfach durch eine Gruppe L substituiert sein können,
d) der Gruppe bestehend aus gesättigten, teilweise ungesättigten oder vollständig ungesättigten, und optional substituierten, polycyclischen Resten mit 5 bis 20 cyclischen C-Atomen, von denen auch eines oder mehrere durch Heteroatome ersetzt sein können, vorzugsweise ausgewählt aus der Gruppe bestehend aus Bicyclo[1.1,1]pentan-1,3-diyl, Bicyclo[2.2.2]octan-1,4-diyl, Spiro[3.3]heptan-2,6-diyl, 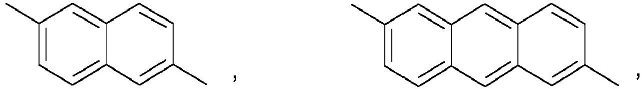 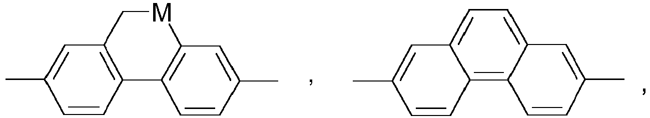 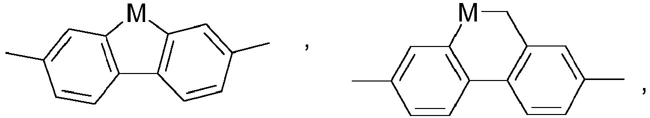 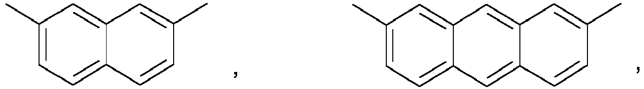 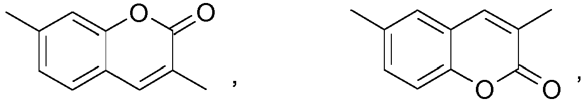 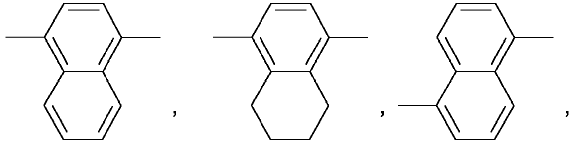 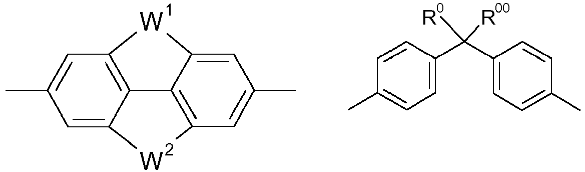 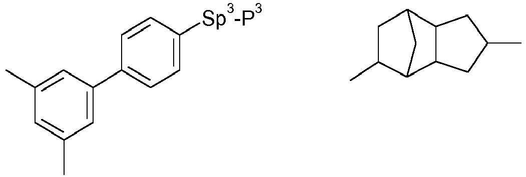 wobei in diesen Resten auch ein oder mehrere H- Atome durch eine Gruppe L oder -Sp³-P ersetzt sein können, und/oder eine oder mehrere Doppelbindungen durch Einfachbindungen ersetzt sein können, und/oder ein oder mehrere CH-Gruppen durch N ersetzt sein können,
- P³: eine polymerisierbare Gruppe,
- Sp³: eine Abstandsgruppe,
- n: 0, 1, 2 oder 3, bevorzugt 1 oder 2,
- Z¹: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist, -O-, -CO-, -C(R^{c}R^{d})-, -CH₂CF₂-, -CF₂CF₂-, oder eine Einfachbindung,
- L: bei jedem Auftreten gleich oder verschieden F, Cl, CN, SCN, SF₅ oder unverzweigtes oder verzweigtes, jeweils optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen,
- R⁰, R⁰⁰: jeweils unabhängig voneinander H, F oder unverzweigtes oder verzweigtes Alkyl mit 1 bis 12 C-Atomen, worin auch ein oder mehrere H-Atome durch F ersetzt sein können,
- M: -O-, -S-, -CH₂-, -CHY¹- oder -CY¹Y²- , und
- Y¹, und Y²: jeweils unabhängig voneinander eine der oben für R⁰ angegebenen Bedeutungen, Cl oder CN, und vorzugsweise H, F, Cl, CN, OCF₃ oder CF₃,
- W¹, W²: jeweils unabhängig voneinander -CH₂CH₂-, -CH=CH-, -CH₂-O-, -O-CH₂-, -C(R^{c}R^{d})- oder -O- bedeuten,
- R^{c} und R^{d}: jeweils unabhängig voneinander H oder Alkyl mit 1 bis 6 C-Atomen, vorzugsweise H, Methyl oder Ethyl, bedeuten,
wobei eine oder mehrere der Gruppen P¹-Sp¹-, -Sp²-P² und -Sp³-P³ einen Rest R^{aa} bedeuten können, mit der Maßgabe, dass mindestens eine der vorhandenen Gruppen P¹-Sp¹-, -Sp²-P² und -Sp³-P³ nicht R^{aa} bedeutet,
- R^{aa}: H, F, Cl, CN oder unverzweigtes oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹- ersetzt sein können, besonders bevorzugt unverzweigtes oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen), wobei die Gruppen -OH, -NH₂, -SH, -NHR, -C(O)OH und -CHO in R^{aa} nicht enthalten sind.

Die polymerisierbare Gruppe P, P¹, P² bzw. P³ in den vorangehenden und nachfolgenden Formeln ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine polymerisierbare C=C-Doppelbindung oder -C≡C-Dreifachbindung, sowie zur Polymerisation unter Ringöffnung geeignete Gruppen wie beispielsweise Oxetan- oder Epoxygruppen.

Bevorzugte Gruppen P/P¹/P²/P³ sind ausgewählt aus der Gruppe bestehend aus CH₂=CW¹-CO-O-, CH₂=CW¹-CO-, 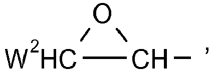 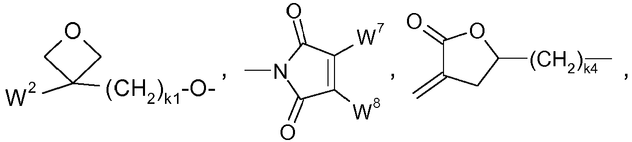 CH₂=CW²-O-, CW¹=CH-CO-(O)ₖ₃-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CH-(COO)ₖ₁-Phe-(O)ₖ₂-, CH₂=CH-(CO)ₖ₁-Phe-(O)ₖ₂-, Phe-CH=CH-, HOOC- und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit einem oder mehreren, von P-Sp- verschiedenen Resten L wie oben definiert substituiert ist, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Besonders bevorzugte Gruppen P/P¹/P²/P³ sind ausgewählt aus der Gruppe bestehend aus CH₂=CW¹-CO-O-, CH₂=CW¹-CO-, 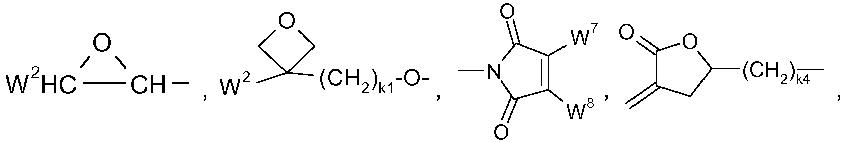 CH₂=CW²-O-, CW¹=CH-CO-(O)ₖ₃-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH- und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Ganz besonders bevorzugte Gruppen P/P¹/P²/P³ sind ausgewählt aus der Gruppe bestehend aus CH₂=CW¹-CO-O-, insbesondere CH₂=CH-CO-O-, CH₂=C(CH₃)-CO-O- und CH₂=CF-CO-O-, ferner CH₂=CH-O-, (CH₂=CH)₂CH-O-CO-, (CH₂=CH)₂CH-O-, 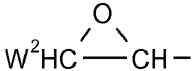 und

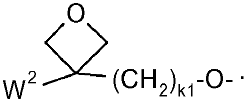

Ganz besonders bevorzugte Gruppen P/P¹/P²/P³ sind daher ausgewählt aus der Gruppe bestehend aus Acrylat-, Methacrylat-, Ethylacrylat-, Fluoracrylat-, ferner Vinyloxy-, Chloracrylat-, Oxetan- und Epoxygruppen, und unter diesen wiederum bevorzugt eine Acrylat- oder Methacrylatgruppe.

Bevorzugte Abstandsgruppen Sp, Sp¹, Sp² bzw. Sp³ sind eine Einfachbindung oder ausgewählt aus der Formel Sp"-X", so dass der Rest P^{(1/2)}-S_{P}^{(1/2)}- der Formel P^{1/2}-Sp"-X"- bzw. P-Sp"-X"- entspricht, wobei
- Sp": Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C≡C- ersetzt sein können, dass O- und/oder S-Atome nicht direkt miteinander verknüpft sind,
- X": -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- oder eine Einfachbindung bedeutet,
- R⁰⁰: jeweils unabhängig Alkyl mit 1 bis 12 C-Atomen bedeutet,
- R⁰⁰⁰: jeweils unabhängig H oder Alkyl mit 1 bis 12 C-Atomen bedeuten, und
- Y² und Y³: jeweils unabhängig voneinander H, F, Cl oder CN bedeuten.

X" ist vorzugsweise -O-, -S -CO-, -COO-, -OCO-, -O-COO-, oder eine Einfachbindung.

Typische Abstandsgruppen Sp" sind beispielsweise eine Einfachbindung, -(CH₂)ₚ₁-, -O-(CH₂)ₚ₁-, -(CH₂CH₂O)_{q1}-CH₂CH₂-, -CH₂CH₂-S-CH₂CH₂-, oder -(SiR⁰⁰R⁰⁰⁰-O)p1-, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -Sp"-X"- sind -(CH₂)ₚ₁-, -(CH₂)ₚ₁-O-, -(CH₂)ₚ₁-O-CO-, -(CH₂)ₚ₁-O-CO-O-, worin p1 und q1 die oben angegebene Bedeutung haben.

Besonders bevorzugte Gruppen Sp" sind beispielsweise jeweils unverzweigtes Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

Die Substanzen der Formel M enthalten im Allgemeinen und bevorzugt keine Ankergruppe, also z. B. keine Gruppe -OH, -NH₂, -SH, -C(O)OH und -CHO.

Geeignete und bevorzugte (Co-)Monomere für die Verwendung in erfindungsgemäßen Anzeigen sind beispielsweise ausgewählt aus den folgenden Formeln: 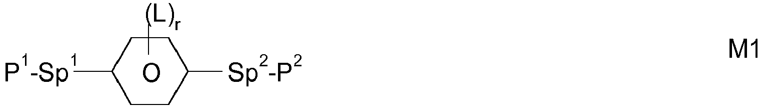 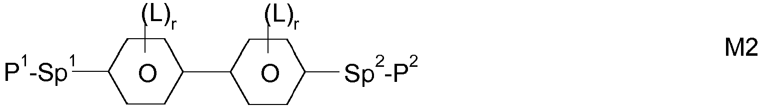 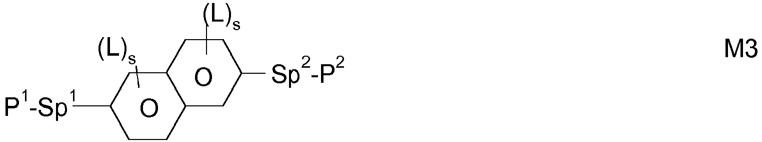 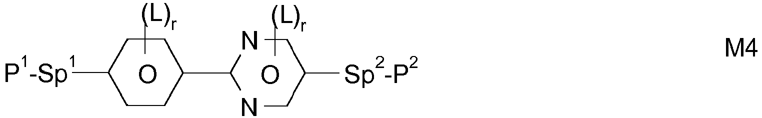 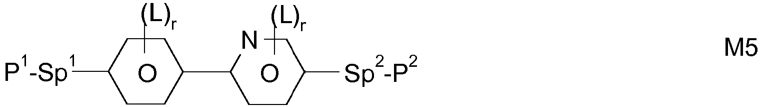 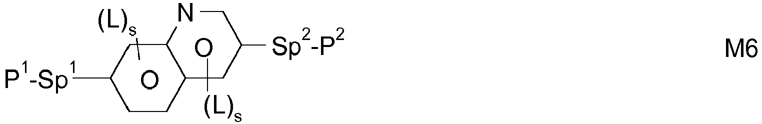 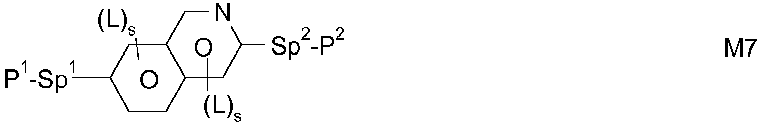 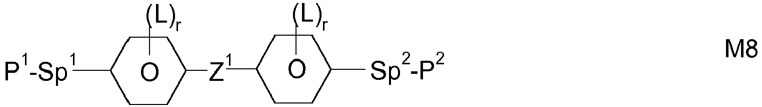 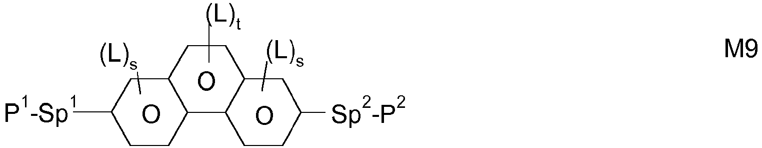 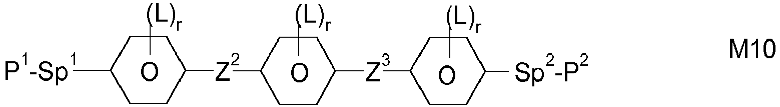 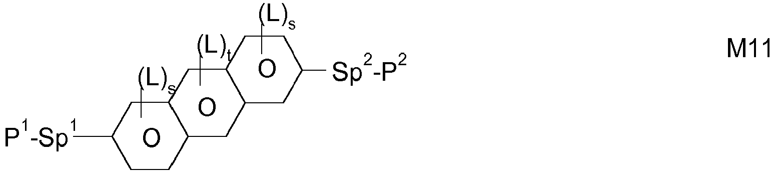 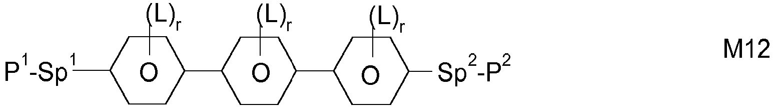 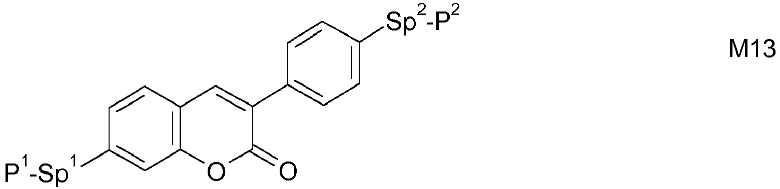 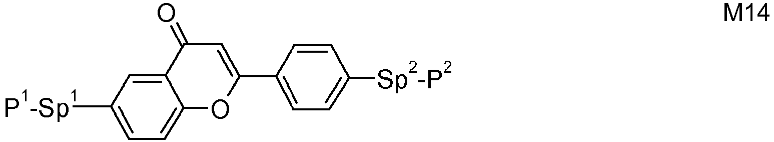 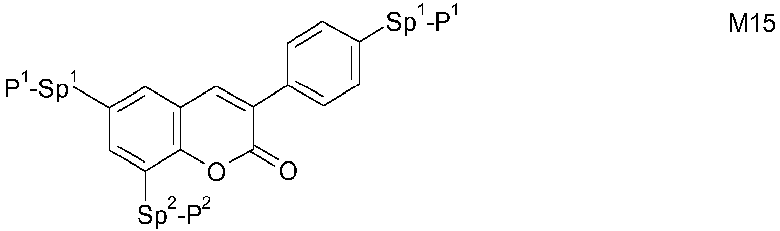 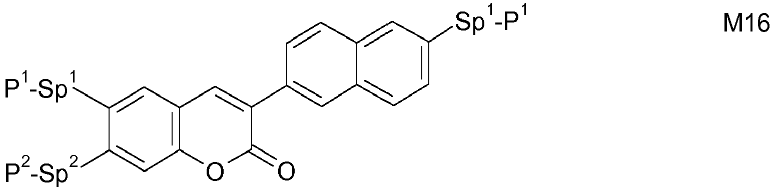 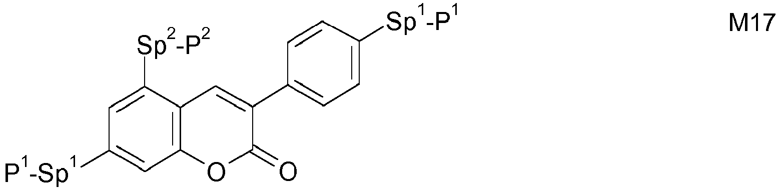 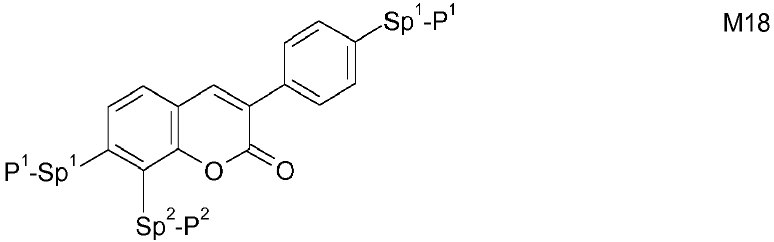 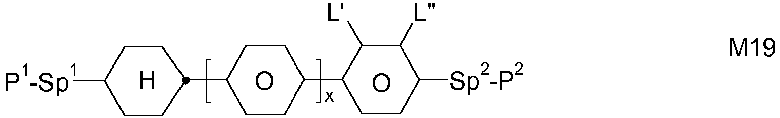 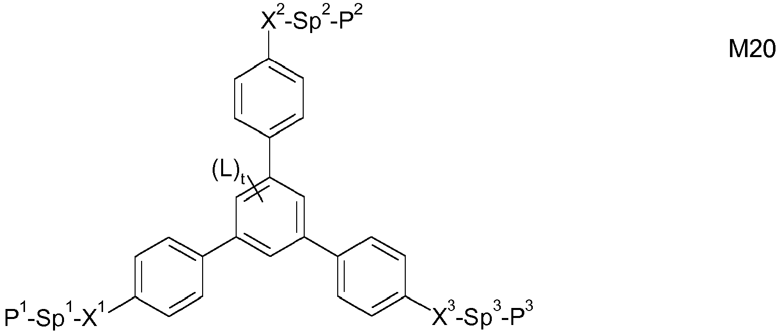 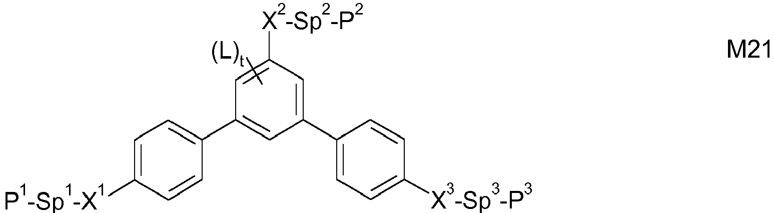 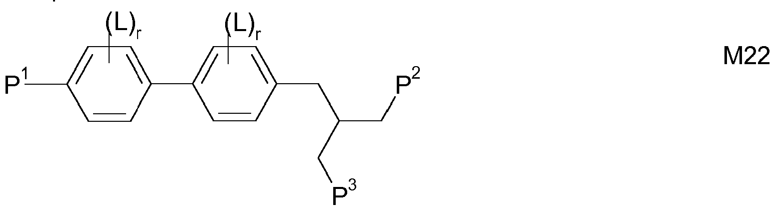 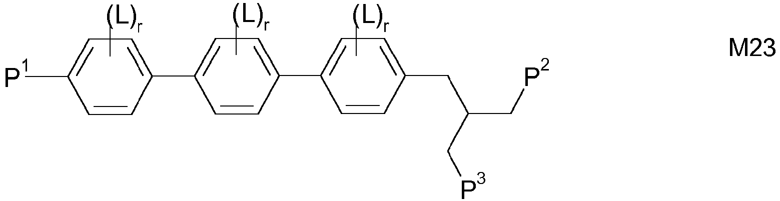 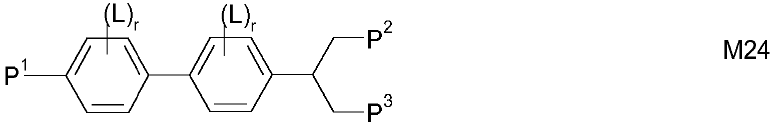 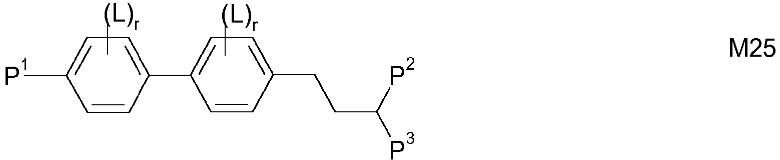 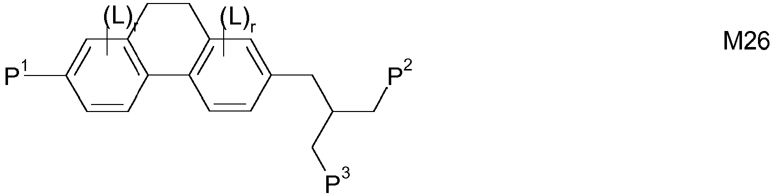 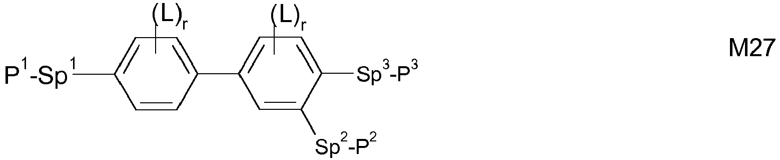 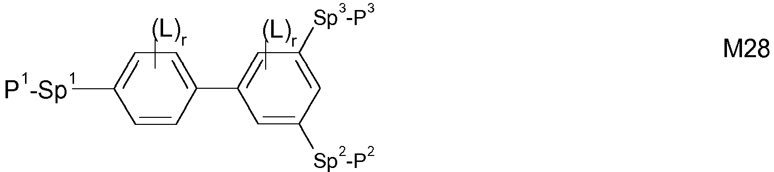 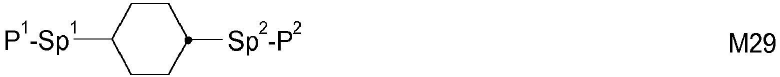 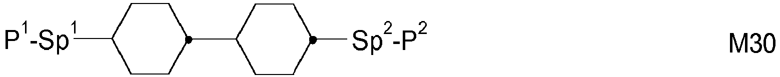 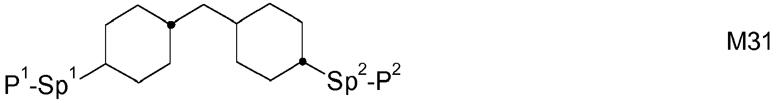 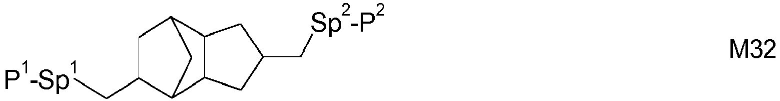 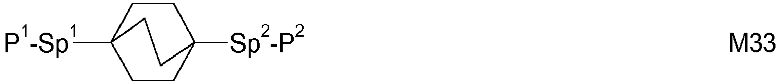 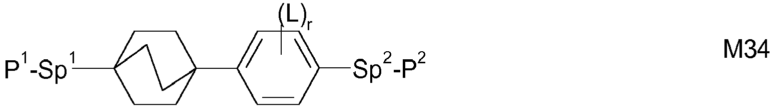 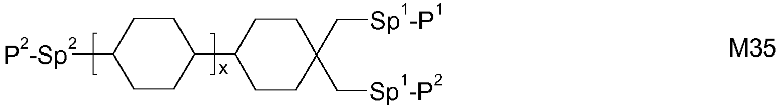 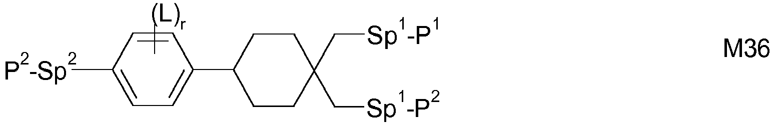 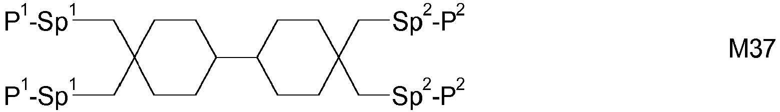 worin die einzelnen Reste folgende Bedeutung besitzen:
- P¹_{,}P² und P³: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Ethylacrylat-, Fluoracrylat-, Oxetan-, Vinyloxy- oder Epoxygruppe,
- Sp¹, Sp² und Sp³: jeweils unabhängig voneinander eine Einfachbindung oder eine Abstandsgruppe, vorzugsweise mit einer der vor- und nachstehend für Formel M angegebenen Bedeutungen, und besonders bevorzugt -(CH₂)ₚ₁-, -(CH₂)ₚ₁-O-, -(CH₂)ₚ₁-CO-O- oder -(CH₂)ₚ₁-O-CO-O-, worin p1 eine ganze Zahl von 1 bis 12 ist, und wobei in den letztgenannten Gruppen die Anbindung zum benachbarten Ring über das O-Atom erfolgt,
wobei auch einer oder mehrere der Reste P¹-Sp¹-, P²-Sp²- und P³-Sp³- einen Rest R^{aa} bedeuten können, mit der Maßgabe dass mindestens einer der vorhandenen Reste P¹-Sp¹-, P²-Sp²- und P³-Sp³- nicht R^{aa} bedeutet,
- R^{aa}: H, F, Cl, CN oder unverzweigtes oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -O-, -S-, -CO-, -CO-O-, -O-CO-, -O-CO-O-so ersetzt sein können, dass O- und/oder S-Atome nicht direkt miteinander verknüpft sind, und worin auch ein oder mehrere H-Atome durch F, Cl, CN oder P¹-Sp¹- ersetzt sein können, bevorzugt unverzweigtes oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen),
wobei -OH, -NH₂, -SH, -NHR, -C(O)OH und -CHO in der Gruppe R^{aa} nicht enthalten sind,
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- X¹, X² und X³: jeweils unabhängig voneinander -CO-O-, O-CO- oder eine Einfachbindung,
- Z¹: -O-, -CO-, -C(R^{y}R^{z})-, oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, SCN, SF₅ oder unverzweigtes oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen, vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

In den Verbindungen der Formeln M1 bis M37 bedeutet die Ringgruppe 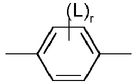 vorzugsweise 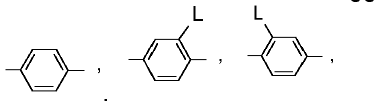 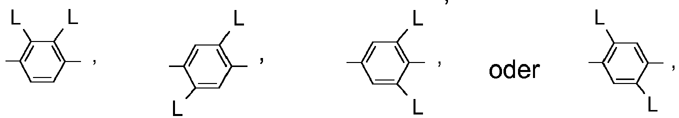 worin L, bei jedem Auftreten gleich oder verschieden, eine der vorstehenden Bedeutungen hat und vorzugsweise F, Cl, CN, NO₂, CH₃, C₂H₅, C(CH₃)₃, CH(CH₃)₂, CH₂CH(CH₃)C₂H₅, OCH₃, OC₂H₅, COCH₃, COC₂H₅, COOCH₃, COOC₂H₅, CF₃, OCF₃, OCHF₂, OC₂F₅ oder P-Sp-, besonders bevorzugt F, Cl, CN, CH₃, C₂H₅, OCH₃, COCH₃, OCF₃ oder P-Sp-, ganz besonders bevorzugt F, Cl, CH₃, OCH₃, COCH₃ oder OCF₃, insbesondere F oder CH₃ bedeutet.

Vorzugsweise umfasst das FK-Medium oder die polymerisierbare Komponente eine oder mehrere Verbindungen ausgewählt aus der Gruppe der Formeln M1-M28, besonders bevorzugt der Formeln M2-M15, darunter besonders bevorzugt der Formeln M2, M3, M9, M14 und M15. Vorzugsweise umfasst das FK-Medium oder die polymerisierbare Komponente keine Verbindungen der Formel M10, worin Z² und Z³ -(CO)O- oder -O(CO)-bedeuten.

Besonders bevorzugt sind somit beispielsweise die polymerisierbaren Verbindungen der Formel:

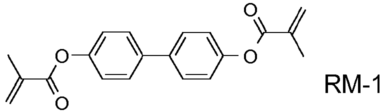

Zur Herstellung von PS-Anzeigen werden die polymerisierbaren Verbindungen im FK-Medium zwischen den Substraten der FK-Anzeige, optional unter Anlegen einer Spannung, durch in-situ-Polymerisation polymerisiert oder vernetzt (falls eine polymerisierbare Verbindung zwei oder mehr polymerisierbare Gruppen enthält). Die Polymerisation kann in einem Schritt durchgeführt werden. Es ist auch möglich, zunächst in einem ersten Schritt die Polymerisation unter Anlegen einer Spannung durchzuführen, um einen Pretilt-Winkel zu erzeugen, und anschließend in einem zweiten Polymerisationsschritt ohne anliegende Spannung die im ersten Schritt nicht abreagierten Verbindungen zu polymerisieren bzw. zu vernetzen ("end curing").

Geeignete und bevorzugte Polymerisationsmethoden sind beispielsweise die thermische oder Photopolymerisation, vorzugsweise Photopolymerisation, insbesondere UV-Photopolymerisation. Dabei können gegebenenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren lrgacure651®, lrgacure184®, lrgacure907®, lrgacure369®, oder Darocure1173® (Ciba AG). Falls ein Initiator eingesetzt wird, beträgt dessen Anteil vorzugsweise 0,001 bis 5 Gew.-%, besonders bevorzugt 0,001 bis 1 Gew.-%.

Die polymerisierbare Komponente oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox® (Ciba AG), wie beispielsweise Irganox® 1076. Falls Stabilisatoren eingesetzt werden, beträgt deren Anteil, bezogen auf die Gesamtmenge der RMs beziehungsweise der polymerisierbaren Komponente, vorzugsweise 10 - 10000 ppm, besonders bevorzugt 50 - 500 ppm.

Die FK-Medien zur Verwendung in den erfindungsgemäßen FK-Anzeigen enthalten, neben den oben beschriebenen Selbstorientierungsadditiven und den optionalen polymerisierbaren Verbindungen (M) eine niedermolekulare, nicht polymerisierbare Komnponente (FK-Mischung, "Host-Mischung") enthaltend eine oder mehr, vorzugsweise zwei oder mehr niedermolekulare (d.h. monomere bzw. unpolymerisierte, nicht polymerisierbare) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA- und VA-IPS-Anzeigen geeignete dielektrisch negative bzw. positive FK-Mischung. Der Anteil der Host-Mischung beträgt für Flüssigkristallanzeigen in der Regel 95 Gew.-% oder mehr, bevorzugt 97 Gew.-% oder mehr.

Geeignete FK-Mischungen sind dem Fachmann bekannt und in der Literatur beschrieben. FK-Medien für VA-Anzeigen mit negativer dielektrischer Anisotropie sind z. B. in EP 1 378 557 A1 oder WO 2013/004372 beschrieben. Geeignete FK-Mischungen mit positiver dielektrischer Anisotropie, die sich für LCDs und speziell für IPS-Anzeigen eignen, sind z.B. aus JP 07-181 439 (A), EP 0 667 555, EP 0 673 986, DE 195 09 410, DE 195 28 106, DE 195 28 107, WO 96/23 851 und WO 96/28 521 bekannt.

Im Folgenden werden bevorzugte Ausführungsformen für das erfindungsgemäße flüssigkristalline Medium mit negativer dielektrischer Anisotropie angeführt:
Das FK-Medium enthält vorzugsweise zusätzlich eine oder mehrere Verbindungen ausgewählt aus der Gruppe der Verbindungen der Formeln A, B und C, 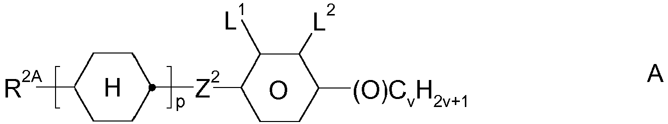 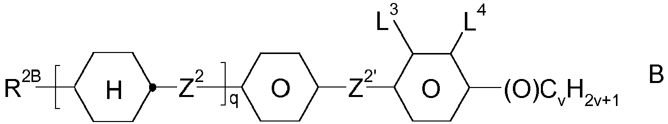 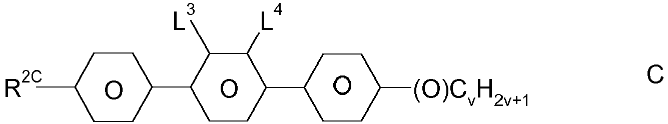 worin
- R^{2A}, R^{2B} und R^{2C}: jeweils unabhängig voneinander H, einen unsubstituierten, einen einfach durch CN oder CF₃ oder mindestens einfach durch Halogen substituierten Alkylrest mit bis zu 15 C-Atomen, wobei in diesen Resten auch eine oder mehrere CH₂-Gruppen durch -O-, -S-, 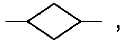 -C≡C-, -CH=CH-, -CF₂O-, -OCF₂-, -OC-O- oder -O-CO- so ersetzt sein können, dass O-Atome nicht direkt miteinander verknüpft sind,
- L¹⁻⁴: jeweils unabhängig voneinander F, Cl, CF₃ oder CHF₂,
- Z² und Z^{2'}: jeweils unabhängig voneinander Einfachbindung, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
- p: 1 oder 2, bevorzugt 1,
- q: 0 oder 1,
- (O): -O- oder eine Einfachbindung, und
- v: 1 bis 6
bedeuten.

In den Verbindungen der Formeln A und B können Z² gleiche oder unterschiedliche Bedeutungen haben. In den Verbindungen der Formel B können Z² und Z²' gleiche oder verschiedene Bedeutungen aufweisen. In den Verbindungen der Formeln A, B und C bedeuten R^{2A}, R^{2B} und R^{2C} jeweils vorzugsweise Alkyl mit 1-6 C-Atomen, insbesondere CH₃, C₂H₅, n-C₃H₇, n-C₄H₉, n-C₅H₁₁.

In den Verbindungen der Formeln A und B bedeuten L¹, L², L³ und L⁴ vorzugsweise L¹ = L² = F und L³ = L⁴ = F, ferner L¹ = F und L² = Cl, L¹ = Cl und L² = F, L³ = F und L⁴ = Cl, L³ = Cl und L⁴ = F. Z² und Z²' bedeuten in den Formeln A und B vorzugsweise jeweils unabhängig voneinander eine Einfachbindung, ferner eine -C₂H₄-Brücke.

Sofern in der Formel B Z² = -C₂H₄- ist, ist Z²' vorzugsweise eine Einfachbindung bzw. falls Z²' = -C₂H₄- bedeutet, ist Z² vorzugsweise eine Einfachbindung. In den Verbindungen der Formeln A und B bedeutet (O)CᵥH₂ᵥ₊₁ vorzugsweise OCᵥH₂ᵥ₊₁, ferner CᵥH₂ᵥ₊₁. In den Verbindungen der Formel C bedeutet (O)CᵥH₂ᵥ₊₁ vorzugsweise CᵥH₂ᵥ₊₁. In den Verbindungen der Formel C bedeuten L³ und L⁴ vorzugsweise jeweils F.

Bevorzugte Verbindungen der Formeln A, B und C sind beispielsweise: 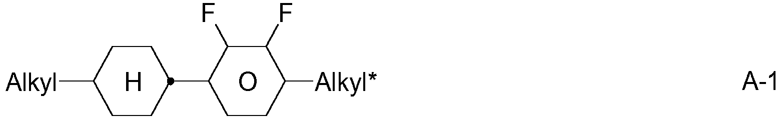 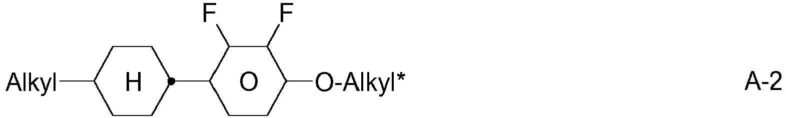 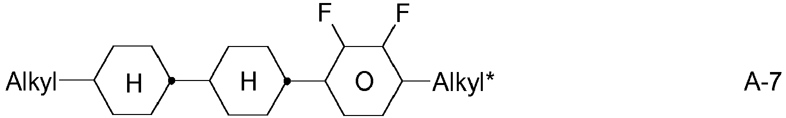 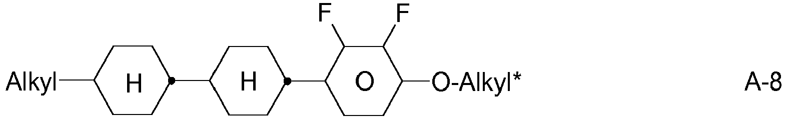 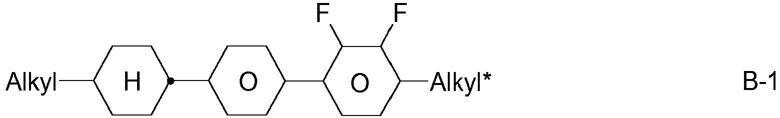 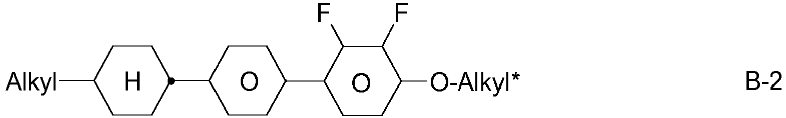 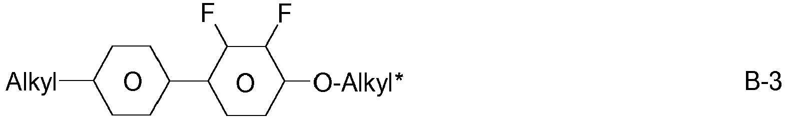 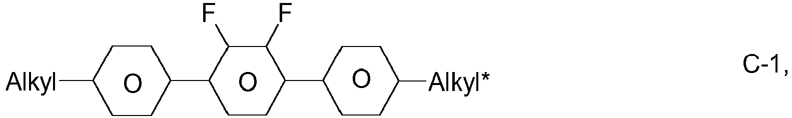 worin Alkyl und Alkyl* jeweils unabhängig voneinander einen unverzweigten Alkylrest mit 1-6 C-Atomen bedeuten.

Das FK-Medium enthält bevorzugt zusätzlich eine oder mehrere Verbindungen der Formel D, 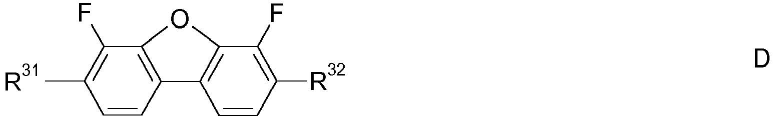 worin
- R³¹, R³²: unabhängig voneinander einen unsubstituierten Alkylrest mit 1 bis 7 C-Atomen, bevorzugt einen n-Alkylrest, besonders bevorzugt mit 2 bis 5 C-Atomen, oder einen unsubstituierten Alkoxyrest mit 2 bis 7 C-Atomen, besonders bevorzugt mit 2 bis 5 C-Atomen,
bedeuten,
wobei bevorzugt mindestens einer der Reste R³¹ und R³² Alkoxy bedeutet.

Das FK-Medium weist vorzugsweise ein Δε von -1,5 bis -8,0, insbesondere von -2,5 bis -6,0 auf.

In einer weiteren bevorzugten Ausführungsform enthält das Medium eine oder mehrere Verbindungen der Formeln D-1 bis D-3 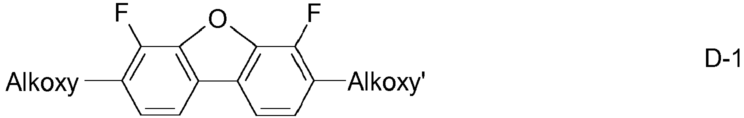 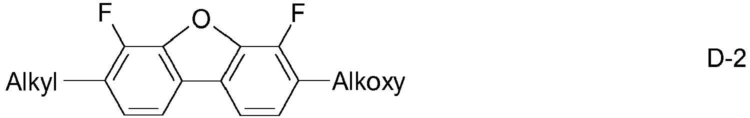 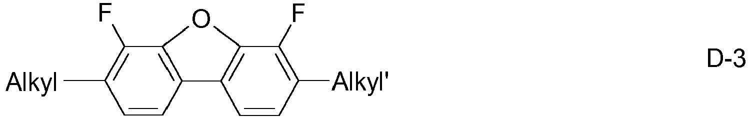 worin
Alkyl, Alkyl' Alkyl mit 1 bis 7 C-Atomen, bevorzugt mit 2-5 C-Atomen, und Alkoxy, Alkoxy' Alkoxy mit 1 bis 7 C-Atomen, bevorzugt mit 2 bis 5 C-Atomen, bedeuten.

Bevorzugt enthält das Medium eine oder mehrere Verbindungen der Formel E: 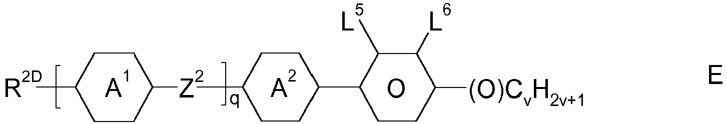 worin
- A¹, A²: unabhängig eine Ringgruppe ausgewählt aus den Formeln 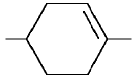 oder 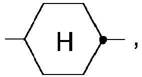 wobei Formel D mindestens eine Gruppe 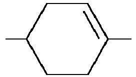 umfasst,
- R^{2D}: unabhängig H, einen unsubstituierten, einen einfach durch CN oder CF₃ oder mindestens einfach durch Halogen substituierten Alkylrest mit bis zu 15 C-Atomen, wobei in diesen Resten auch eine oder mehrere CH₂-Gruppen durch -O-, -S-, 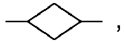 -C≡C-, -CF₂O-, -OCF₂-, -OC-O- oder -O-CO- so ersetzt sein können, dass O-Atome nicht direkt miteinander verknüpft sind,
- L⁵, L⁶: jeweils unabhängig voneinander F, Cl, CF₃ oder CHF₂,
- Z²: unabhängig eine Einfachbindung, -CH₂CH₂-, -CH=CH-, -CF₂O-, -OCF₂-, -CH₂O-, -OCH₂-, -COO-, -OCO-, -C₂F₄-, -CF=CF- oder -CH=CHCH₂O-,
- q: 0 oder 1,
- (O): -O- oder eine Einfachbindung, und
- v: 1 bis 6
bedeuten.

Die Werte der Doppelbrechung Δn in der Flüssigkristallmischung liegen in der Regel zwischen 0,07 und 0,16, vorzugsweise zwischen 0,08 und 0,12. Die Rotationsviskosität γ₁ bei 20 °C vor der Polymerisation ist vorzugsweise ≤ 165 mPa·s, insbesondere ≤ 140 mPa·s.

Im Folgenden werden bevorzugte Ausführungsformen für das erfindungsgemäße flüssigkristalline Medium mit negativer oder positiver dielektrischer Anisotropie angeführt:

LC-Medium, welches zusätzlich eine oder mehrere Verbindungen der Formel II und/oder III enthält: 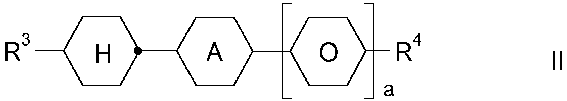 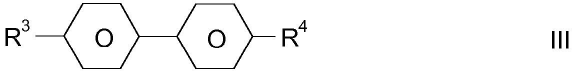 worin
- Ring A: 1,4-Phenylen oder trans-1,4-Cyclohexylen bedeutet,
- a: 0 oder 1 ist,
- R³: jeweils unabhängig voneinander Alkyl mit 1 bis 9 C-Atomen oder Alkenyl mit 2 bis 9 C-Atomen bedeutet, vorzugsweise Alkenyl mit 2 bis 9 C-Atomen, und
- R⁴: jeweils unabhängig voneinander einen unsubstituierten oder halogenierten Alkylrest mit 1 bis 12 C-Atomen, wobei auch eine oder zwei nicht benachbarte CH₂-Gruppen durch -O-, -CH=CH-, -CH=CF-, -(CO)-, -O(CO)- oder -(CO)O- so ersetzt sein können, dass O-Atome nicht direkt miteinander verknüpft sind, und vorzugsweise Alkyl mit 1 bis 12 C-Atomen oder Alkenyl mit 2 bis 9 C-Atomen bedeutet.

Die Verbindungen der Formel II sind vorzugsweise ausgewählt aus der Gruppe bestehend aus folgenden Formeln: 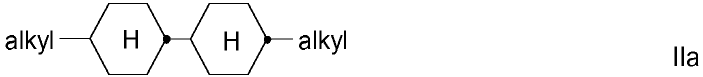 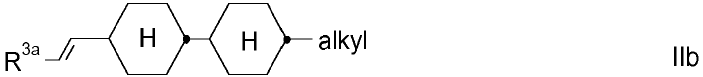 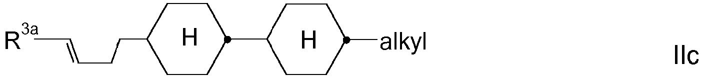 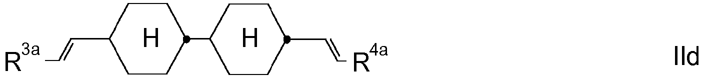 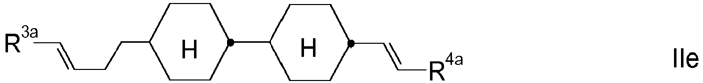 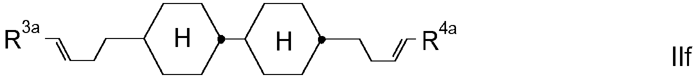 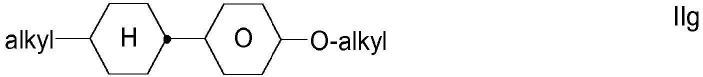 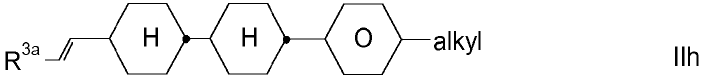 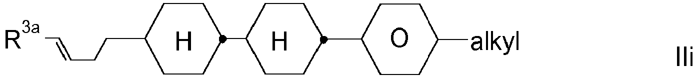 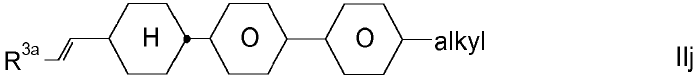 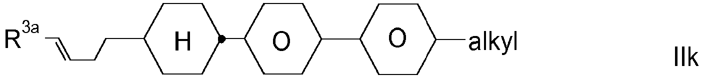 worin R^{3a} und R^{4a} jeweils unabhängig voneinander H, CH₃, C₂H₅ oder C₃H₇ bedeuten, und "alkyl" jeweils unabhängig eine unverzweigte Alkylgruppe mit 1 bis 8, vorzugsweise 1, 2, 3, 4 oder 5 C-Atomen bedeutet. Besonders bevorzugt sind Verbindungen der Formel IIa, IIb, IIg und IIh, insbesondere solche, worin R^{3a} H oder CH₃, vorzugsweise H, bedeutet, und Verbindungen der Formel IId, insbesondere solche, worin R^{3a} und R^{4a} H, CH₃ oder C₂H₅ bedeuten.

Im Folgenden werden bevorzugte Ausführungsformen für das erfindungsgemäße flüssigkristalline Medium mit positiver dielektrischer Anisotropie angeführt:

Das FK-Medium enthält bevorzugt eine oder mehrere Verbindungen der Formeln IV und V: 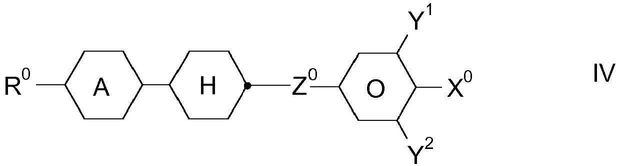 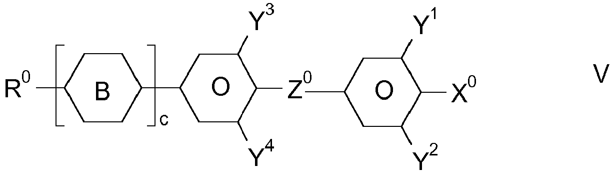 worin
- R⁰: Alkyl- oder Alkoxyrest mit 1 bis 15 C Atomen, worin optional zusätzlich eine oder mehrere CH₂ Gruppen in diesen Resten, unabhängig voneinander, durch -C≡C-, -CF₂O-, -CH=CH-, 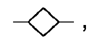 , 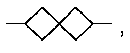 , -O-, -(CO)O- oder -O(CO)- substituiert sind, so dass O-Atome nicht direkt miteinander verknüpft sind, und in denen zusätzlich ein oder mehrere H-Atome optional durch Halogen ersetzt sein können,
- Ring A: 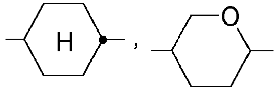 oder 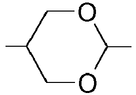
- Ring B: unabhängig voneinander 1,4-Phenylen, optional substituiert durch ein oder zwei F oder Cl, 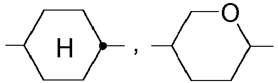 oder 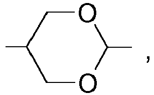
- X⁰: F, Cl, CN, SF₅, SCN, NCS, eine halogenierte Alkylgruppe, eine halogenierte Alkenylgruppe, eine halogenierte Alkoxygruppe oder eine halogenierte Alkenyloxygruppe, jeweils mit bis zu 6 C-Atomen,
- Y¹⁻⁴: jeweils unabhängig voneinander H oder F,
- Z⁰: -CF₂O-, -(CO)O- oder eine Einfachgruppe, und
- c: 0, 1 oder 2, bevorzugt 1 oder 2. 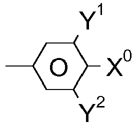 bedeutet bevorzugt 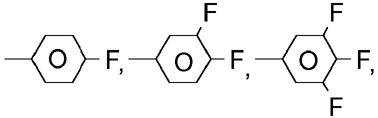 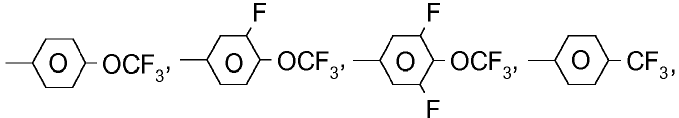 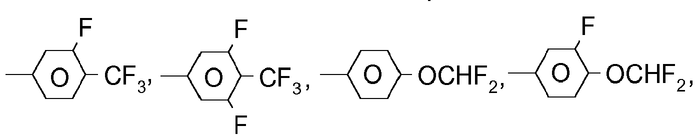 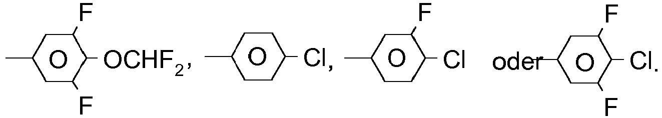
- R⁰: bedeutet bevorzugt unverzweigtes Alkyl oder Alkenyl mit 2 bis 7 C-Atomen;
- X⁰: bedeutet bevorzugt F, OCF₃, Cl oder CF₃, insbesondere F.

Die nematische Phase des dielektrisch negativen oder positiven FK-Mediums gemäß der Erfindung hat bevorzugt eine nematische Phasen in einem Temperaturbereich von 10 °C oder weniger bis 60 °C oder mehr, besonders bevorzugt von 0 oder weniger bis 70 °C oder mehr.

Im Rahmen der vorliegenden Anmeldung sind die beiden Formeln für substituierte Benzolringe 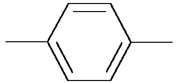 und 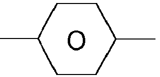 gleichbedeutend. 1,4-substituiertes Cyclohexan wir durch 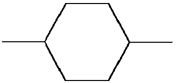 oder 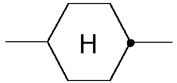 wiedergegeben, welches vorzugweise 1,4-trans-konfiguriert ist.

Ein durch die Gruppe L substituierter Phenylenring der
Formel 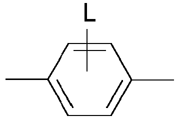 ist an genau einer beliebigen Stelle durch eine Gruppe L substituiert. Der Substituent (L)ᵣ steht entsprechend für Anzahl r der Substituenten L an verschiedenen freien Stellen.

In der vorliegenden Erfindung und insbesondere in den folgenden Beispielen sind die Strukturen der mesogenen Verbindungen durch Abkürzungen angegeben, die auch als Akronyme bezeichnet werden. In diesen Akronymen sind die chemischen Formeln unter Verwendung der folgenden Tabellen A bis C wie folgt abgekürzt. Alle Gruppen CₙH₂ₙ₊₁, CₘH₂ₘ₊₁ und CₗH₂ₗ₊₁ bzw. CₙH₂ₙ₋₁, CₘH₂ₘ₋₁ und CₗH₂ₗ₋₁ bedeuten unverzweigtes Alkyl bzw. Alkenyl, vorzugsweise 1-E-Alkenyl, jeweils mit n, m bzw. I C-Atomen. In der Tabelle A werden die für die Ringelemente der Kernstrukturen der Verbindungen verwendeten Codes aufgeführt, während in der Tabelle B die Verknüpfungsgruppen gezeigt sind. Tabelle C gibt die Bedeutungen der Codes für die Endgruppen der linken bzw. rechten Seite. Die Akronyme werden aus den Codes für die Ringelemente mit optionalen Verknüpfungsgruppen, gefolgt von einem ersten Bindestrich und den Codes für die linke Endgruppe, sowie einem zweiten Bindestrich und den Codes für die rechts Endgruppe, zusammengesetzt. In Tabelle D sind Beispielstrukturen von Verbindungen mit ihren jeweiligen Abkürzungen zusammengestellt.

**Tabelle A: Ringelemente**

| | | | |
|---|---|---|---|
| **C** | 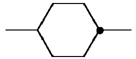 | **P** | 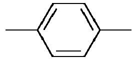 |
| **D** | 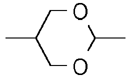 | **DI** | 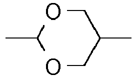 |
| **A** | 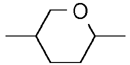 | **AI** | 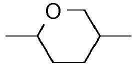 |
| **G** | 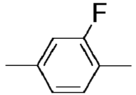 | **Gl** | 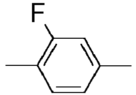 |
| **U** | 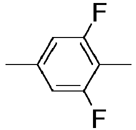 | **UI** | 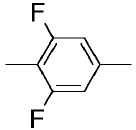 |
| **M** | 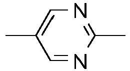 | **MI** | 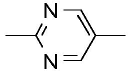 |
| **N** | 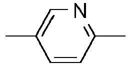 | **NI** | 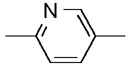 |
| **Y** | 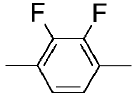 | | |
| **P(F, CI)Y** | 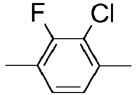 | **P(CI,F)Y** | 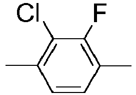 |
| **Np** | 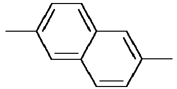 | **dH** | 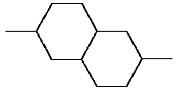 |
| **n3f** | 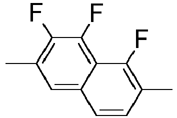 | **n3fI** | 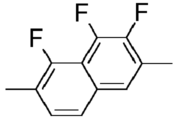 |
| **tH** | 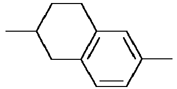 | **tHI** | 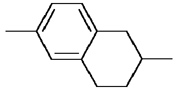 |
| **tH2f** | 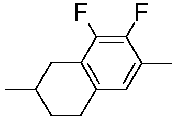 | **tH2fI** | 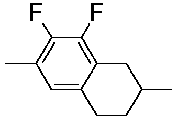 |
| **o2f** | 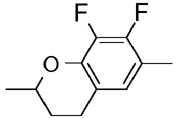 | **o2fI** | 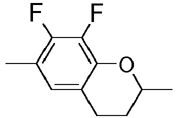 |
| **dh** | 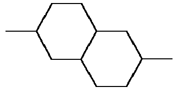 | **B** | 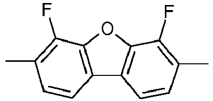 |
| **K** | 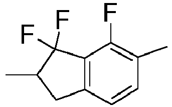 | **KI** | 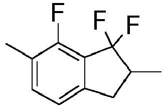 |
| **L** | 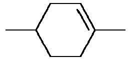 | **LI** | 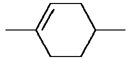 |
| **F** | 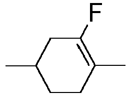 | **FI** | 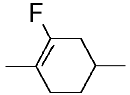 |
| **Nf** | 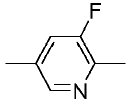 | **Nfl** | 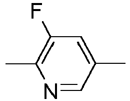 |

**Tabelle B: Brückenglieder**

| | | | |
|---|---|---|---|
| **E** | -CH₂-CH₂- | | |
| **V** | -CH=CH- | | |
| **T** | -C≡C- | | |
| **W** | -CF₂-CF₂- | | |
| **B** | -CF=CF- | | |
| **Z** | -CO-O- | **ZI** | -O-CO- |
| **X** | -CF=CH- | **XI** | -CH=CF- |
| **O** | -CH₂-O- | **OI** | -O-CH₂- |
| **Q** | -CF₂-O- | **QI** | -O-CF₂- |

**Tabelle C: Endgruppen**

| **Links einzelstehend oder in Kombination** | | **Rechts einzelstehend oder in Kombination** | |
|---|---|---|---|
| **-**n- | CₙH₂ₙ₊₁- | **-n** | -CₙH₂ₙ₊₁ |
| -**nO**- | CₙH₂ₙ₊₁-O- | -**On** | -O-CₙH₂ₙ₊₁ |
| **-V-** | CH₂=CH- | **-V** | -CH=CH₂ |
| **-nV-** | CₙH₂ₙ₊₁-CH=CH- | **-nV** | -CₙH₂ₙ-CH=CH₂ |
| **-Vn-** | CH₂=CH-CₙH₂ₙ- | **-Vn** | -CH=CH-CₙH₂ₙ₊₁ |
| **-nVm-** | CₙH₂ₙ₊₁-CH=CH-CₘH₂ₘ- | **-nVm** | -CₙH₂ₙ-CH=CH-CₘH₂ₘ₊₁ |
| **-N-** | N≡C- | **-N** | -C≡N |
| **-S-** | S=C=N- | **-S** | -N=C=S |
| **-F-** | F- | **-F** | -F |
| **-CL-** | Cl- | **₋CL** | -Cl |
| **-M-** | CFH₂- | **-M** | -CFH₂ |
| **-D-** | CF₂H- | **-D** | -CF₂H |
| **-T-** | CF₃- | **-T** | -CF₃ |
| **-MO-** | CFH₂O - | **-OM** | -OCFH₂ |
| **-DO-** | CF₂HO - | **-OD** | -OCF₂H |
| **-TO-** | CF₃O - | **-OT** | -OCF₃ |
| **-A-** | H-C≡C- | **-A** | -C≡C-H |
| **-nA-** | CₙH₂ₙ₊₁-C≡C- | **-An** | -C≡C-CₙH₂ₙ₊₁ |
| **-NA-** | N≡C-C≡C- | **-AN -OXF** | -C≡C-C≡N -O-CH=CF2 |

| **Links nur in Kombination** | | **Rechts nur in Kombination** | |
|---|---|---|---|
| **-...n...-** | -CₙH₂ₙ- | **-...n...** | -CₙH₂ₙ- |
| **-...M...-** | -CFH- | **-...M...** | -CFH- |
| **-...D...-** | -CF₂- | **-...D...** | -CF₂- |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W... -...X...** | -CF=CF- -CH=CF- |

worin n und m jeweils ganze Zahlen und die drei Punkte "..." Platzhalter für andere Abkürzungen aus dieser Tabelle sind.

In der folgenden Tabelle werden Beispielstrukturen zusammen mit ihren jeweiligen Abkürzungen angegeben. Diese werden gezeigt, um die Bedeutung der Regeln für die Abkürzungen zu demonstrieren. Vorzugsweise enthalten die erfindungsgemäßen Mischungen neben den Verbindungen der Formeln I eine oder mehrere Verbindungen der nachfolgend genannten Verbindungen.

Folgende Abkürzungen werden verwendet:
(n, m und z unabhängig voneinander jeweils eine ganze Zahl, bevorzugt 1 bis 6).

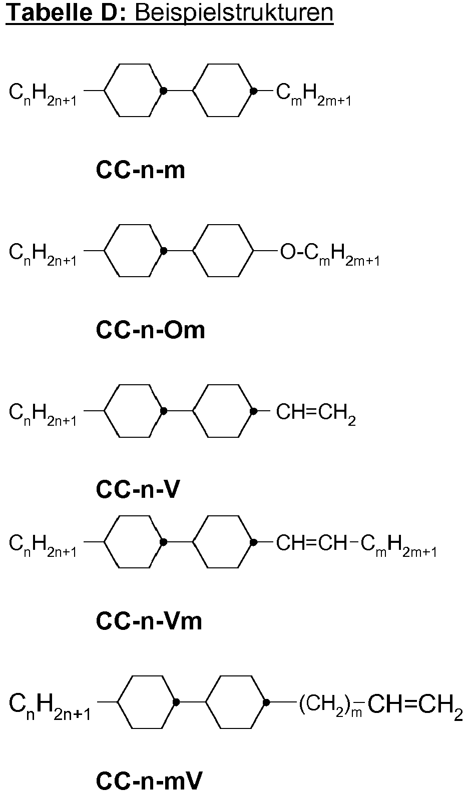

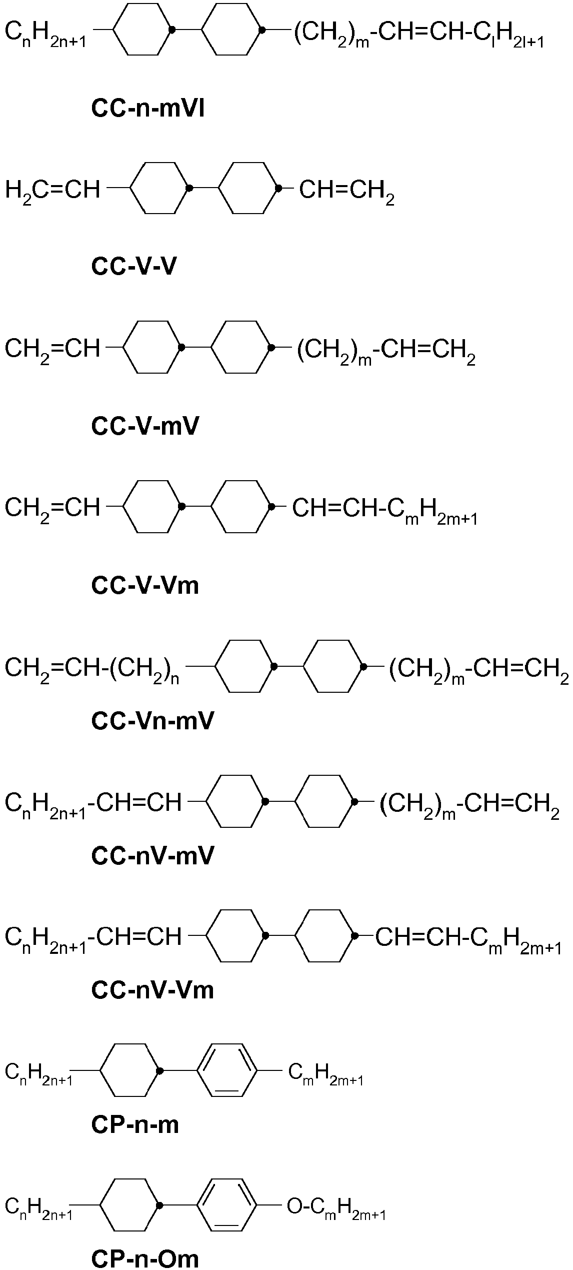

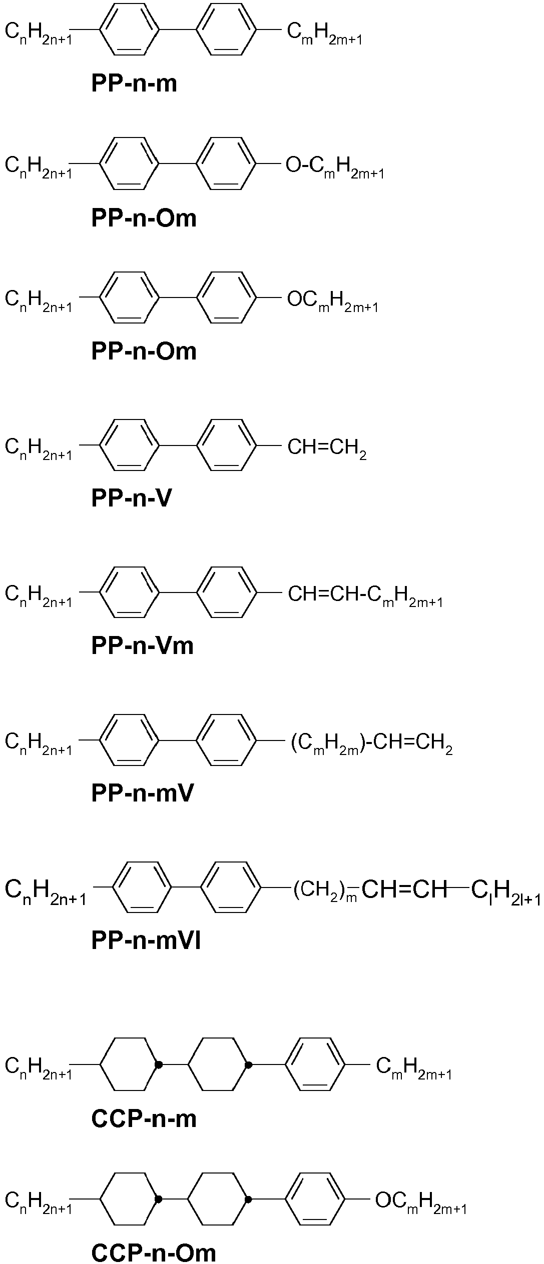

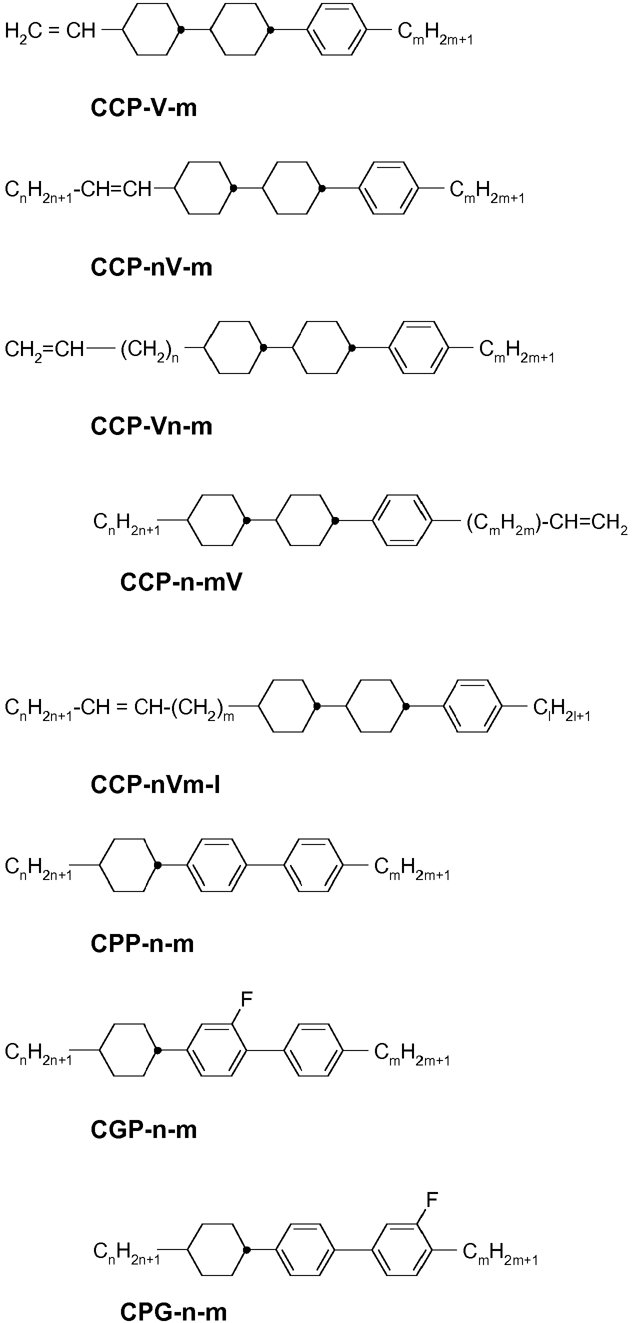

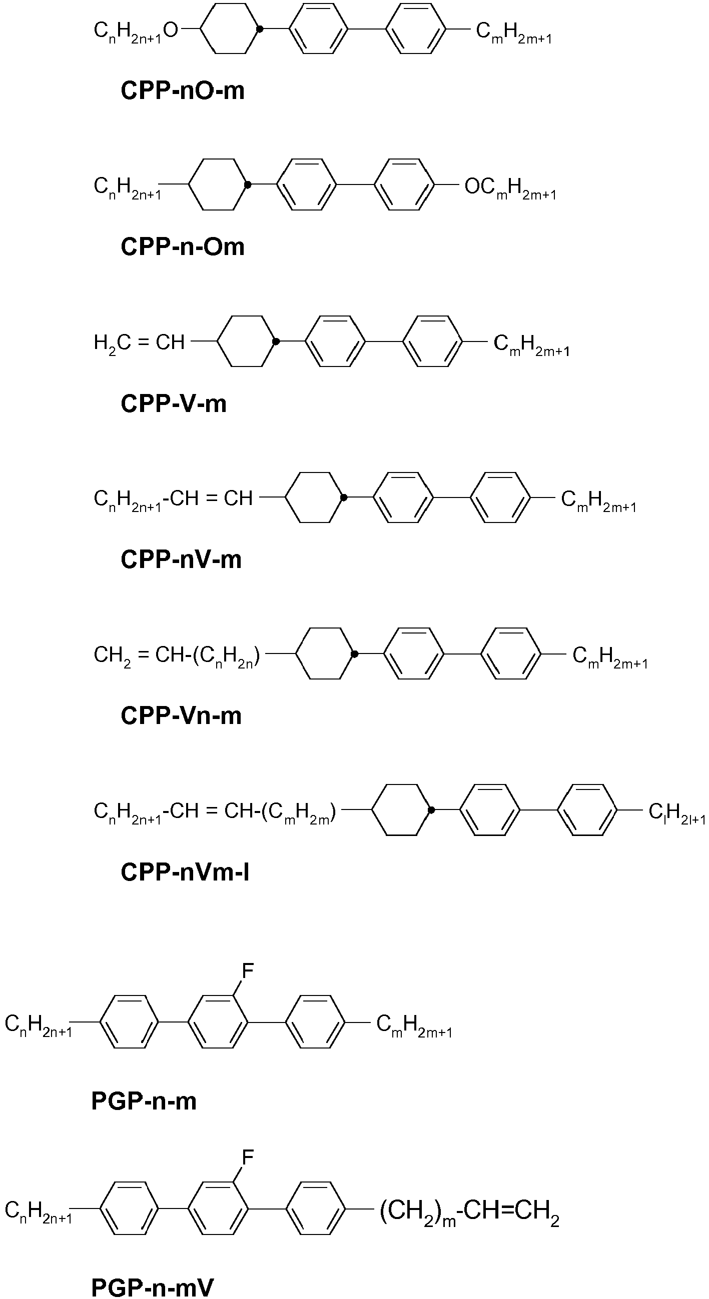

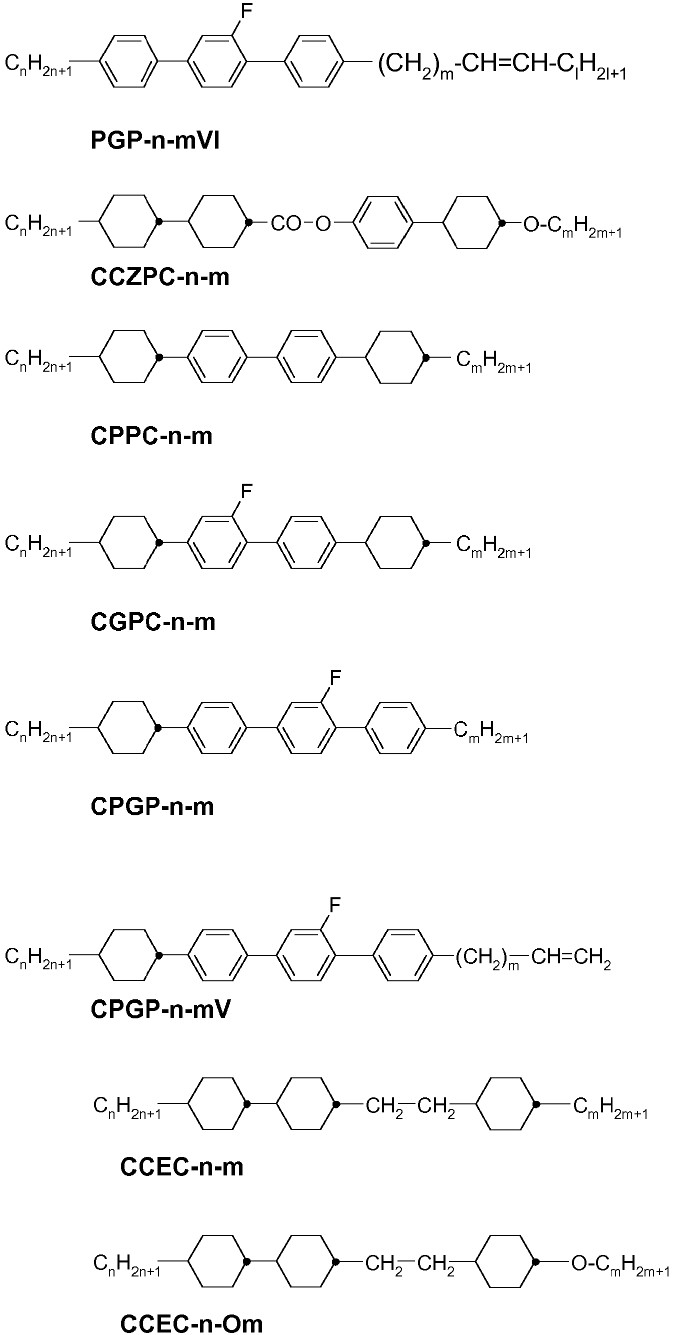

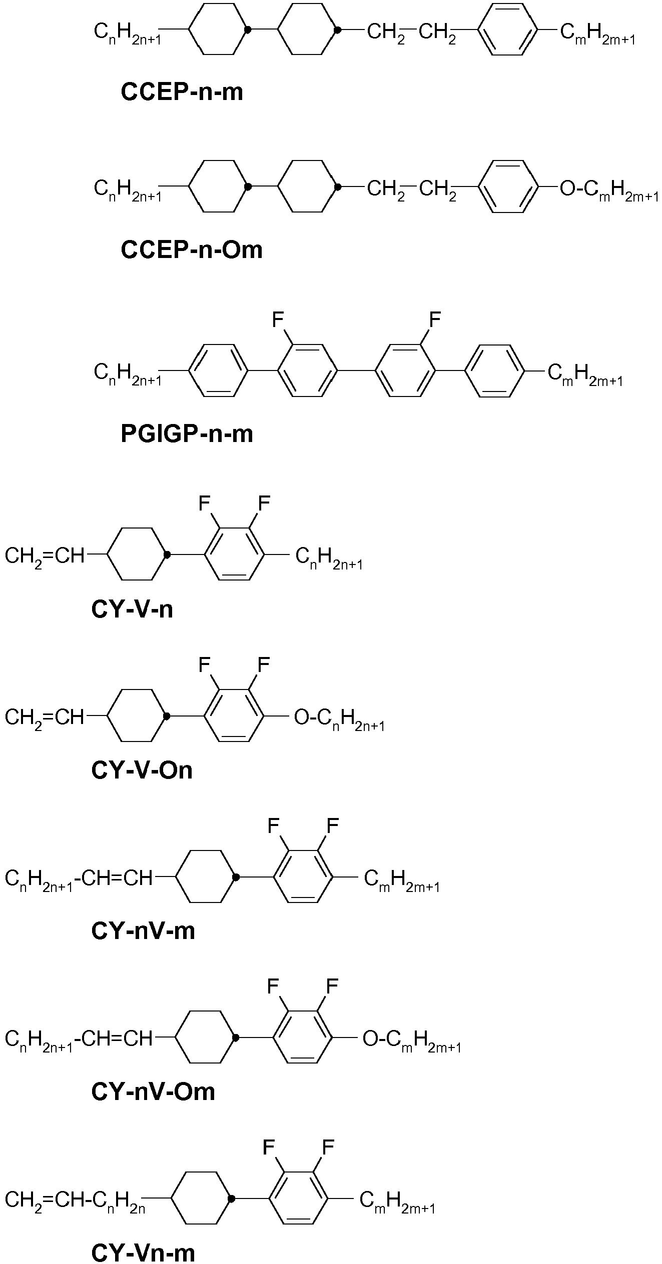

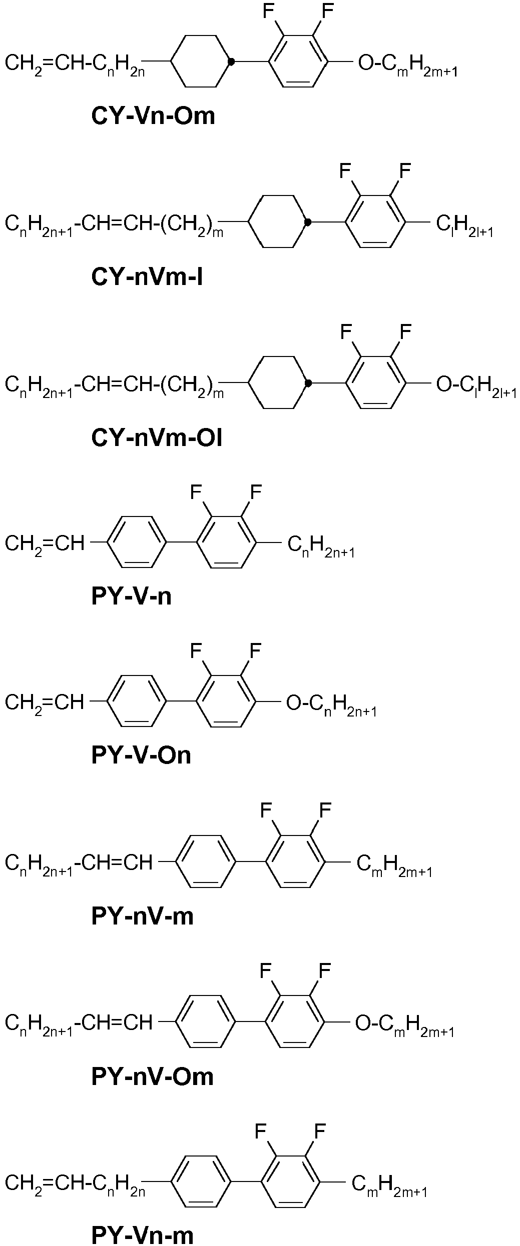

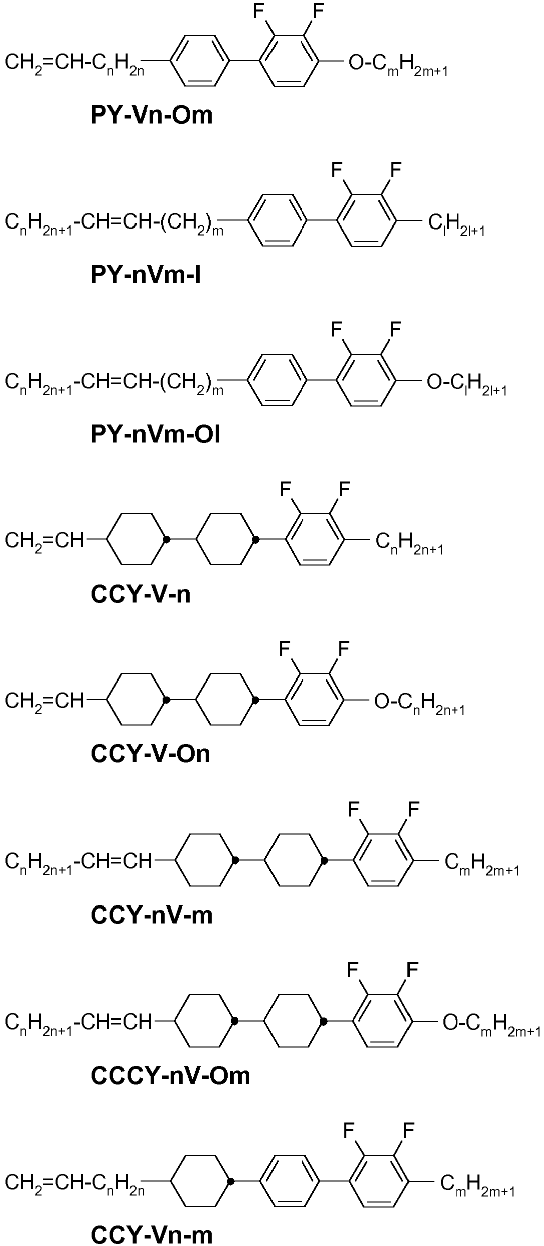

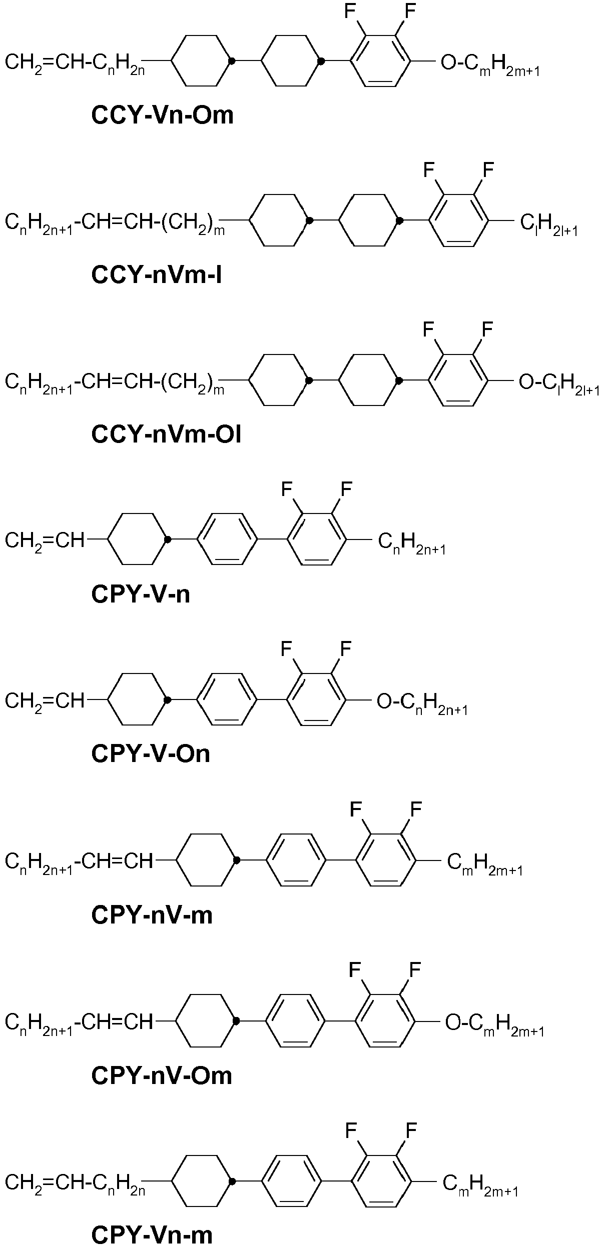

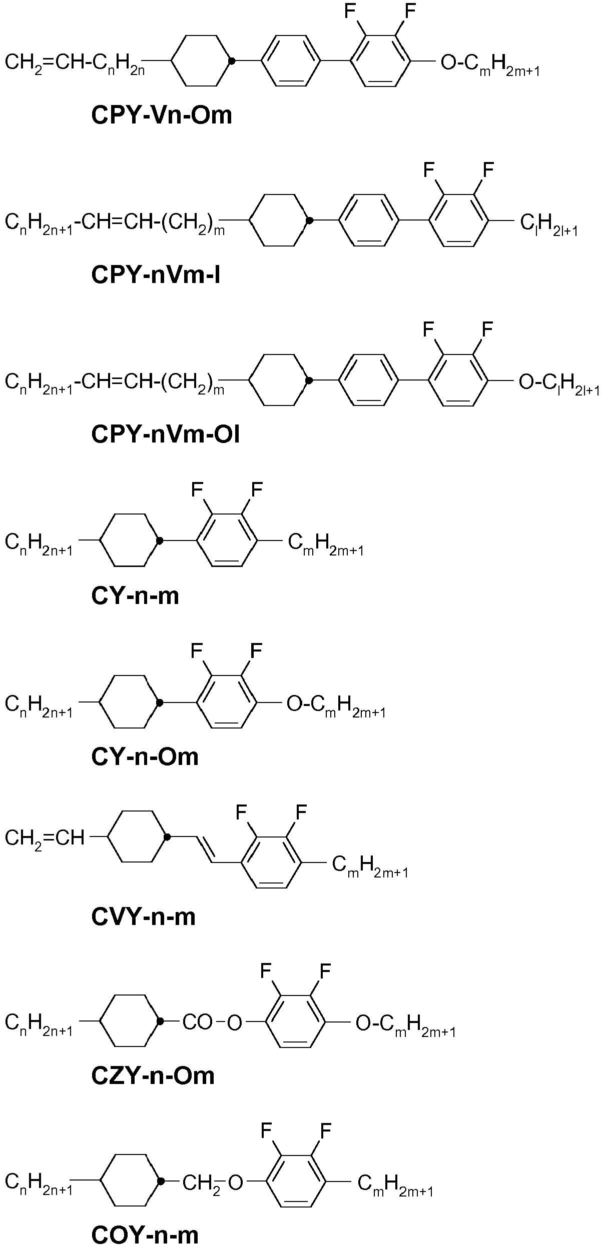

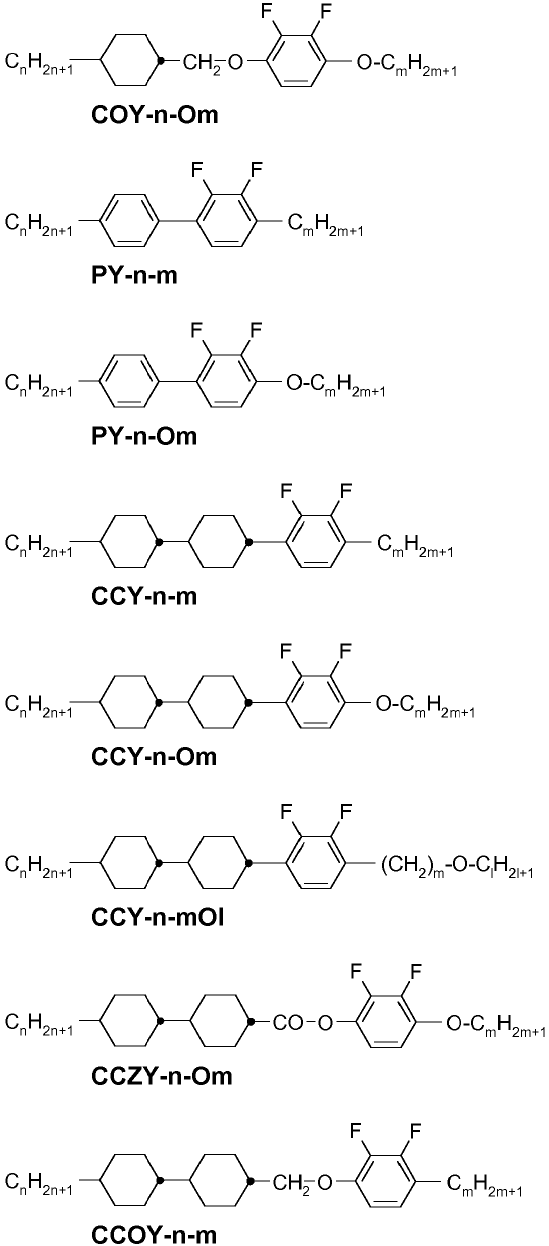

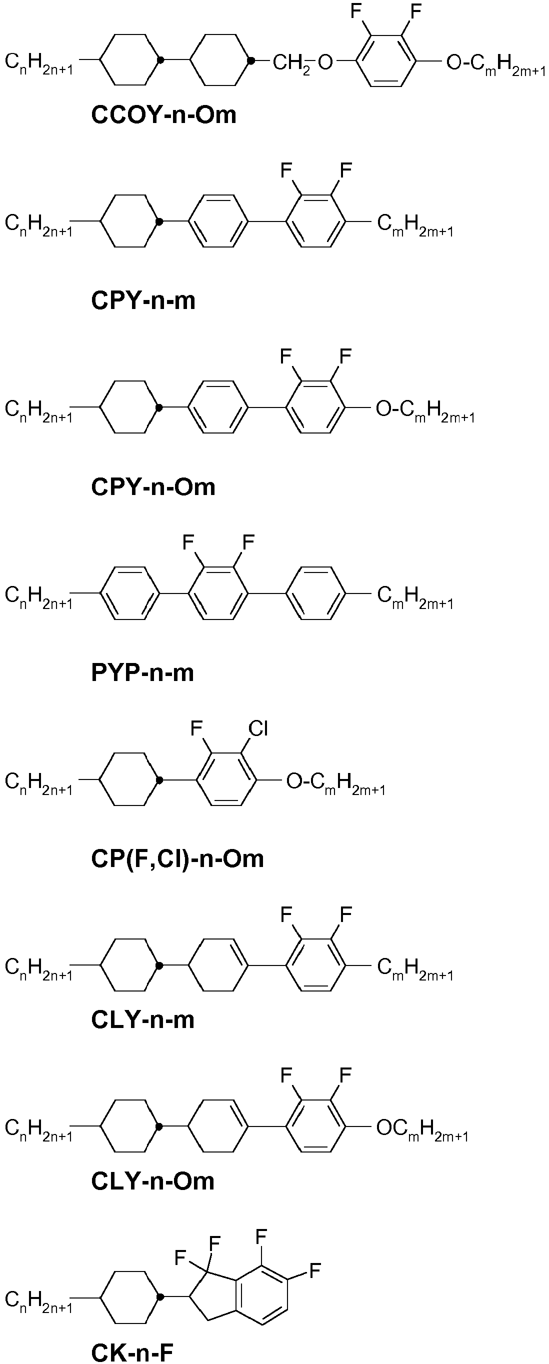

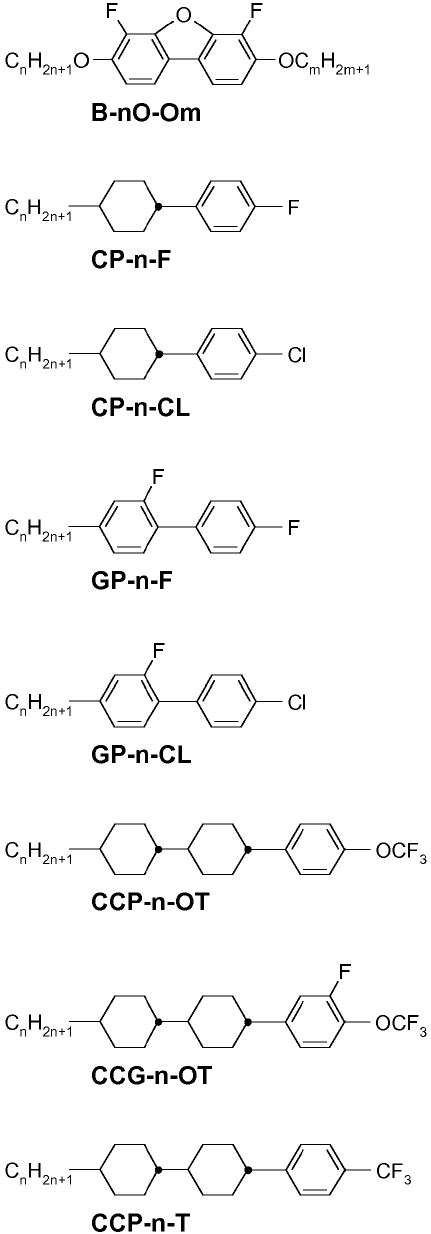

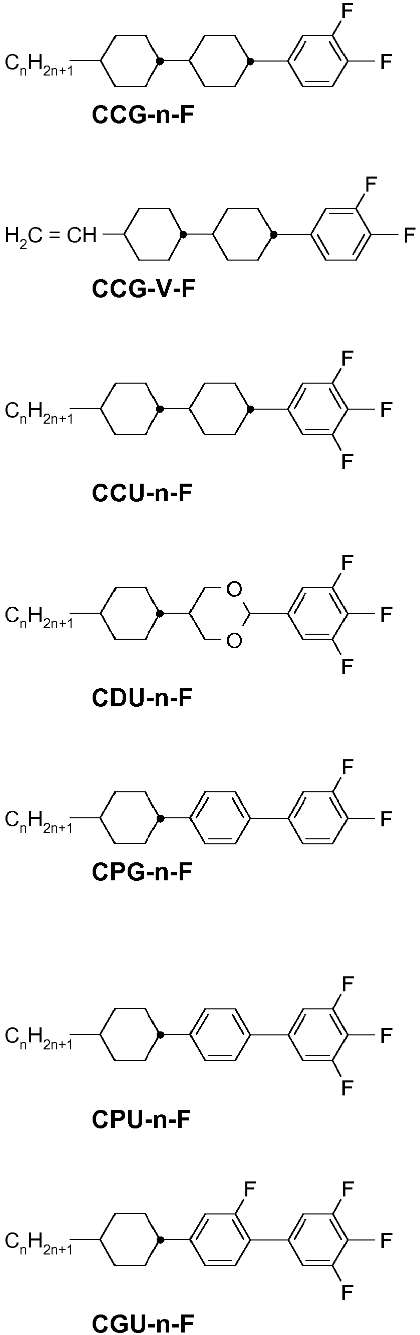

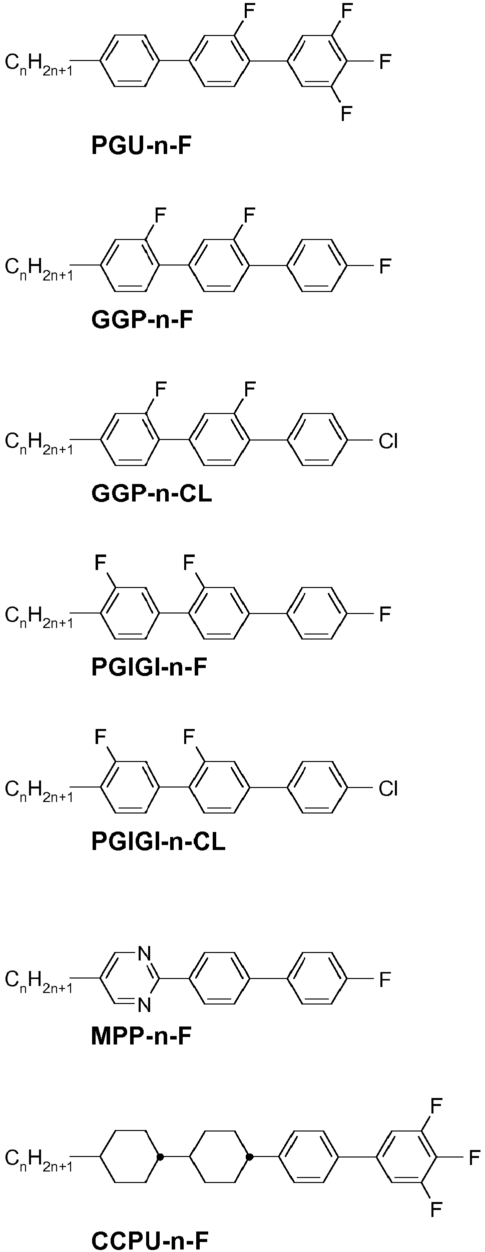

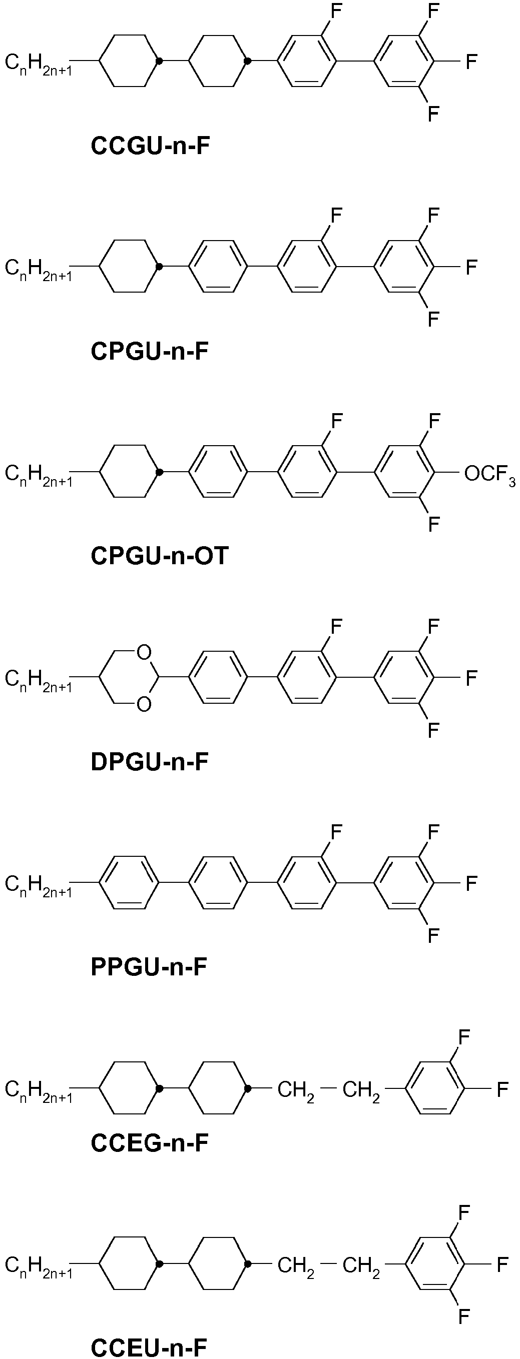

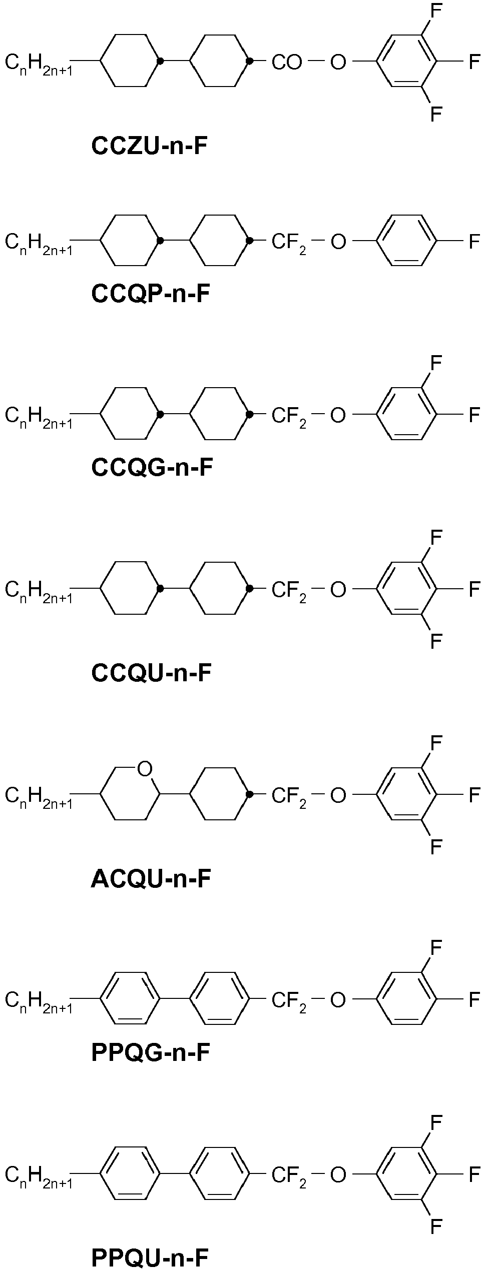

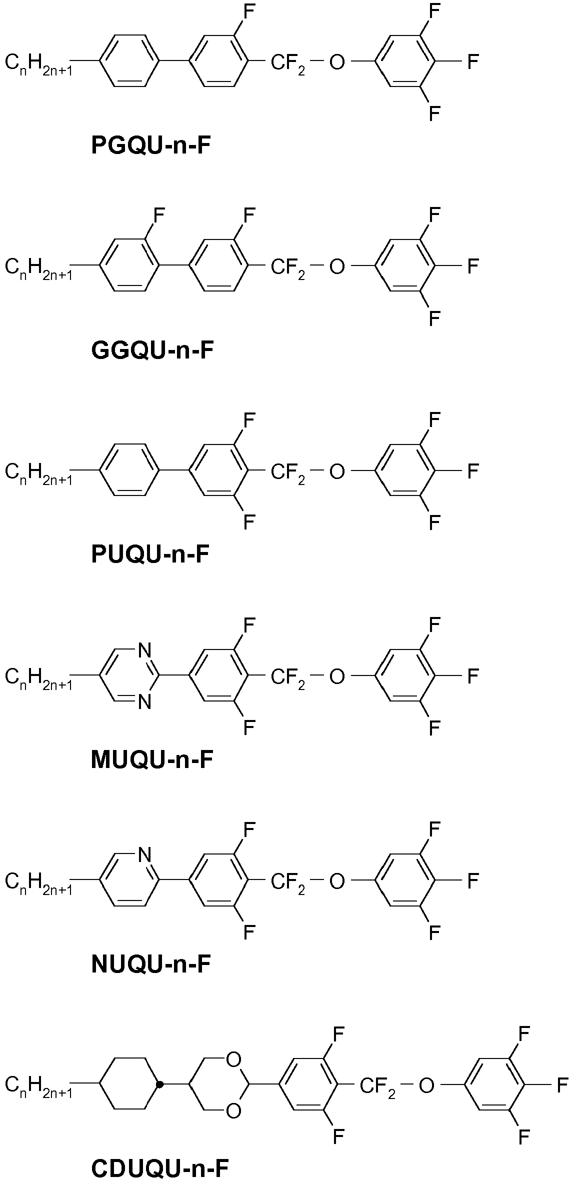

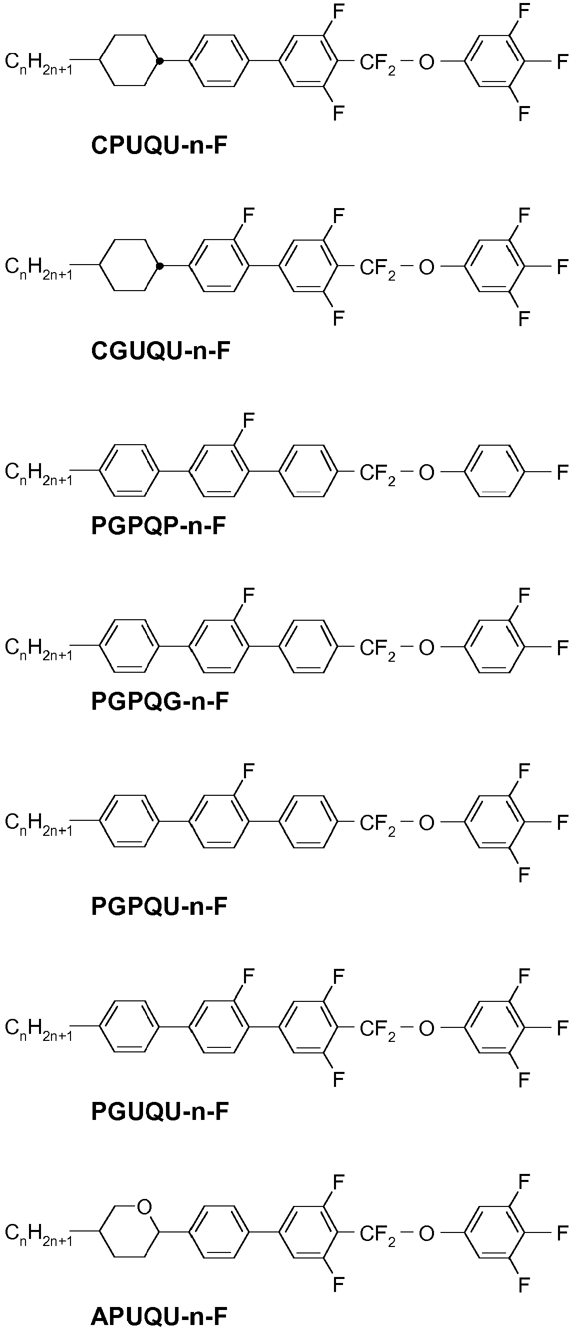

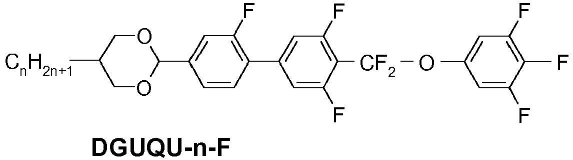

**Tabelle E**

| | |
|---|---|
| In der Tabelle E werden mögliche chirale Dotierstoffe angegeben, die den erfindungsgemäßen FK-Medien zugesetzt werden können. | |
| 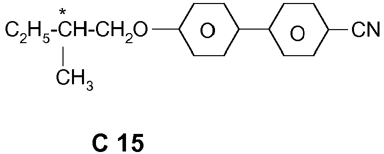 | 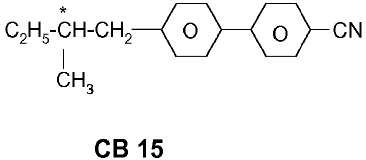 |
| 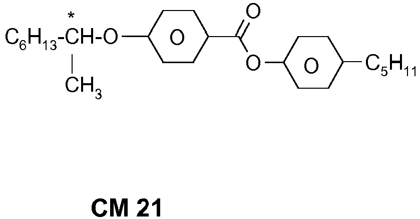 | 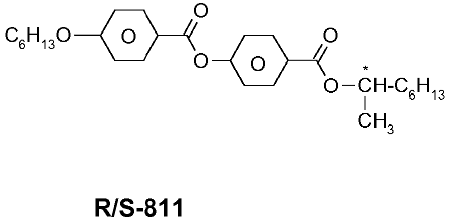 |
| 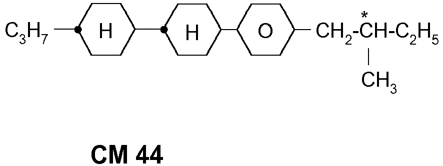 | 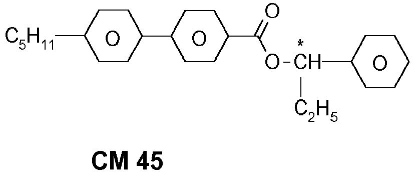 |
| 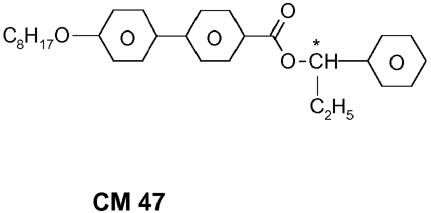 | 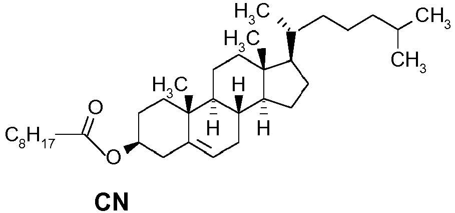 |
| 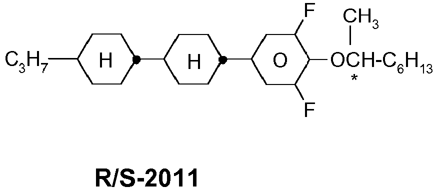 | 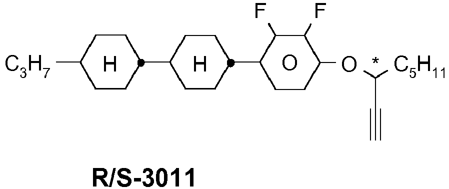 |
| 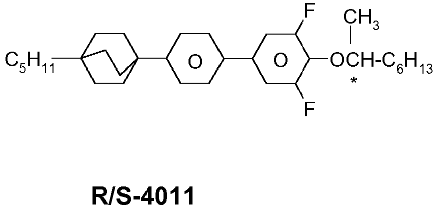 | 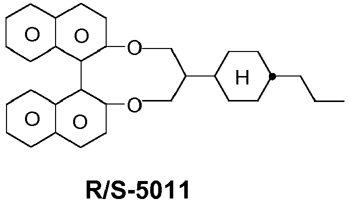 |
| 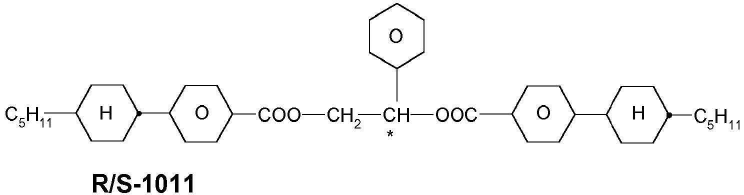 | |

Optional enthalten die FK-Medien 0 bis 10 Gew.-%, insbesondere 0,01 bis 5 Gew.-%, besonders bevorzugt 0,1 bis 3 Gew.-% an Dotierstoffen, vorzugsweise ausgewählt aus der Gruppe bestehend aus Verbindungen der Tabelle E.

**Tabelle F**

| | |
|---|---|
| In der Tabelle F werden mögliche Stabilisatoren angegeben, die den erfindungsgemäßen FK-Medien zugesetzt werden können. (n bedeutet hier eine ganze Zahl von 1 bis 12, vorzugsweise 1, 2, 3, 4, 5, 6, 7 oder 8, endständige Methylgruppen sind nicht gezeigt). | |
| 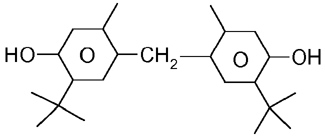 | 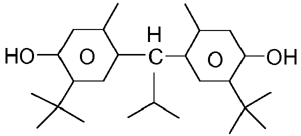 |
| 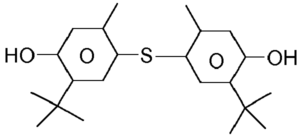 | 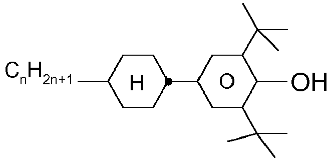 |
| 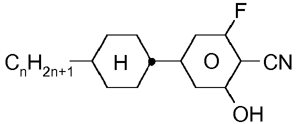 | 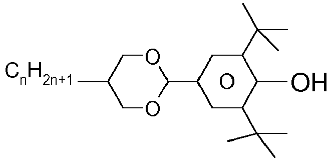 |
| 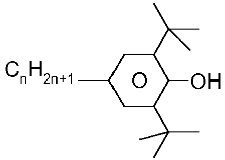 | 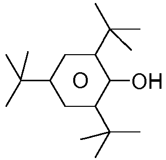 |
| 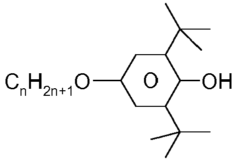 | 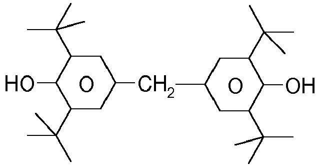 |
| 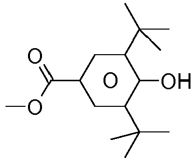 | 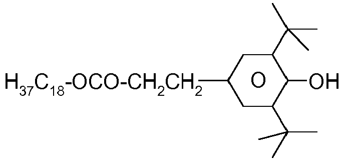 |
| 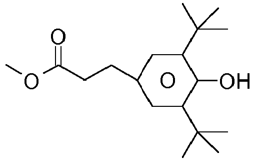 | 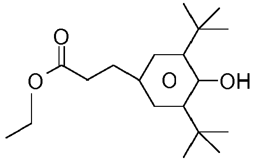 |
| 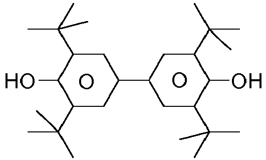 | 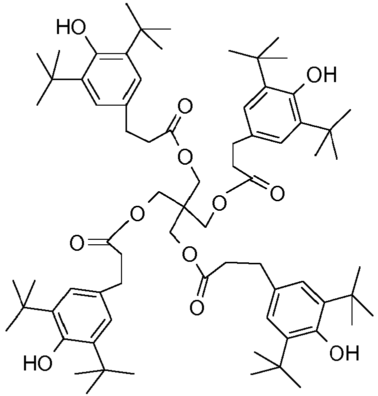 |
| 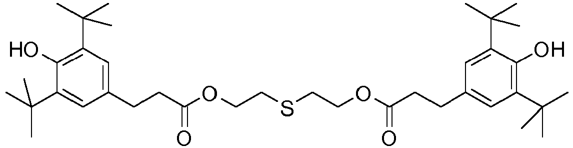 | |
| 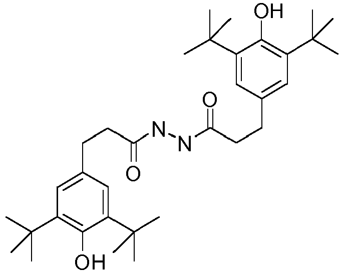 | 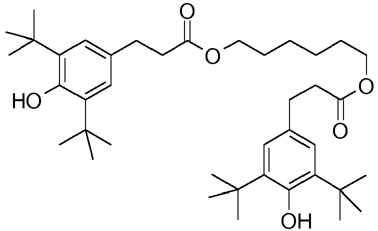 |
| 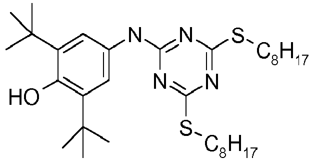 | 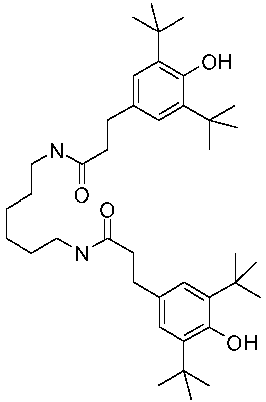 |
| 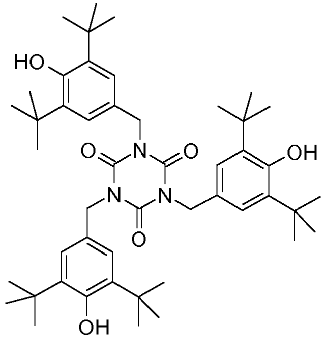 | 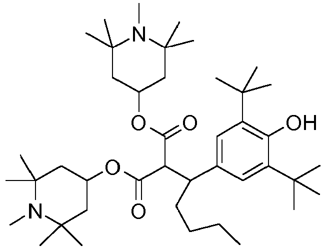 |
| 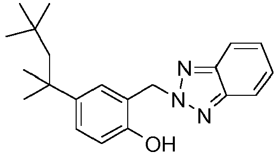 | 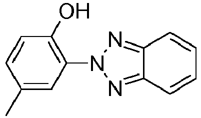 |
| 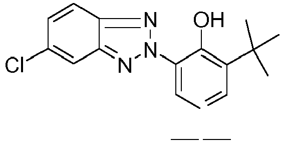 | 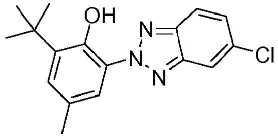 |
| 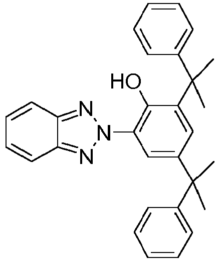 | 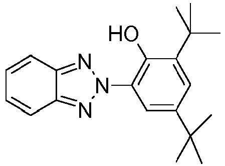 |
| 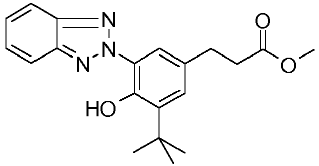 | 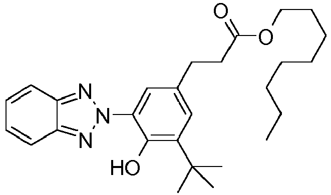 |
| 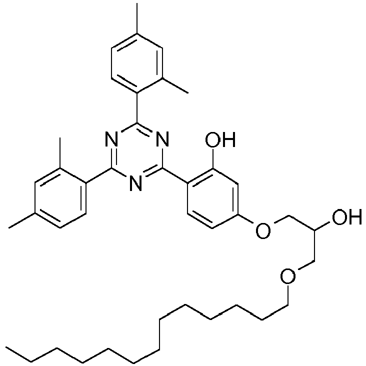 | 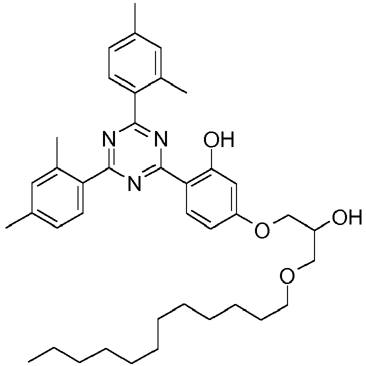 |
| 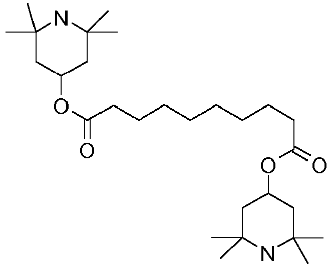 | 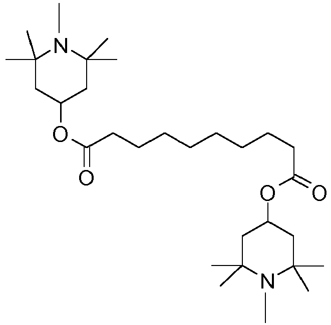 |

Vorzugsweise enthalten die FK-Medien 0 bis 10 Gew.-%, insbesondere 1 ppm bis 5 Gew.-%, besonders bevorzugt 1 ppm bis 1 Gew.-% an Stabilisatoren. Vorzugsweise enthalten die FK-Medien einen oder mehrere Stabilisatoren ausgewählt aus der Gruppe bestehend aus Verbindungen der Tabelle F.

**Tabelle G**

| |
|---|
| In der Tabelle G sind Beispielverbindungen zusammengestellt, die in den FK-Medien gemäß der vorliegenden Erfindung vorzugsweise als polymerisierbare Verbindungen verwendet werden können. |
| 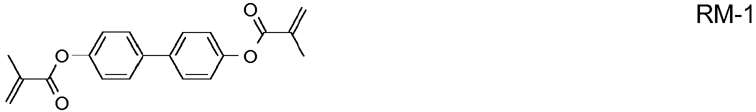 |
| 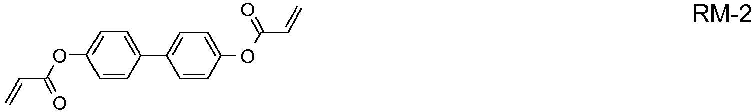 |
| 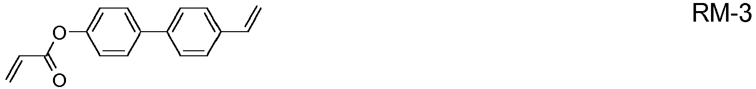 |
| 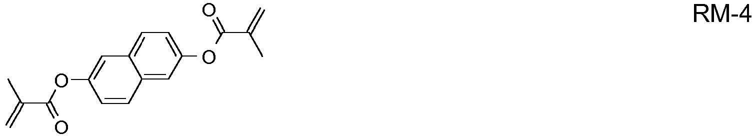 |
| 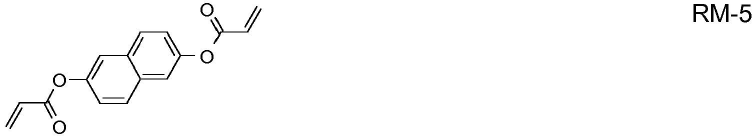 |
| 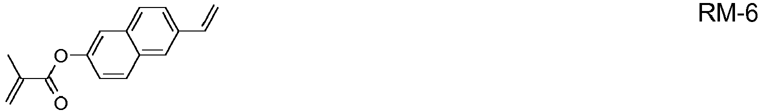 |
| 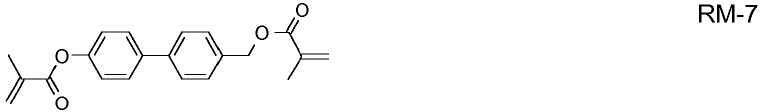 |
| 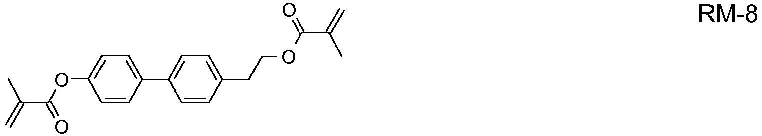 |
| 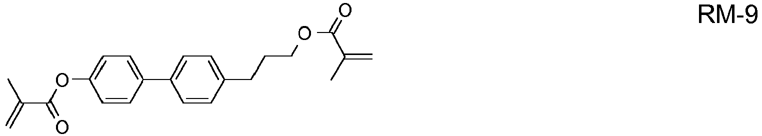 |
| 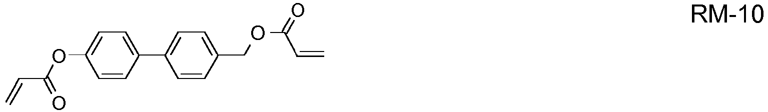 |
| 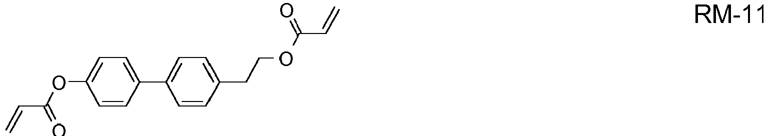 |
| 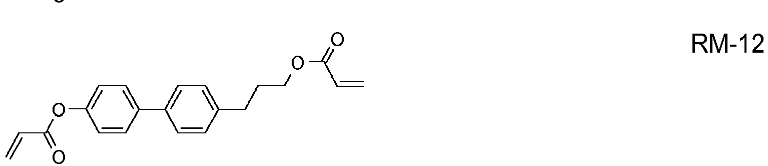 |
| 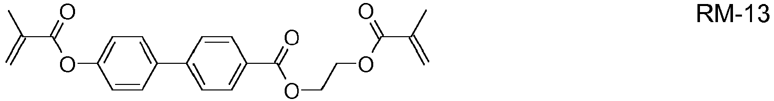 |
| 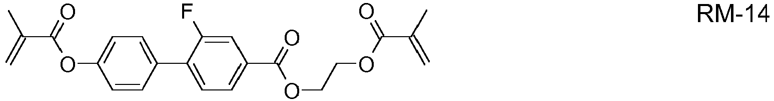 |
| 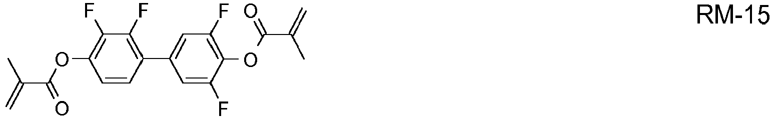 |
| 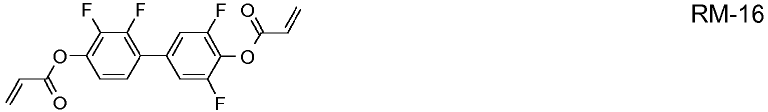 |
| 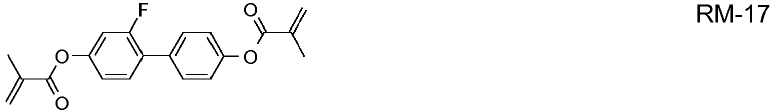 |
| 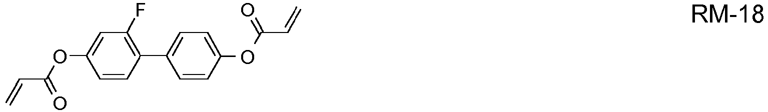 |
| 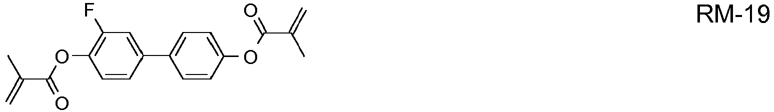 |
| 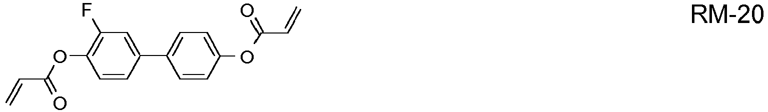 |
| 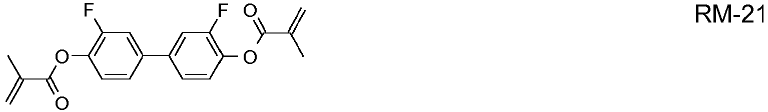 |
| 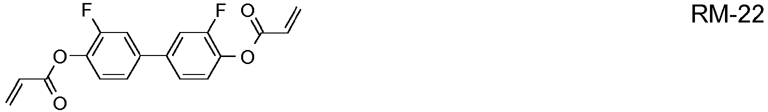 |
| 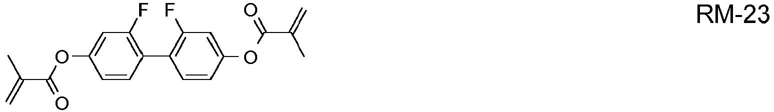 |
| 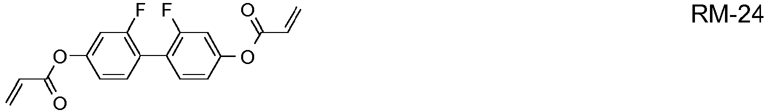 |
| 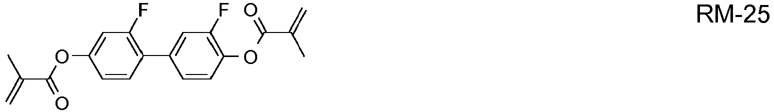 |
| 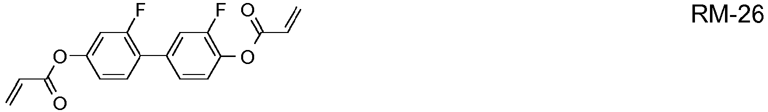 |
| 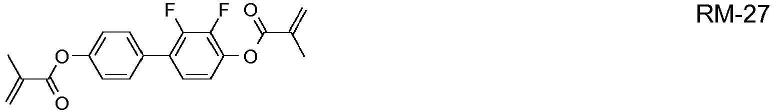 |
| 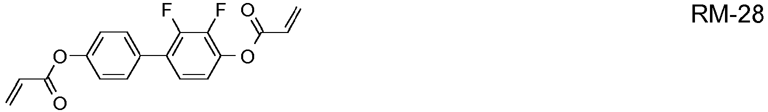 |
| 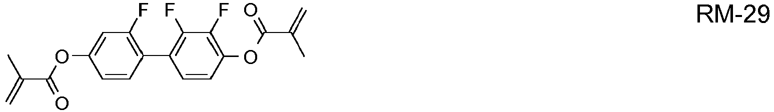 |
| 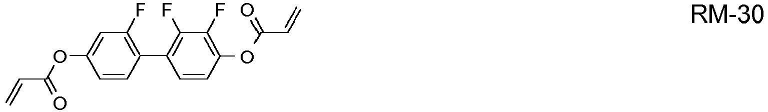 |
| 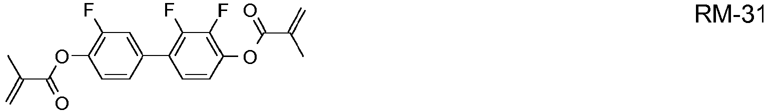 |
| 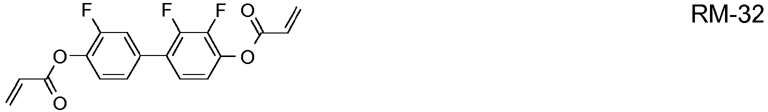 |
| 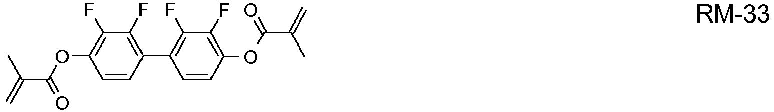 |
| 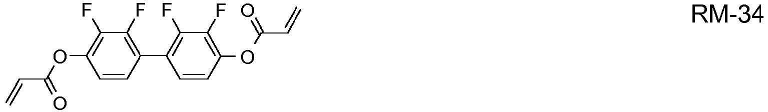 |
| 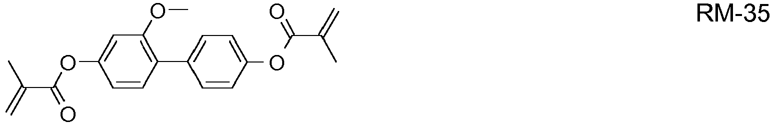 |
| 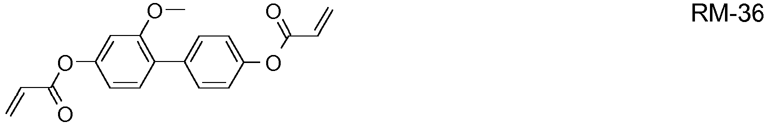 |
| 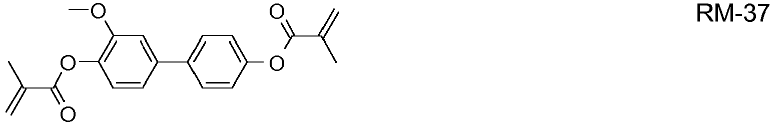 |
| 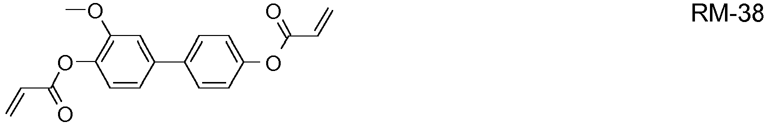 |
| 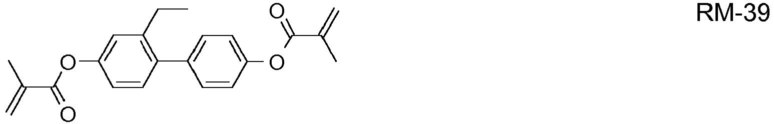 |
| 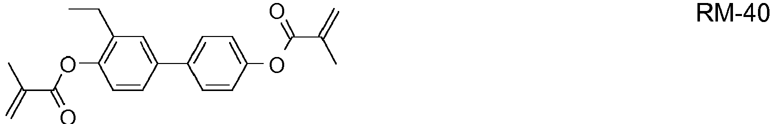 |
| 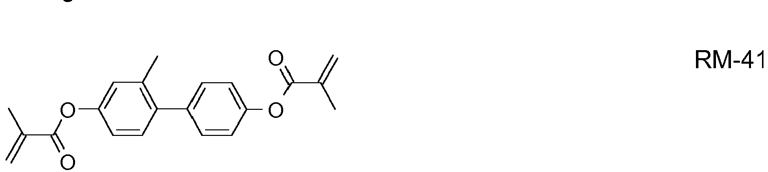 |
| 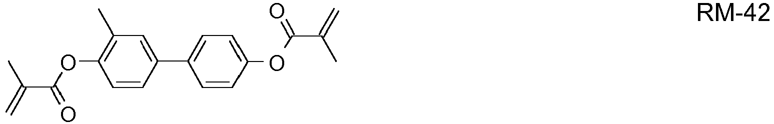 |
| 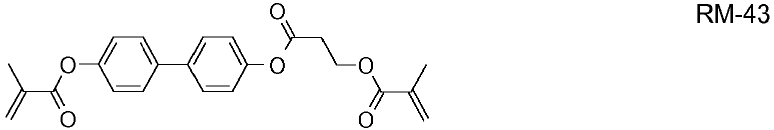 |
| 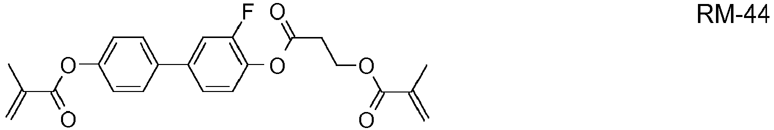 |
| 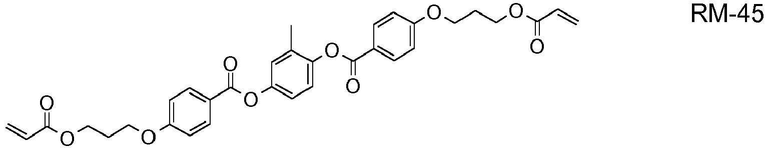 |
| 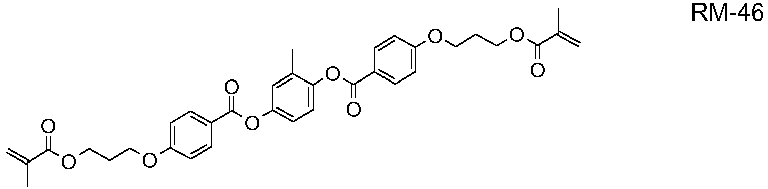 |
| 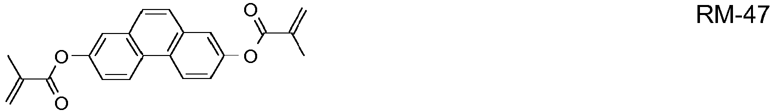 |
| 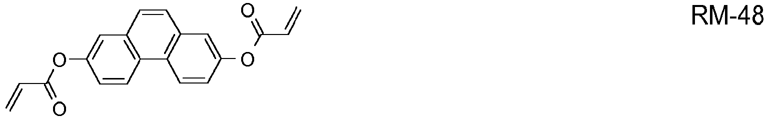 |
| 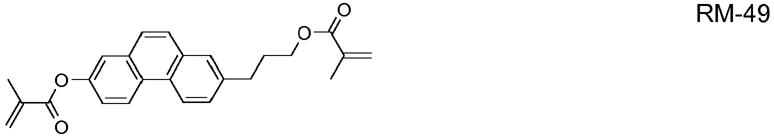 |
| 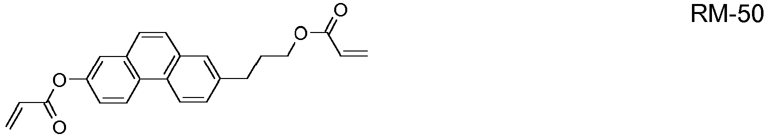 |
| 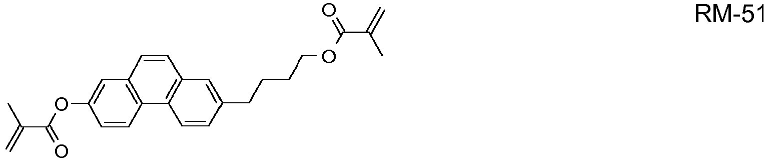 |
| 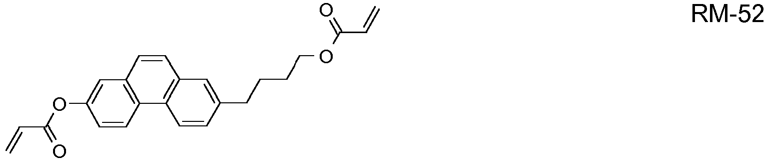 |
| 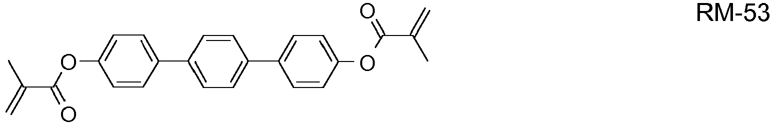 |
| 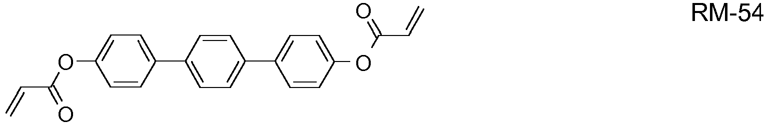 |
| 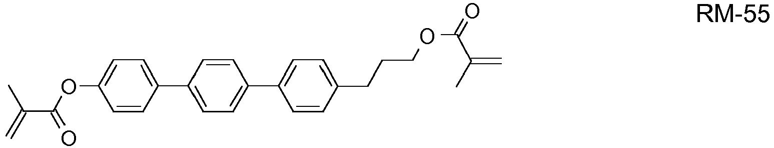 |
| 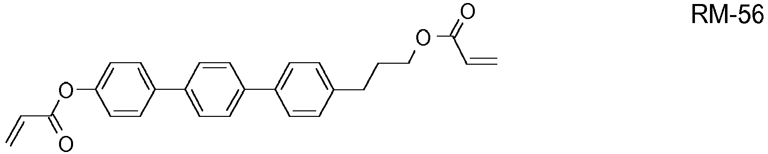 |
| 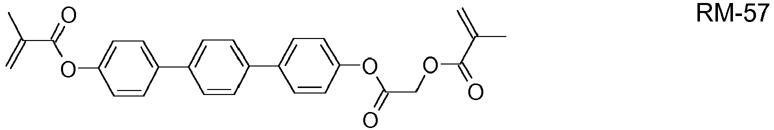 |
| 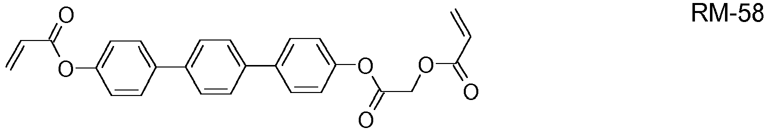 |
| 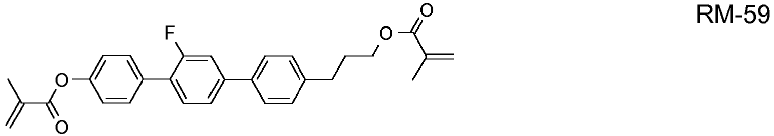 |
| 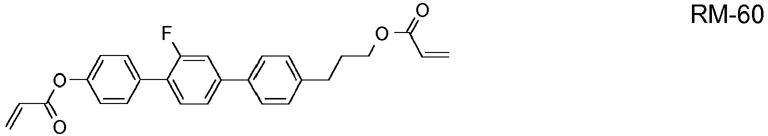 |
| 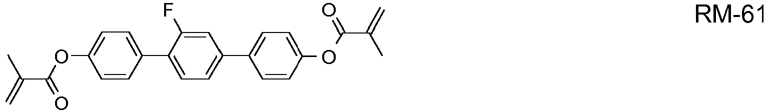 |
| 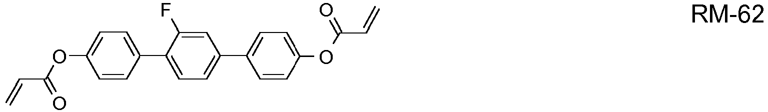 |
| 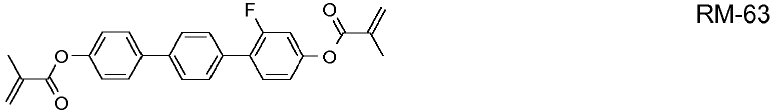 |
| 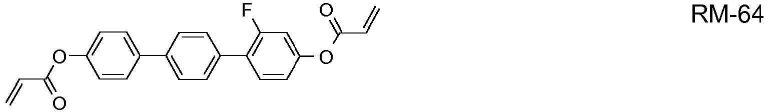 |
| 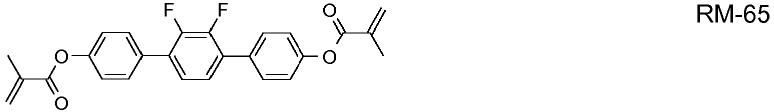 |
| 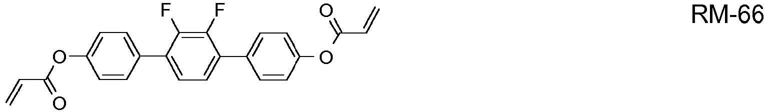 |
| 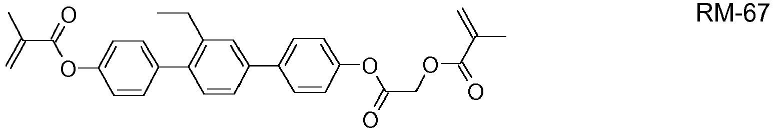 |
| 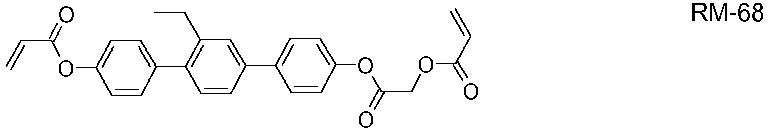 |
| 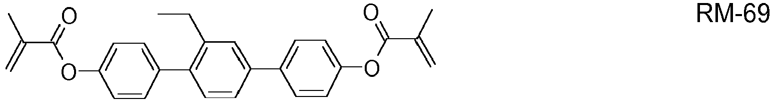 |
| 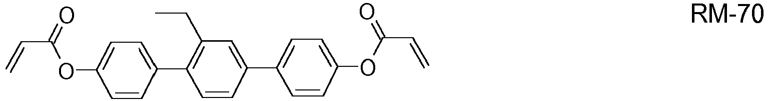 |
| 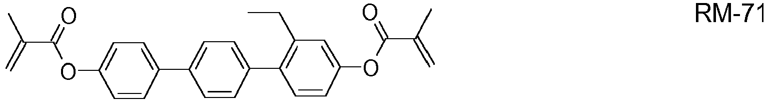 |
| 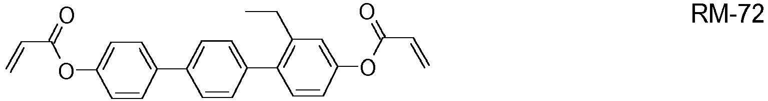 |
| 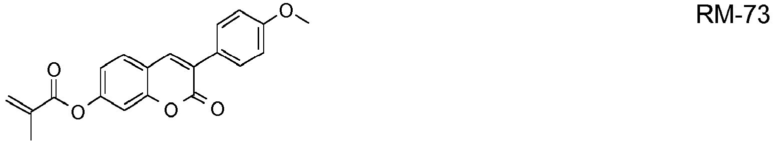 |
| 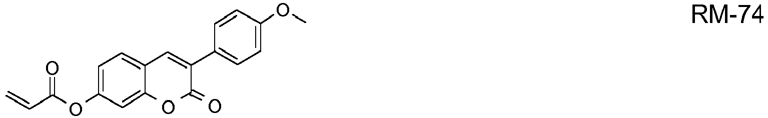 |
| 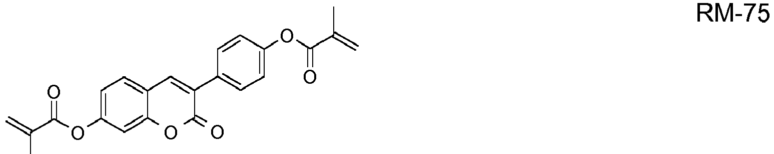 |
| 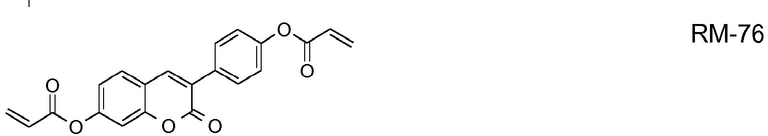 |
| 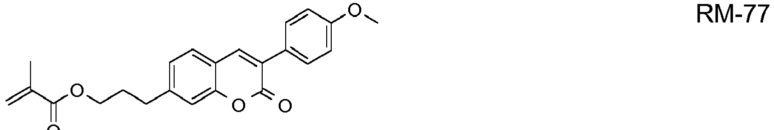 |
| 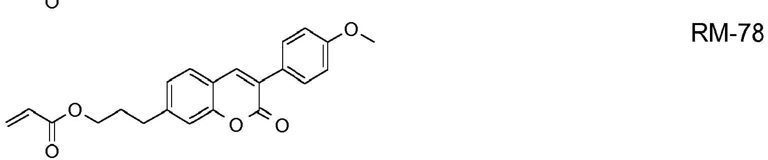 |
| 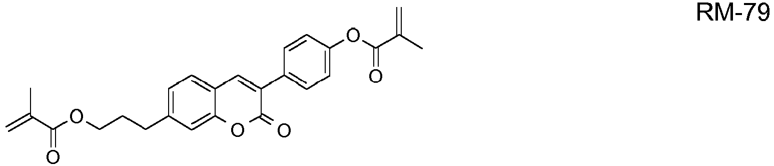 |
| 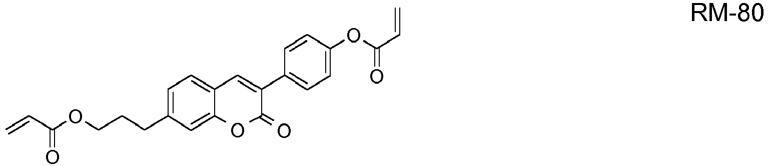 |
| 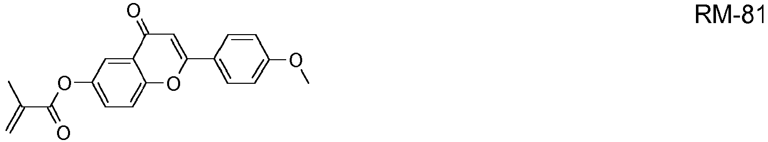 |
| 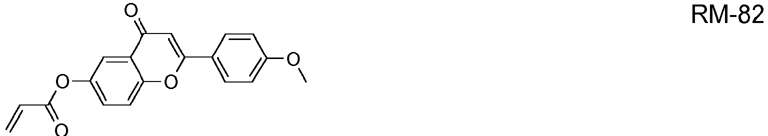 |
| 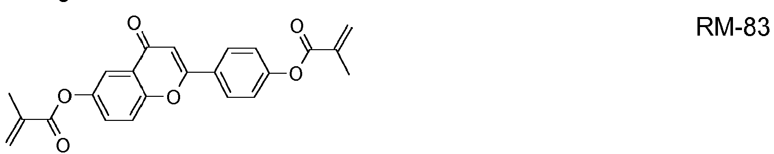 |
| 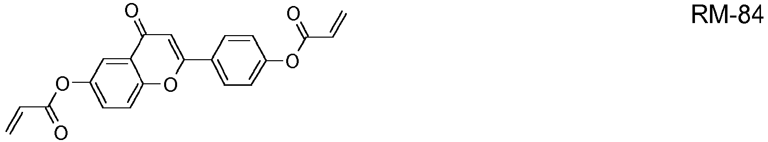 |
| 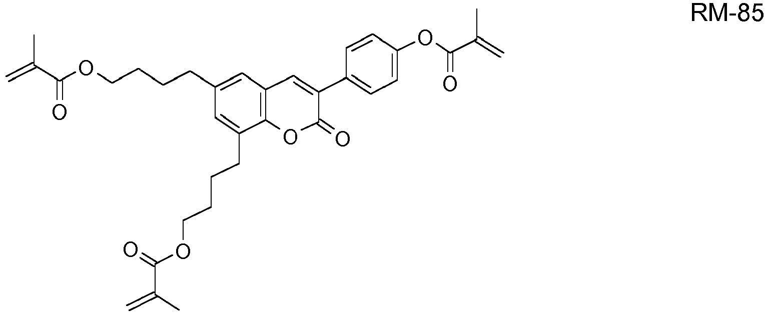 |
| 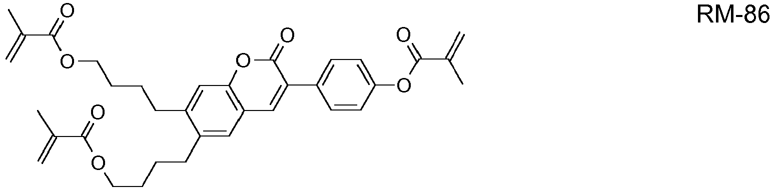 |
| 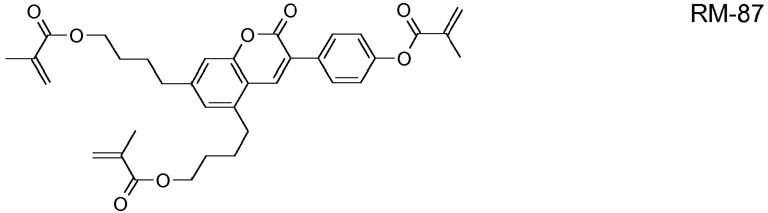 |
| 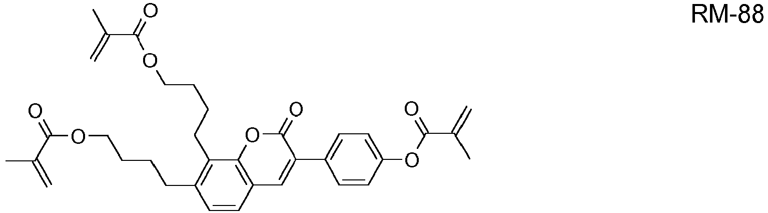 |
| 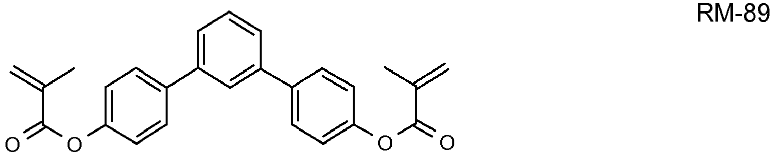 |
| 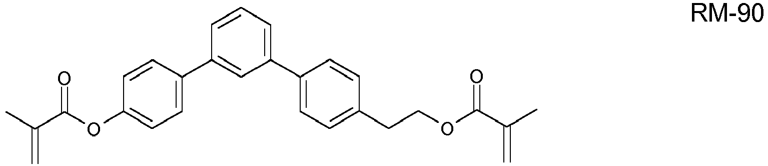 |
| 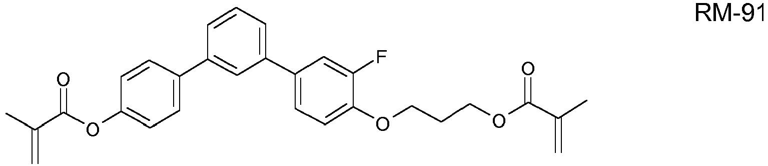 |
| 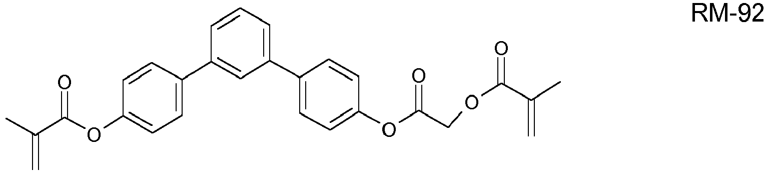 |
| 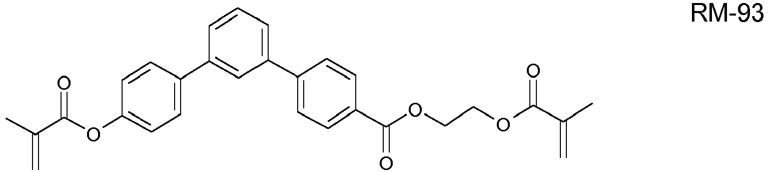 |
| 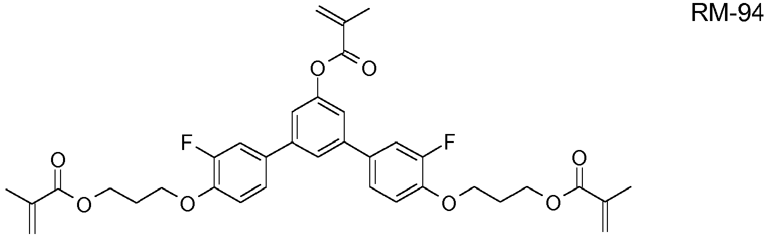 |
| 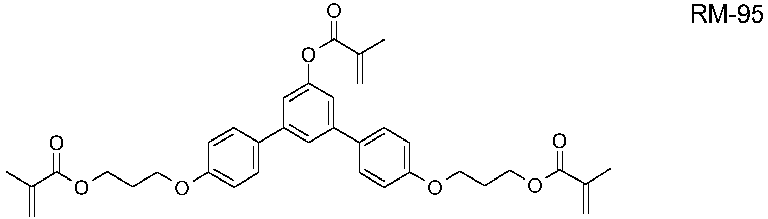 |
| 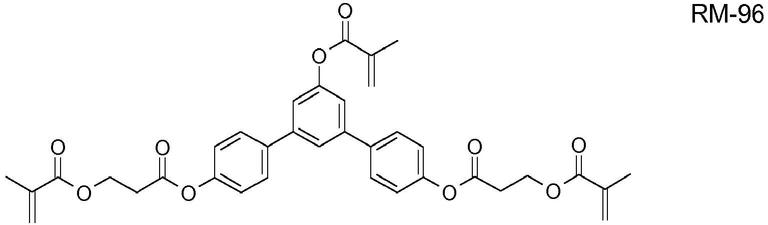 |
| 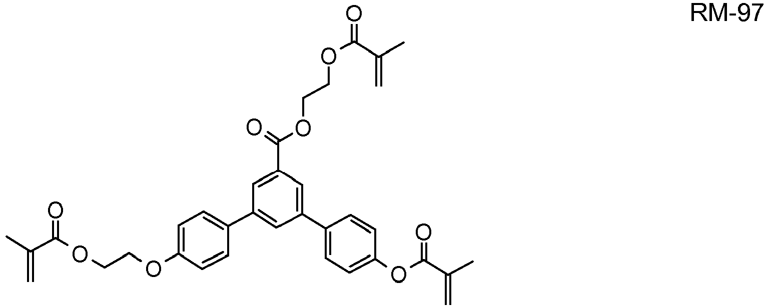 |
| 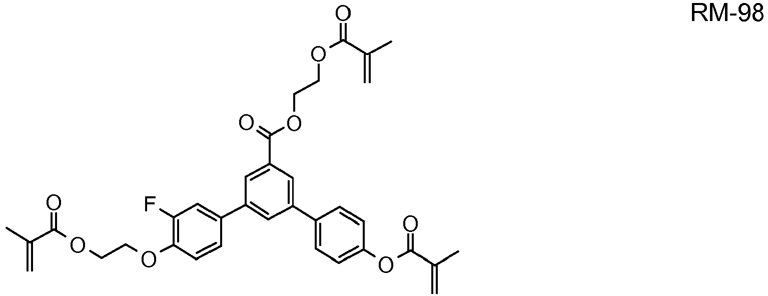 |
| 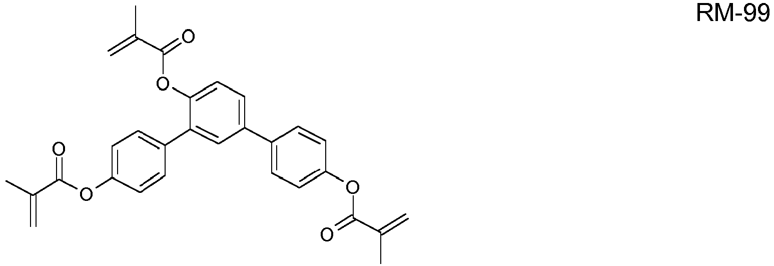 |
| 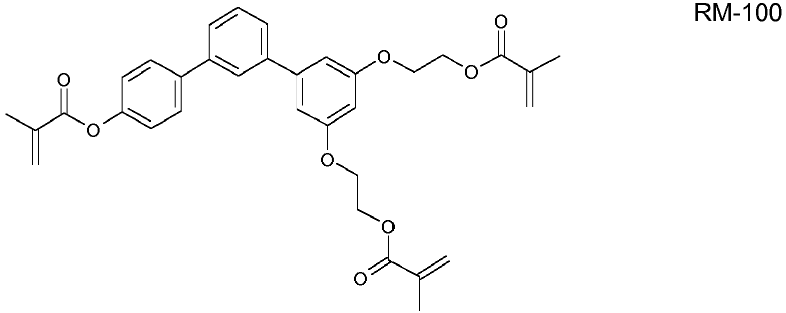 |
| 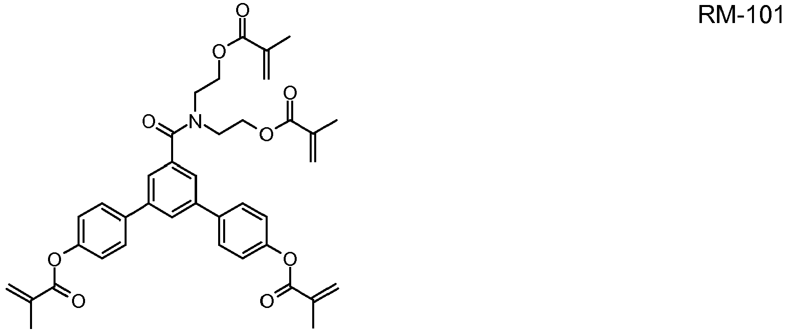 |
| 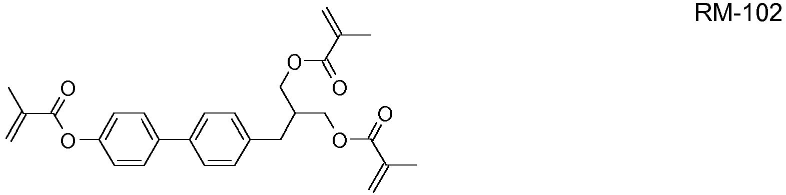 |
| 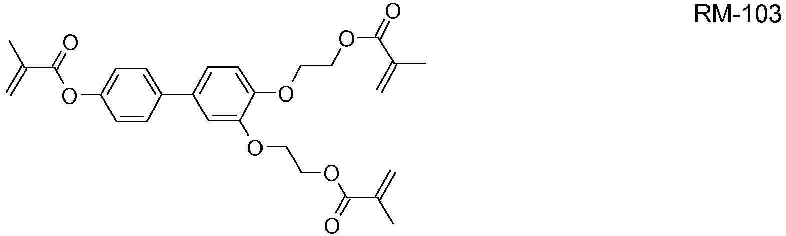 |
| 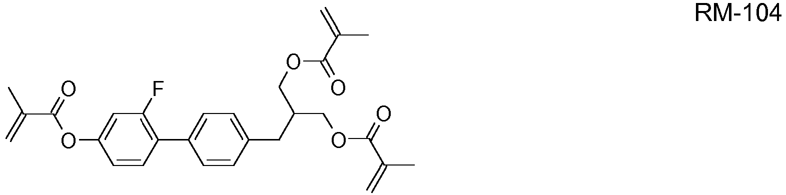 |
| 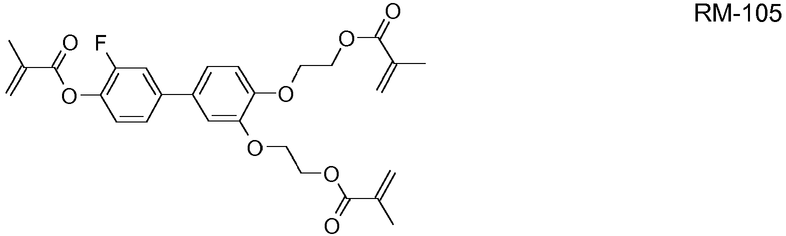 |
| 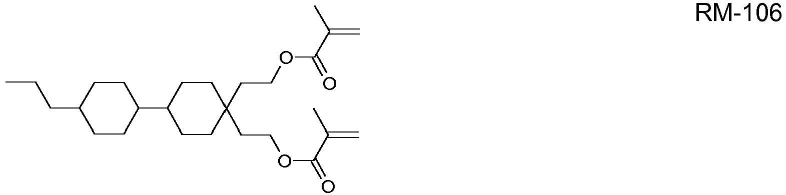 |
| 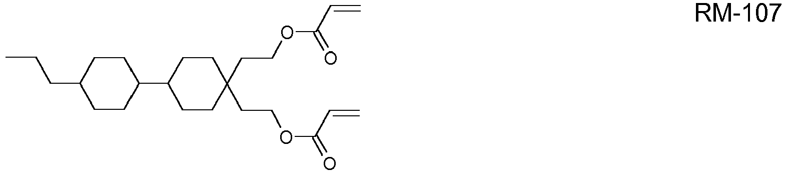 |
| 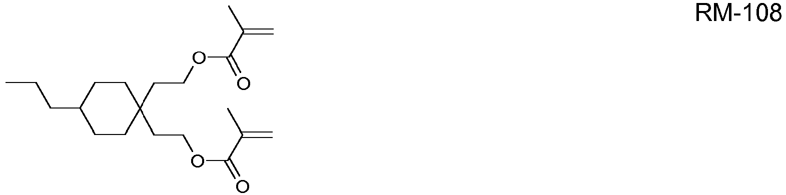 |
| 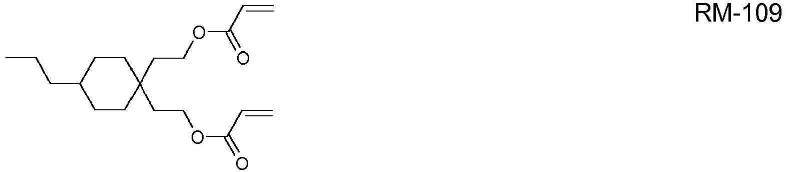 |
| 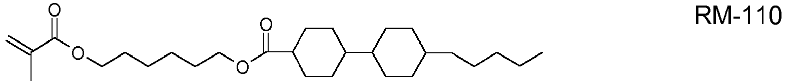 |
| 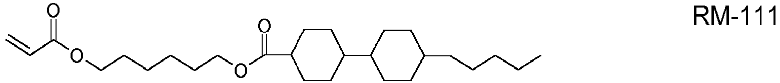 |
| 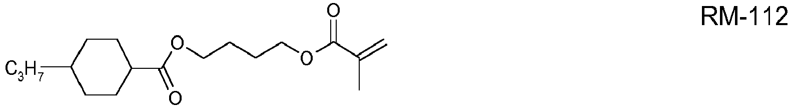 |
| 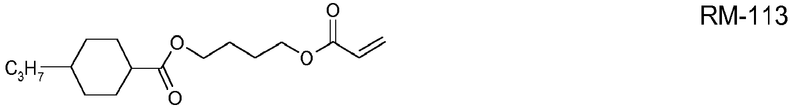 |
| 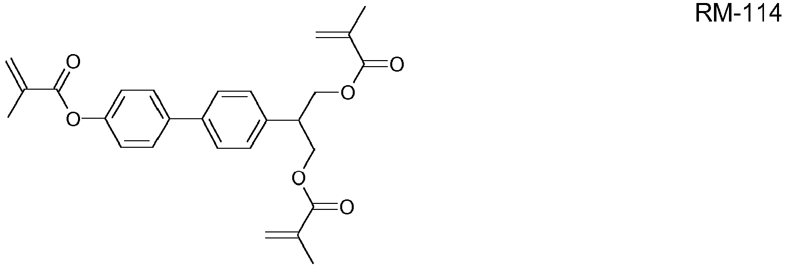 |
| 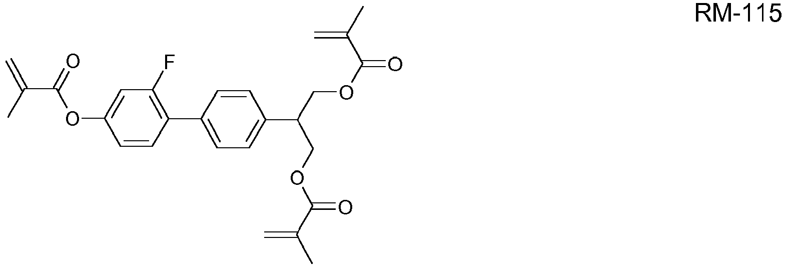 |
| 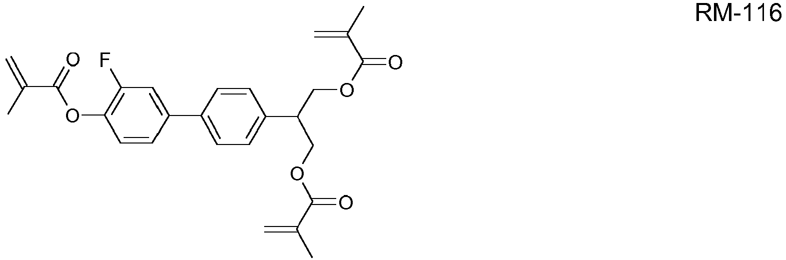 |
| 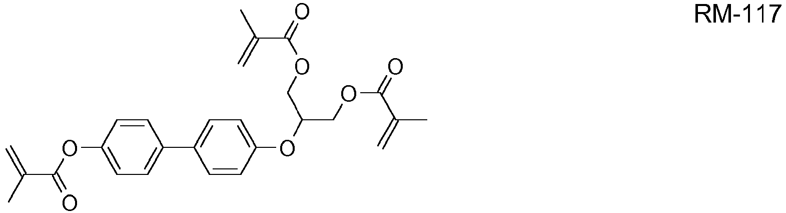 |
| 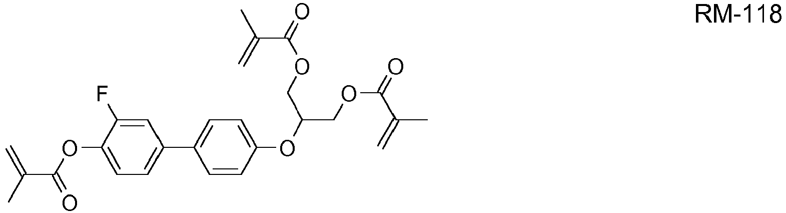 |
| 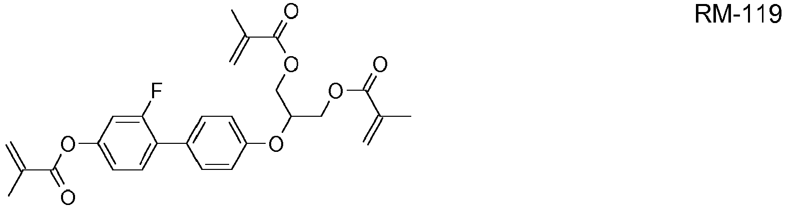 |
| 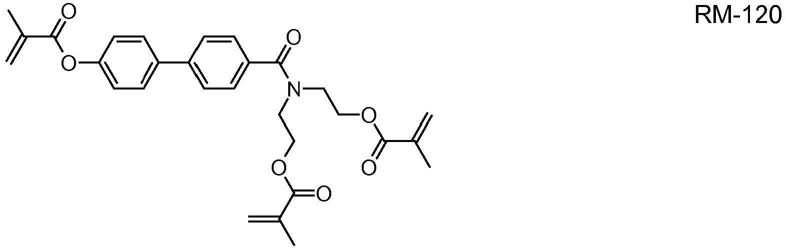 |
| 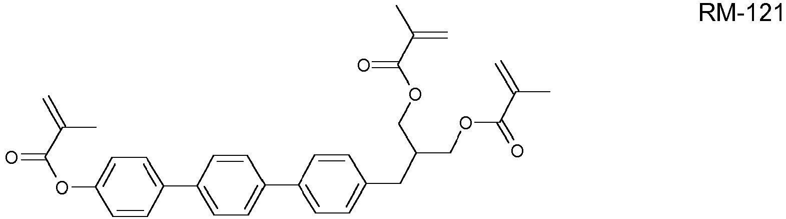 |
| 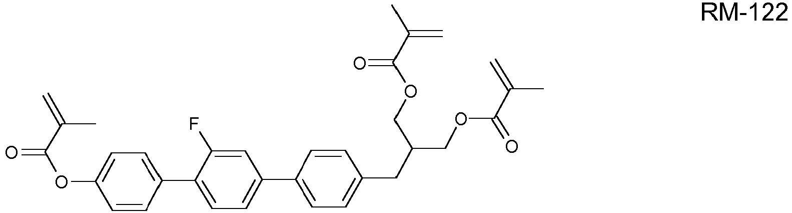 |
| 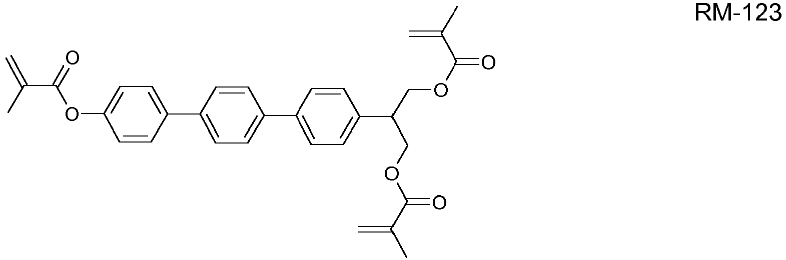 |
| 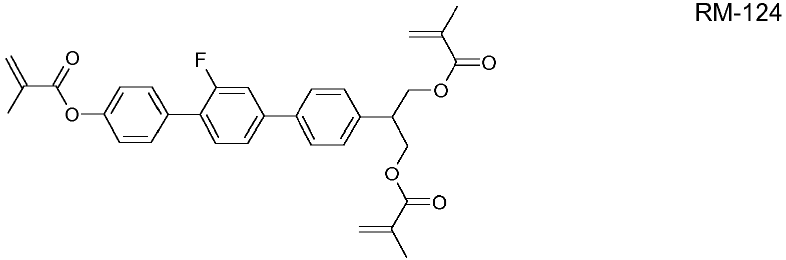 |
| 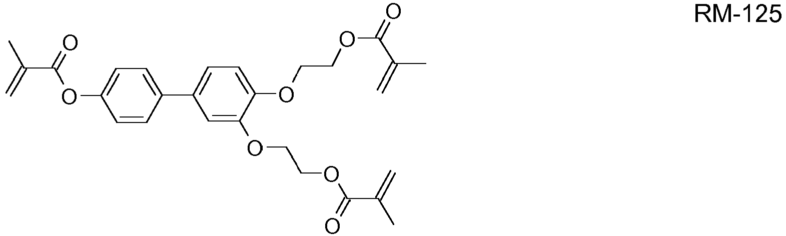 |
| 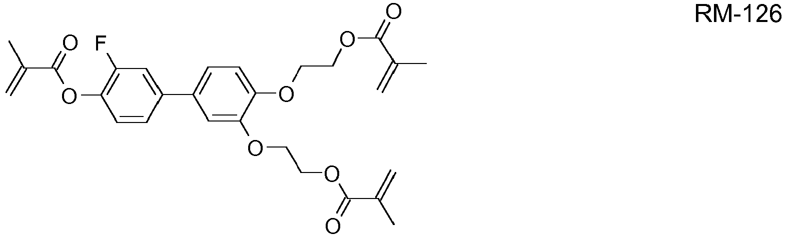 |
| 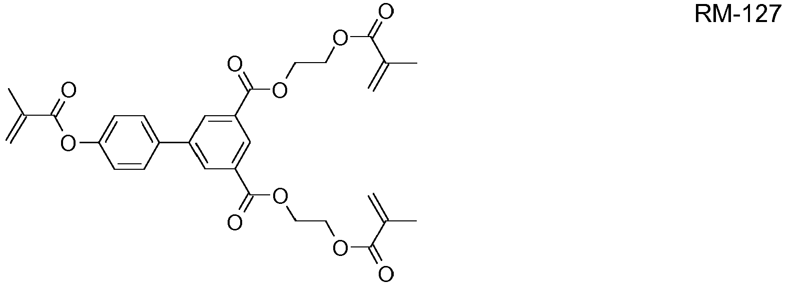 |
| 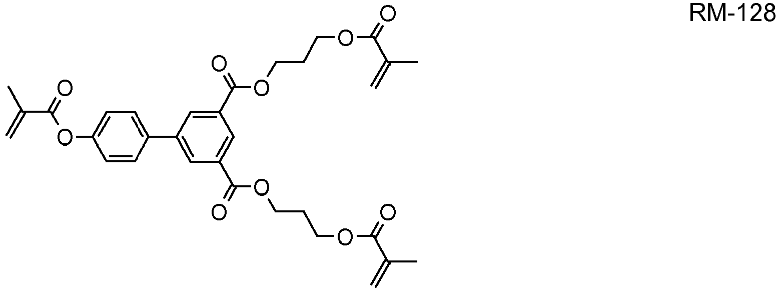 |
| 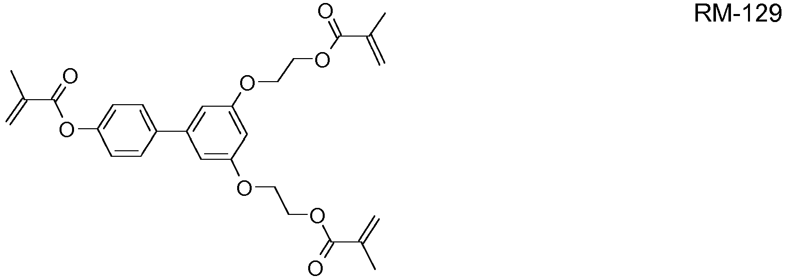 |
| 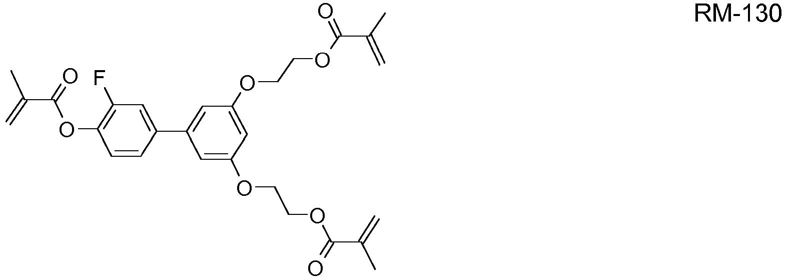 |
| 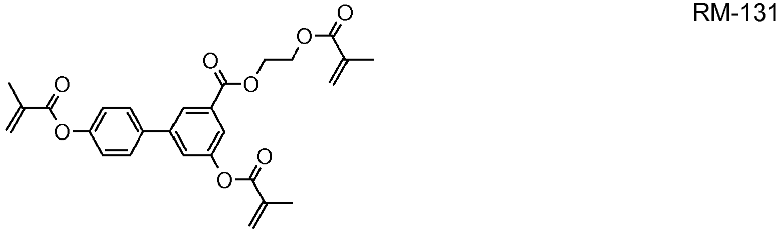 |
| 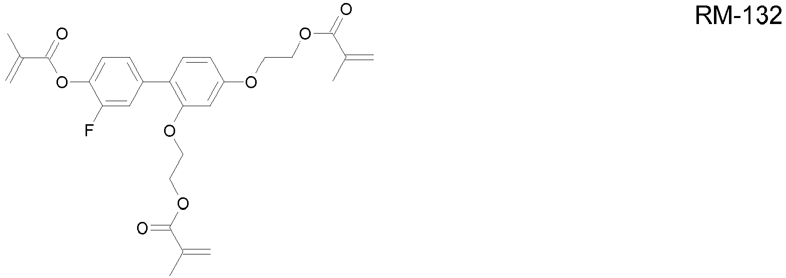 |
| 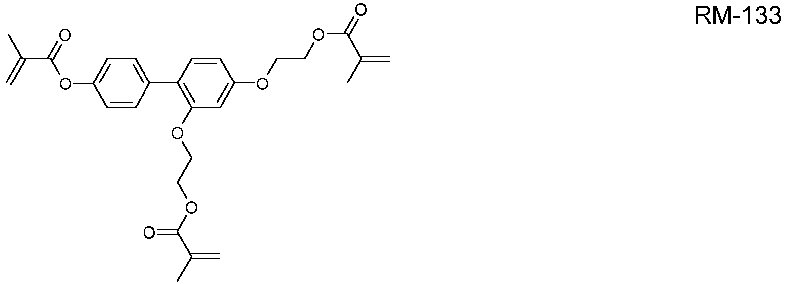 |
| 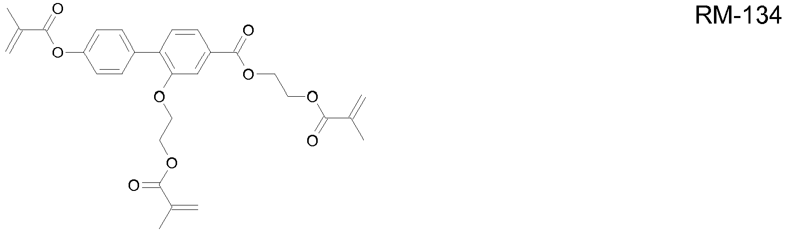 |
| 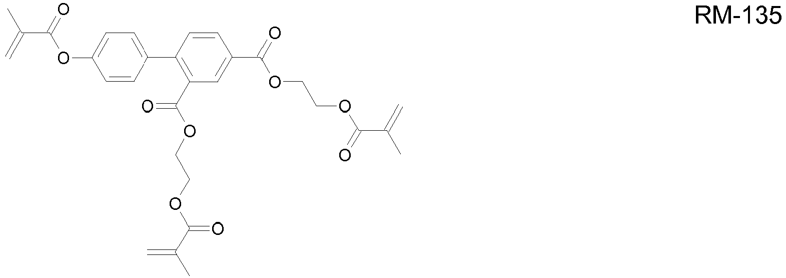 |
| 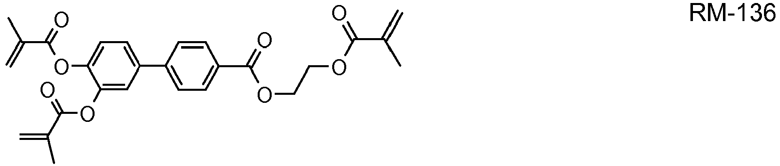 |
| 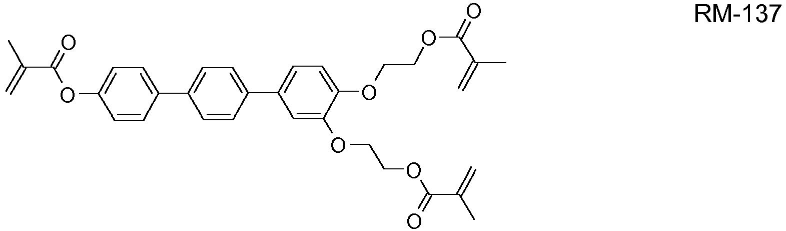 |
| 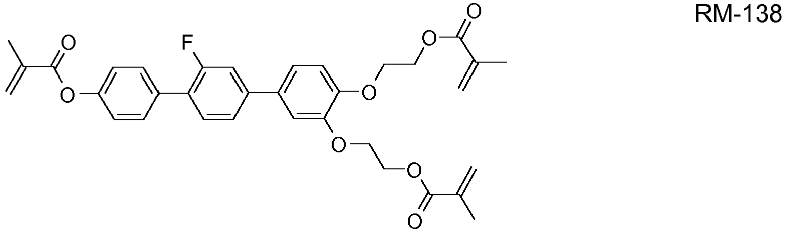 |
| 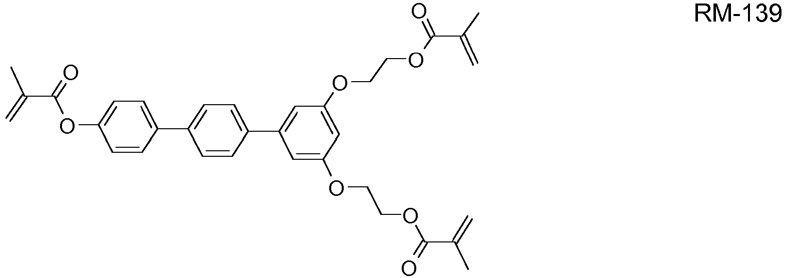 |
| 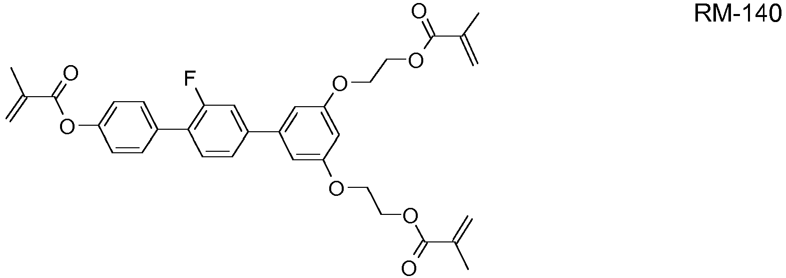 |
| 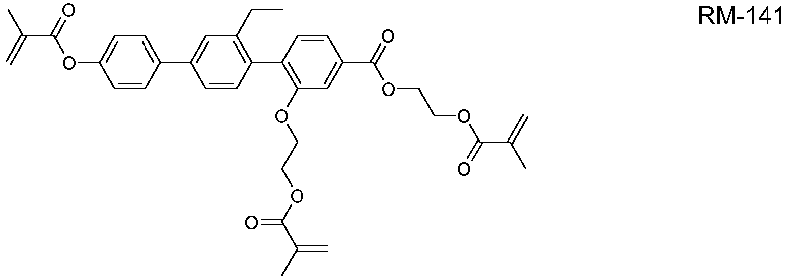 |
| 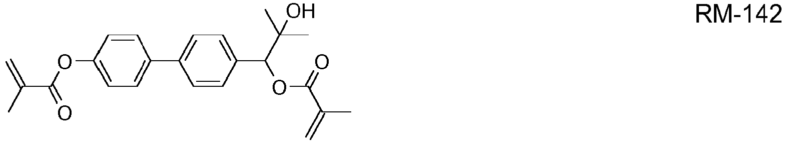 |
| 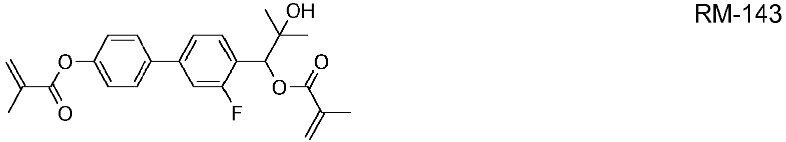 |
| 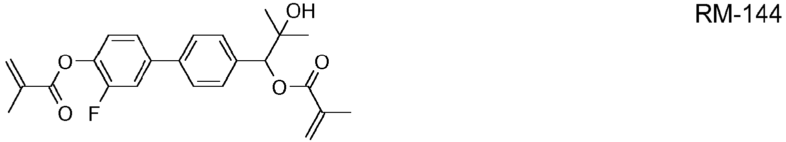 |

In einer bevorzugten Ausführungsform der vorliegenden Erfindung enthalten die mesogenen Medien eine oder mehrere Verbindungen ausgewählt aus der Gruppe der Verbindungen der Tabelle G.

In der vorliegenden Anmeldung bedeutet der Begriff "Verbindungen", auch geschrieben als "Verbindung(en)", sofern nicht explizit anders angegeben, sowohl eine als auch mehrere Verbindungen. Umgekehrt schließt der Begriff "Verbindung" generell auch mehrere Verbindungen ein, sofern dies laut Definition möglich und nicht anders angegeben ist. Gleiches gilt für die Begriffe FK-Medien und FK-Medium. Der Begriff "Komponente" umfasst jeweils eine oder mehrere Stoffe, Verbindungen und/oder Teilchen.

Außerdem werden folgende Abkürzungen und Symbole verwendet:
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Permittivität senkrecht zum Direktor bei 20°C und 1 kHz,
- ε∥: dielektrische Permittivität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa.s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN],
- V₀: Kapazitive Schwelle (Freedericks Schwelle) bei 20°C [V].

Soweit nicht explizit anders vermerkt, sind in der vorliegenden Anmeldung alle Konzentrationen in Gewichtsprozent angegeben und beziehen sich auf die entsprechende Gesamtmischung, enthaltend alle festen oder flüssigkristallinen Komponenten, ohne Lösungsmittel.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei optional gleichzeitig eine Spannung an die Anzeige angelegt wird (üblicherweise 10 bis 30 V Wechselstrom, 1 kHz). In den Beispielen wird, falls nicht anders angegeben, eine Quecksilberdampflampe mit 100 mW/cm² verwendet, die Intensität wird mit einem Standard-UV-Meter (Fabrikat Ushio UNI Meter) gemessen, der mit einem Bandpassfilter bei 320 nm (optional 340 nm) ausgerüstet ist.

Die folgenden Beispiele erläutern die vorliegende Erfindung, ohne sie in irgendeiner Weise beschränken zu sollen. Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

Weitere Kombinationen der Ausführungsformen und Varianten der Erfindung gemäß der Beschreibung ergeben sich auch aus den Ansprüchen.

### Beispiele

Die eingesetzten Verbindungen, soweit nicht kommerziell erhältlich, werden nach Standard-Laborvorschriften synthetisiert. Die FK-Medien stammen von der Merck KGaA, Deutschland.

### A) Synthesebeispiele

### Beispiel 1.

### Schritt 1: Synthese von Zwischenstufe 5 (Boronsäure)

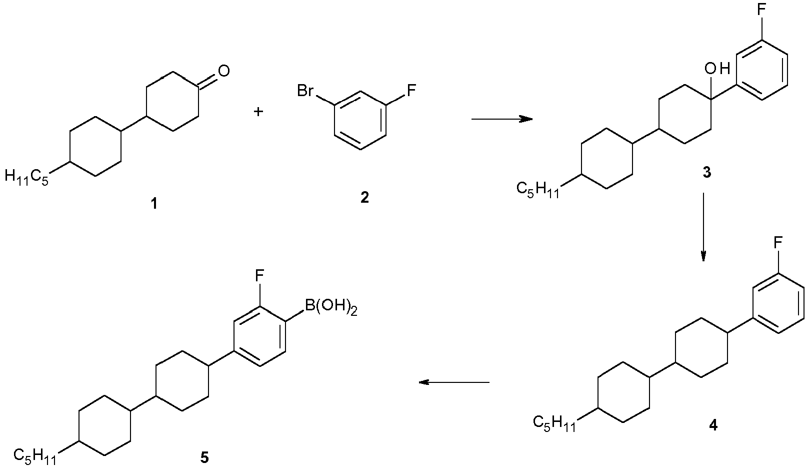

9.7 g (0.4 mol) Magnesium werden mit 40 ml THF überschichtet und beides auf 50 °C erhitzt. Danach werden ca. 10 % einer Lösung des Bromaromaten **2** (69.9 g, 0.4 mol) in 100 ml THF zugetropft. Nach Anspringen der Reaktion wird **2** so zudosiert, dass das Gemisch kontinuierlich siedet. Nach beendeter Zugabe lässt man auf 65 °C abkühlen und gibt 100 g (0.4 mol) Keton **1** gelöst in 100 ml THF tropfenweise hinzu. Nach dem Aufarbeiten erhält man 128.3 g vom Alkohol **3,** welcher ohne weitere Aufreinigung in 800 ml Toluol gelöst und nach Zugabe von 3.2 g p-Toluolsulfonsäurehydrat 2 h unter Rückfluss gekocht wird. Nach dem Aufarbeiten erhält man 98 g rohes Olefin, welches durch katalytische Hydrierung zu **4** umgesetzt wird.

71.7 g (217 mmol) **4** werden in 200 ml THF gelöst und auf -70 °C abgekühlt. Nun werden 170 ml (0.239 mol) einer 1.4 molaren Lösung von s-Butyllithium in Hexan bei dieser Temperatur zugetropft. Nach beendeter Zugabe wird eine Stunde bei -70 °C nachgerüht und anschließend eine Lösung von 28.3 ml (0.249 mol) Trimethylborat in 100 ml THF zugetropft. Nach dem Aufarbeiten und Kristallisieren aus Heptan erhält man 65.8 g Boronsäure **5.**

### Schritt 2: Synthese von Zwischenstufe 8

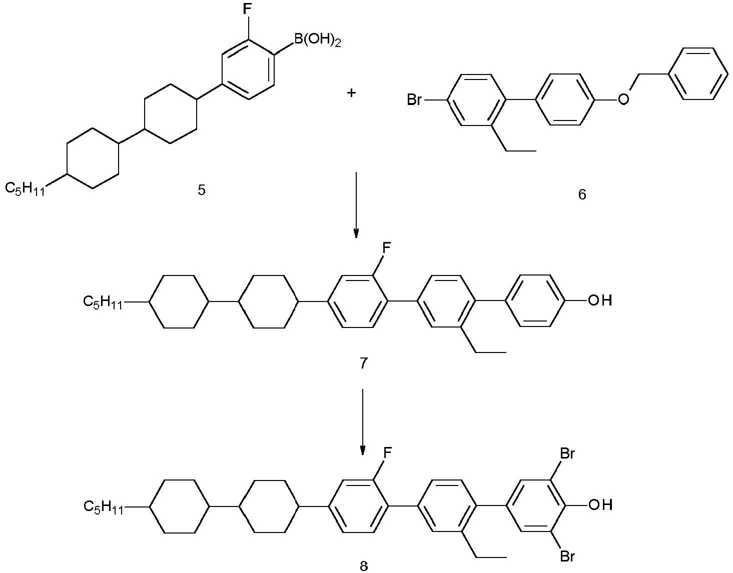

65.2 g (172 mmol) Boronsäure **5,** 63 g (172 mmol) Bromid **6,** 5.95 g (5.1 mmol) Tetrakis(triphenylphosphino)palladium(0) und 47.3 g Natriumcarbonat werden in 53 ml Ethanol, 360 ml Toluol und 170 ml Wasser suspendiert und zum Rückfluss erhitzt. Nach dem Aufarbeiten werden 90.0 g farbloser Feststoff erhalten, der in 1.8 L Tetrahydrofuran über 30 g Palladium auf Kohle (5%) hydriert wird. Das Rohprodukt wird aus Heptan/Toluol (1:1) umkristallisiert. Man erhält 55 g Phenol **7.**
40 g (76 mmol) 7 werden in 500 ml Tetrahydrofuran vorgelegt, mit 2 ml Diisopropylamin versetzt und bei -5 °C werden 27.0 g (152 mmol) N-Bromsuccinimid portionsweise zugegeben. Es wird eine Stunde bei 0 °C gerührt und danach auf RT kommen gelassen. Nach Aufarbeitung werden 56.8 g Dibromid 8 erhalten.

### Schritt 3: Synthese von Zwischenstufe 11

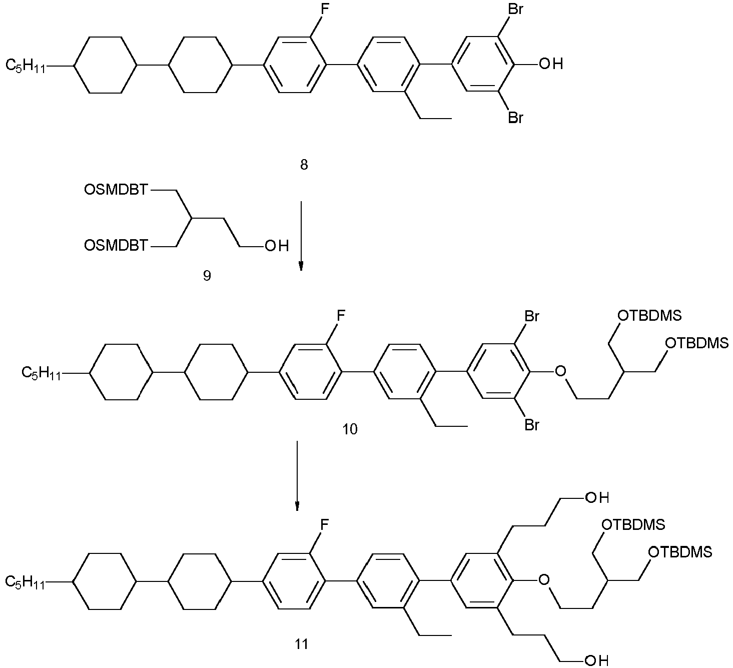

15 g (21.9 mmol) 8 und 9.2 g (26.3 mmol) 9 werden mit 6.9 g (26.3 mmol) Triphenylphosphin in 120 ml Tetrahydrofuran gelöst und bei RT werden 5.5 ml (26.3 mmol) Diisopropylazodicarboxylat zugetropft. Das Gemisch wird über Nacht bei RT nachgerührt. Nach dem Aufarbeiten erhält man 11.7 g **10** als weißen, wachsartigen Feststoff.
11.7 g (11.5 mmol) **10** werden mit 6.2 g (43.8 mmol) 2-Butoxy-1,2-oxaborolan, 14 g Kaliumphosphat, 26.1 mg (0.115 mmol) Palladiumacetat, 113.2 mg (0.230 mmol) Ruphos (CAS 787618-22-8), 35 ml Wasser und 170 ml Tetrahydrofuran vermischt und 3 h zum Rückfluss erhitzt. Nach dem Aufarbeiten erhält man 8.9 g 11 als weiße, wachsartige Substanz.

### Schritt 4: Synthese der Endverbindung 12 (Selbstorientierungsadditiv Nr. 2)

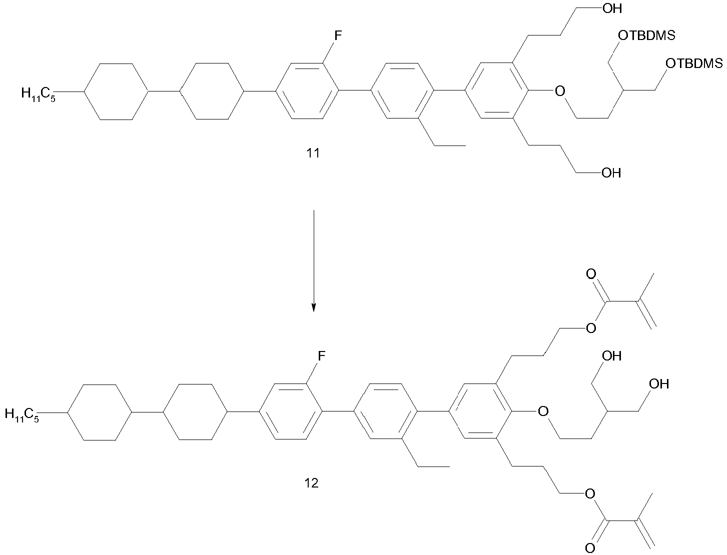

8.9 g (9.1 mmol) 11, 3.9 ml (45.7 mmol) Methacrylsäure sowie 223 mg (1.8 mmol) DMAP werden in 90 ml Dichlormethan gelöst und bei 5 °C werden 7.9 ml (45.7 mmol) EDC (1-[3-(Dimethylamino)propyl]-3-ethylcarbodiimid Hydrochlorid) zugetropft. Man lässt über Nacht bei RT nachrühren und arbeitet wie üblich auf. Das Rohprodukt (6.2 g) wird in 50 ml Tetrahydrofuran gelöst und bei 5 °C mit 8.3 ml (16.8 mmol) 2-molarer Salzsäure versetzt. Danach lässt man bei RT über Nacht nachrühren und arbeitet wie üblich auf. Nach Kristallisation aus Heptan werden 3.0 g **12** als weißes Pulver vom Schmelzpunkt 72 °C erhalten.

### Phasen Glastemperatur (Tg) -26°C, Schmelzpunkt 72°C

¹H NMR (700 MHz, Chloroform-d) δ 7.41 - 7.36 (m, 2H), 7.03 (s, 2H), 7.01 (dd, *J* = 12.0, 1.7 Hz, 1H), 6.10 (s, 2H), 5.56 (q, *J* = 1.7 Hz, 2H), 4.24 (t, *J* = 6.7 Hz, 4H), 3.95 - 3.89 (m, 4H), 3.80 (dd, *J* = 10.8, 6.7 Hz, 2H), 2.77 - 2.72 (m, 4H), 2.69 (s, 1H), 2.63 (q, *J* = 7.6 Hz, 2H), 2.49 (tt, *J* = 12.1, 3.5 Hz, 1H), 2.12 (qq, *J =* 10.7, 6.5, 5.4 Hz, 1H), 2.07 - 2.00 (m, 4H), 2.00 - 1.95 (m, 2H), 1.94 (s, 6H), 1.87 (q, *J* = 6.3 Hz, 4H), 1.81 - 1.72 (m, 4H), 1.49 - 1.41 (m, 2H), 1.34 - 1.27 (m, 4H), 1.27 - 1.21 (m, 2H), 1.21 - 1.05 (m, 10H), 1.01 (qd, *J* = 12.0, 2.8 Hz, 2H), 0.88 (q, *J* = 8.9, 8.1 Hz, 5H).

Analog nach beschriebener Synthese lassen sich folgende Verbindungen herstellen:

### Beispiel 2. (Selbstorientierungsadditiv Nr. 1)

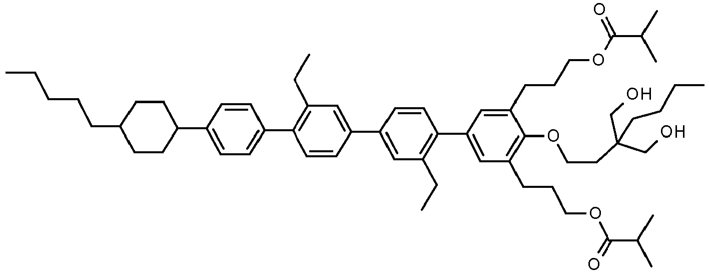

### Phasen Glastemperatur (Tg) -9°C, Schmelzpunkt 80°C.

¹H NMR (500 MHz, Chloroform-d) δ 7.57 (d, *J* = 1.9 Hz, 2H), 7.50 (dd, *J* = 7.8, 1.9 Hz, 2H), 7.38 - 7.20 (m, 6H), 7.07 (s, 2H), 6.13 (t, *J* = 1.3 Hz, 2H), 5.58 (p, *J* = 1.7 Hz, 2H), 4.26 (t, *J* = 6.5 Hz, 4H), 3.99 (t, *J* = 6.3 Hz, 2H), 3.71 (s, 4H), 2.96 - 2.63 (m, 9H), 2.55 (tt, *J* = 12.1, 3.4 Hz, 1H), 2.17 - 1.84 (m, 16H), 1.53 (qd, *J* = 12.8, 3.2 Hz, 3H), 1.41 - 1.01 (m, 23H), 0.93 (dt, *J* = 8.7, 7.0 Hz, 6H).

### Beispiel 3.

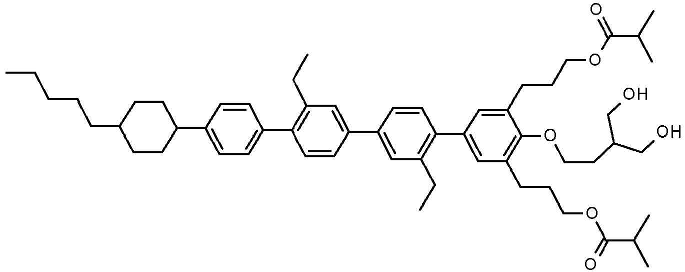

### Phasen: Glastemperatur (Tg) -17°C Schmelzpunkt 65°C

¹H NMR (500 MHz, Chloroform-d) δ 7.58 (d, *J* = 1.8 Hz, 2H), 7.51 (dd, *J* = 7.9, 2.0 Hz, 2H), 7.36 - 7.21 (m, 6H), 7.07 (s, 2H), 6.14 (s, 2H), 5.59 (q, *J* = 1.7 Hz, 2H), 4.28 (t, *J* = 6.6 Hz, 4H), 3.95 (dt, *J* = 10.4, 5.3 Hz, 4H), 3.84 (dd, *J =* 10.8, 6.7 Hz, 2H), 2.86 - 2.60 (m, 10H), 2.56 (tt, *J =* 12.1, 3.4 Hz, 1H), 2.15 (tt, *J* = 6.5, 4.2 Hz, 1H), 2.11 - 2.04 (m, 4H), 2.03 - 1.85 (m, 12H), 1.54 (qd, *J* = 12.8, 3.3 Hz, 3H), 1.32 (dddd, *J* = 26.7, 15.0, 8.8, 6.3 Hz, 9H), 1.20 (td, *J =* 7.5,4.6 Hz, 6H), 1.11 (qd, J = 13.1, 3.3 Hz, 2H), 0.93 (t, *J* = 7.0 Hz, 3H).

### Beispiel 4:

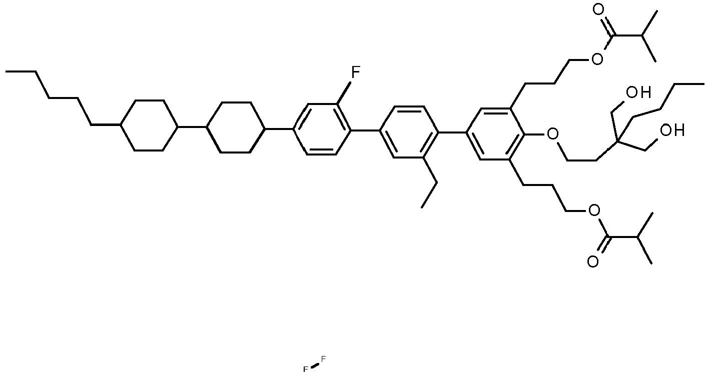

### Phasen: Glastemperatur (Tg) -20°C, Schmelzpunkt 71 °C, Phasenübergang Nematisch-Isotrop 120.0°C

¹H NMR (500 MHz, Chloroform-d) δ 7.49 (d, *J* = 1.6 Hz, 1H), 7.41 (ddd, *J =* 8.0, 5.0, 3.1 Hz, 2H), 7.25 (d, *J =* 7.9 Hz, 1H), 7.12 - 6.99 (m, 4H), 6.13 (t, *J =* 1.3 Hz, 2H), 5.58 (p, *J* = 1.6 Hz, 2H), 4.26 (t, *J* = 6.5 Hz, 4H), 3.99 (t, *J* = 6.3 Hz, 2H), 3.71 (s, 4H), 2.90 - 2.70 (m, 5H), 2.66 (q, *J* = 7.6 Hz, 2H), 2.57 - 2.48 (m, 1H), 2.12 - 1.94 (m, 13H), 1.90 (d, *J* = 9.1 Hz, 2H), 1.85 - 1.74 (m, 4H), 1.48 (q, *J* = 12.1 Hz, 2H), 1.32 (ddt, *J* = 23.3, 9.4, 6.4 Hz, 12H), 1.23 - 1.13 (m, 9H), 1.06 (dtd, *J =* 23.8, 13.2, 11.3, 2.4 Hz, 3H), 0.93 (dt, *J =* 16.1, 7.0 Hz, 8H).

### Beispiel 5:

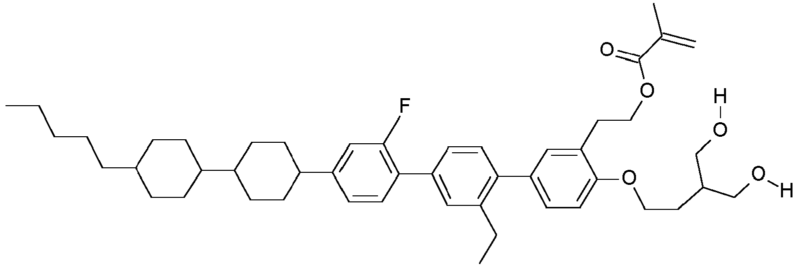

¹H NMR (500 MHz, Chloroform-d) δ 7.46 (t, *J =* 1.7 Hz, 1H), 7.39 (t, *J =* 8.0 Hz, 2H), 7.23 - 7.12 (m, 3H), 7.06 (dd, *J* = 8.0, 1.7 Hz, 1H), 7.01 (dd, *J =* 12.1, 1.7 Hz, 1H), 6.89 (d, *J* = 8.3 Hz, 1H), 6.08 (dd, *J* = 1.7, 1.0 Hz, 1H), 5.54 (p, *J* = 1.6 Hz, 1H), 4.41 (t, *J =* 7.2 Hz, 2H), 4.12 (t, *J =* 5.9 Hz, 2H), 3.90 (dd, *J =* 10.8, 4.1 Hz, 2H), 3.79 (dd, *J* = 10.8, 6.8 Hz, 2H), 3.04 (t, *J* = 7.2 Hz, 2H), 2.64 (q, *J* = 7.5 Hz, 2H), 2.50 (qd, *J* = 8.6, 7.6, 3.0 Hz, 3H), 2.14 (tdd, *J* = 6.8, 5.4, 2.8 Hz, 1H), 1.97 (dd, *J* = 12.9, 3.3 Hz, 2H), 1.93 - 1.83 (m, 7H), 1.82 - 1.70 (m, 4H), 1.51 - 1.39 (m, 2H), 1.35 - 1.20 (m, 6H), 1.20 - 0.95 (m, 12H), 0.88 (q, *J =* 7.0 Hz, 5H).

### Beispiel 6:

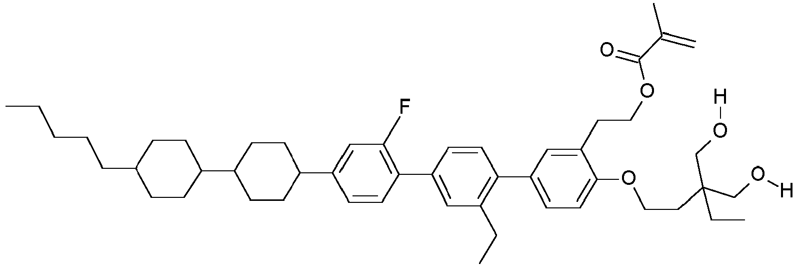

¹H NMR (500 MHz, Chloroform-d) δ 7.49 (s, 1H), 7.42 (t, *J* = 7.9 Hz, 2H), 7.26 - 7.16 (m, 3H), 7.09 (dd, *J* = 8.0, 1.6 Hz, 1H), 7.04 (dd, *J* = 12.0, 1.6 Hz, 1H), 6.96 (d, *J =* 8.3 Hz, 1H), 6.11 (s, 1H), 5.56 (t, *J =* 1.7 Hz, 1H), 4.42 (t, *J =* 7.1 Hz, 2H), 4.18 (t, *J* = 6.2 Hz, 2H), 3.93 - 3.48 (m, 4H), 3.06 (t, *J* = 7.1 Hz, 2H), 2.83 - 2.12 (m, 5H), 2.08 - 1.96 (m, 4H), 1.96 - 1.84 (m, 5H), 1.84 - 1.69 (m, 4H), 1.54 - 1.38 (m, 4H), 1.38 - 0.98 (m, 19H), 0.92 (dq, *J =* 14.9, 7.6 Hz, 8H).

### Weitere Beispiele (Synthese analog zu Beispiel 1):

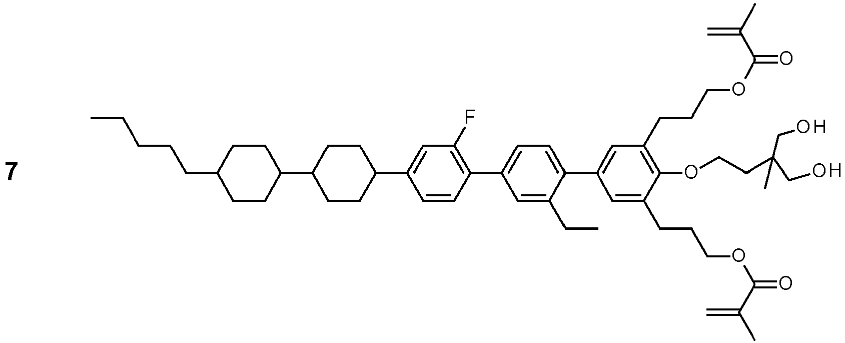

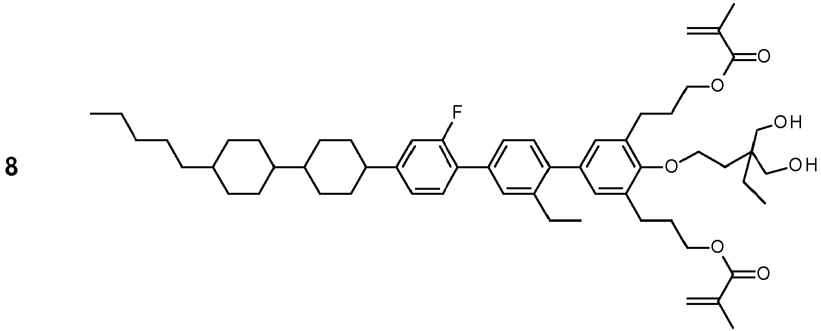

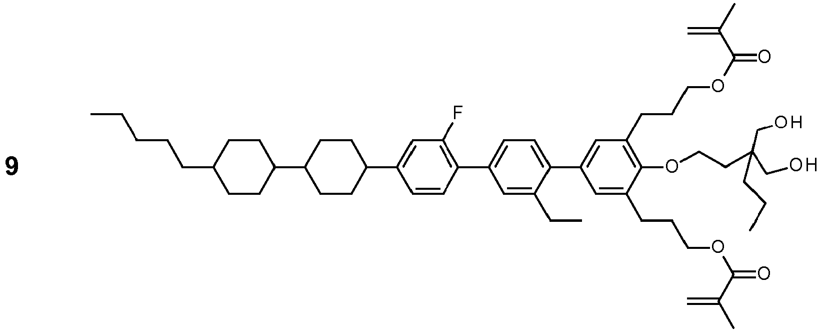

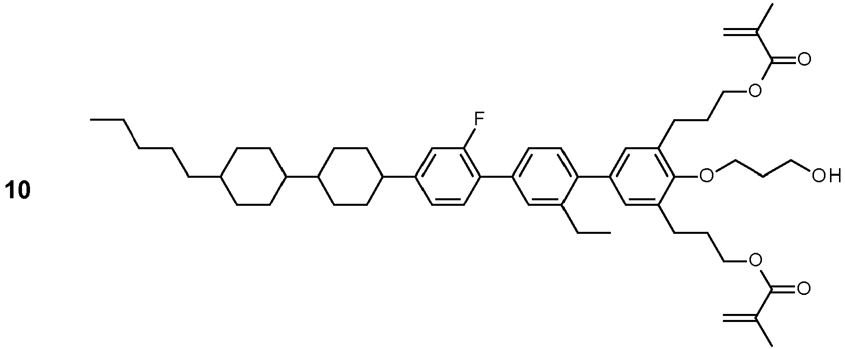

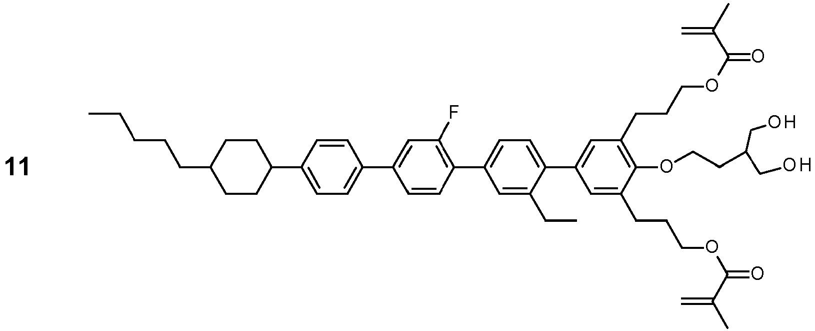

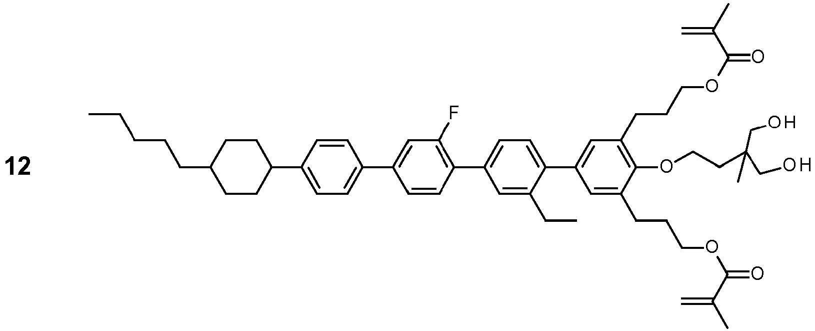

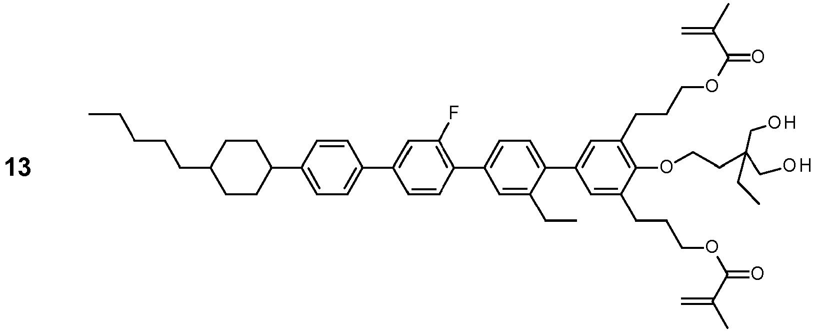

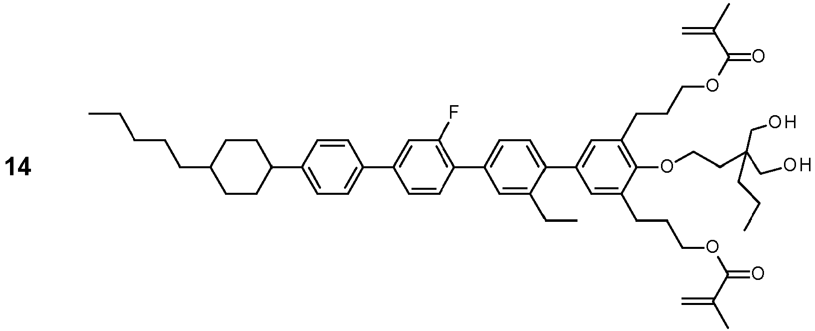

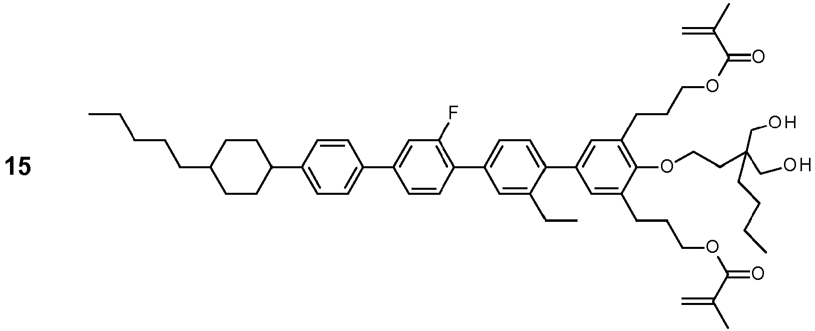

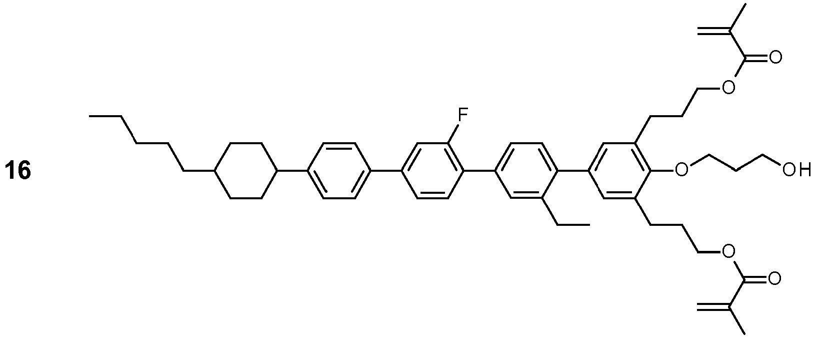

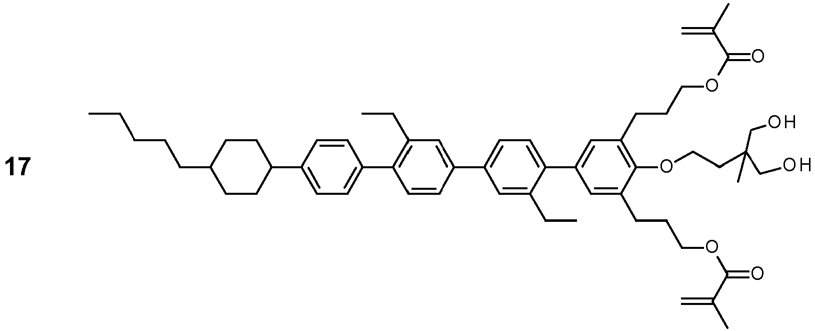

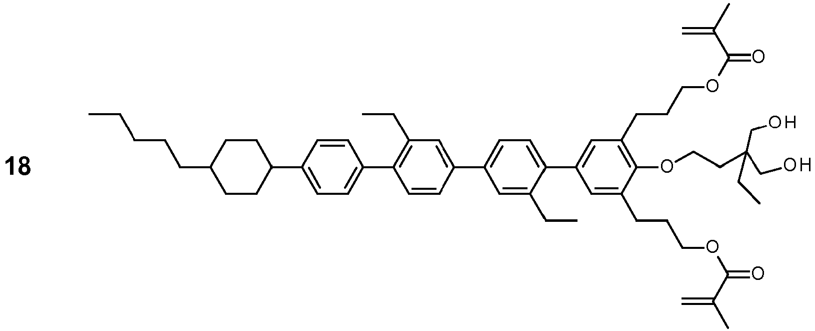

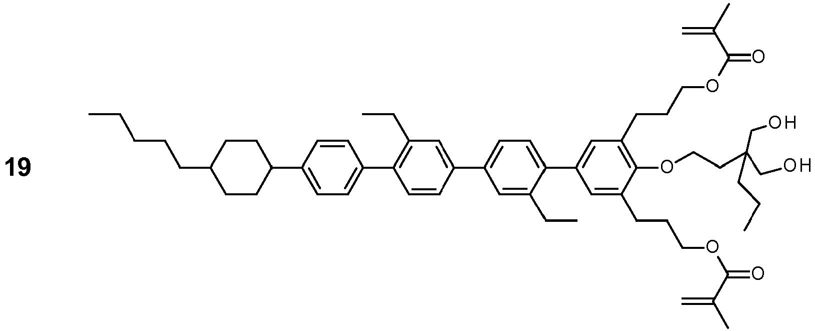

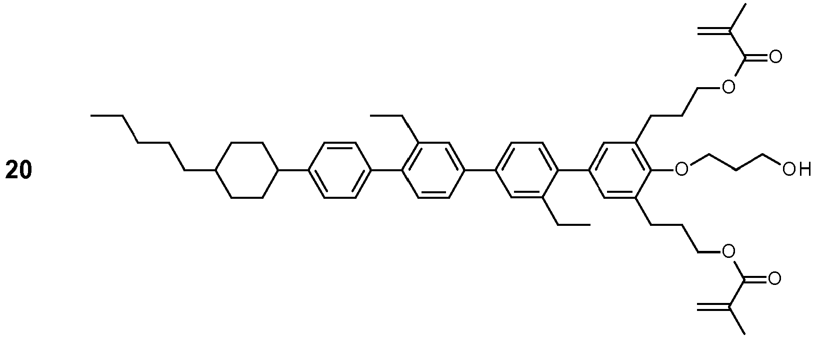

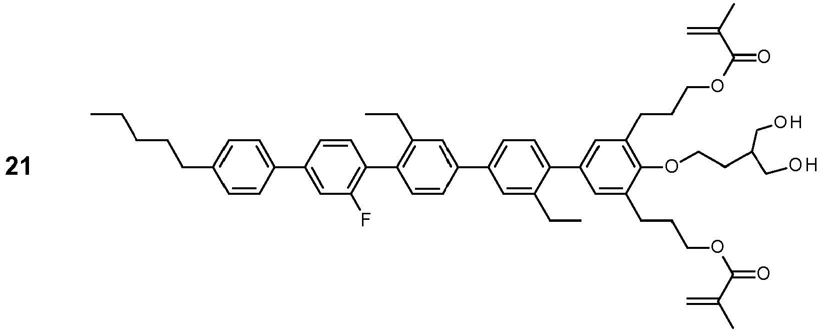

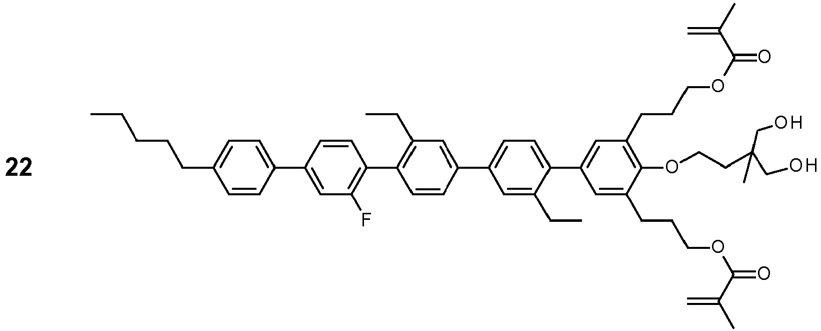

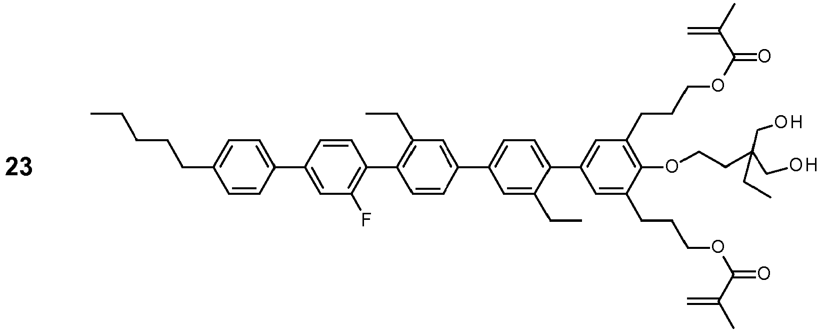

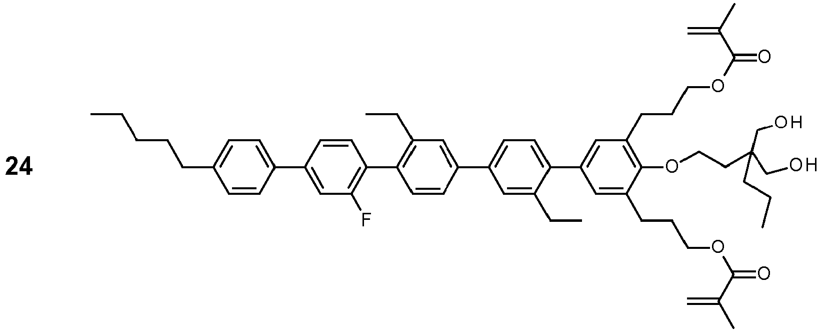

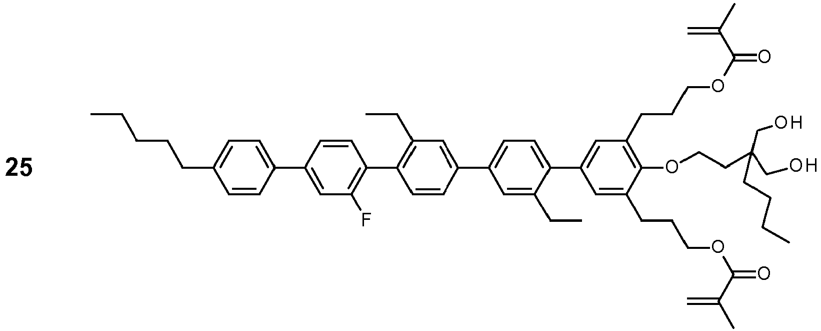

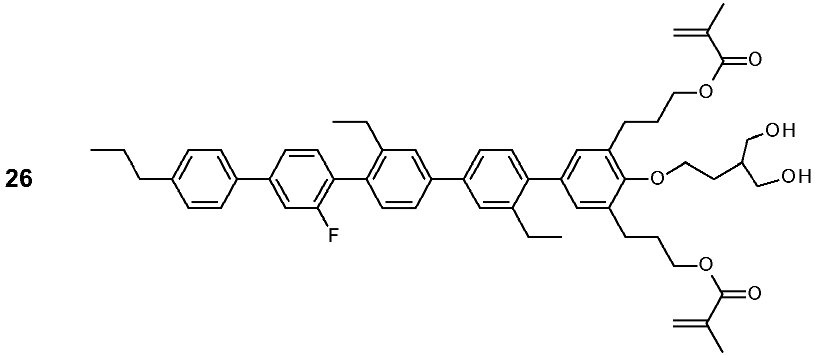

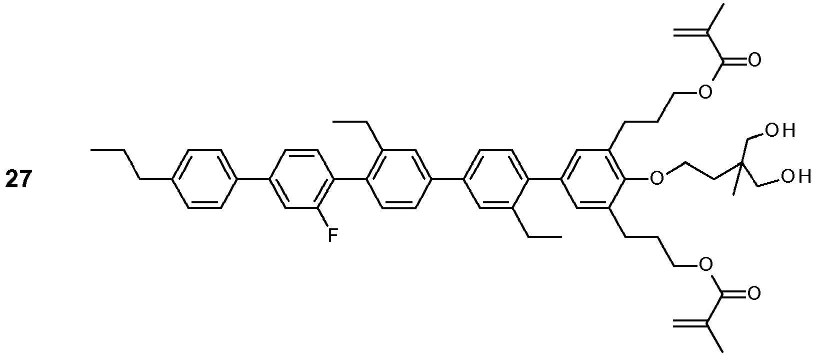

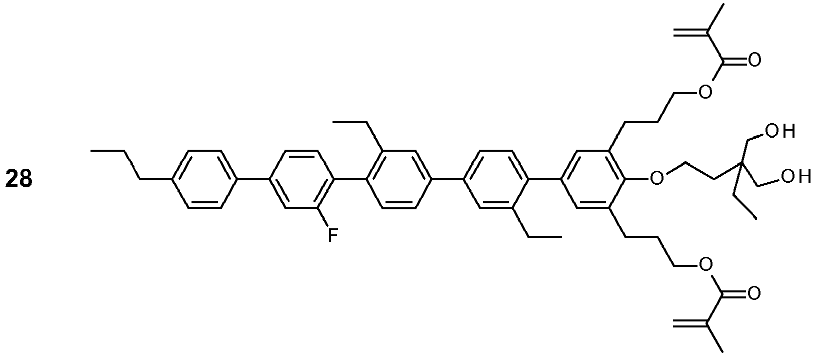

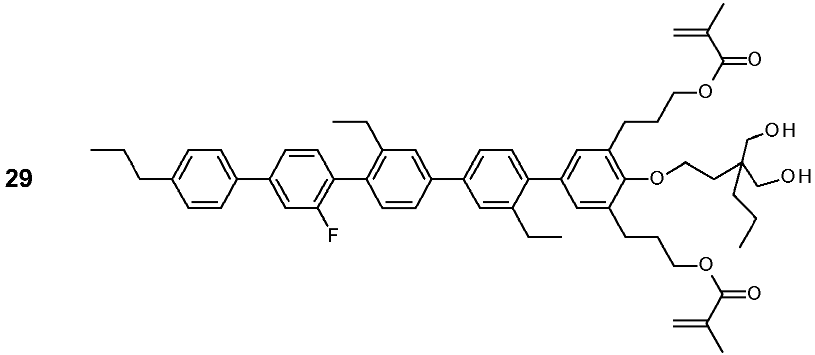

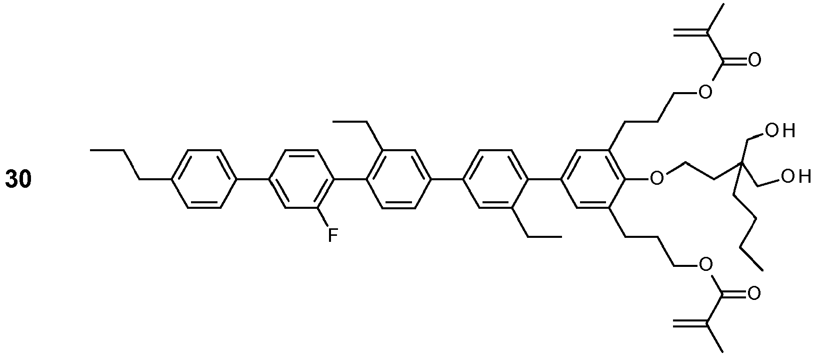

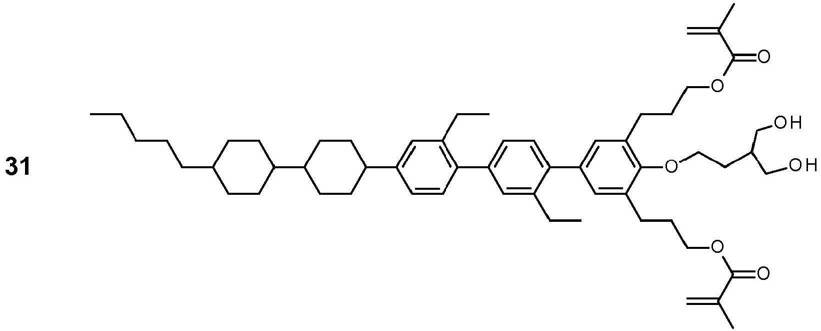

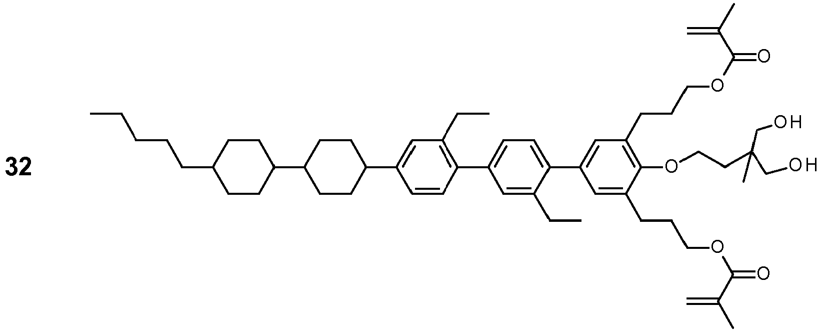

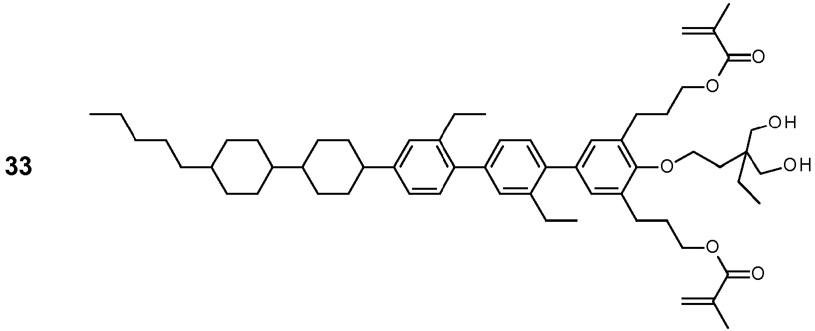

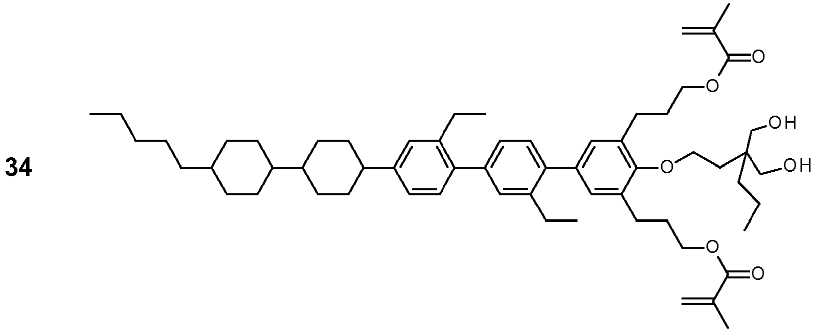

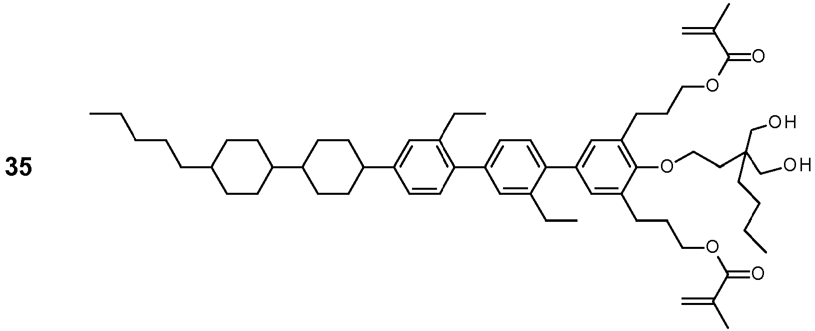

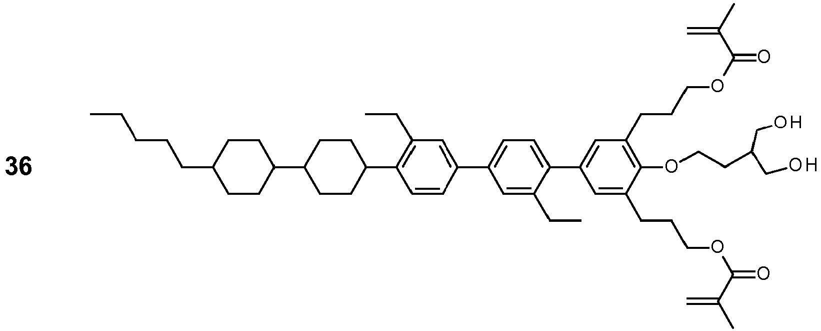

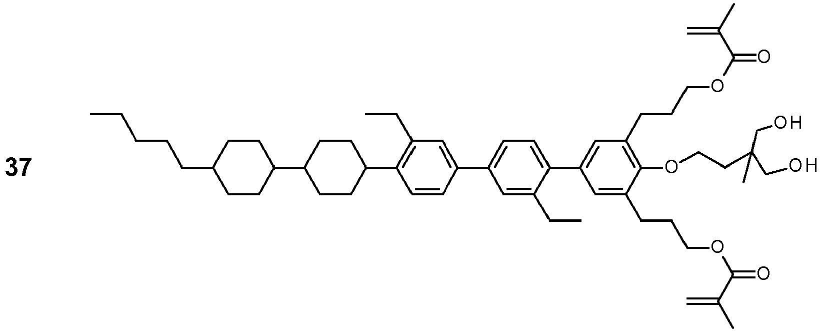

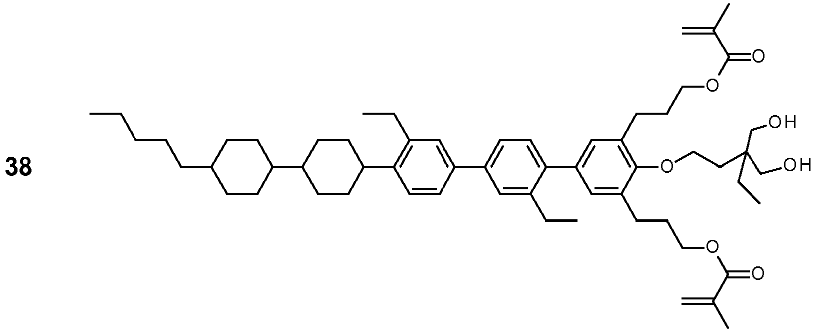

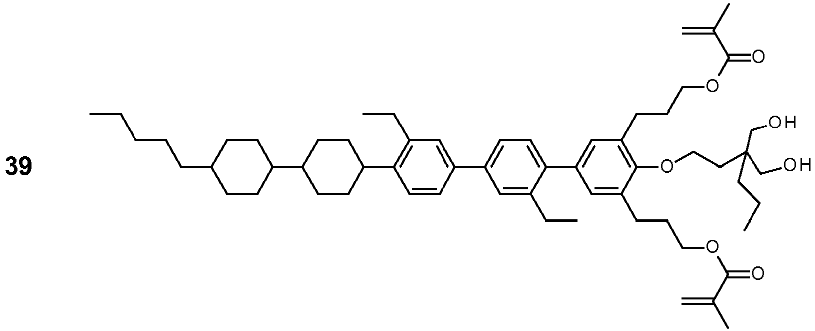

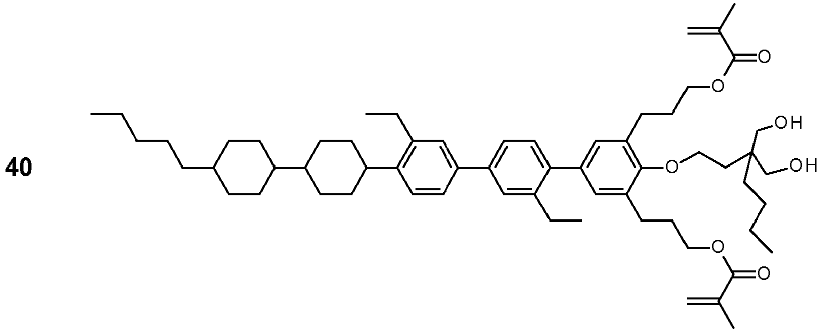

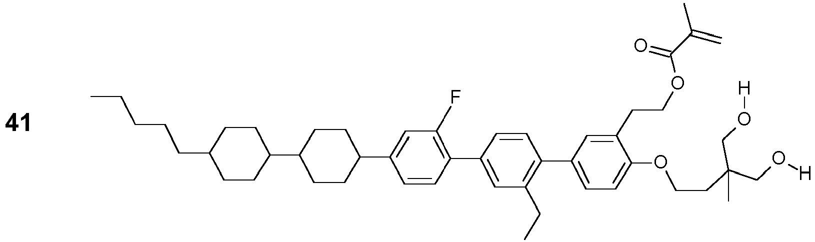

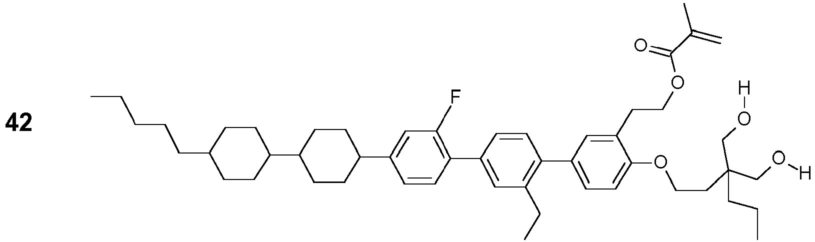

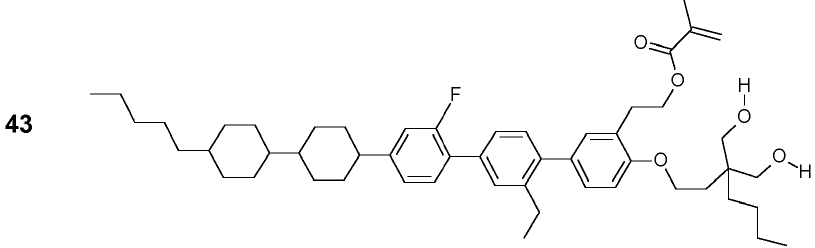

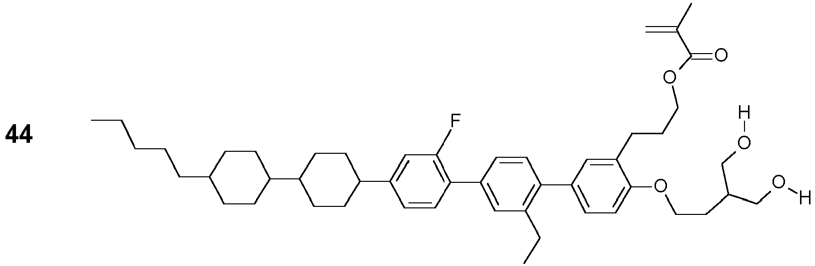

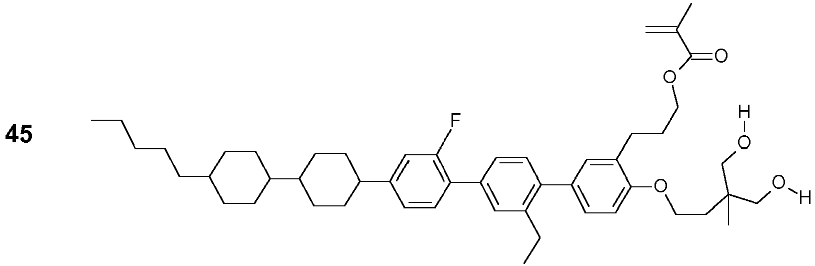

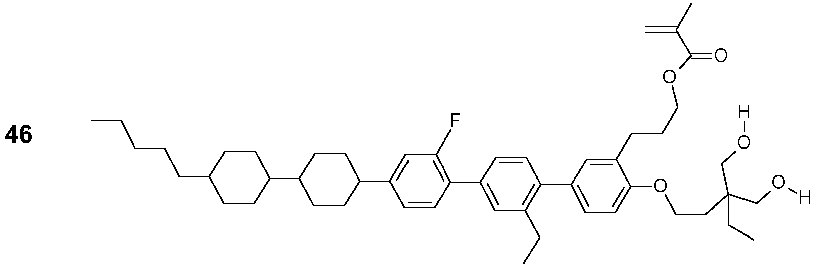

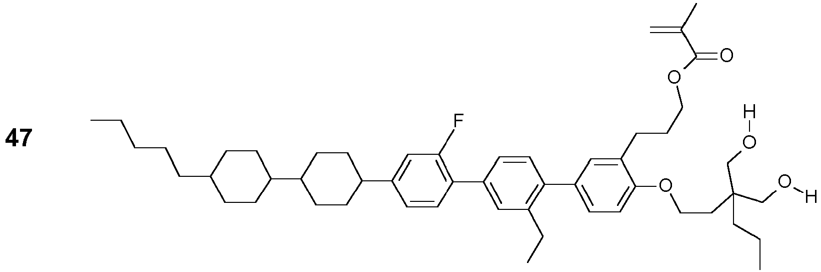

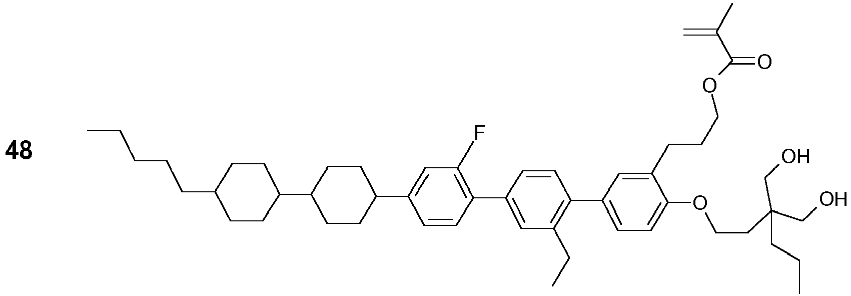

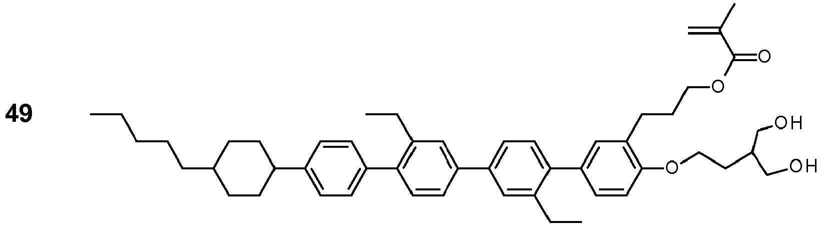

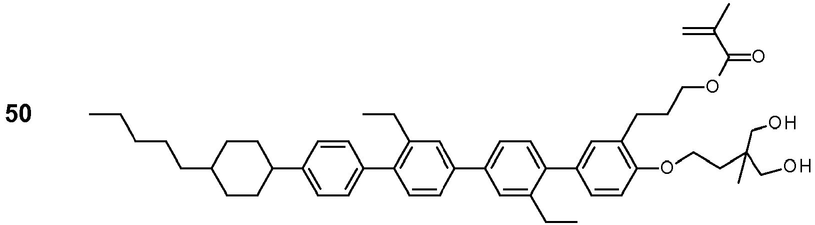

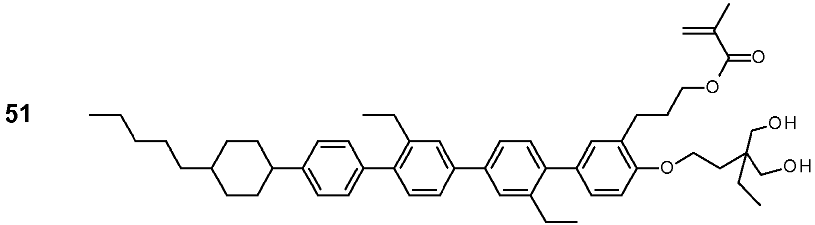

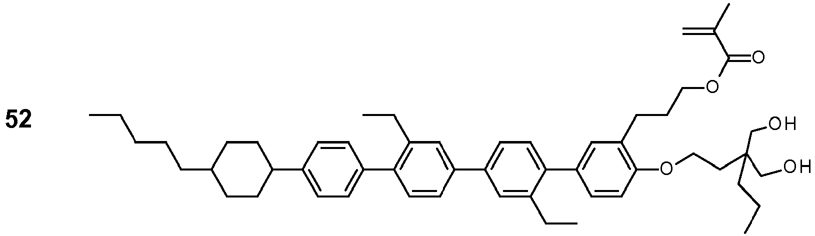

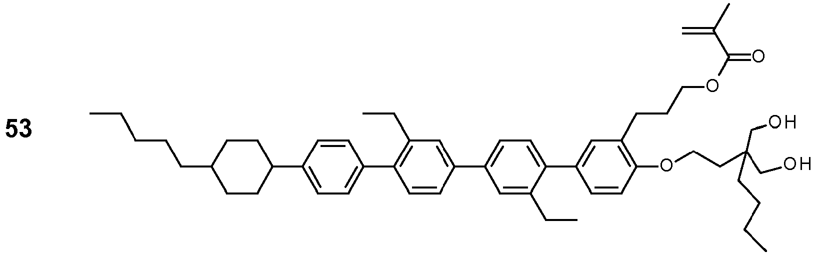

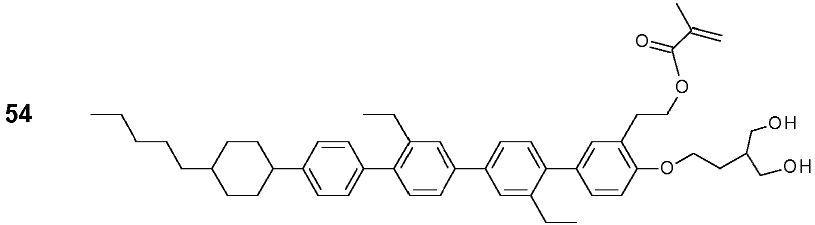

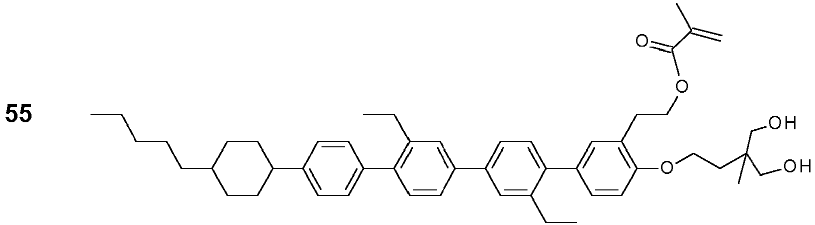

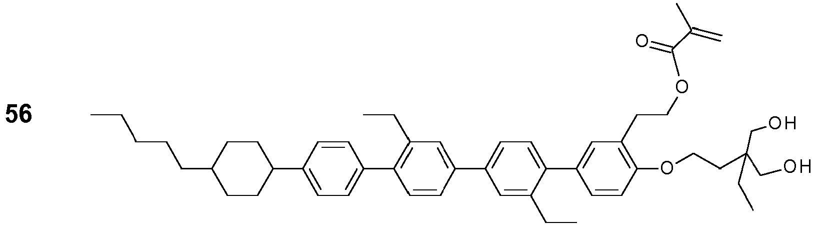

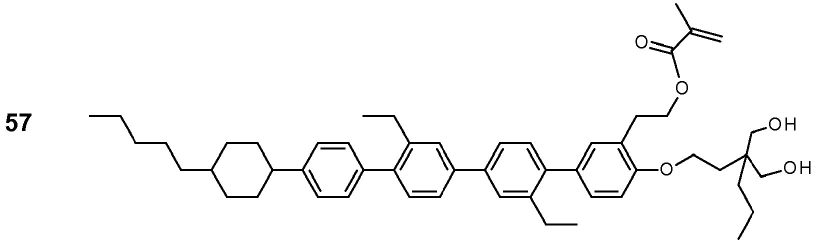

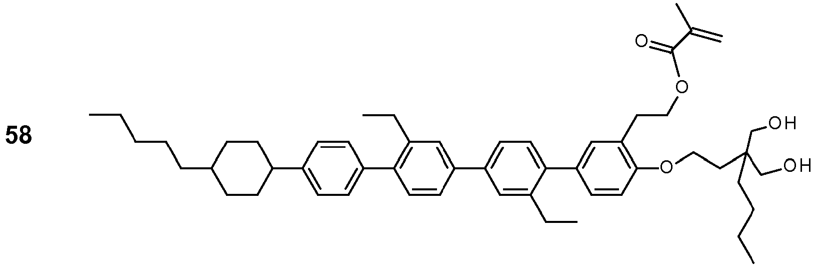

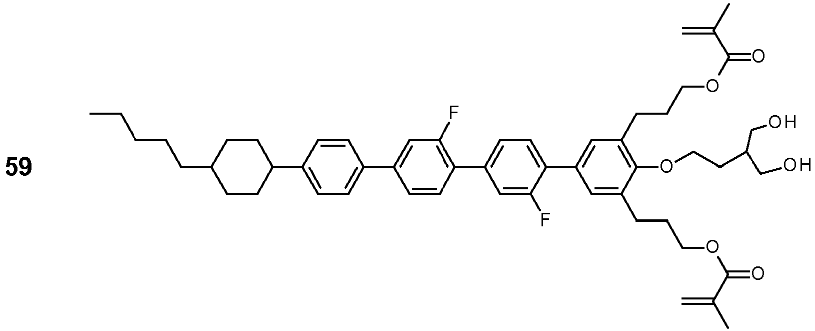

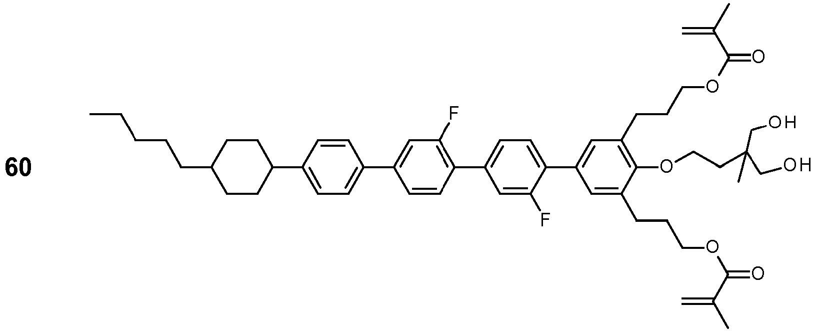

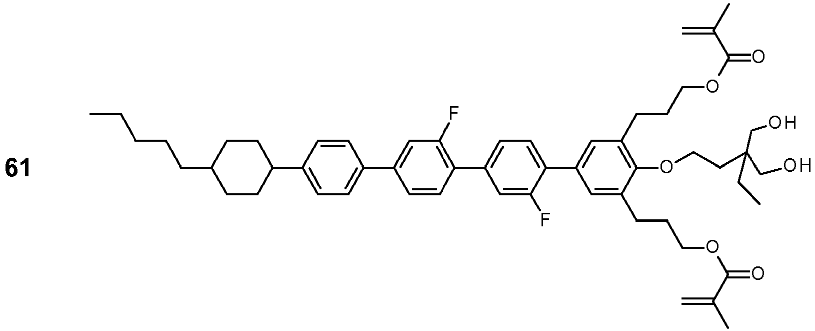

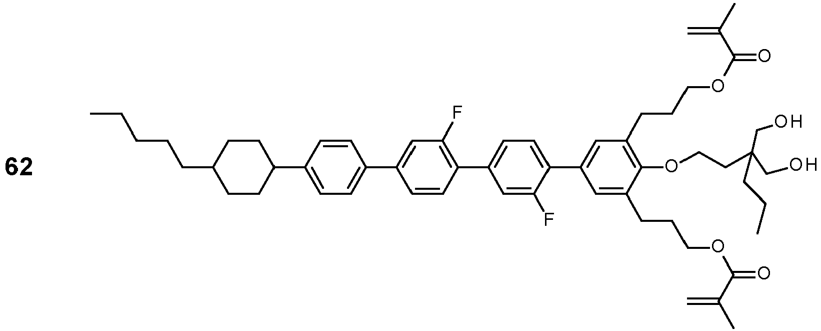

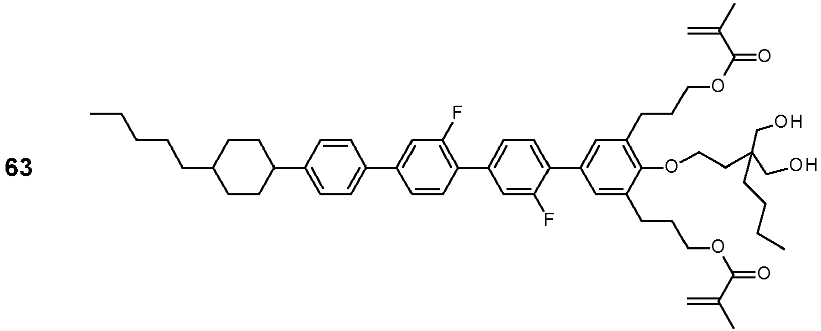

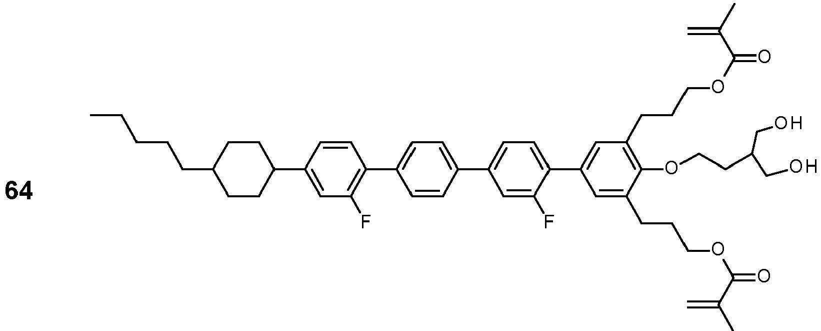

### (Vergleichsbeispiel)

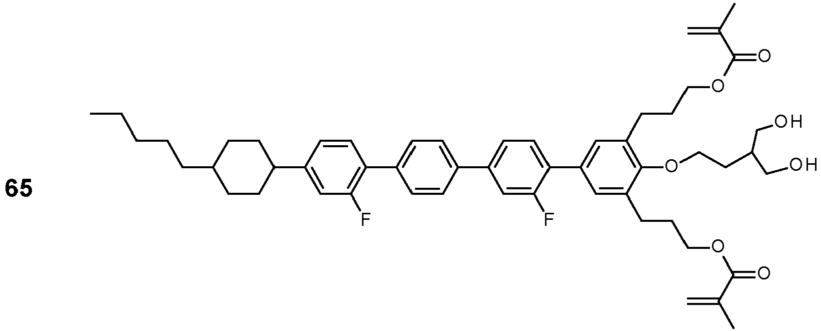

### (Vergleichsbeispiel)

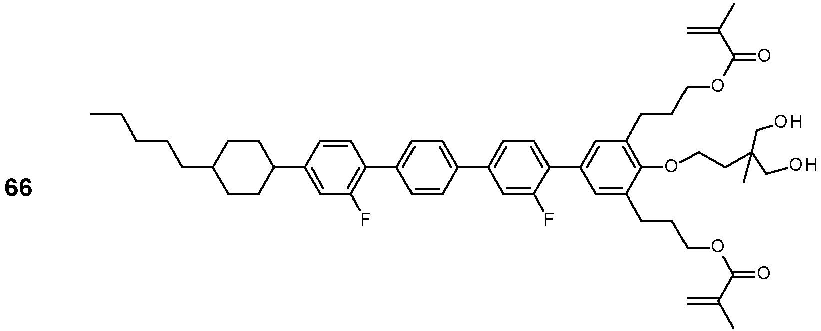

### (Vergleichsbeispiel)

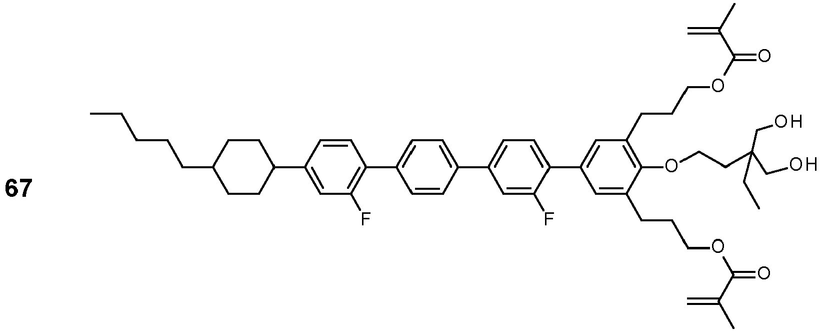

### (Vergleichsbeispiel)

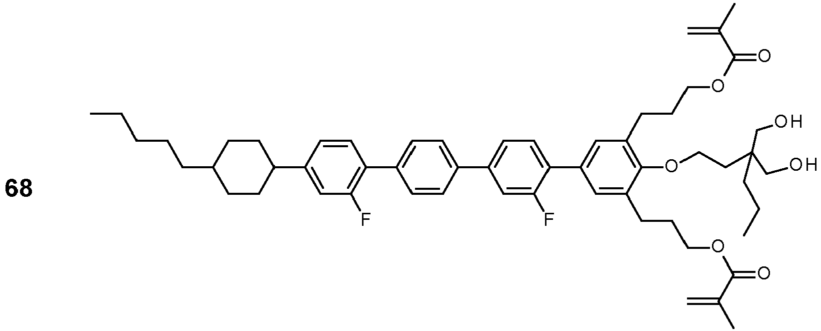

### (Vergleichsbeispiel)

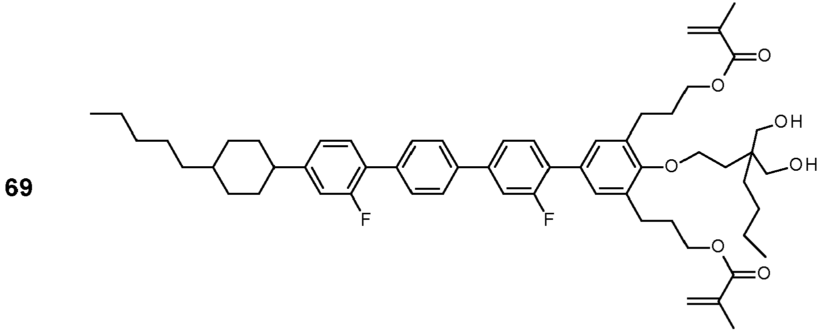

### (Vergleichsbeispiel)

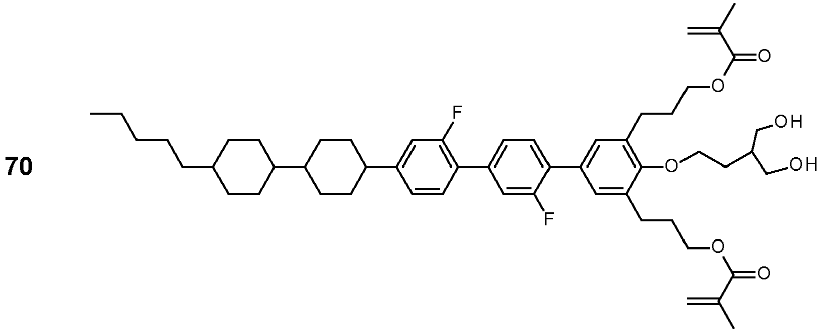

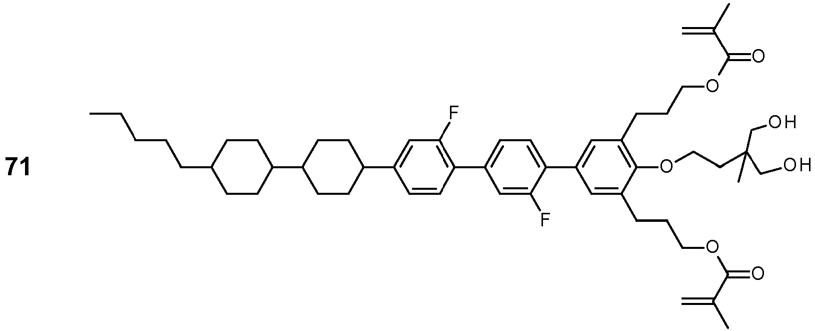

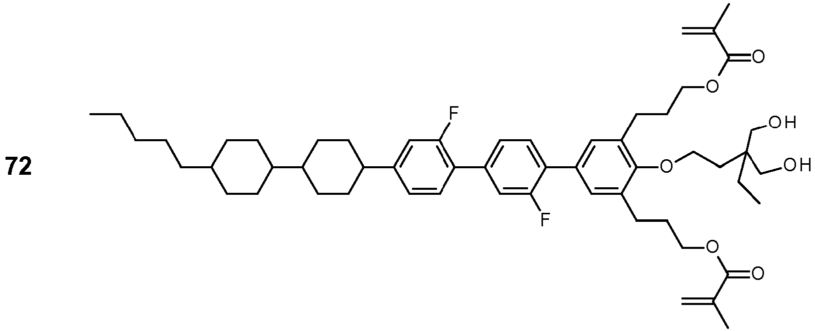

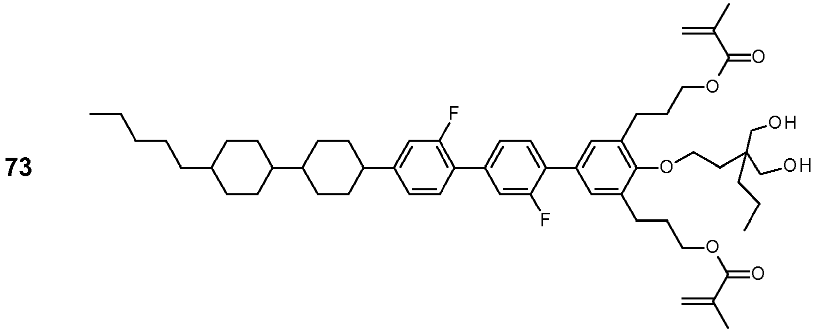

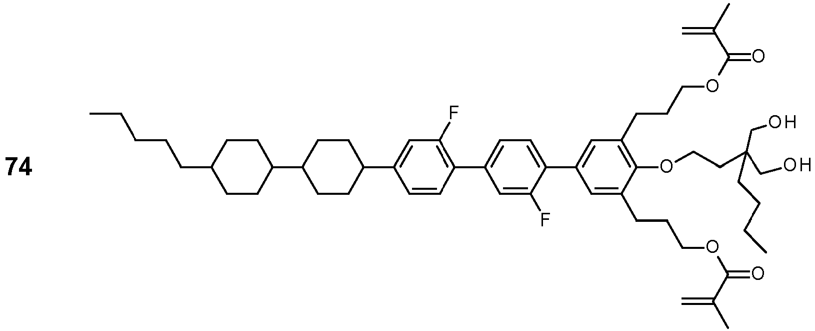

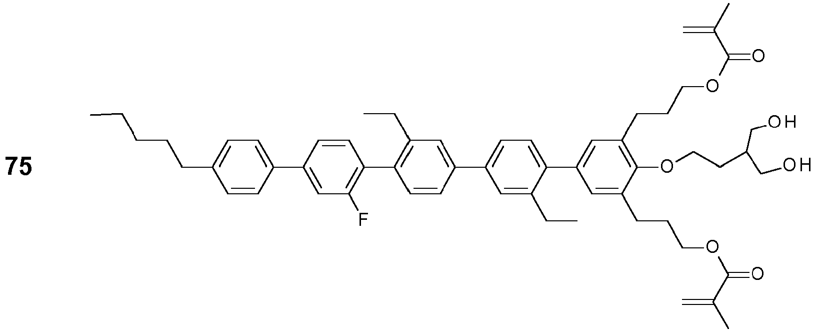

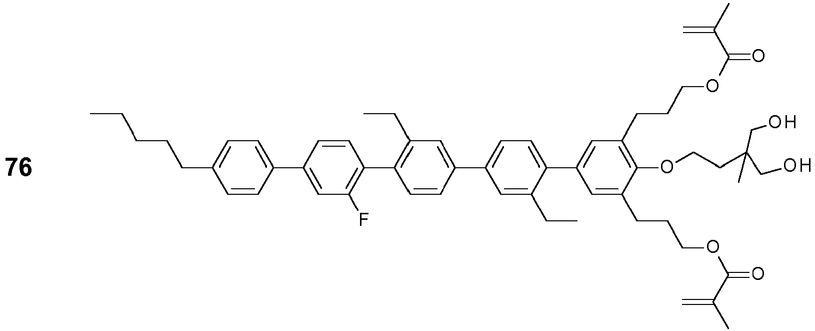

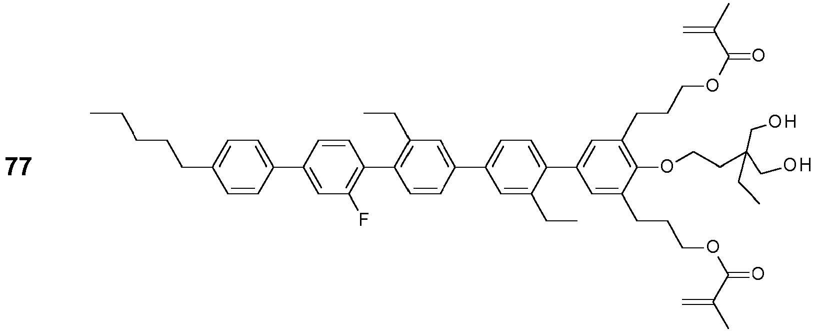

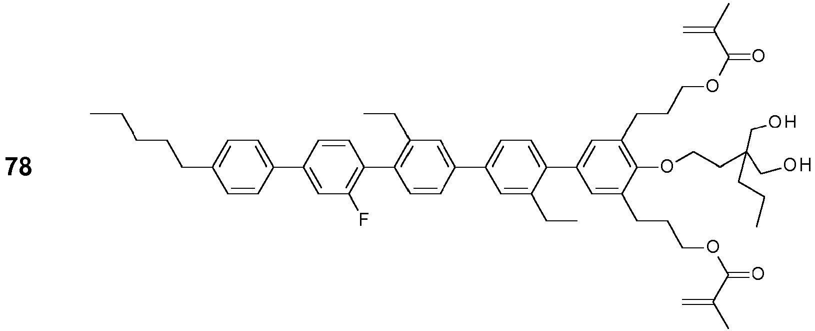

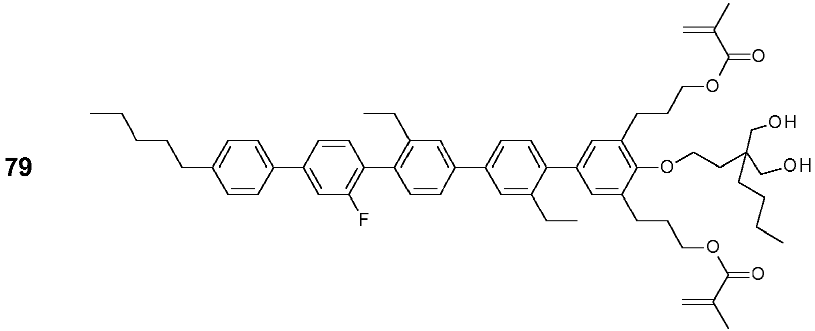

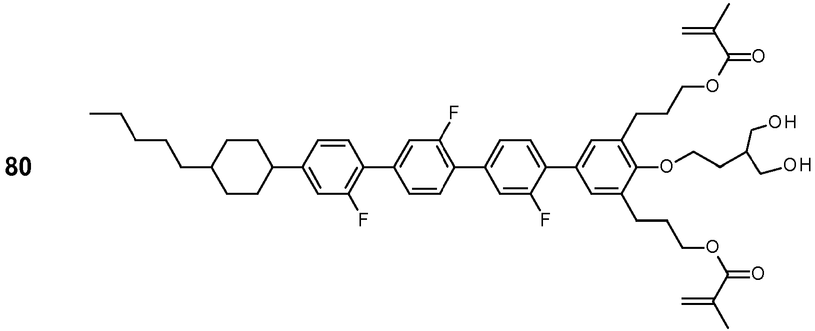

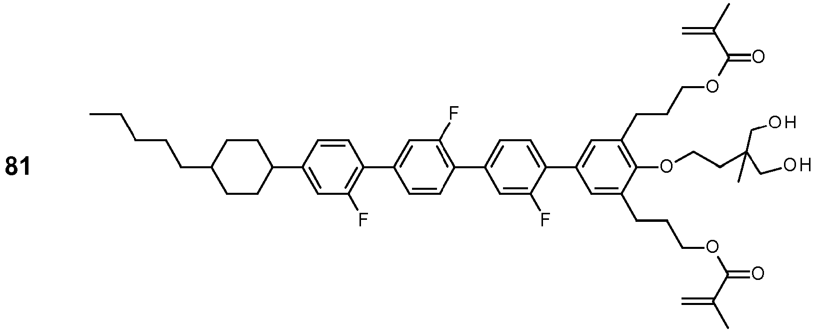

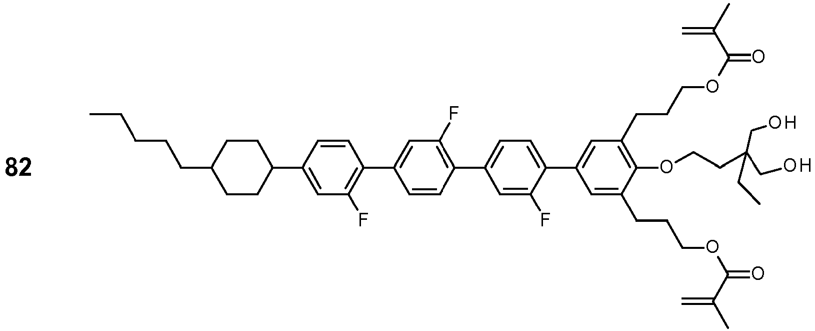

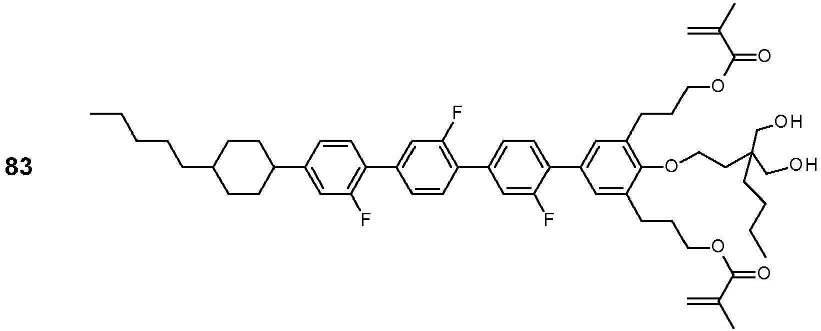

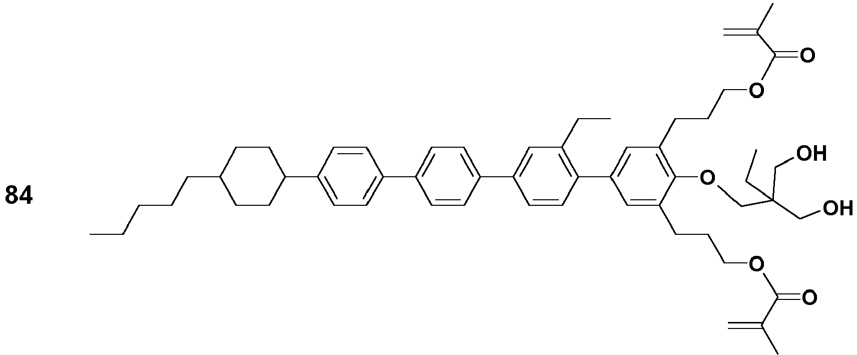

### (Vergleichsbeispiel)

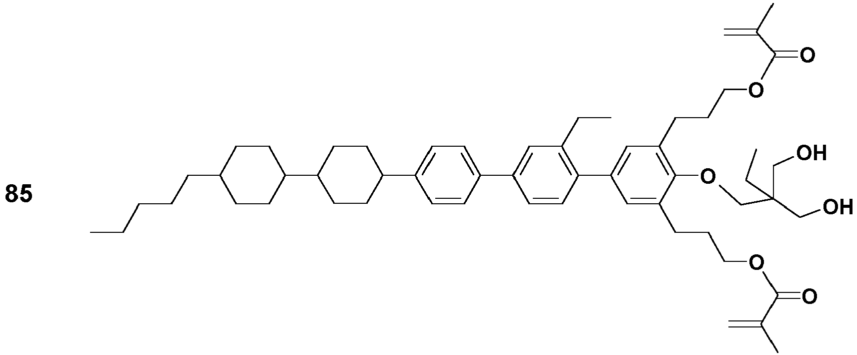

### (Vergleichsbeispiel)

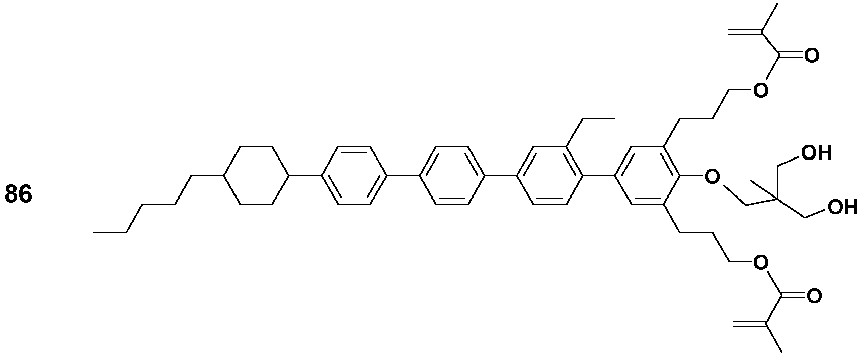

### (Vergleichsbeispiel)

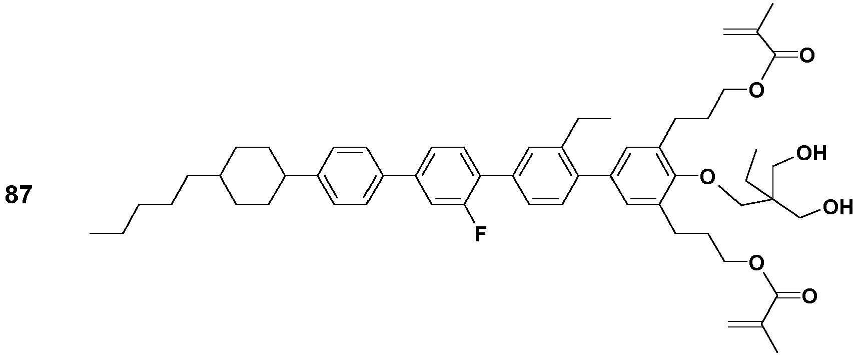

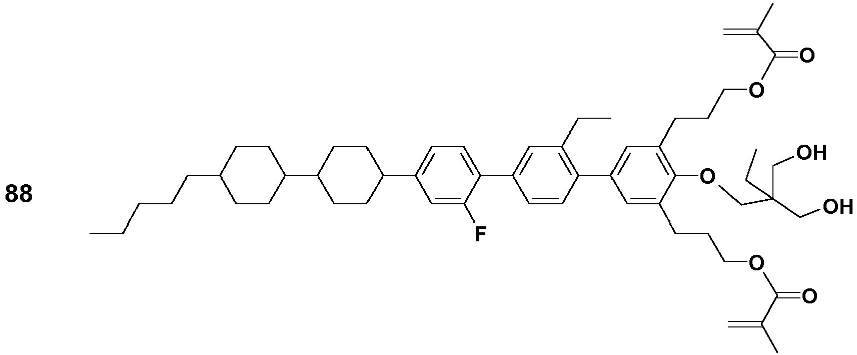

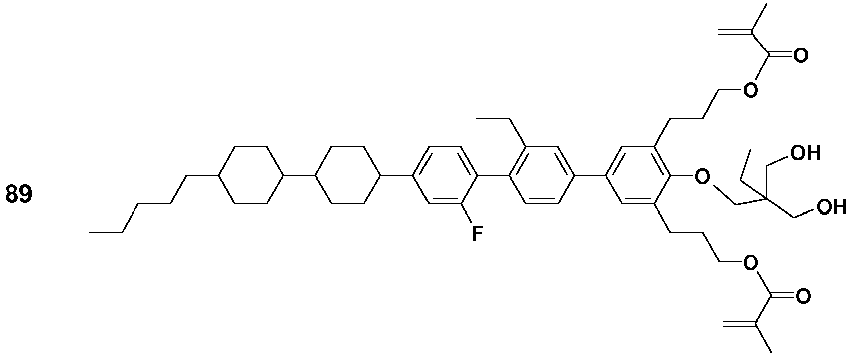

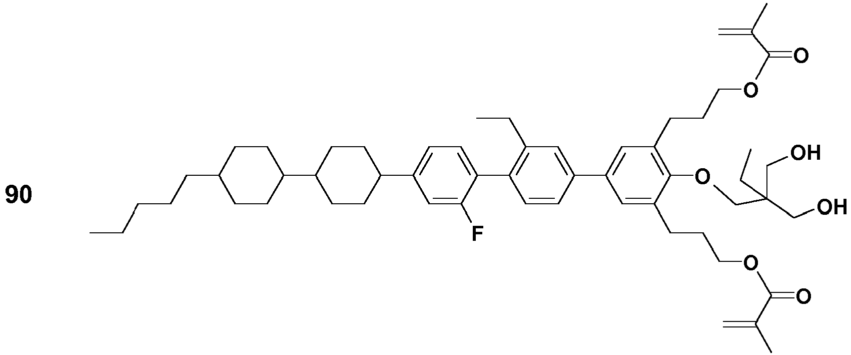

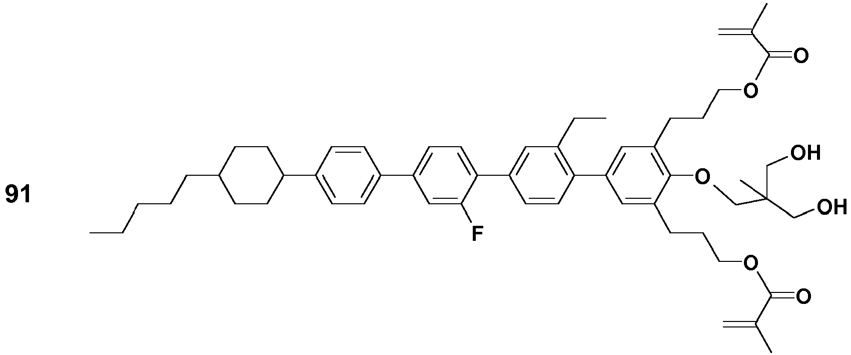

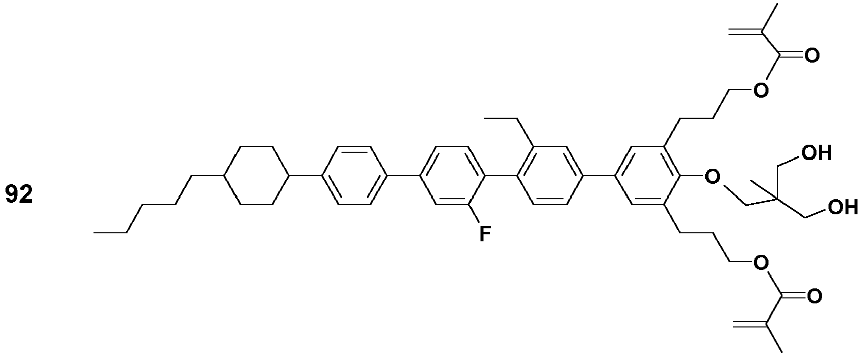

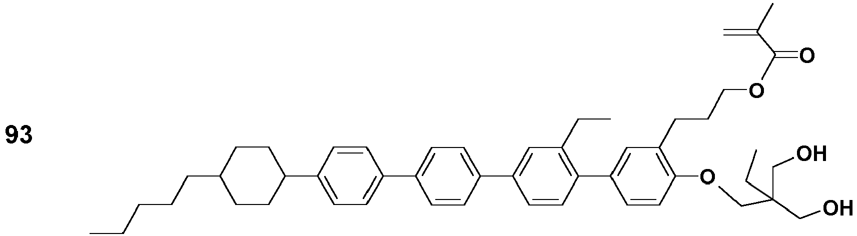

### (Vergleichsbeispiel)

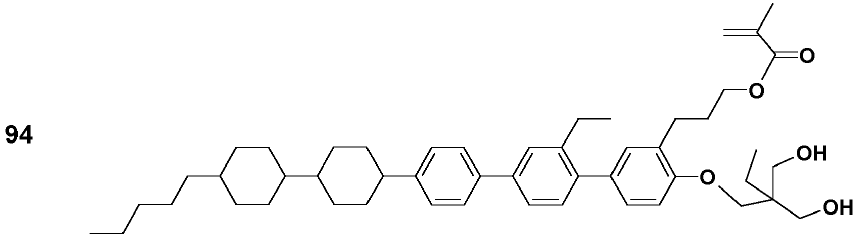

### (Vergleichsbeispiel)

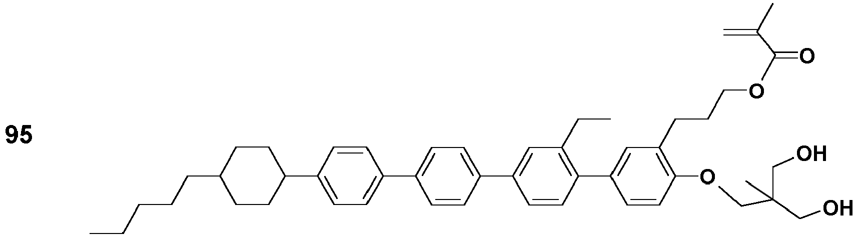

### (Vergleichsbeispiel)

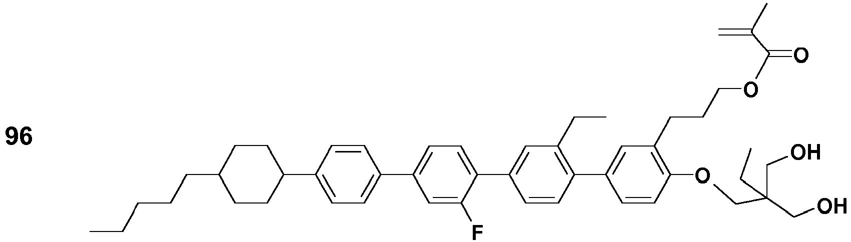

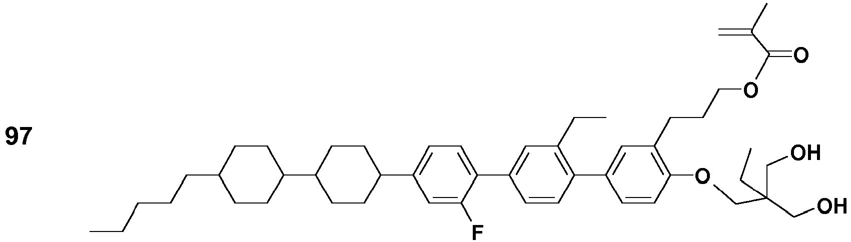

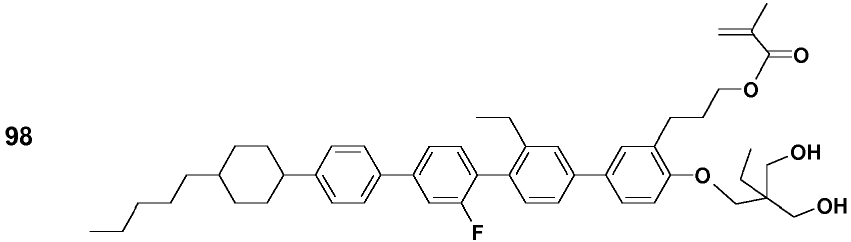

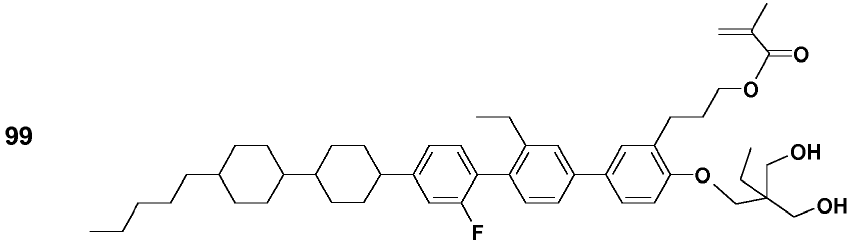

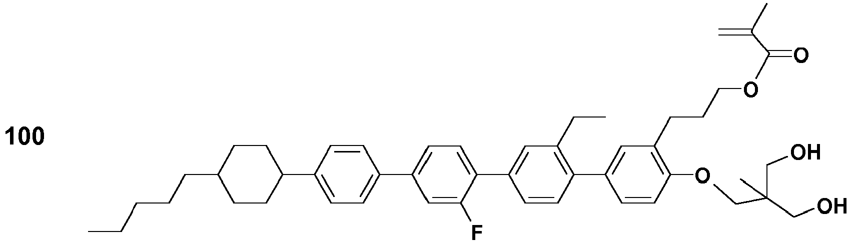

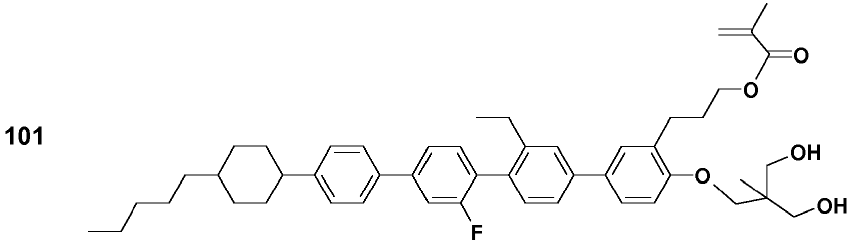

### B) Mischungsbeispiele

Zur Herstellung von erfindungsgemäßen FK-Medien werden die folgenden flüssigkristallinen Mischungen bestehend aus niedermolekularen Komponenten in den angegebenen prozentualen Gewichtsanteilen verwendet (Akronyme vgl. Tabellen A - D oben).
**H1:** Nematische Hostmischung (Δε < 0)

| | | | |
|---|---|---|---|
| CY-3-O2 | 10.0 % | Klärpunkt [°C]: | 81 |
| CCY-3-O1 | 8.0 % | Δn (589 nm, 20°C): | 0.103 |
| CCY-3-O2 | 11.0 % | Δε (1 kHz, 20°C): | -3.8 |
| CCY-4-O2 | 5.0 % | | |
| CCY-5-O2 | 2.0 % | | |
| CPY-2-O2 | 9.0 % | K₁ (20°C) [pN]: | 13.9 |
| CPY-3-O2 | 9.0 % | K₃ (20°C) [pN]: | 15.0 |
| CCH-34 | 9.0 % | γ₁ (20°C) [mPa·s]: | 133 |
| CCH-23 | 17.5 % | V₀ (20°C) [V]: | 2.10 |
| CP-3-O1 | 9.0 % | | |
| PYP-2-3 | 2.5 % | | |
| PY-3-O2 | 8.0 % | | |

**H2:** Nematische Hostmischung (Δε < 0)

| | | | |
|---|---|---|---|
| B-2O-O5 | 4.00 % | Klärpunkt [°C]: | 74.2 |
| CPP-3-2 | 8.00 % | Δn (589 nm, 20°C): | 0.109 |
| CC-3-V1 | 9.00 % | Δε (1 kHz, 20°C): | -3.1 |
| CC-3-O1 | 2.00 % | ε_{∥} (1 kHz, 20°C): | 3.6 |
| CC-3-4 | 8.00 % | ε_{⊥} (1 kHz, 20°C): | 6.7 |
| CC-3-5 | 7.00 % | K₁ (20°C) [pN]: | 14.5 |
| CCP-3-1 | 8.00 % | K₃ (20°C) [pN]: | 16.5 |
| CCP-V2-1 | 5.00 % | γ₁ (20°C) [mPa·s]: | 108 |
| CCY-3-O2 | 10.50 % | V₀ (20°C) [V]: | 2.41 |
| CLY-3-O2 | 1.00 % | | |
| CPY-3-O2 | 2.50 % | | |
| CY-3-O2 | 11.5 % | | |
| CP-3-O1 | 5.50 % | | |
| PY-3-O2 | 18.0 % | | |

**H3:** Nematische Hostmischung (Δε < 0)

| | | | |
|---|---|---|---|
| CPP-3-2 | 6.0 % | Klärpunkt [°C]: | 74.8 |
| CC-3-V1 | 6.0 % | Δn (589 nm, 20°C): | 0.107 |
| CC-3-4 | 9.0 % | Δε (1 kHz, 20°C): | -3.3 |
| CC-3-5 | 7.0 % | ε_{∥} (1 kHz, 20°C): | 3.6 |
| CCP-3-1 | 8.0 % | ε_{⊥} (1 kHz, 20°C): | 6.9 |
| CCP-3-3 | 3.0 % | K₁ (20°C) [pN]: | 14.2 |
| CCY-3-1 | 2.0 % | K₃ (20°C) [pN]: | 16.5 |
| CCY-3-O2 | 10.5 % | γ₁ (20°C) [mPa·s]: | 118 |
| CCY-4-O2 | 5.0 % | V₀ (20°C) [V]: | |
| CPY-3-O2 | 3.5 % | | |
| CY-3-O2 | 14 % | | |
| CP-3-O1 | 5.5 % | | |
| PY-1-O4 | 6.5 % | | |
| PY-3-O2 | 14 % | | |

**H4:** Nematische Hostmischung (Δε < 0)

| | | | |
|---|---|---|---|
| CY-3-O2 | 15.5 % | Klärpunkt [°C]: | 75.1 |
| CCY-3-O3 | 8.00 % | Δn (589 nm, 20°C): | 0.098 |
| CCY-4-O2 | 10.0 % | Δε (1 kHz, 20°C): | -3.0 |
| CPY-2-O2 | 5.50 % | ε_{∥} (1 kHz, 20°C): | 3.4 |
| CPY-3-O2 | 11.5 % | ε_{⊥} (1 kHz, 20°C): | 6.4 |
| CC-3-4 | 9.25 % | K₁ (20°C) [pN]: | 13.1 |
| CC-2-3 | 24.5 % | K₃ (20°C) [pN]: | 13.3 |
| PYP-2-3 | 8.75 % | γ₁ (20°C) [mPa·s]: | 113 |
| CP-3-O1 | 7.00 % | V₀ (20°C) [V]: | 2.22 |

**H5:** Nematische Hostmischung (Δε < 0)

| | | | |
|---|---|---|---|
| CY-3-O4 | 14.0 % | Klärpunkt [°C]: | 80.0 |
| CCY-3-O2 | 9.00 % | Δn (589 nm, 20°C): | 0.090 |
| CCY-3-O3 | 9.00 % | Δε (1 kHz, 20°C): | -3.3 |
| CPY-2-O2 | 10.0 % | ε_{∥} (1 kHz, 20°C): | 3.4 |
| CPY-3-O2 | 10.0 % | ε_{⊥} (1 kHz, 20°C): | 6.7 |
| CCY-3-1 | 8.00 % | K₁ (20°C) [pN]: | 15.1 |
| CC-3-4 | 9.00 % | K₃ (20°C) [pN]: | 14.6 |
| CC-3-5 | 6.00 % | γ₁ (20°C) [mPa·s]: | 140 |
| CP-5-3 | 10.0 % | V₀ (20°C) [V]: | 2.23 |
| CC-3-O1 | 6.00 % | | |
| CC-3-O3 | 9.00 % | | |

**H6:** Nematische Hostmischung (Δε < 0)

| | | | |
|---|---|---|---|
| CC-3-V1 | 9.00 % | Klärpunkt [°C]: | 74.7 |
| CC-2-3 | 18.0 % | Δn (589 nm, 20°C): | 0.098 |
| CC-3-4 | 3.00 % | Δε (1 kHz, 20°C): | -3.4 |
| CC-3-5 | 7.00 % | ε_{∥} (1 kHz, 20°C): | 3.5 |
| CCP-3-1 | 5.50 % | ε_{⊥} (1 kHz, 20°C): | 6.9 |
| CCY-3-O2 | 11.5 % | K₁ (20°C) [pN]: | 14.9 |
| CPY-2-O2 | 8.00 % | K₃ (20°C) [pN]: | 15.9 |
| CPY-3-O2 | 11.0 % | γ₁ (20°C) [mPa·s]: | 108 |
| CY-3-O2 | 15.5 % | V₀ (20°C) [V]: | 2.28 |
| PY-3-O2 | 11.5 % | | |

**H7:** Nematische Hostmischung (Δε < 0)

| | | | |
|---|---|---|---|
| CC-3-V | 37.5 % | Klärpunkt [°C]: | 74.8 |
| CC-3-V1 | 2.00 % | Δn (589 nm, 20°C): | 0.099 |
| CCY-4-O2 | 14.5 % | Δε (1 kHz, 20°C): | -2.9 |
| CPY-2-O2 | 10.5 % | ε_{∥} (1 kHz, 20°C): | 3.7 |
| CPY-3-O2 | 9.50 % | ε_{⊥} (1 kHz, 20°C): | 6.6 |
| CY-3-O2 | 15.0 % | K₁ (20°C) [pN]: | 12.2 |
| CY-3-O4 | 4.50 % | K₃ (20°C) [pN]: | 13.4 |
| PYP-2-4 | 5.50 % | γ₁ (20°C) [mPa·s]: | 92 |
| PPGU-3-F | 1.00 % | V₀ (20°C) [V]: | 2.28 |

**H8:** Nematische Hostmischung (Δε < 0)

| | | | |
|---|---|---|---|
| CC-2-3 | 20.0 % | Klärpunkt [°C]: | 74.8 |
| CC-3-O1 | 6.00 % | Δn (589 nm, 20°C): | 0.105 |
| CC-3-4 | 6.00 % | Δε (1 kHz, 20°C): | -3.2 |
| CCP-3-1 | 3.00 % | ε_{∥} (1 kHz, 20°C): | 3.5 |
| CCY-3-O2 | 11.0 % | ε_{⊥} (1 kHz, 20°C): | 6.8 |
| CPY-2-O2 | 12.0 % | K₁ (20°C) [pN]: | 12.7 |
| CPY-3-O2 | 11.0 % | K₃ (20°C) [pN]: | 13.6 |
| CY-3-O2 | 14.0 % | γ₁ (20°C) [mPa·s]: | 120 |
| CY-3-O4 | 4.00 % | V₀ (20°C) [V]: | 2.16 |
| CP-3-O1 | 4.00 % | | |
| PYP-2-3 | 9.00 % | | |

**H9:** Nematische Hostmischung (Δε < 0)

| | | | |
|---|---|---|---|
| CC-4-V | 17.0 % | Klärpunkt [°C]: | 106.1 |
| CCP-V-1 | 15.0 % | Δn (589 nm, 20°C): | 0.120 |
| CCEPC-3-3 | 2.50 % | Δε (1 kHz, 20°C): | -3.6 |
| CCY-3-O2 | 4.00 % | ε_{∥} (1 kHz, 20°C): | 3.5 |
| CCY-3-O3 | 5.00 % | ε_{⊥} (1 kHz, 20°C): | 7.0 |
| CCY-4-O2 | 5.00 % | K₁ (20°C) [pN]: | 16.8 |
| CLY-3-O2 | 3.50 % | K₃ (20°C) [pN]: | 17.3 |
| CLY-3-O3 | 2.00 % | γ₁ (20°C) [mPa·s]: | 207 |
| CPY-2-O2 | 8.00 % | V₀ (20°C) [V]: | 2.33 |
| CPY-3-O2 | 10.0 % | | |
| CY-3-O4 | 17.0 % | | |
| PYP-2-3 | 11.0 % | | |

**H10:** Nematische Hostmischung (Δε < 0)

| | | | |
|---|---|---|---|
| CY-3-O2 | 15.0 % | Klärpunkt [°C]: | 75.5 |
| CCY-4-O2 | 9.50 % | Δn (589 nm, 20°C): | 0.108 |
| CCY-5-O2 | 5.00 % | Δε (1 kHz, 20°C): | -3.0 |
| CPY-2-O2 | 9.00 % | ε_{∥} (1 kHz, 20°C): | 3.5 |
| CPY-3-O2 | 9.00 % | ε_{⊥} (1 kHz, 20°C): | 6.5 |
| CC-3-4 | 9.00 % | K₁ (20°C) [pN]: | 12.9 |
| CC-2-3 | 22.0 % | K₃ (20°C) [pN]: | 13.0 |
| PYP-2-3 | 7.00 % | γ₁ (20°C) [mPa·s]: | 115 |
| PYP-2-4 | 7.50 % | V₀ (20°C) [V]: | 2.20 |
| CP-3-O1 | 7.00 % | | |

**H11:** Nematische Hostmischung (Δε < 0)

| | | | |
|---|---|---|---|
| CY-3-O2 | 15.0 % | Klärpunkt [°C]: | 74.7 |
| CY-5-O2 | 6.50 % | Δn (589 nm, 20°C): | 0.108 |
| CCY-3-O2 | 11.0 % | Δε (1 kHz, 20°C): | -3.0 |
| CPY-2-O2 | 5.50 % | ε_{∥} (1 kHz, 20°C): | 3.6 |
| CPY-3-O2 | 10.5 % | ε_{⊥} (1 kHz, 20°C): | 6.6 |
| CC-3-V | 28.5 % | K₁ (20°C) [pN]: | 12.9 |
| CC-3-V1 | 10.0 % | K₃ (20°C) [pN]: | 15.7 |
| PYP-2-3 | 12.5 % | γ₁ (20°C) [mPa·s]: | 97 |
| PPGU-3-F | 0.50 % | V₀ (20°C) [V]: | 2.42 |

**H12:** Nematische Hostmischung (Δε < 0)

| | | | |
|---|---|---|---|
| CC-3-5 | 9.50 % | Klärpunkt [°C]: | 79.1 |
| CC-5-O1 | 5.00 % | Δn (589 nm, 20°C): | 0.091 |
| CCY-2-1 | 9.50 % | Δε (1 kHz, 20°C): | -3.6 |
| CCY-3-1 | 10.5 % | ε_{∥} (1 kHz, 20°C): | 3.5 |
| CCY-3-O2 | 10.5 % | ε_{⊥} (1 kHz, 20°C): | 7.1 |
| CCY-5-O2 | 9.50 % | K₁ (20°C) [pN]: | 14.6 |
| CPY-2-O2 | 12.0 % | K₃ (20°C) [pN]: | 14.5 |
| CY-3-O4 | 9.00 % | γ₁ (20°C) [mPa·s]: | 178 |
| CY-5-O4 | 11.0 % | V₀ (20°C) [V]: | 2.12 |
| CP-5-3 | 13.5 % | | |

**H13:** Nematische Hostmischung (Δε < 0)

| | | | |
|---|---|---|---|
| CPP-3-2 | 4.00 % | Klärpunkt [°C]: | 74.8 |
| CC-3-V1 | 8.00 % | Δn (589 nm, 20°C): | 0.106 |
| CC-2-3 | 13.0 % | Δε (1 kHz, 20°C): | -3.5 |
| CC-3-4 | 7.00 % | ε_{∥} (1 kHz, 20°C): | 3.6 |
| CC-3-5 | 7.00 % | ε_{⊥} (1 kHz, 20°C): | 7.1 |
| CCY-3-O2 | 13.0 % | K₁ (20°C) [pN]: | 14.8 |
| CPY-2-O2 | 7.00 % | K₃ (20°C) [pN]: | 15.8 |
| CPY-3-O2 | 12.0 % | γ₁ (20°C) [mPa·s]: | 115 |
| CY-3-O2 | 12.0 % | V₀ (20°C) [V]: | 2.23 |
| CP-3-O1 | 2.00 % | | |
| PY-3-O2 | 15.0 % | | |

**H14:** Nematische Hostmischung (Δε < 0)

| | | | |
|---|---|---|---|
| CY-3-O4 | 22.0 % | Klärpunkt [°C]: | 86.9 |
| CY-5-O4 | 12.0 % | Δn (589 nm, 20°C): | 0.111 |
| CCY-3-O2 | 6.00 % | Δε (1 kHz, 20°C): | -4.9 |
| CCY-3-O3 | 6.00 % | ε_{∥} (1 kHz, 20°C): | 3.8 |
| CCY-4-O2 | 6.00 % | ε_{⊥} (1 kHz, 20°C): | 8.7 |
| CPY-2-O2 | 10.0 % | K₁ (20°C) [pN]: | 14.9 |
| CPY-3-O2 | 10.0 % | K₃ (20°C) [pN]: | 15.9 |
| PYP-2-3 | 7.00 % | γ₁ (20°C) [mPa·s]: | 222 |
| CC-3-V1 | 7.00 % | V₀ (20°C) [V]: | 1.91 |
| CC-5-V | 10.0 % | | |
| CCEPC-3-3 | 2.00 % | | |
| CCEPC-3-5 | 2.00 % | | |

**H15:** Nematische Hostmischung (Δε < 0)

| | | | |
|---|---|---|---|
| CY-3-O4 | 12.0 % | Klärpunkt [°C]: | 86.0 |
| CY-5-O2 | 10.0 % | Δn (589 nm, 20°C): | 0.110 |
| CY-5-O4 | 8.00 % | Δε (1 kHz, 20°C): | -5.0 |
| CCY-3-O2 | 8.00 % | ε_{∥} (1 kHz, 20°C): | 3.8 |
| CCY-4-O2 | 7.00 % | ε_{⊥} (1 kHz, 20°C): | 8.8 |
| CCY-5-O2 | 6.00 % | K₁ (20°C) [pN]: | 14.7 |
| CCY-2-1 | 8.00 % | K₃ (20°C) [pN]: | 16.0 |
| CCY-3-1 | 7.00 % | γ₁ (20°C) [mPa·s]: | 250 |
| CPY-3-O2 | 9.00 % | V₀ (20°C) [V]: | 1.90 |
| CPY-3-O2 | 9.00 % | | |
| CPP-3-2 | 6.00 % | | |
| CP-5-3 | 10.0 % | | |

**H16:** Nematische Hostmischung (Δε < 0)

| | | | |
|---|---|---|---|
| CC-3-V1 | 10.25 % | Klärpunkt [°C]: | 74.7 |
| CC-2-3 | 18.5 % | Δn (589 nm, 20°C): | 0.103 |
| CC-3-5 | 6.75 % | Δε (1 kHz, 20°C): | -3.1 |
| CCP-3-1 | 6.00 % | ε_{∥} (1 kHz, 20°C): | 3.4 |
| CCY-3-1 | 2.50 % | ε_{⊥} (1 kHz, 20°C): | 6.4 |
| CCY-3-O2 | 12.0 % | K₁ (20°C) [pN]: | 15.4 |
| CPY-2-O2 | 6.00 % | K₃ (20°C) [pN]: | 16.8 |
| CPY-3-O2 | 9.75 % | γ₁ (20°C) [mPa·s]: | 104 |
| CY-3-O2 | 11.5 % | V₀ (20°C) [V]: | 2.46 |
| PP-1-2V1 | 3.75 % | | |
| PY-3-O2 | 13.0 % | | |

**H17:** Nematische Hostmischung (Δε < 0)

| | | | |
|---|---|---|---|
| CC-3-V | 27.5 % | Klärpunkt [°C]: | 74.7 |
| CC-3-V1 | 10.0 % | Δn (589 nm, 20°C): | 0.104 |
| CC-3-5 | 8.00 % | Δε (1 kHz, 20°C): | -3.0 |
| CCY-3-O2 | 9.25 % | ε_{∥} (1 kHz, 20°C): | 3.4 |
| CLY-3-O2 | 10.0 % | ε_{⊥} (1 kHz, 20°C): | 6.4 |
| CPY-3-O2 | 11.75 % | K₁ (20°C) [pN]: | 15.3 |
| PY-3-O2 | 14.0 % | K₃ (20°C) [pN]: | 16.2 |
| PY-4-O2 | 9.00 % | γ₁ (20°C) [mPa·s]: | 88 |
| PYP-2-4 | 0.50 % | V₀ (20°C) [V]: | 2.44 |

**H18:** Nematische Hostmischung (Δε < 0)

| | | | |
|---|---|---|---|
| CPP-3-2 | 6.50 % | Klärpunkt [°C]: | 74.7 |
| CC-3-V1 | 8.00 % | Δn (589 nm, 20°C): | 0.104 |
| CC-2-3 | 17.0 % | Δε (1 kHz, 20°C): | -3.0 |
| CC-3-4 | 6.50 % | ε_{∥} (1 kHz, 20°C): | 3.4 |
| CCY-3-O1 | 3.50 % | ε_{⊥} (1 kHz, 20°C): | 6.3 |
| CCY-3-O2 | 12.5 % | K₁ (20°C) [pN]: | 14.8 |
| CPY-2-O2 | 5.50 % | K₃ (20°C) [pN]: | 15.8 |
| CPY-3-O2 | 10.0 % | γ₁ (20°C) [mPa·s]: | 106 |
| CY-3-O2 | 15.5 % | | |
| CP-3-O1 | 4.50 % | | |
| PP-1-2V1 | 5.00 % | | |
| PY-3-O2 | 5.50 % | | |

**H19:** Nematische Hostmischung (Δε < 0)

| | | | | |
|---|---|---|---|---|
| CPP-3-2 | 10.5 | % | Klärpunkt [°C]: | 74.5 |
| CC-3-4 | 9.0 | % | Δn (589 nm, 20°C): | 0.104 |
| CC-3-5 | 9.0 | % | Δε (1 kHz, 20°C): | -3.4 |
| CCP-3-1 | 8.0 | % | ε_{∥} (1 kHz, 20°C): | 3.7 |
| CCY-3-O2 | 9.5 | % | ε_{⊥} (1 kHz, 20°C): | 7 |
| CCY-4-O2 | 5.5 | % | K₁ (20°C) [pN]: | 14 |
| CPY-3-O2 | 5.5 | % | K₃ (20°C) [pN]: | 15.7 |
| CY-3-O2 | 15 | % | γ₁ (20°C) [mPa·s]: | 128 |
| CY-5-O2 | 5.0 | % | | |
| CP-3-O1 | 7.0 | % | | |
| PY-3-O2 | 16 | % | | |

**H20:** Nematische Hostmischung (Δε > 0)

| | | | |
|---|---|---|---|
| CC-4-V | 10.0 % | Klärpunkt [°C]: | 77.0 |
| CC-5-V | 13.5 % | Δn (589 nm, 20°C): | 0.113 |
| PGU-3-F | 6.50 % | Δε (1 kHz, 20°C): | 19.2 |
| ACQU-2-F | 10.0 % | ε_{∥} (1 kHz, 20°C): | 23.8 |
| ACQU-3-F | 12.0 % | ε_{⊥} (1 kHz, 20°C): | 4.6 |
| PUQU-3-F | 11.0 % | K₁ (20°C) [pN]: | 11.5 |
| CCP-V-1 | 12.0 % | K₃ (20°C) [pN]: | 11.1 |
| APUQU-2-F | 6.00 % | γ₁ (20°C) [mPa·s]: | 122 |
| APUQU-3-F | 7.00 % | V₀ (20°C) [V]: | 0.81 |
| PGUQU-3-F | 8.00 % | | |
| CPGU-3-OT | 4.00 % | | |

**H21:** Nematische Hostmischung (Δε > 0)

| | | | |
|---|---|---|---|
| PGU-2-F | 3.50 % | Klärpunkt [°C]: | 77.0 |
| PGU-3-F | 7.00 % | Δn (589 nm, 20°C): | 0.105 |
| CC-3-V1 | 15.0 % | Δε (1 kHz, 20°C): | 7.2 |
| CC-4-V | 18.0 % | ε_{∥} (1 kHz, 20°C): | 10.3 |
| CC-5-V | 20.0 % | ε_{⊥} (1 kHz, 20°C): | 3.1 |
| CCP-V-1 | 6.00 % | K₁ (20°C) [pN]: | 15.3 |
| APUQU-3-F | 15.0 % | K₃ (20°C) [pN]: | 13.5 |
| PUQU-3-F | 5.50 % | γ₁ (20°C) [mPa·s]: | 63 |
| PGP-2-4 | 3.00 % | V₀ (20°C) [V]: | 1.53 |
| CPP-3-2 | 7.00 % | | |

**H22:** Nematische Hostmischung (Δε > 0)

| | | | |
|---|---|---|---|
| APUQU-2-F | 6.00 % | Klärpunkt [°C]: | 74.0 |
| APUQU-3-F | 12.0 % | Δn (589 nm, 20°C): | 0.120 |
| PUQU-3-F | 18.0 % | Δε (1 kHz, 20°C): | 17.4 |
| CPGU-3-OT | 9.00 % | ε_{∥} (1 kHz, 20°C): | 22.0 |
| CCGU-3-F | 3.00 % | ε_{⊥} (1 kHz, 20°C): | 4.5 |
| CPU-3-F | 14.0 % | K₁ (20°C) [pN]: | 10.1 |
| CCQU-3-F | 10.0 % | K₃ (20°C) [pN]: | 10.8 |
| CC-3-V | 25.0 % | γ₁ (20°C) [mPa·s]: | 111 |
| PGP-2-2V | 3.00 % | V₀ (20°C) [V]: | 0.80 |

**H23:** Nematische Hostmischung (Δε > 0)

| | | | |
|---|---|---|---|
| PUQU-3-F | 15.0 % | Klärpunkt [°C]: | 74.3 |
| APUQU-2-F | 5.00 % | Δn (589 nm, 20°C): | 0.120 |
| APUQU-3-F | 12.0 % | Δε (1 kHz, 20°C): | 14.9 |
| CCQU-3-F | 11.0 % | ε_{∥} (1 kHz, 20°C): | 19.1 |
| CCQU-5-F | 1.50 % | ε_{⊥} (1 kHz, 20°C): | 4.3 |
| CPGU-3-OT | 5.00 % | K₁ (20°C) [pN]: | 11.2 |
| CPP-3-OT | 4.50 % | K₃ (20°C) [pN]: | 10.8 |
| CGU-3-F | 10.0 % | γ₁ (20°C) [mPa·s]: | 98 |
| PGP-2-3 | 1.50 % | V₀ (20°C) [V]: | 0.91 |
| PGP-2-2V | 8.00 % | | |
| CC-3-V | 26.5 % | | |

**H24:** Nematische Hostmischung (Δε > 0)

| | | | |
|---|---|---|---|
| CCQU-3-F | 9.00 % | Klärpunkt [°C]: | 94.5 |
| CCQU-5-F | 9.00 % | Δn (589 nm, 20°C): | 0.121 |
| PUQU-3-F | 16.0 % | Δε (1 kHz, 20°C): | 20.4 |
| APUQU-2-F | 8.00 % | ε_{∥} (1 kHz, 20°C): | 24.7 |
| APUQU-3-F | 9.00 % | ε_{⊥} (1 kHz, 20°C): | 4.3 |
| PGUQU-3-F | 8.00 % | K₁ (20°C) [pN]: | 12.1 |
| CPGU-3-OT | 7.00 % | K₃ (20°C) [pN]: | 13.9 |
| CC-4-V | 18.0 % | γ₁ (20°C) [mPa·s]: | 163 |
| CC-5-V | 5.00 % | V₀ (20°C) [V]: | 0.81 |
| CCP-V-1 | 6.00 % | | |
| CCEPC-3-3 | 3.00 % | | |
| PPGU-3-F | 2.00 % | | |

**H25:** Nematische Hostmischung (Δε > 0)

| | | | |
|---|---|---|---|
| CC-3-V | 28.50 % | Klärpunkt [°C]: | 85.6 |
| CCP-V-1 | 3.00 % | Δn (589 nm, 20°C): | 0.121 |
| CCEPC-3-3 | 2.00 % | Δε (1 kHz, 20°C): | 19.5 |
| PGU-2-F | 4.00 % | ε_{∥} (1 kHz, 20°C): | 23.8 |
| CCQU-3-F | 8.00 % | ε_{⊥} (1 kHz, 20°C): | 4.3 |
| CCQU-5-F | 6.00 % | K₁ (20°C) [pN]: | 11.6 |
| CCGU-3-F | 3.00 % | K₃ (20°C) [pN]: | 12.7 |
| PUQU-2-F | 2.00 % | γ₁ (20°C) [mPa·s]: | 126 |
| PUQU-3-F | 10.0 % | V₀ (20°C) [V]: | 0.81 |
| APUQU-2-F | 6.00 % | | |
| APUQU-3-F | 9.00 % | | |
| PGUQU-3-F | 5.00 % | | |
| PGUQU-4-F | 5.00 % | | |
| PGUQU-5-F | 4.00 % | | |
| CPGU-3-OT | 4.00 % | | |
| PPGU-3-F | 0.50 % | | |

**H26:** Nematische Hostmischung (Δε < 0)

| | | | |
|---|---|---|---|
| CC-3-V1 | 9.00 % | Klärpunkt [°C]: | 74.6 |
| CC-3-O1 | 3.50 % | Δn (589 nm, 20°C): | 0.0984 |
| CC-3-4 | 8.00 % | Δε (1 kHz, 20°C): | -3.6 |
| CC-3-5 | 8.00 % | ε_{∥} (1 kHz, 20°C): | 3.6 |
| CCP-3-1 | 6.00 % | ε_{⊥} (1 kHz, 20°C): | 7.1 |
| CCY-3-O1 | 6.50 % | K₁ (20°C) [pN]: | 14.1 |
| CCY-3-O2 | 12.5 % | K₃ (20°C) [pN]: | 17 |
| CPY-3-O2 | 10.0 % | γ₁ (20°C) [mPa·s]: | 119 |
| CY-3-O2 | 15.5 % | V₀ (20°C) [V]: | 2.31 |
| CP-3-O1 | 8.5 % | | |
| PY-3-O2 | 12.5 % | | |

**H27:** Nematische Hostmischung (Δε < 0)

| | | | |
|---|---|---|---|
| CC-3-5 | 9.50 % | Klärpunkt [°C]: | 79.1 |
| CC-5-O1 | 5.00 % | Δn (589 nm, 20°C): | 0.0911 |
| CCY-2-1 | 9.50 % | Δε (1 kHz, 20°C): | -3.6 |
| CCY-3-1 | 10.5 % | ε_{∥} (1 kHz, 20°C): | 3.5 |
| CCY-3-O2 | 10.5 % | ε_{⊥} (1 kHz, 20°C): | 7.1 |
| CCY-5-O2 | 9.50 % | K₁ (20°C) [pN]: | 14.6 |
| CPY-2-O2 | 12.0 % | K₃ (20°C) [pN]: | 14.5 |
| CY-3-O4 | 9.00 % | γ₁ (20°C) [mPa·s]: | 178 |
| CY-5-O4 | 11.0 % | V₀ (20°C) [V]: | 2.12 |
| CP-5-3 | 13.5 % | | |

**H28:** Nematische Hostmischung (Δε < 0)

| | | | |
|---|---|---|---|
| CC-3-V | 37.5 % | Klärpunkt [°C]: | 74.8 |
| CC-3-V1 | 2.00 % | Δn (589 nm, 20°C): | 0.0987 |
| CCY-4-O2 | 14.5 % | Δε (1 kHz, 20°C): | -2.9 |
| CPY-2-O2 | 10.5 % | ε_{∥} (1 kHz, 20°C): | 3.7 |
| CPY-3-O2 | 9.5 % | ε_{⊥} (1 kHz, 20°C): | 6.6 |
| CY-3-O2 | 15.0 % | K₁ (20°C) [pN]: | 12.2 |
| CY-3-O4 | 4.50 % | K₃ (20°C) [pN]: | 13.4 |
| PYP-2-4 | 5.50 % | γ₁ (20°C) [mPa·s]: | 92 |
| PPGU-3-F | 1.00 % | V₀ (20°C) [V]: | 2.28 |

**H29:** Nematische Hostmischung (Δε < 0)

| | | | |
|---|---|---|---|
| CC-3-V | 37.5 % | Klärpunkt [°C]: | 75.4 |
| CC-5-O1 | 2.00 % | Δn (589 nm, 20°C): | 0.1034 |
| CCY-3-O2 | 12.0 % | Δε (1 kHz, 20°C): | -3.3 |
| CCY-3-O3 | 6.50 % | ε_{∥} (1 kHz, 20°C): | 3.6 |
| CPY-2-O2 | 12.0 % | ε_{⊥} (1 kHz, 20°C): | 6.9 |
| CPY-3-O2 | 10.0 % | K₁ (20°C) [pN]: | 13.4 |
| CY-3-O2 | 2.00 % | K₃ (20°C) [pN]: | 15 |
| PY-3-O2 | 16.0 % | γ₁ (20°C) [mPa·s]: | 95 |
| CP-3-O1 | 2.00 % | V₀ (20°C) [V]: | 2.24 |

**H30:** Nematische Hostmischung (Δε < 0)

| | | | |
|---|---|---|---|
| CC-3-V | 22.5 % | Klärpunkt [°C]: | 74.8 |
| CC-3-V1 | 9.75 % | Δn (589 nm, 20°C): | 0.1027 |
| CC-1-3 | 0.75 % | Δε (1 kHz, 20°C): | -3.2 |
| CC-3-4 | 5.5 % | ε_{∥} (1 kHz, 20°C): | 3.5 |
| CC-3-5 | 4.00 % | ε_{⊥} (1 kHz, 20°C): | 6.8 |
| CCY-3-O1 | 10 % | K₁ (20°C) [pN]: | 14.4 |
| CCY-3-O2 | 12 % | K₃ (20°C) [pN]: | 15.2 |
| CPY-2-O2 | 10 % | γ₁ (20°C) [mPa·s]: | |
| CPY-3-O2 | 2.0 % | V₀ (20°C) [V]: | 2.29 |
| CY-3-O2 | 0.5 % | | |
| PP-1-2V1 | 0.25 % | | |
| PY-1-O4 | 4.25 % | | |
| PY-3-O2 | 17 % | | |
| PYP-2-3 | 1.5 % | | |

**H31:** Nematische Hostmischung (Δε < 0)

| | | | |
|---|---|---|---|
| CPP-3-2 | 4.0 % | Klärpunkt [°C]: | 74.6 |
| CC-3-V | 10 % | Δn (589 nm, 20°C): | 0.099 |
| CC-3-V1 | 8.5 % | Δε (1 kHz, 20°C): | -3.4 |
| CC-3-4 | 4.5 % | ε_{∥} (1 kHz, 20°C): | 3.6 |
| CC-3-5 | 8.0 % | ε_{⊥} (1 kHz, 20°C): | 7 |
| CCP-3-1 | 4.25 % | K₁ (20°C) [pN]: | 14.2 |
| CCY-3-O1 | 6.5 % | K₃ (20°C) [pN]: | 15.9 |
| CCY-3-O2 | 12.75 % | γ₁ (20°C) [mPa·s]: | 108 |
| CCY-4-O2 | 6.0 % | V₀ (20°C) [V]: | 2.28 |
| CY-3-O2 | 15.5 % | | |
| CP-3-O1 | 2.0 % | | |
| PY-3-O2 | 16 % | | |
| PYP-2-3 | 2.0 % | | |

**H32:** Nematische Hostmischung (Δε < 0)

| | | | |
|---|---|---|---|
| CC-3-V | 15 % | Klärpunkt [°C]: | 74.4 |
| CC-3-V1 | 9.0 % | Δn (589 nm, 20°C): | 0.1086 |
| CC-2-3 | 8.0 % | Δε (1 kHz, 20°C): | -3.2 |
| CC-3-4 | 7.5 % | ε_{∥} (1 kHz, 20°C): | 3.5 |
| CCY-3-O2 | 10 % | ε_{⊥} (1 kHz, 20°C): | 6.7 |
| CCY-5-O2 | 8.0 % | K₁ (20°C) [pN]: | 14.3 |
| CPY-2-O2 | 3.0 % | K₃ (20°C) [pN]: | 15.7 |
| CPY-3-O2 | 8.5 % | γ₁ (20°C) [mPa·s]: | 102 |
| CY-3-O2 | 7.0 % | V₀ (20°C) [V]: | 2.33 |
| PY-3-O2 | 16 % | | |
| PYP-2-3 | 8.0 % | | |

**H33:** Nematische Hostmischung (Δε < 0)

| | | | |
|---|---|---|---|
| CPP-3-2 | 6.0 % | Klärpunkt [°C]: | 75.2 |
| CC-3-O1 | 4.0 % | Δn (589 nm, 20°C): | 0.1095 |
| CC-3-4 | 9.0 % | Δε (1 kHz, 20°C): | -3.1 |
| CC-3-5 | 9.0 % | ε_{∥} (1 kHz, 20°C): | 3.6 |
| CCP-3-1 | 8.0 % | ε_{⊥} (1 kHz, 20°C): | 6.7 |
| CCP-3-3 | 1.0 % | K₁ (20°C) [pN]: | 13.8 |
| CCY-3-O2 | 12 % | K₃ (20°C) [pN]: | 16.5 |
| CLY-3-O2 | 1.0 % | γ₁ (20°C) [mPa·s]: | 119 |
| CPY-3-O2 | 11 % | V₀ (20°C) [V]: | 2.41 |
| CY-3-O2 | 9.5 % | | |
| CP-3-O1 | 11.5 % | | |
| PY-3-O2 | 18 % | | |

**H34:** Nematische Hostmischung (Δε < 0)

| | | | |
|---|---|---|---|
| CPP-3-2 | 3.0 % | Klärpunkt [°C]: | 75.2 |
| CC-3-V1 | 9.0 % | Δn (589 nm, 20°C): | 0.1098 |
| CC-3-O1 | 2.5 % | Δε (1 kHz, 20°C): | -3.1 |
| CC-3-4 | 9.0 % | ε_{∥} (1 kHz, 20°C): | 3.6 |
| CC-3-5 | 9.0 % | ε_{⊥} (1 kHz, 20°C): | 6.7 |
| CCP-3-1 | 7.5 % | K₁ (20°C) [pN]: | 14.6 |
| CCP-V2-1 | 5.0 % | K₃ (20°C) [pN]: | 16.6 |
| CCY-3-O2 | 4.0 % | γ₁ (20°C) [mPa·s]: | 114 |
| CPY-2-O2 | 5.5 % | V₀ (20°C) [V]: | 2.43 |
| CPY-3-O2 | 10.5 % | | |
| CY-3-O2 | 15 % | | |
| CP-3-O1 | 1.5 % | | |
| PY-3-O2 | 18 % | | |
| PPGU-3-F | 0.5 % | | |

**H35:** Nematische Hostmischung (Δε < 0)

| | | | |
|---|---|---|---|
| CPP-3-2 | 8.5 % | Klärpunkt [°C]: | 74.7 |
| CC-3-V1 | 9.0 % | Δn (589 nm, 20°C): | 0.1097 |
| CC-3-O1 | 2.0 % | Δε (1 kHz, 20°C): | -3.1 |
| CC-3-4 | 9.0 % | ε_{∥} (1 kHz, 20°C): | 3.5 |
| CC-3-5 | 9.0 % | ε_{⊥} (1 kHz, 20°C): | 6.6 |
| CCP-3-1 | 2.5 % | K₁ (20°C) [pN]: | 14.2 |
| CCP-V2-1 | 5.0 % | K₃ (20°C) [pN]: | 16.6 |
| CCY-3-O2 | 7.5 % | γ₁ (20°C) [mPa·s]: | 112 |
| CLY-3-O2 | 1.0 % | V₀ (20°C) [V]: | 2.44 |
| CPY-3-O2 | 10.5 % | | |
| CY-3-O2 | 15 % | | |
| CP-3-O1 | 3.0 % | | |
| PY-3-O2 | 18 % | | |

**H36:** Nematische Hostmischung (Δε < 0)

| | | | |
|---|---|---|---|
| B-2O-O5 | 4.0 % | Klärpunkt [°C]: | 75 |
| CPP-3-2 | 2.0 % | Δn (589 nm, 20°C): | 0.1094 |
| CC-3-O1 | 5.0 % | Δε (1 kHz, 20°C): | -3.1 |
| CC-3-4 | 9.0 % | ε_{∥} (1 kHz, 20°C): | 3.6 |
| CC-3-5 | 9.0 % | ε_{⊥} (1 kHz, 20°C): | 6.7 |
| CCP-3-1 | 8.0 % | K₁ (20°C) [pN]: | 13.9 |
| CCP-3-3 | 5.0 % | K₃ (20°C) [pN]: | 16.4 |
| CCY-3-O2 | 11.5 % | γ₁ (20°C) [mPa·s]: | 117 |
| CLY-3-O2 | 1.0 % | V₀ (20°C) [V]: | 2.42 |
| CPY-3-O2 | 10.5 % | | |
| CY-3-O2 | 2.0 % | | |
| CP-3-O1 | 15 % | | |
| PY-3-O2 | 18 % | | |

**H37:** Nematische Hostmischung (Δε < 0)

| | | | |
|---|---|---|---|
| CPP-3-2 | 7.5 % | Klärpunkt [°C]: | 74.8 |
| CC-3-V1 | 9.0 % | Δn (589 nm, 20°C): | 0.1098 |
| CC-3-O1 | 1.5 % | Δε (1 kHz, 20°C): | -3.1 |
| CC-3-4 | 9.0 % | ε_{∥} (1 kHz, 20°C): | 3.5 |
| CC-3-5 | 9.0 % | ε_{⊥} (1 kHz, 20°C): | 6.6 |
| CCP-3-1 | 4.0 % | K₁ (20°C) [pN]: | 14.4 |
| CCP-V2-1 | 5.0 % | K₃ (20°C) [pN]: | 16.6 |
| CCY-3-O2 | 7.0 % | γ₁ (20°C) [mPa·s]: | 112 |
| CPY-2-O2 | 2.0 % | V₀ (20°C) [V]: | 2.44 |
| CPY-3-O2 | 10 % | | |
| CY-3-O2 | 15 % | | |
| CP-3-O1 | 3.0 % | | |
| PY-3-O2 | 18 % | | |

**H38:** Nematische Hostmischung (Δε < 0)

| | | | |
|---|---|---|---|
| CY-3-O2 | 10 % | Klärpunkt [°C]: | 100 |
| CY-3-O4 | 20 % | Δn (589 nm, 20°C): | 0.0865 |
| CY-5-O4 | 20 % | Δε (1 kHz, 20°C): | -5.4 |
| CCY-3-O2 | 6.0 % | ε_{∥} (1 kHz, 20°C): | 3.9 |
| CCY-3-O3 | 6.0 % | ε_{⊥} (1 kHz, 20°C): | 9.3 |
| CCY-4-O2 | 6.0 % | K₁ (20°C) [pN]: | 15.6 |
| CCY-5-O2 | 6.0 % | K₃ (20°C) [pN]: | 16.6 |
| CCZC-3-3 | 3.0 % | γ₁ (20°C) [mPa·s]: | 347 |
| CCZC-3-5 | 3.5 % | V₀ (20°C) [V]: | 1.84 |
| CCZC-4-3 | 3.5 % | | |
| CCZC-4-5 | 3.5 % | | |
| CCEPC-3-3 | 4.0 % | | |
| CCEPC-3-4 | 4.5 % | | |
| CCEPC-3-5 | 4.0 % | | |

**H39:** Nematische Hostmischung (Δε < 0)

| | | | |
|---|---|---|---|
| Y-4O-O4 | 12.5 % | Klärpunkt [°C]: | 105 |
| CY-3-O4 | 5.0 % | Δn (589 nm, 20°C): | 0.0868 |
| CY-5-O4 | 18 % | Δε (1 kHz, 20°C): | -5.4 |
| CCY-3-O1 | 4.0 % | ε_{∥} (1 kHz, 20°C): | 4.2 |
| CCY-3-O2 | 6.0 % | ε_{⊥} (1 kHz, 20°C): | 9.6 |
| CCY-3-O3 | 6.0 % | K₁ (20°C) [pN]: | 16.7 |
| CCY-4-O2 | 6.0 % | K₃ (20°C) [pN]: | 16.5 |
| CCY-5-O2 | 6.0 % | γ₁ (20°C) [mPa·s]: | |
| CPY-3-O2 | 4.5 % | V₀ (20°C) [V]: | 1.85 |
| CCZC-3-3 | 4.0 % | | |
| CCZC-3-5 | 4.0 % | | |
| CCZC-4-3 | 4.0 % | | |
| CCZC-4-5 | 4.0 % | | |
| CCOC-3-3 | 2.0 % | | |
| CCOC-4-3 | 2.0 % | | |
| CCEPC-3-3 | 4.0 % | | |
| CCEPC-3-4 | 4.0 % | | |
| CCEPC-3-5 | 4.0 % | | |

**H40:** Nematische Hostmischung (Δε < 0)

| | | | |
|---|---|---|---|
| Y-4O-O4 | 3.0 % | Klärpunkt [°C]: | 108 |
| CY-3-O4 | 8.0. % | Δn (589 nm, 20°C): | 0.1096 |
| CCY-3-O1 | 4.0 % | Δε (1 kHz, 20°C): | -2.4 |
| CCY-3-O2 | 6.0 % | ε_{∥} (1 kHz, 20°C): | 3.2 |
| CCY-3-O3 | 6.0 % | ε_{⊥} (1 kHz, 20°C): | 5.6 |
| CPY-2-O2 | 8.0 % | K₁ (20°C) [pN]: | 16.3 |
| CPY-3-O2 | 8.0 % | K₃ (20°C) [pN]: | 18.9 |
| CP-3-O1 | 5.5 % | γ₁ (20°C) [mPa·s]: | |
| CC-4-V | 15 % | V₀ (20°C) [V]: | 2.99 |
| CC-3-V1 | 5.5 % | | |
| CCP-V-1 | 13 % | | |
| CCP-V2-1 | 13 % | | |
| CPTP-3-O1 | 5.0 % | | |

**H41:** Nematische Hostmischung (Δε < 0)

| | | | |
|---|---|---|---|
| CY-3-O4 | 16 % | Klärpunkt [°C]: | 109 |
| CCY-3-O1 | 4.0 % | Δn (589 nm, 20°C): | 0.0854 |
| CCY-3-O2 | 6.0 % | Δε (1 kHz, 20°C): | -2.3 |
| CCY-3-O3 | 6.0 % | ε_{∥} (1 kHz, 20°C): | 3.1 |
| CCY-4-O2 | 6.0 % | ε_{⊥} (1 kHz, 20°C): | 5.4 |
| CCY-5-O2 | 5.0 % | K₁ (20°C) [pN]: | 16.3 |
| CC-3-O1 | 6.0 % | K₃ (20°C) [pN]: | 19.4 |
| CC-4-V | 15 % | γ₁ (20°C) [mPa·s]: | |
| CC-3-V1 | 6.0 % | V₀ (20°C) [V]: | 3.08 |
| CCP-V-1 | 13 % | | |
| CCP-V2-1 | 13 % | | |
| CCEPC-3-3 | 4.0 % | | |

**H42:** Nematische Hostmischung (Δε < 0)

| | | | |
|---|---|---|---|
| Y-4O-O4 | 10 % | Klärpunkt [°C]: | 107 |
| CY-3-O2 | 7.0 % | Δn (589 nm, 20°C): | 0.1104 |
| CY-3-O4 | 15 % | Δε (1 kHz, 20°C): | -6 |
| CCY-3-O1 | 4.0 % | ε_{∥} (1 kHz, 20°C): | 4.3 |
| CCY-3-O2 | 6.0 % | ε_{⊥} (1 kHz, 20°C): | 10.3 |
| CCY-3-O3 | 6.0 % | K₁ (20°C) [pN]: | 15.7 |
| CCY-4-O2 | 6.0 % | K₃ (20°C) [pN]: | 19.1 |
| CCY-5-O2 | 6.0 % | γ₁ (20°C) [mPa·s]: | |
| CPY-2-O2 | 9.0 % | V₀ (20°C) [V]: | 1.88 |
| CPY-3-O2 | 9.0 % | | |
| CCP-V-1 | 8.5 % | | |
| CCEPC-3-3 | 4.0 % | | |
| CCEPC-3-4 | 4.0 % | | |
| CCEPC-3-5 | 3.5 % | | |
| CGPC-3-3 | 2.0 % | | |

**H43:** Nematische Hostmischung (Δε < 0)

| | | | |
|---|---|---|---|
| Y-4O-O4 | 10 % | Klärpunkt [°C]: | 108 |
| CY-3-O2 | 4.0 % | Δn (589 nm, 20°C): | 0.1403 |
| CY-3-O4 | 15 % | Δε (1 kHz, 20°C): | -6.4 |
| CCY-3-O1 | 4.0 % | ε_{∥} (1 kHz, 20°C): | 4.3 |
| CCY-3-O2 | 6.0 % | ε_{⊥} (1 kHz, 20°C): | 10.7 |
| CCY-3-O3 | 6.0 % | K₁ (20°C) [pN]: | 16.8 |
| CCY-4-O2 | 6.0 % | K₃ (20°C) [pN]: | 20.5 |
| CLY-3-O2 | 5.0 % | γ₁ (20°C) [mPa·s]: | |
| CPY-2-O2 | 5.0 % | V₀ (20°C) [V]: | 1.89 |
| CPY-3-O2 | 5.0 % | | |
| PTY-3-O2 | 10 % | | |
| PTY-5-O2 | 10 % | | |
| CCP-V-1 | 7.0 % | | |
| CCP-V2-1 | 7.0 % | | |

**H44:** Nematische Hostmischung (Δε < 0)

| | | | |
|---|---|---|---|
| Y-4O-O4 | 10 % | Klärpunkt [°C]: | 109 |
| CCY-3-O1 | 5.0 % | Δn (589 nm, 20°C): | 0.1405 |
| PTY-3-O2 | 3.0 % | Δε (1 kHz, 20°C): | -2 |
| PTY-3-O2 | 10 % | ε_{∥} (1 kHz, 20°C): | 3.4 |
| PTY-5-O2 | 10 % | ε_{⊥} (1 kHz, 20°C): | 5.4 |
| CP-3-O1 | 4.0 % | K₁ (20°C) [pN]: | 16.5 |
| CC-4-V | 15 % | K₃ (20°C) [pN]: | 19.9 |
| CC-3-V1 | 8.0 % | γ₁ (20°C) [mPa·s]: | |
| CCP-V-1 | 13 % | V₀ (20°C) [V]: | 3.34 |
| CCP-V2-1 | 13 % | | |
| CPTP-3-1 | 4.5 % | | |
| CPTP-3-2 | 4.5 % | | |

**H45:** Nematische Hostmischung (Δε < 0)

| | | | |
|---|---|---|---|
| CY-3-O4 | 13 % | Klärpunkt [°C]: | 107 |
| CCY-3-O1 | 4.0 % | Δn (589 nm, 20°C): | 0.082 |
| CCY-3-O2 | 5.0 % | Δε (1 kHz, 20°C): | -2 |
| CCY-3-O3 | 5.0 % | ε_{∥} (1 kHz, 20°C): | 3 |
| CCY-4-O2 | 5.0 % | ε_{⊥} (1 kHz, 20°C): | 5 |
| CCY-5-O2 | 5.0 % | K₁ (20°C) [pN]: | 16.3 |
| CC-3-O1 | 13 % | K₃ (20°C) [pN]: | 19.2 |
| CC-4-V | 12 % | γ₁ (20°C) [mPa·s]: | |
| CC-3-V1 | 6.0 % | V₀ (20°C) [V]: | 3.29 |
| CCP-V-1 | 13 % | | |
| CCP-V2-1 | 13 % | | |
| CCZC-3-3 | 3.0 % | | |
| CCEPC-3-3 | 3.0 % | | |

**H46:** Nematische Hostmischung (Δε < 0)

| | | | |
|---|---|---|---|
| Y-4O-O4 | 5.0 % | Klärpunkt [°C]: | 107 |
| CY-3-O4 | 15 % | Δn (589 nm, 20°C): | 0.0821 |
| CY-5-O4 | 14.5 % | Δε (1 kHz, 20°C): | -4.5 |
| CCY-3-O1 | 5.0 % | ε_{∥} (1 kHz, 20°C): | 3.7 |
| CCY-3-O2 | 6.0 % | ε_{⊥} (1 kHz, 20°C): | 8.2 |
| CCY-3-O3 | 6.0 % | K₁ (20°C) [pN]: | 16 |
| CCY-4-O2 | 6.0 % | K₃ (20°C) [pN]: | 17 |
| CCY-5-O2 | 6.0 % | γ₁ (20°C) [mPa·s]: | |
| CC-4-V | 8.5 % | V₀ (20°C) [V]: | 2.04 |
| CCZC-3-3 | 3.0 % | | |
| CCZC-3-5 | 3.0 % | | |
| CCZC-4-3 | 3.0 % | | |
| CCZC-4-5 | 3.0 % | | |
| CCOC-3-3 | 4.0 % | | |
| CCEPC-3-3 | 4.0 % | | |
| CCEPC-3-4 | 4.0 % | | |
| CCEPC-3-5 | 4.0 % | | |

**H47:** Nematische Hostmischung (Δε < 0)

| | | | |
|---|---|---|---|
| B-2O-O5 | 4.0 % | Klärpunkt [°C]: | 75 |
| CPP-3-2 | 4.5 % | Δn (589 nm, 20°C): | 0.1095 |
| CC-3-V1 | 9.0 % | Δε (1 kHz, 20°C): | -3.1 |
| CC-3-O1 | 3.0 % | ε_{∥} (1 kHz, 20°C): | 3.6 |
| CC-3-4 | 9.0 % | ε_{⊥} (1 kHz, 20°C): | 6.7 |
| CC-3-5 | 9.0 % | K₁ (20°C) [pN]: | 14.5 |
| CCP-3-1 | 8.0 % | K₃ (20°C) [pN]: | 16.7 |
| CCP-V2-1 | 5.0 % | γ₁ (20°C) [mPa·s]: | 109 |
| CCY-3-O2 | 6.0 % | V₀ (20°C) [V]: | 2.43 |
| CPY-3-O2 | 10.5 % | | |
| CY-3-O2 | 9.5 % | | |
| CP-3-O1 | 4.5 % | | |
| PY-3-O2 | 18 % | | |

**H48:** Nematische Hostmischung (Δε < 0)

| | | | |
|---|---|---|---|
| B-2O-O5 | 4.0 % | Klärpunkt [°C]: | 75.2 |
| CPP-3-2 | 12 % | Δn (589 nm, 20°C): | 0.1101 |
| CC-3-V1 | 9.0 % | Δε (1 kHz, 20°C): | -3.1 |
| CC-3-5 | 5.5 % | ε_{∥} (1 kHz, 20°C): | 3.6 |
| CCP-3-1 | 5.5 % | ε_{⊥} (1 kHz, 20°C): | 6.7 |
| CCP-V2-1 | 5.0 % | K₁ (20°C) [pN]: | 13 |
| CCY-3-O2 | 4.0 % | K₃ (20°C) [pN]: | 16.3 |
| CLY-3-O2 | 1.0 % | γ₁ (20°C) [mPa·s]: | 121 |
| CPY-2-O2 | 2.5 % | V₀ (20°C) [V]: | 2.39 |
| CPY-3-O2 | 10.5 % | | |
| CY-3-O2 | 15 % | | |
| CY-3-O4 | 11 % | | |
| CP-3-O1 | 15 % | | |

Die folgenden (polymerisierbaren) Selbstorientierungsadditive werden verwendet:

| Nr. | Struktur Selbstorientierungsadditiv |
|---|---|
| **1** | 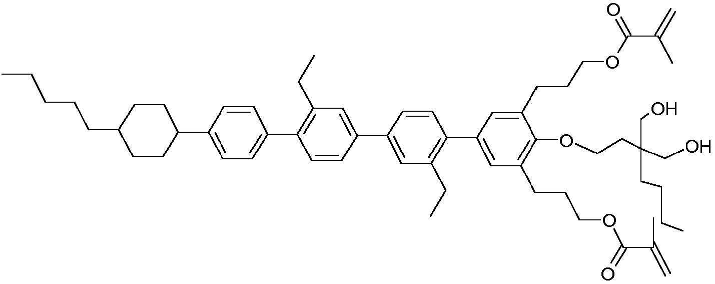 |
| **2** | 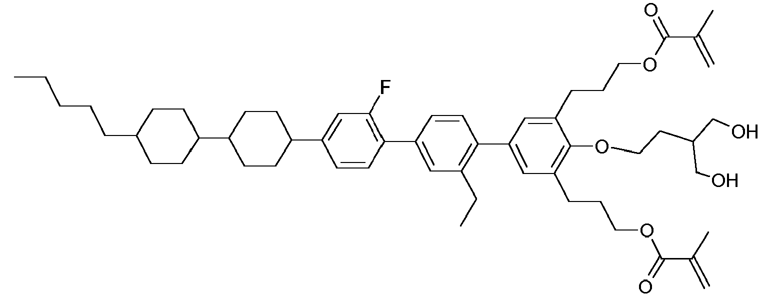 |
| **V1** | 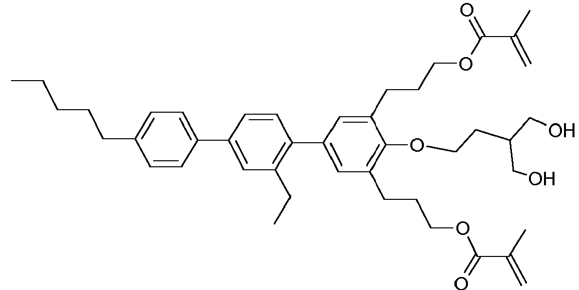 |

Die folgende polymerisierbare Verbindung wird verwendet:

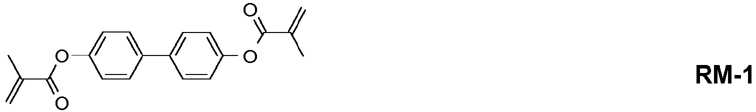

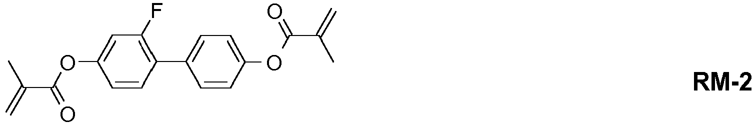

### Ausführungsbeispiele:

Die Selbstorientierungsadditive Nr. **1** und **2** werden allgemein zu 0.02 - 1.5 Gewichts% in einer der Hostmischungen H1 bis H47 gelöst.

### Allgemeine Durchführung für Mischungsbeispiele:

Zu der Hostmischung werden zunächst 0.3 % der polymerisierbaren Verbindung **RM-1** hinzugegeben. Anschließend wird dieser Host-Mischung das erfindungsgemäße Selbstorientierungsadditiv in der angegebenen Menge (in der Regel 0.02 - 2.5 Gewichts%) hinzugefügt.

Die entstandene Mischung wird in eine Testzelle gefüllt (ohne Polyimid-Orientierungsschicht, Schichtdicke d ≈ 4,0 µm, beidseitige ITO-Beschichtung für VHR Messungen). Das FK-Medium weist eine spontane homöotrope (vertikale) Orientierung zu den Substratoberflächen auf.

Unter Anlegen einer Spannung größer als die optische Schwellenspannung (z.B. 14 Vpp) wird die VA-Zelle mit UV-Licht der Intensität 100 mW/cm² bei 20°C bzw. 40°C und 320 nm Bandpassfilter bestrahlt. Dadurch erfolgt Polymerisation der polymerisierbaren Verbindungen. Hierdurch wird ein 'Pretilt' generiert.

Bedingungen für den UV Prozess zur Pre-Tilt Einstellung: Metall-Halid-Lampe, (100 mW/cm³, Cut-off Filter 320 nm, 60 Minuten Bestrahlung, Temperierung der Probe auf 40°C, angelegte Spannung: 20 V (200 Hz AC).

Pre-Tilt Messungen: Die Pretilt Winkel der Zellen werden direkt nach der Einstellung des Pretilts mit einem AXOSCAN (Axometrics, Inc. 103 Quality Circle, Suite 215 Huntsville, AL 35806 U.S.A.) bei einer Wellenlänge von 578 nm gemessen.

Bedingungen zur Bestimmung der Pretiltstabilität: Die Zellen werden für 60 Stunden mit 60 Vpp belastet. Der Pre-Tilt wird vor und nach Belastung gemessen. Eine Änderung des Pretilts nach Belastung ist ein Maß der Stabilität des Pretilts.

UV Prozess für VHR Messungen: Metall Halid Lampe, (100 mW/cm³, mit einem 320 nm Cut-off Filter für 60 Minuten) bei 40°C mit vollflächig ITObeschichteten Testzellen.

VHR Messungen werden mit einem Toyo VHR Gerät durchgeführt: VHR wird eine Stunde nach dem Prozessieren der Testzellen gemessen, mit den folgenden Bedingungen: Angelegte Spannung: 1V, Frequenz: 0.6 Hz, im BiPolar-Modus bei 60°C.

Backlight Test: 7 Tage zwischen zwei Backlight-Modulen mit angelegter Spannung (30 V(pp), 1 MHz, ca. 50°C).

Messungen des "Additive Spreading" (Verteilungsverhalten des Additivs): Testzellen (8 cm x 4 cm) werden mit der Testmischung befüllt. Der untere Teil (nahe der Füllöffnung) weist gute Orientierung auf; der obere Teil (gegenüber der Füllöffnung) weist gegebenenfalls schlechte Orientierung auf, die durch eine höhere Transmission zwischen gekreuzten Polarisatoren charakterisiert wird. Das Verhältnis dieser beiden Bereiche ist ein Maß für die Spreading-Eigenschaften des Additivs.

### Mischungsbeispiel V1 (zum Vergleich)

Zu einem nematischen FK-Medium **H1** des VA-Typs (Δε < 0) werden eine polymerisierbare Verbindung **RM-1** (0,3 Gew.%) und das auf einer Terphenylstruktur basierende polymerisierbare Selbstorientierungsadditiv **V1** (0,3 Gew.%) zugesetzt und homogenisiert.

Charakterisierung der Mischung siehe die folgenden Mischungsbeispiele 1 und 2.

### Mischungsbeispiel M1:

Hinzufügen von 0.7 Gew.% Selbstorientierungsadditiv Nr. **1** zu einem nematischen FK-Medium **H1** des VA-Typs (Δε < 0), das zusätzlich 0,3 % **RM-1** enthält. Das resultierende Medium wird wie angegeben mit UV-Licht unter angelegter Spannung polymerisiert.

Alignment (Optische Beurteilung): Sehr gute vertikale Ausrichtung. Die Zelle ohne Polyimidschicht lässt sich reversibel schalten.

"Additive spreading": 95% der Fläche der Testzelle zeigen gute VA Orientierung (Vergleich mit **V1:** 95%)

### VHR-Messung:

Die VHR-Werte (voltage holding ratio) der Testzellen werden vor und nach dem Polymerisationsvorgang (PS-Stabilisierung), der durch UV-Bestrahlung eingeleitet wird, gemessen (Tabelle 1).

**Tabelle 1: Vergleich VHR-Werte (100°C, 0,6 Hz) in Testzellen mit Hostmischung H1 vor und nach Belastung mit Backlight.**

| **VHR** [%] | **Mischungsbeispiel** | |
|---|---|---|
| | **M1** | **V1** |
| Vor Belastung | 90 | 84 |
| **Nach Belastung** | **96** | **83** |

LTS (-20°C) >1000 h (Vergleich mit **V1**, 0.3 Gew.%: 144 h)
Tiltwinkelgenerierung (20°C): 11° (Vergleich mit **V1:** 3°)
,Pre-Tilt' Stabilität (nach 60 min. Bestrahlung): 0.4° Änderung nach Stress (Vergleich mit **V1:** 0.4°)

Im Vergleich mit **V1** (3 Gew.% eines Selbstorientierungsadditivs mit Terphenylstruktur) ist insbesondere die Tieftemperaturstabilität, VHR und Tiltwinkelgenerierung verbessert.

### Mischungsbeispiel M2:

Hinzufügen von 0.6 Gew.% Selbstorientierungsadditiv Nr. **2** zu einem nematischen FK-Medium **H1** des VA-Typs (Δε < 0), das zusätzlich 0,3 % **RM-1** enthält. Das resultierende Medium wird wie angegeben mit UV-Licht unter angelegter Spannung polymerisiert.

Alignment (Optische Beurteilung): Sehr gute vertikale Ausrichtung. Die Zelle ohne Polyimidschicht lässt sich reversibel schalten.

Additive spreading: 95% der Fläche der Testzelle zeigen gute VA Orientierung (Vergleich mit **V1:** 95%)
LTS (-20°C) >600 h (Vergleich mit **V1,** 0.3%: 144 h)
Tiltwinkelgenerierung (20°C): 10.5° (Vergleich mit **V1:** 3°)
,Pre-Tilt' Stabilität (nach 60 min. Bestrahlung): 0.3° Änderung nach Stress (Vergleich mit **V1:** 0.4°)

### VHR-Messung:

Die VHR-Werte (voltage holding ratio) der Testzellen werden vor und nach dem Polymerisationsvorgang (PS-Stabilisierung), der durch UV-Bestrahlung eingeleitet wird, gemessen (Tabelle 2).

**Tabelle 2: Vergleich VHR-Werte (100°C, 0,6 Hz) in Testzellen mit Hostmischung H1 vor und nach Belastung mit Backlight.**

| **VHR** [%] | **Mischungsbeispiel** | |
|---|---|---|
| | **M2** | **V1** |
| Vor Belastung | 92 | 84 |
| **Nach Belastung** | **83** | **83** |

## Patentansprüche

1. Verbindungen der Formel I-A 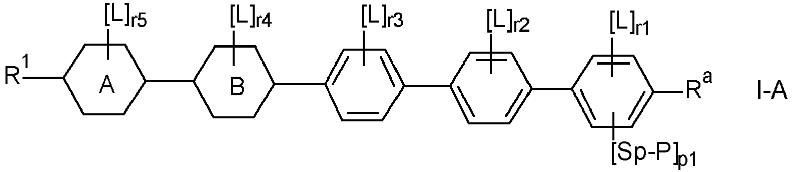 worin
Ringe A, B jeweils unabhängig einen Ring der Formeln der Formel 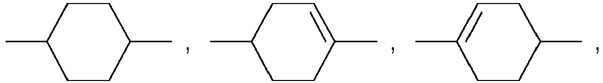 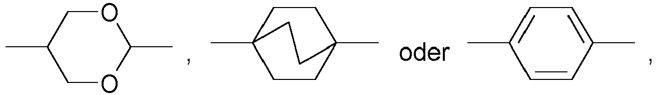
L jeweils unabhängig voneinander unverzweigtes oder verzweigtes Alkyl, Alkenyl, Alkoxy, Alkylcarbonyl, Alkoxycarbonyl, Alkylcarbonyloxy oder Alkoxycarbonyloxy mit 1 bis 25 C-Atomen, worin auch ein oder mehrere H-Atome durch F oder Cl ersetzt sein können, F, Cl, Br, I, -CN, -NO₂, -NCO, -NCS, -OCN, -SCN, -C(=O)N(R⁰)R⁰⁰ oder Cycloalkyl mit 3 bis 6 C-Atomen,
r1 0,
r2 1,
r3 1,
r4 0 oder 1,
r5 0 oder 1,
P eine polymerisierbare Gruppe,
Sp eine Abstandsgruppe oder eine Einfachbindung,
p1 1 oder 2,
R¹ einen Alkylrest mit 1 bis 25 C-Atomen, wobei in diesem Rest auch eine oder mehrere CH₂-Gruppen jeweils unabhängig voneinander durch -CH=CH-, -C≡C-, -CF₂O-, -OCF₂-, 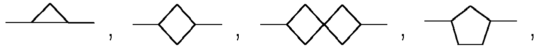 -O-, -S-, -CO-, -CO-O- oder -O-CO- so ersetzt sein können, dass O/S-Atome nicht direkt miteinander verknüpft sind, und worin auch ein oder mehrere H-Atome durch F oder Cl ersetzt sein können, H,
oder eine Gruppe -Sp-P,
R^{a} eine Ankergruppe der Formel 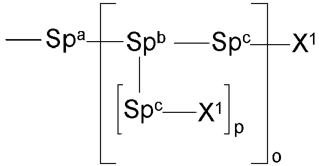 oder 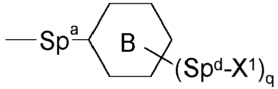
p 1 oder 2,
q 2 oder 3,
B ein substituiertes oder unsubstituiertes Ringsystem oder kondensiertes Ringsystem,
o 0 oder 1,
X¹ unabhängig voneinander OH, SH, NH₂, NHR¹¹, NR¹¹₂, C(O)OH oder -CHO,
R¹¹ Alkyl mit 1 bis 12 C-Atomen, welches linear oder verzweigt sein kann, und worin H durch Fluor oder Alkoxy mit 1 bis 8 C-Atomen substituiert sein kann,
Sp^{a}, Sp^{c}, Sp^{d} jeweils unabhängig voneinander eine Abstandsgruppe oder eine Einfachbindung, und
Sp^{b} eine tri- oder tetravalente Gruppe
bedeuten.

2. Verbindungen nach Anspruch 1, **dadurch gekennzeichnet, dass** 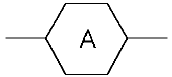 einen Ring der Formel 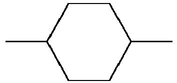 und 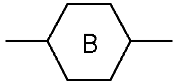 einen Ring der Formel 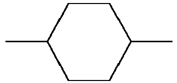 oder 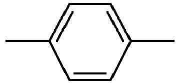 bedeutet.

3. Verbindungen nach Anspruch 1 oder 2, **dadurch gekennzeichnet, dass**
Sp^{a} eine unverzweigte oder verzweigte Alkylenkette mit 1 bis 8 C-Atomen, worin eine oder mehrere CH₂-Gruppen durch -O-, -NH-, -NR³-, -S- und -(CO)- ersetzt sein können, so dass O/S-Atome nicht direkt miteinander verknüpft sind, und worin auch ein oder mehrere H-Atome durch F, Cl oder -OH ersetzt sein können,
oder, für o = 1, zusätzlich eine Einfachbindung,
bedeutet.

4. Verbindungen nach einem oder mehreren der Ansprüche 1 bis 3,
**dadurch gekennzeichnet, dass** in der Gruppe R^{a}
Sp^{b} -CH oder -CR³ (für p = 1) oder C (für p = 2), worin R³ ein unverzweigter oder verzweigter Alkylrest mit 1 bis 10 C-Atomen ist,
und Sp^{c} bevorzugt eine unverzweigte oder verzweigte Alkylenkette mit 1 bis 8 C-Atomen, worin eine oder mehrere CH₂-Gruppen durch -O- ersetzt sein können, und worin auch ein oder mehrere H-Atome durch F, Cl oder -OH ersetzt sein können, oder eine Einfachbindung,
bedeuten.

5. Verbindungen nach einem oder mehreren der Ansprüche 1 bis 4, **dadurch gekennzeichnet, dass** in den jeweiligen Formeln
L F, Cl, CH₃, Ethyl, Propyl, Cyclopropyl oder Isopropyl bedeutet.

6. Verbindungen nach einem oder mehreren der Ansprüche 1 bis 5, **dadurch gekennzeichnet, dass** die Gruppe R^{a} ausgewählt ist aus den folgenden Formeln: 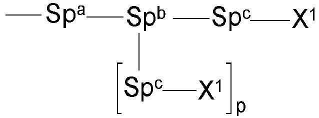 oder
-Sp^{a}-X¹
worin Sp^{a}, Sp^{b}, Sp^{c}, X¹ und p wie in Anspruch 1 definiert sind.

7. Verbindungen nach einem oder mehreren der Ansprüche 1 bis 6, ausgewählt aus den folgenden Formeln: 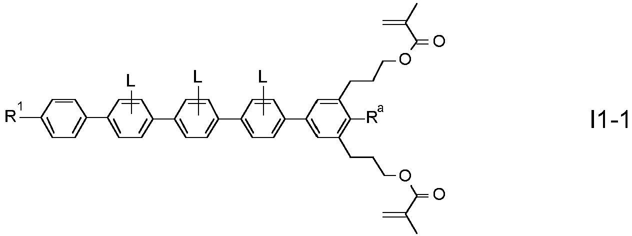 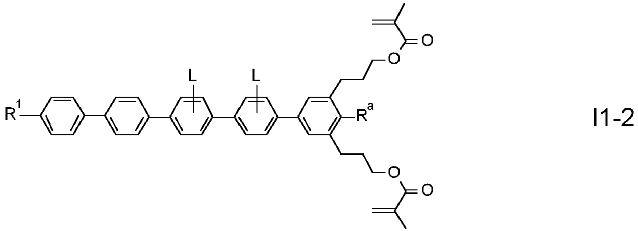 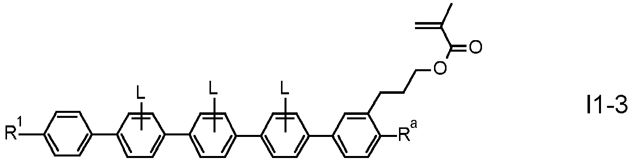 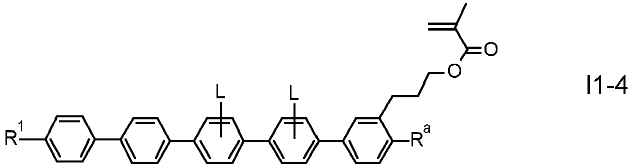 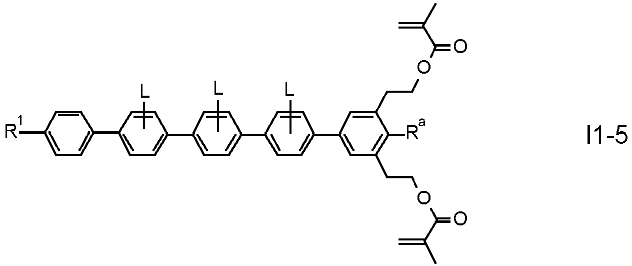 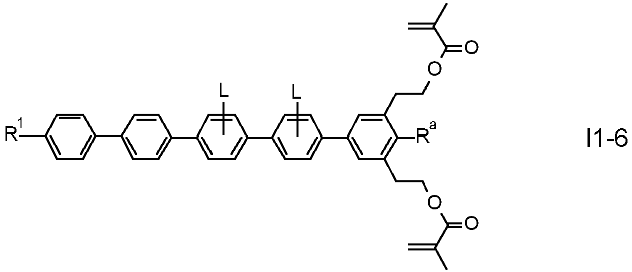 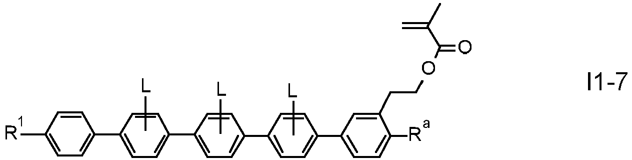 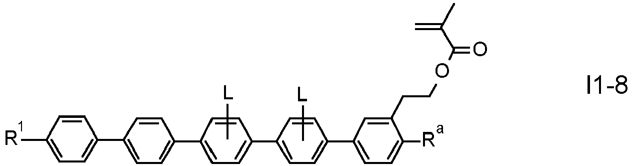 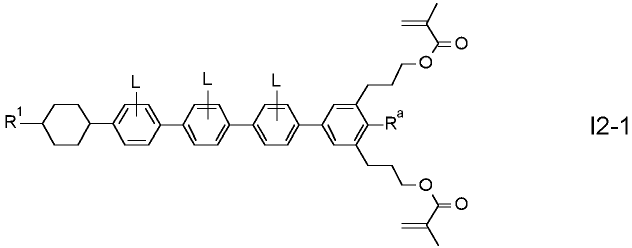 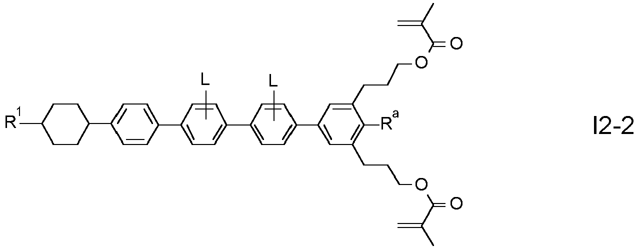 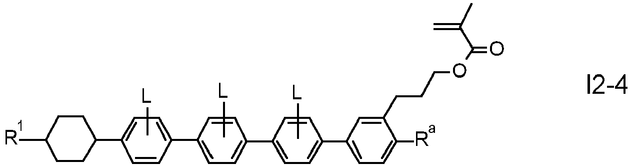 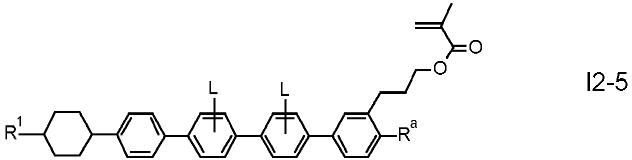 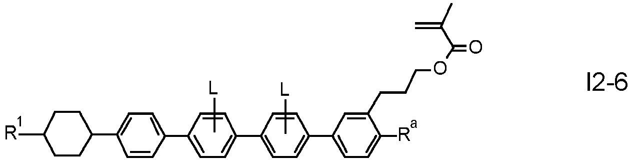 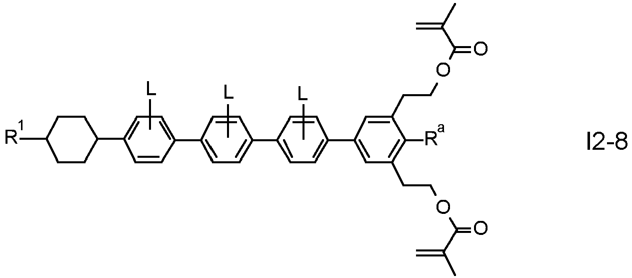 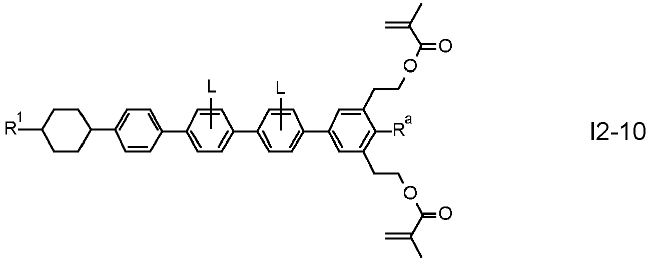 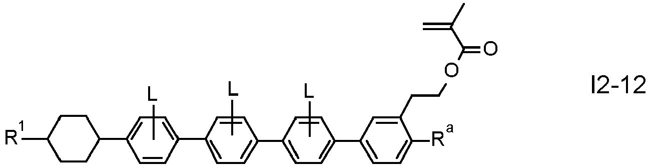 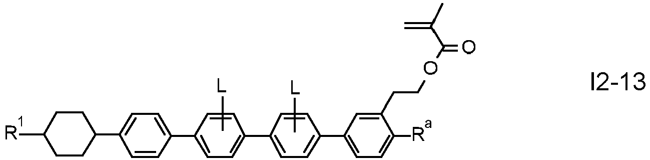 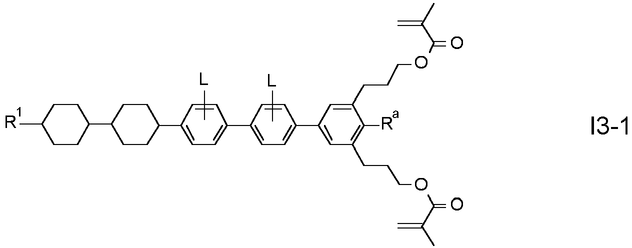 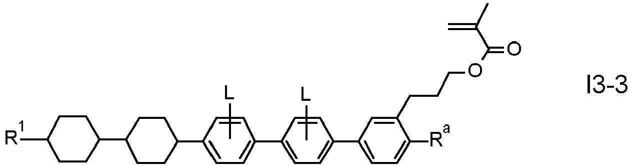 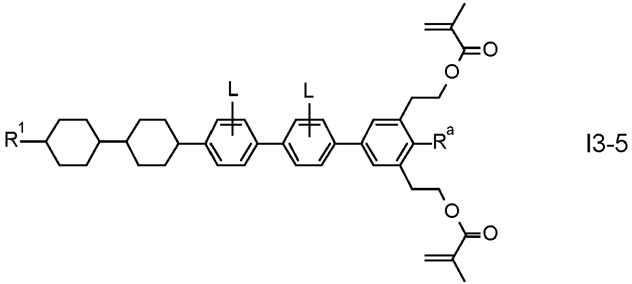 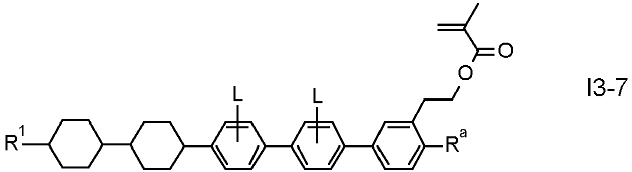 worin R¹, L und R^{a} jeweils unabhängig wie für Formel I-A nach Anspruch 1 definiert sind.

8. Verbindungen nach einem oder mehreren der Ansprüche 1 bis 7, **dadurch gekennzeichnet, dass** die Gruppe R^{a} eine Gruppe ausgewählt aus den Teilformeln 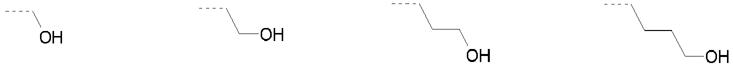 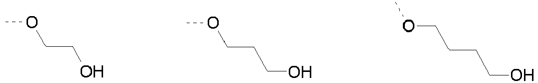 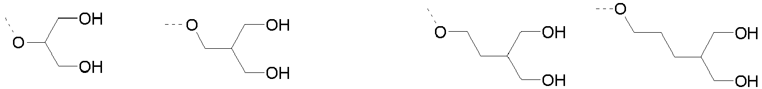 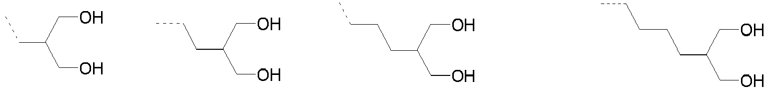 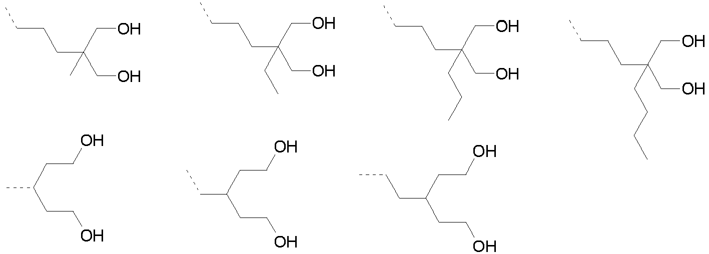 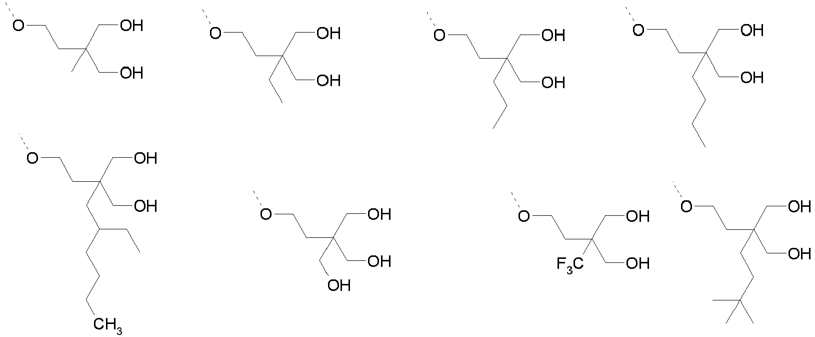 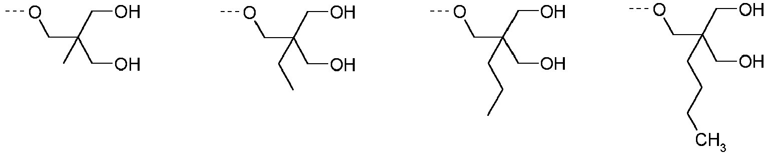 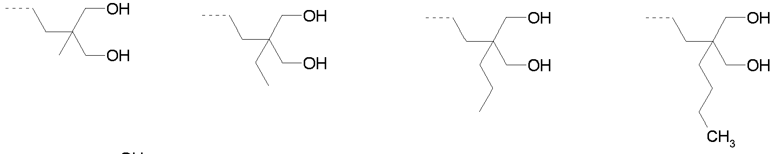 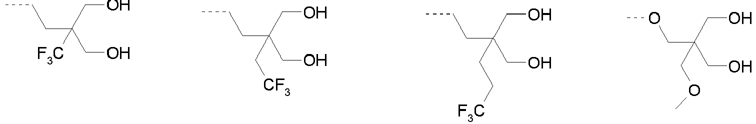 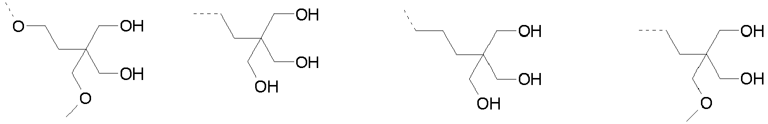 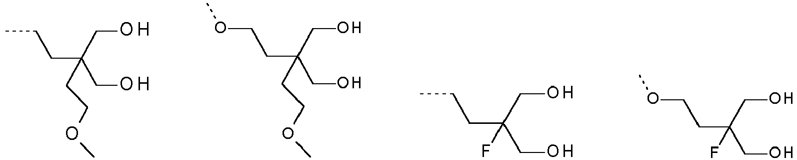 bedeutet.

9. Verbindungen nach einem oder mehreren der Ansprüche 1 bis 8, **dadurch gekennzeichnet, dass** für die Verbindung der Formel I-A die eine oder die mehreren polymerisierbaren Gruppen P ausgewählt sind aus Acrylat, Methacrylat, Ethylacrylat, Fluoroacrylat, Chloroacrylat, Vinyloxy, Oxetan und Epoxid.

10. Verwendung von Verbindungen der Formel I-A nach einem oder mehreren der Ansprüche 1 bis 9 als Additiv für FK-Medien zur Herbeiführung einer homöotropen Orientierung gegenüber einer das FK-Medium begrenzenden Oberfläche, wobei die Verbindungen nach Herbeiführung der homöotropen Orientierung optional polymerisiert werden.

11. FK-Medium enthaltend eine niedermolekulare, nicht polymerisierbare, flüssigkristalline Komponente und eine oder mehrere Verbindungen der Formel I-A nach einem der Ansprüche 1 bis 10, oder, falls möglich, ein (Co-)Polymer umfassend die Verbindung(en) der Formel I-A in polymerisierter Form.

12. FK-Medium nach Anspruch 11, **dadurch gekennzeichnet, dass** es eine polymerisierbare oder polymerisierte Komponente umfasst, wobei die polymerisierbare oder polymerisierte Komponente eine Verbindung der Formel I-A mit einer, zwei oder mehreren polymerisierbaren Gruppen P umfasst, wobei die polymerisierte Komponente durch Polymerisieren der polymerisierbaren Komponente erhältlich ist.

13. FK-Medium nach Anspruch 11 oder 12, **dadurch gekennzeichnet, dass** es eine oder mehrere polymerisierbare Verbindungen der Formel M oder ein (Co-)Polymer umfassend Verbindungen der Formel M in polymerisierter Form enthält:
P¹-Sp¹-A²-(Z¹-A¹)ₙ-Sp²-P² M
worin die einzelnen Reste folgende Bedeutung besitzen:
P¹, P² jeweils unabhängig eine polymerisierbare Gruppe,
Sp¹, Sp² jeweils unabhängig eine Abstandsgruppe,
A¹, A², jeweils unabhängig voneinander einen Rest ausgewählt aus folgenden Gruppen
a) der Gruppe bestehend aus trans-1,4-Cyclohexylen, 1,4-Cyclohexenylen und 4,4'-Bicyclohexylen, worin auch eine oder mehrere nicht benachbarte CH₂-Gruppen durch -O- und/oder -S- ersetzt sein können und worin auch ein oder mehrere H-Atome durch eine Gruppe L ersetzt sein können, oder ausgewählt aus 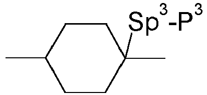
b) der Gruppe bestehend aus 1,4-Phenylen und 1,3-Phenylen, worin auch eine oder zwei CH-Gruppen durch N ersetzt sein können und worin auch ein oder mehrere H-Atome durch eine Gruppe L oder -Sp³-P ersetzt sein können,
c) der Gruppe bestehend aus Tetrahydropyran-2,5-diyl, 1,3-Dioxan-2,5-diyl, Tetrahydrofuran-2,5-diyl, Cyclobutan-1,3-diyl, Piperidin-1,4-diyl, Thiophen-2,5-diyl und Selenophen-2,5-diyl, welche auch ein oder mehrfach durch eine Gruppe L substituiert sein können,
d) der Gruppe bestehend aus gesättigten, teilweise ungesättigten oder vollständig ungesättigten, und optional substituierten, polycyclischen Resten mit 5 bis 20 cyclischen C-Atomen, von denen auch eines oder mehrere durch Heteroatome ersetzt sein können,
P³ eine polymerisierbare Gruppe,
Sp³ eine Abstandsgruppe,
n 0, 1, 2 oder 3,
Z¹ jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist, -O-, -CO-, -C(R^{c}R^{d})-, -CH₂CF₂-, -CF₂CF₂-, oder eine Einfachbindung,
L bei jedem Auftreten gleich oder verschieden F, Cl, CN, SCN, SF₅ oder unverzweigtes oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
R^{c} und R^{d} jeweils unabhängig voneinander H oder Alkyl mit 1 bis 6 C-Atomen, vorzugsweise H, Methyl oder Ethyl, bedeuten.
wobei eine oder mehrere der Gruppen P¹-Sp¹-, -Sp²-P² und -Sp³-P³ einen Rest R^{aa} bedeuten können, mit der Maßgabe, dass mindestens eine der vorhandenen Gruppen P¹-Sp¹-, -Sp²-P² und -Sp³-P³ nicht R^{aa} bedeutet,
R^{aa} H, F, Cl, CN oder unverzweigtes oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹- ersetzt sein können, wobei die Gruppen -OH, -NH₂, -SH, -NHR, -C(O)OH und -CHO in R^{aa} nicht enthalten sind.

14. FK-Medium nach einem oder mehreren der Ansprüche 11 bis 13, **dadurch gekennzeichnet, dass** es
- 95 Gew.-% oder mehr einer niedermolekularen, nicht polymerisierbaren, flüssigkristallinen Komponente, und
- 5 Gew.-% oder weniger einer polymerisierbaren oder polymerisierten Komponente
enthält.

15. FK-Medium nach einem oder mehreren der Ansprüche 11 bis 14, **dadurch gekennzeichnet, dass** es eine oder mehrere Verbindungen ausgewählt aus der Gruppe der Verbindungen der Formeln A, B und C enthält: 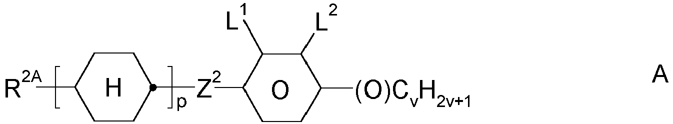 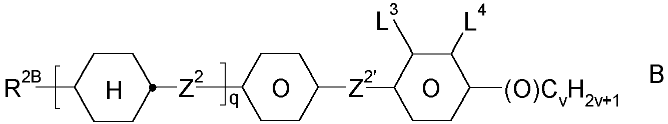 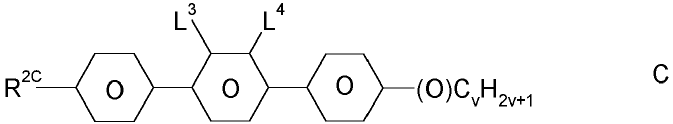 worin
R^{2A}, R^{2B} und R^{2C} jeweils unabhängig voneinander H, einen unsubstituierten, einen einfach durch CN oder CF₃ oder mindestens einfach durch Halogen substituierten Alkylrest mit bis zu 15 C-Atomen, wobei in diesen Resten auch eine oder mehrere CH₂-Gruppen durch -O-, -S-, 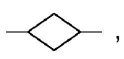 , -C≡C-, -CH=CH-, -CF₂O-, -OCF₂-, -OC-O- oder -O-CO- so ersetzt sein können, dass O-Atome nicht direkt miteinander verknüpft sind,
L¹⁻⁴ jeweils unabhängig voneinander F, Cl, CF₃ oder CHF₂,
Z² und Z² jeweils unabhängig voneinander Einfachbindung, -CH₂CH₂-, -CH=CH-, -CF₂O-, -OCF₂-, -CH₂O-, -OCH₂-, -COO-, -OCO-, -C₂F₄-, -CF=CF- oder -CH=CHCH₂O-,
p 1 oder 2, bevorzugt 1,
q 0 oder 1,
(O) -O- oder eine Einfachbindung, und
v 1 bis 6
bedeuten.

16. FK-Anzeige enthaltend eine FK-Zelle mit zwei Substraten und mindestens zwei Elektroden, wobei mindestens ein Substrat lichtdurchlässig ist und mindestens ein Substrat eine oder zwei Elektroden aufweist, sowie mit einer zwischen den Substraten befindlichen Schicht eines FK-Mediums nach einem oder mehreren der Ansprüche 11 bis 15,
wobei die Verbindung der Formel I-A eine homöotrope Ausrichtung des FK-Mediums gegenüber den Substratoberflächen herbeiführt.

17. FK-Anzeige nach Anspruch 16, **dadurch gekennzeichnet, dass** eines oder beide der Substrate keine Orientierungsschichten zur homöotropen Ausrichtung des FK-Mediums aufweisen.

18. Verfahren zu Herstellung eines FK-Mediums, **dadurch gekennzeichnet, dass** man eine oder mehrere Verbindungen der Formel I-A nach einem der Ansprüche 1 bis 9 mit einer niedermolekularen flüssigkristallinen Komponente mischt und optional eine oder mehrere weitere polymerisierbare Verbindungen und/oder beliebige Additive zugibt.

19. Verfahren zu Herstellung einer FK-Anzeige enthaltend eine FK-Zelle mit zwei Substraten und mindestens zwei Elektroden, wobei mindestens ein Substrat lichtdurchlässig ist und mindestens ein Substrat eine oder zwei Elektroden aufweist, umfassend die Verfahrensschritte:
- Füllen der Zelle mit einem FK-Medium nach einem der Ansprüche 11 bis 15, wobei sich eine homöotrope Ausrichtung des FK-Mediums gegenüber den Substratoberflächen einstellt, und
- optional Polymerisieren der polymerisierbaren Komponente(n), optional unter Anlegen einer Spannung an die Zelle oder unter der Wirkung eines elektrischen Feldes, in einem oder mehreren Verfahrensschritten.

## Claims

1. Compounds of the formula I-A 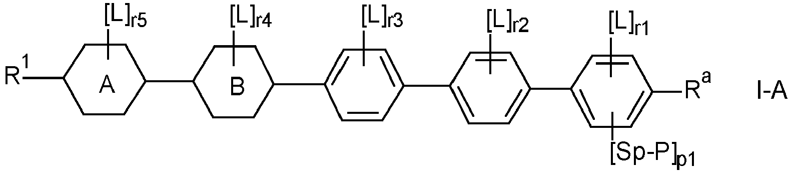 in which
rings A, B in each case independently denote a ring of the formula 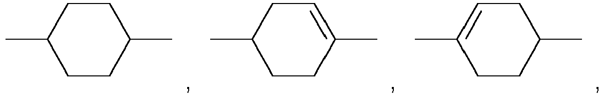 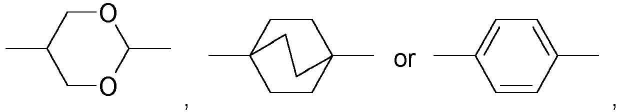
L in each case, independently of one another, denotes unbranched or branched alkyl, alkenyl, alkoxy, alkylcarbonyl, alkoxycarbonyl, alkylcarbonyloxy or alkoxycarbonyloxy having 1 to 25 C atoms, in which, in addition, one or more H atoms may be replaced by F or Cl, or denotes F, Cl, Br, I, -CN, -NO₂, -NCO, -NCS, -OCN, -SCN, -C(=O)N(R⁰)R⁰⁰ or cycloalkyl having 3 to 6 C atoms,
r1 denotes 0,
r2 denotes 1,
r3 denotes 1,
r4 denotes 0 or 1,
r5 denotes 0 or 1,
P denotes a polymerisable group,
Sp denotes a spacer group or a single bond,
p1 denotes 1 or 2,
R¹ denotes an alkyl radical having 1 to 25 C atoms, where, in addition, one or more CH₂ groups in this radical may in each case be replaced, independently of one another, by -CH=CH-, -C≡C-, -CF₂O-, -OCF₂-, 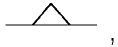 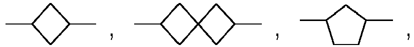 -O-, -S-, -CO-, -CO-O- or -O-CO- in such a way that O/S atoms are not linked directly to one another, and in which, in addition, one or more H atoms may be replaced by F or Cl, or denotes H or a group -Sp-P,
R^{a} denotes an anchor group of the formula 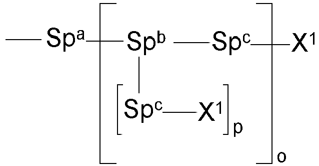 or 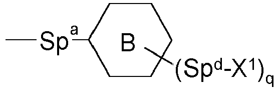
p denotes 1 or 2,
q denotes 2 or 3,
B denotes a substituted or unsubstituted ring system or condensed ring system,
o denotes 0 or 1,
X¹, independently of one another, denotes OH, SH, NH₂, NHR¹¹, NR¹¹₂, C(O)OH or -CHO,
R¹¹ denotes alkyl having 1 to 12 C atoms, which may be linear or branched and in which H may be substituted by fluorine or alkoxy having 1 to 8 C atoms,
Sp^{a}, Sp^{c}, Sp^{d} in each case, independently of one another, denote a spacer group or a single bond, and
Sp^{b} denotes a tri- or tetravalent group.

2. Compounds according to Claim 1, **characterised in that** 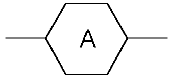 denotes a ring of the formula 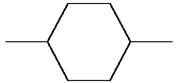 and 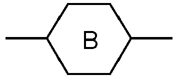 denotes a ring of the formula 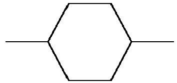 or

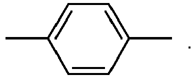

3. Compounds according to Claim 1 or 2, **characterised in that**
Sp^{a} denotes an unbranched or branched alkylene chain having 1 to 8 C atoms, in which one or more CH₂ groups may be replaced by -O-, -NH-, -NR³-, -S- or -(CO)- in such a way that O/S atoms are not linked directly to one another, and in which, in addition, one or more H atoms may be replaced by F, Cl or -OH,
or, for o = 1, additionally denotes a single bond.

4. Compounds according to one or more of Claims 1 to 3, **characterised in that**, in the group R^{a},
Sp^{b} denotes -CH or -CR³ (for p = 1) or C (for p = 2), in which R³ is an unbranched or branched alkyl radical having 1 to 10 C atoms,
and Sp^{c} preferably denotes an unbranched or branched alkylene chain having 1 to 8 C atoms, in which one or more CH₂ groups may be replaced by -O- and in which, in addition, one or more H atoms may be replaced by F, Cl or -OH, or a single bond.

5. Compounds according to one or more of Claims 1 to 4, **characterised in that**, in the respective formulae,
L denotes F, Cl, CH₃, ethyl, propyl, cyclopropyl or isopropyl.

6. Compounds according to one or more of Claims 1 to 5, **characterised in that** the group
R^{a} is selected from the following formulae: 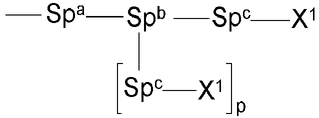 or
-Sp^{a}-X¹
in which Sp^{a}, Sp^{b}, Sp^{c}, X¹ and p are defined as in Claim 1.

7. Compounds according to one or more of Claims 1 to 6, selected from the following formulae: 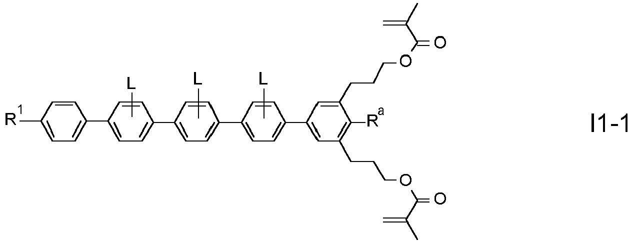 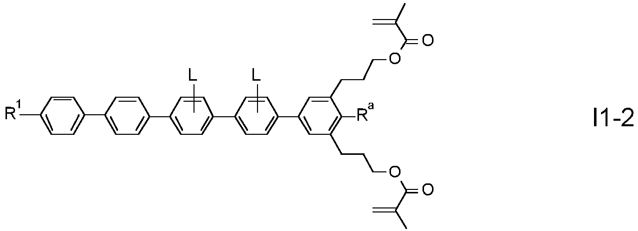 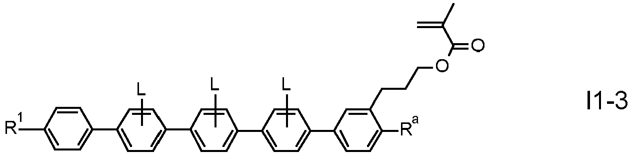 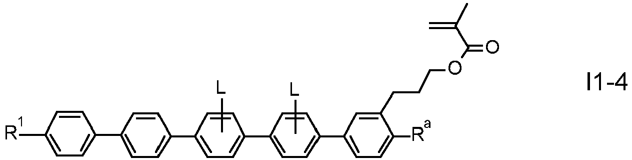 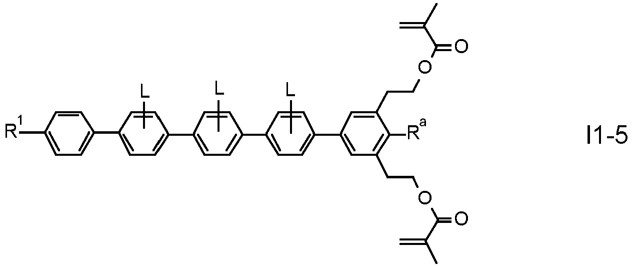 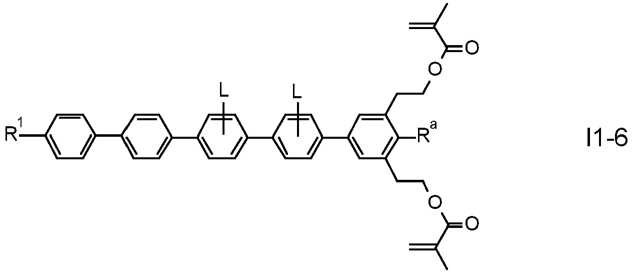 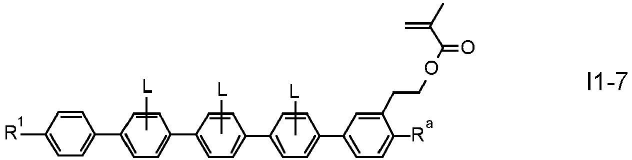 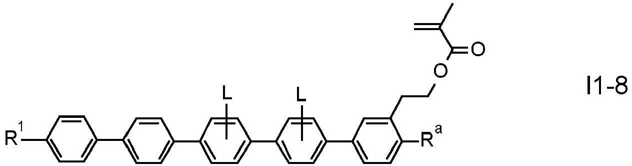 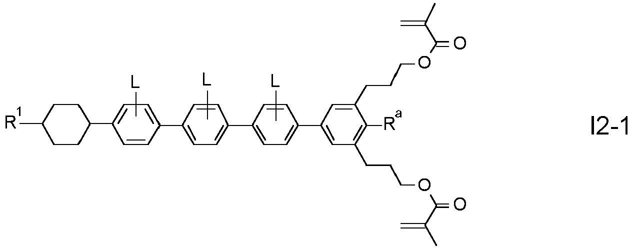 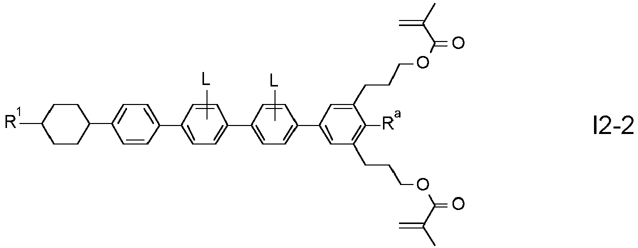 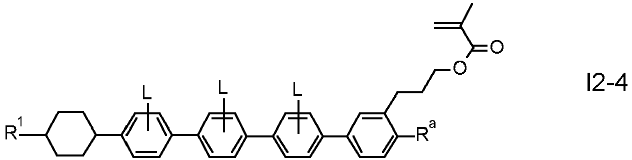 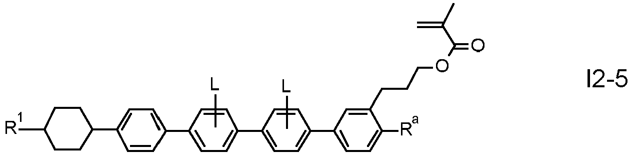 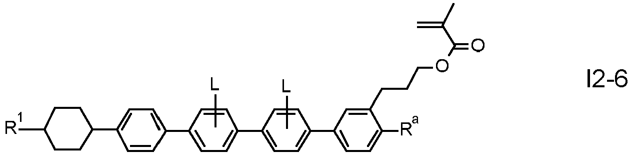 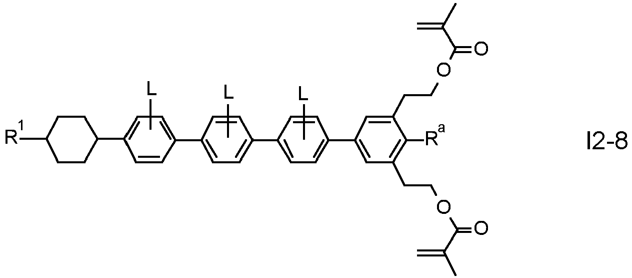 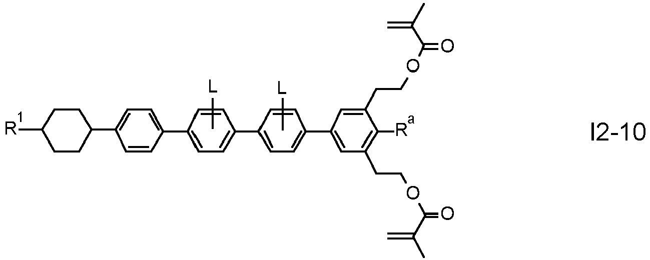 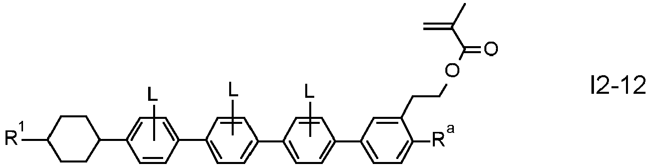 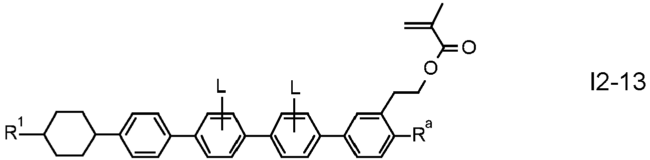 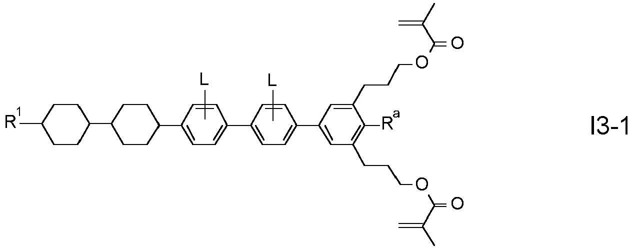 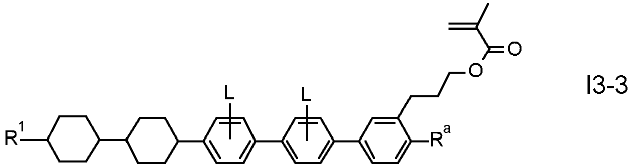 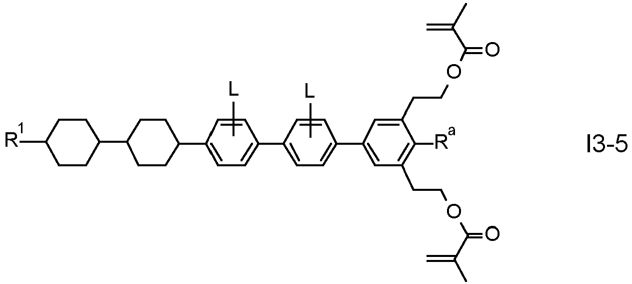 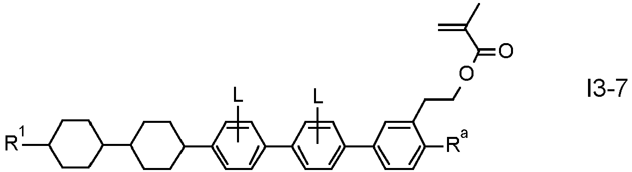 in which R¹, L and R^{a} in each case independently are defined as for formula I-A according to Claim 1.

8. Compounds according to one or more of Claims 1 to 7, **characterised in that** the group R^{a} denotes a group selected from the sub-formulae

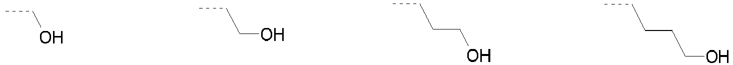

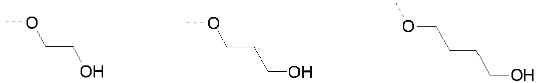

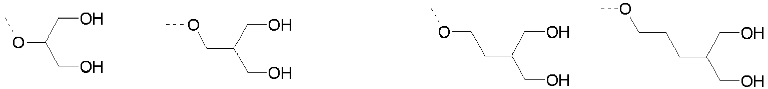

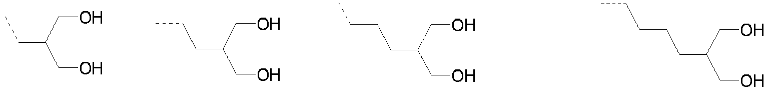

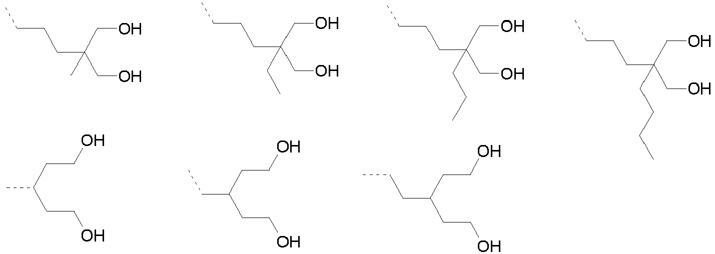

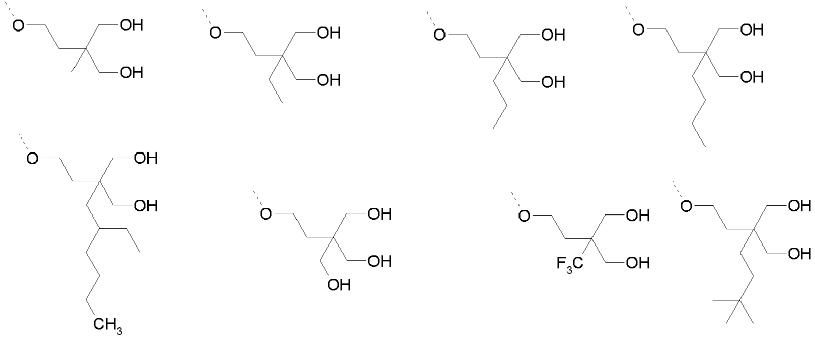

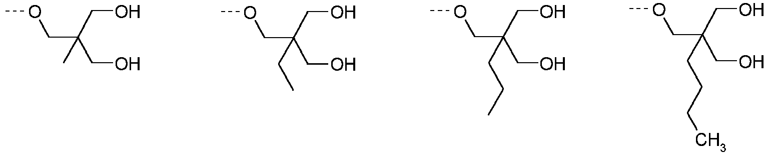

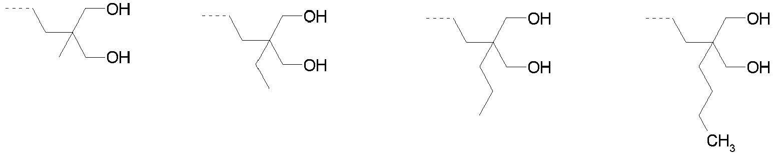

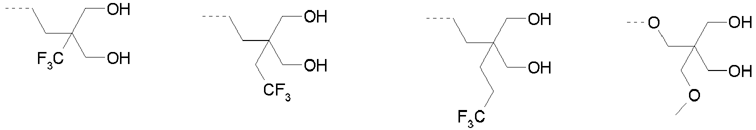

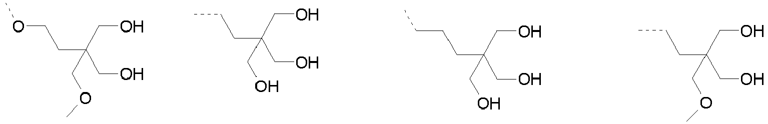

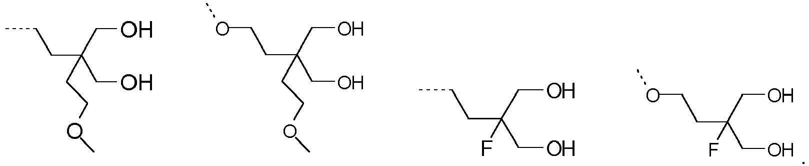

9. Compounds according to one or more of Claims 1 to 8, **characterised in that**, for the compound of the formula I-A, the one or more polymerisable groups P are selected from acrylate, methacrylate, ethylacrylate, fluoroacrylate, chloroacrylate, vinyloxy, oxetane and epoxide.

10. Use of compounds of the formula I-A according to one or more of Claims 1 to 9 as additive for LC media for effecting homeotropic alignment with respect to a surface delimiting the LC medium, where the compounds are optionally polymerised after effecting the homeotropic alignment.

11. LC medium comprising a low-molecular-weight, unpolymerisable, liquid-crystalline component and one or more compounds of the formula I-A according to one of Claims 1 to 10, or, if possible, a (co)polymer comprising the compound(s) of the formula I-A in polymerised form.

12. LC medium according to Claim 11, **characterised in that** it comprises a polymerisable or polymerised component, where the polymerisable or polymerised component comprises a compound of the formula I-A containing one, two or more polymerisable groups P, where the polymerised component is obtainable by polymerisation of the polymerisable component.

13. LC medium according to Claim 11 or 12, **characterised in that** it comprises one or more polymerisable compounds of the formula M or a (co)-polymer comprising compounds of the formula M in polymerised form:
P¹-Sp¹-A²-(Z¹-A¹)ₙ-Sp²-P² M
in which the individual radicals have the following meaning:
P¹, P² in each case independently denote a polymerisable group,
Sp¹, Sp² in each case independently denote a spacer group,
A¹, A² in each case, independently of one another, denote a radical selected from the following groups:
a) the group consisting of trans-1,4-cyclohexylene, 1,4-cyclohex-enylene and 4,4'-bicyclohexylene, in which, in addition, one or more non-adjacent CH₂ groups may be replaced by -O- and/or -S- and in which, in addition, one or more H atoms may be replaced by a group L, or selected from 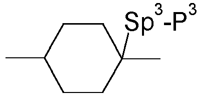
b) the group consisting of 1,4-phenylene and 1,3-phenylene, in which, in addition, one or two CH groups may be replaced by N and in which, in addition, one or more H atoms may be replaced by a group L or -Sp³-P,
c) the group consisting of tetrahydropyran-2,5-diyl, 1,3-dioxane-2,5-diyl, tetrahydrofuran-2,5-diyl, cyclobutane-1,3-diyl, piperidine-1,4-diyl, thiophene-2,5-diyl and selenophene-2,5-diyl, each of which may also be mono- or polysubstituted by a group L,
d) the group consisting of saturated, partially unsaturated or fully unsaturated, and optionally substituted, polycyclic radicals having 5 to 20 cyclic C atoms, one or more of which may, in addition, be replaced by heteroatoms,
P³ denotes a polymerisable group,
Sp³ denotes a spacer group,
n denotes 0, 1, 2 or 3,
Z¹ in each case, independently of one another, denotes -CO-O-, -O-CO-, -CH₂O-, -OCH₂-, -CF₂O-, -OCF₂- or -(CH₂)ₙ-, where n is 2, 3 or 4, -O-, -CO-, -C(R^{c}R^{d})-, -CH₂CF₂-, -CF₂CF₂- or a single bond,
L on each occurrence, identically or differently, denotes F, Cl, CN, SCN, SF₅ or unbranched or branched, in each case optionally fluorinated, alkyl, alkoxy, alkylcarbonyl, alkoxy-carbonyl, alkylcarbonyloxy or alkoxycarbonyloxy having 1 to 12 C atoms,
R^{c} and R^{d} in each case, independently of one another, denote H or alkyl having 1 to 6 C atoms, preferably H, methyl or ethyl,
where one or more of the groups P¹-Sp¹-, -Sp²-P² and -Sp³-P³ may denote a radical R^{aa}, with the proviso that at least one of the groups P¹-Sp¹-, -Sp²-P² and -Sp³-P³ present does not denote R^{aa},
R^{aa} denotes H, F, Cl, CN or unbranched or branched alkyl having 1 to 25 C atoms, in which, in addition, one or more non-adjacent CH₂ groups may in each case be replaced, indepen-dently of one another, by C(R⁰)=C(R⁰⁰)-, -C≡C-, -O-, -S-, -CO-, -CO-O-, -O-CO-, -O-CO-O- in such a way that O and/or S atoms are not linked directly to one another, and in which, in addition, one or more H atoms may be replaced by F, Cl, CN or P¹-Sp¹-, where the groups -OH, -NH₂, -SH, -NHR, -C(O)OH and -CHO are not present in R^{aa}.

14. LC medium according to one or more of Claims 11 to 13, **characterised in that** it comprises
- 95% by weight or more of a low-molecular-weight, unpolymerisable, liquid-crystalline component, and
- 5% by weight or less of a polymerisable or polymerised component.

15. LC medium according to one or more of Claims 11 to 14, **characterised in that** it comprises one or more compounds selected from the group of the compounds of the formulae A, B and C: 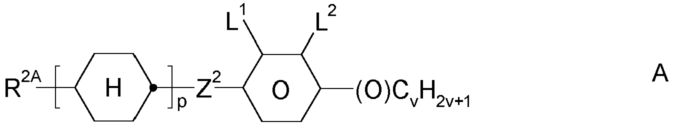 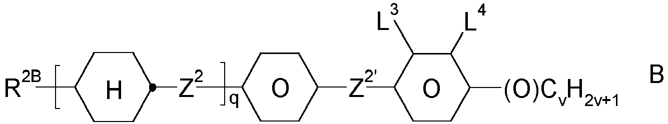 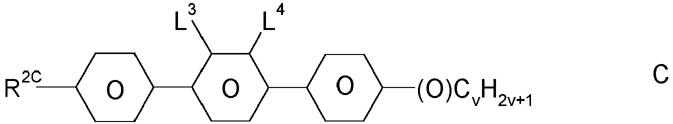 in which
R^{2A}, R^{2B} and R^{2C} in each case, independently of one another, denote H, an alkyl radical having up to 15 C atoms which is unsubstituted, mono-substituted by CN or CF₃ or at least monosubstituted by halogen, where, in addition, one or more CH₂ groups in these radicals may be replaced by -O-, -S-, 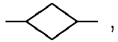 -C≡C-, -CH=CH-, -CF₂O-, -OCF₂-, -OC-O- or -O-CO- in such a way that O atoms are not linked directly to one another,
L¹⁻⁴ in each case, independently of one another, denote F, Cl, CF₃ or CHF₂,
Z² and Z^{2'} in each case, independently of one another, denote a single bond, -CH₂CH₂-, -CH=CH-, -CF₂O-, -OCF₂-, -CH₂O-, -OCH₂-, -COO-, -OCO-, -C₂F₄-, -CF=CF- or -CH=CHCH₂O-,
p denotes 1 or 2, preferably 1,
q denotes 0 or 1,
(O) denotes -O- or a single bond, and
v denotes 1 to 6.

16. LC display containing an LC cell having two substrates and at least two electrodes, where at least one substrate is transparent to light and at least one substrate has one or two electrodes, and having a layer of an LC medium according to one or more of Claims 11 to 15 located between the substrates,
where the compound of the formula I-A effects homeotropic alignment of the LC medium with respect to the substrate surfaces.

17. LC display according to Claim 16, **characterised in that** one or both of the substrates have no alignment layers for homeotropic alignment of the LC medium.

18. Process for the preparation of an LC medium, **characterised in that** one or more compounds of the formula I-A according to one of Claims 1 to 9 are mixed with a low-molecular-weight liquid-crystalline component, and one or more further polymerisable compounds and/or any desired additives are optionally added.

19. Process for the production of an LC display containing an LC cell having two substrates and at least two electrodes, where at least one substrate is transparent to light and at least one substrate has one or two electrodes, comprising the process steps:
- filling of the cell with an LC medium according to one of Claims 11 to 15, where homeotropic alignment of the LC medium with respect to the substrate surfaces occurs, and
- optionally polymerisation of the polymerisable component(s), optionally with application of a voltage to the cell or under the action of an electric field, in one or more process steps.

## Revendications

1. Composés de la formule I-A 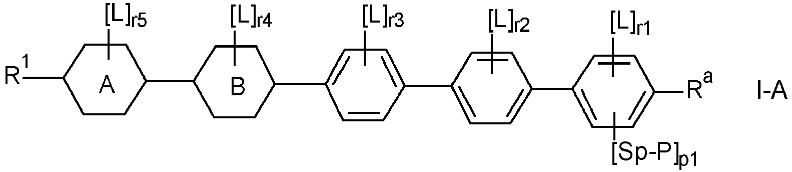 dans laquelle
les cycles A, B représentent dans chaque cas, de manière indépendante, un cycle de la formule 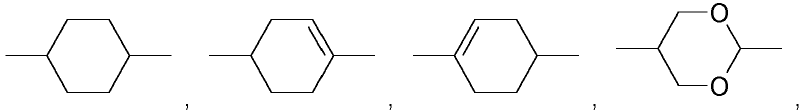 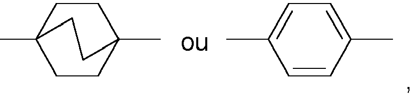
L représente dans chaque cas, de manière indépendante les uns des autres, alkyle, alkényle, alcoxy, alkylcarbonyle, alcoxycarbonyle, alkylcarbonyloxy ou alcoxycarbonyloxy non ramifié ou ramifié qui comporte de 1 à 25 atome(s) de C, où, en outre, un ou plusieurs atome(s) de H peut/ peuvent être remplacé(s) par F ou par Cl, ou représente F, Cl, Br, I, -CN, -NO₂, -NCO, -NCS, -OCN, -SCN, -C(=O)N(R⁰)R⁰⁰ ou cycloalkyle qui comporte de 3 à 6 atomes de C,
r1 représente 0,
r2 représente 1,
r3 représente 1,
r4 représente 0 ou 1,
r5 représente 0 ou 1,
P représente un groupe polymérisable,
Sp représente un groupe d'espaceur ou une liaison simple,
p1 représente 1 ou 2,
R¹ représente un radical alkyle qui comporte de 1 à 25 atome(s) de C, où, en outre, un ou plusieurs groupe(s) CH₂ dans ce radical peut/peuvent dans chaque cas être remplacé(s), de manière indépendante les uns des autres, par -CH=CH-, -C≡C-, -CF₂O-, -OCF₂-, 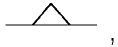 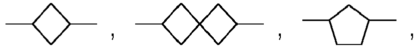 -O-, -S-, -CO-, -CO-O- ou -O-CO- de telle sorte que des atomes de O/S ne soient pas liés directement les uns aux autres, et où, en outre, un ou plusieurs atome(s) de H peut/peuvent être remplacé(s) par F ou par Cl, ou représente H
ou un groupe -Sp-P,
R^{a} représente un groupe d'ancrage de la formule 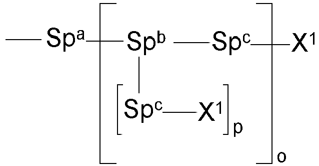 ou 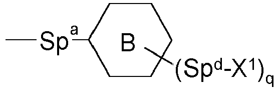
p représente 1 ou 2,
q représente 2 ou 3,
B représente un système de cycle substitué ou non substitué ou un système de cycle condensé,
o représente 0 ou 1,
X¹ représente, de manière indépendante les uns des autres, OH, SH, NH₂, NHR¹¹, NR¹¹₂, C(O)OH ou -CHO,
R¹¹ représente alkyle qui comporte de 1 à 12 atome(s) de C, lequel peut être linéaire ou ramifié et où H peut être substitué par fluor ou par alcoxy qui comporte de 1 à 8 atome(s) de C,
Sp^{a}, Sp^{c}, Sp^{d} représentent dans chaque cas, de manière indépendante les uns des autres, un groupe d'espaceur ou une liaison simple, et
Sp^{b} représente un groupe tri- ou tétravalent.

2. Composés selon la revendication 1, **caractérisés en ce que** 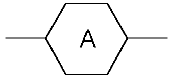 représente un cycle de la formule 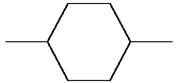 et 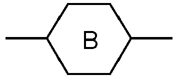 représente un cycle de la formule 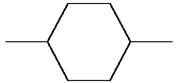 ou

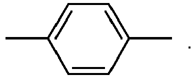

3. Composés selon la revendication 1 ou 2, **caractérisés en ce que**
Sp^{a} représente une chaîne alkylène non ramifiée ou ramifiée qui comporte de 1 à 8 atome(s) de C, où un ou plusieurs groupe(s) CH₂ peut/peuvent être remplacé(s) par -O-, -NH-, -NR³-, -S- ou -(CO)- de telle sorte que des atomes de O/S ne soient pas liés directement les uns aux autres, et où, en outre, un ou plusieurs atome(s) de H peut/peuvent être remplacé(s) par F, par Cl ou par -OH,
ou, pour o = 1, représente de manière additionnelle une liaison simple.

4. Composés selon une ou plusieurs des revendications 1 à 3, **caractérisés en ce que**, dans le groupe R^{a},
Sp^{b} représente -CH ou -CR³ (pour p = 1) ou C (pour p = 2), où R³ est un radical alkyle non ramifié ou ramifié qui comporte de 1 à 10 atome(s) de C,
et
Sp^{c} représente de préférence une chaîne alkylène non ramifiée ou ramifiée qui comporte de 1 à 8 atome(s) de C, où un ou plusieurs groupe(s) CH₂ peut/peuvent être remplacé(s) par -O- et où, en outre, un ou plusieurs atome(s) de H peut/peuvent être remplacé(s) par F, par Cl ou par -OH,
ou une liaison simple.

5. Composés selon une ou plusieurs des revendications 1 à 4, **caractérisés en ce que**, dans les formules respectives,
L représente F, Cl, CH₃, éthyle, propyle, cyclopropyle ou isopropyle.

6. Composés selon une ou plusieurs des revendications 1 à 5, **caractérisés en ce que** le groupe R^{a} est sélectionné parmi les formules qui suivent : 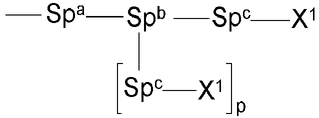 ou
-Sp^{a}-X¹
dans lesquelles Sp^{a}, Sp^{b}, Sp^{c}, X¹ et p sont tels que définis selon la revendication 1.

7. Composés selon une ou plusieurs des revendications 1 à 6, sélectionnés parmi les formules qui suivent : 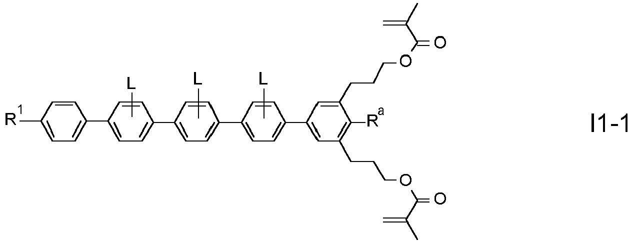 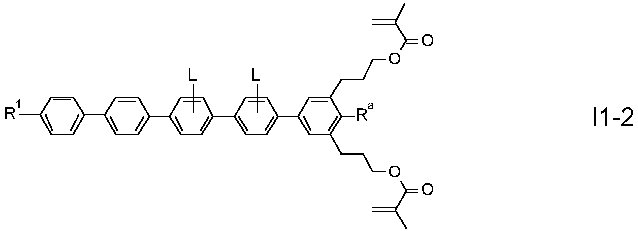 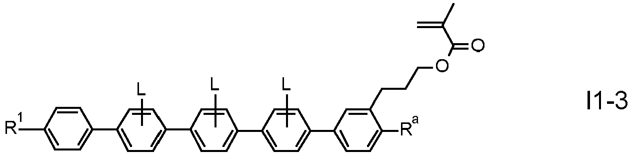 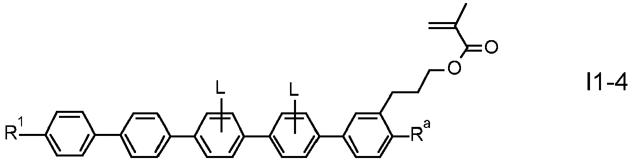 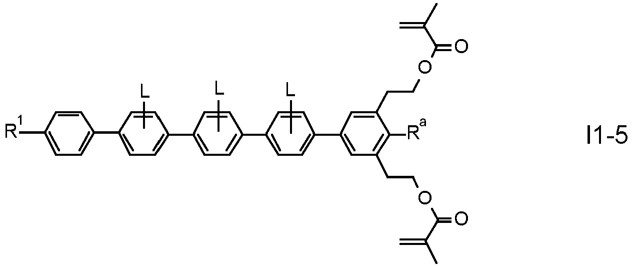 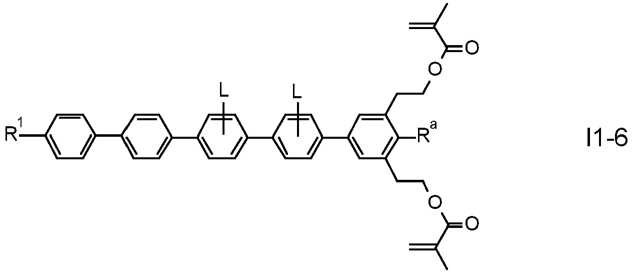 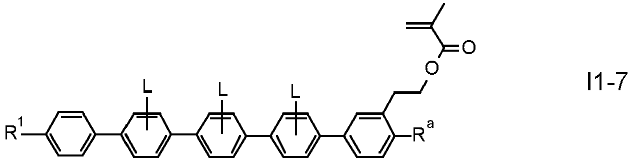 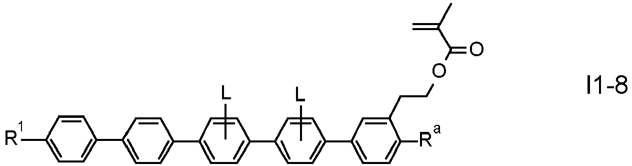 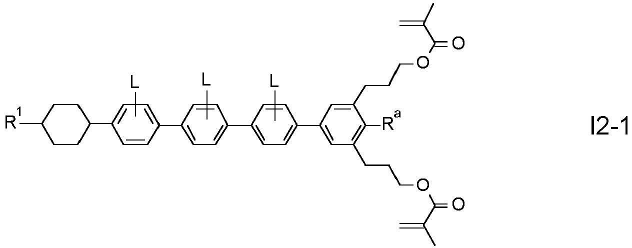 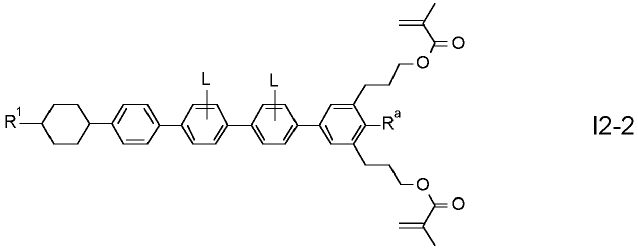 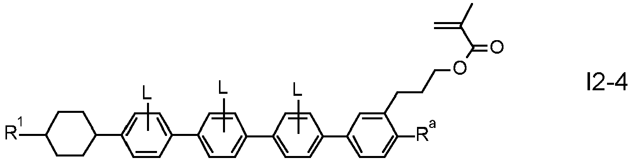 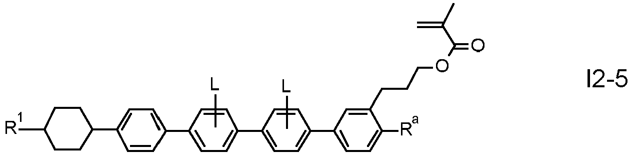 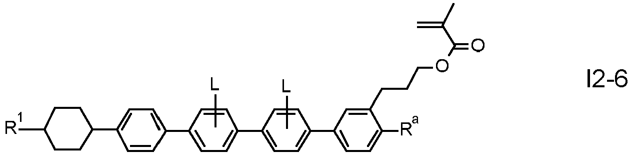 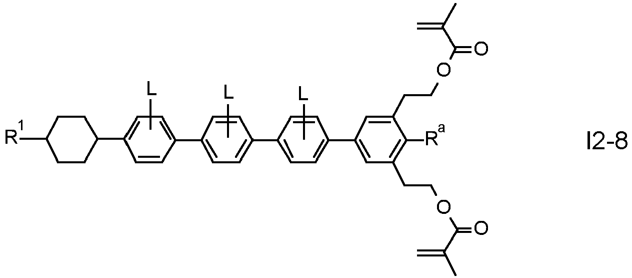 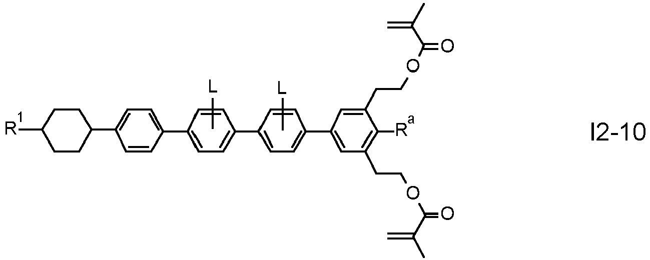 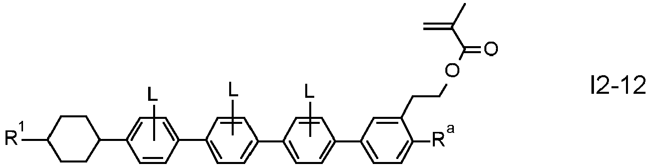 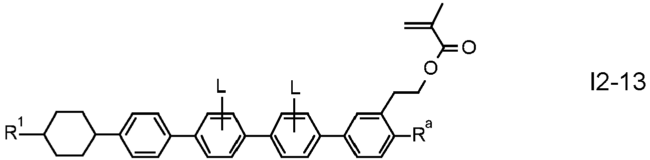 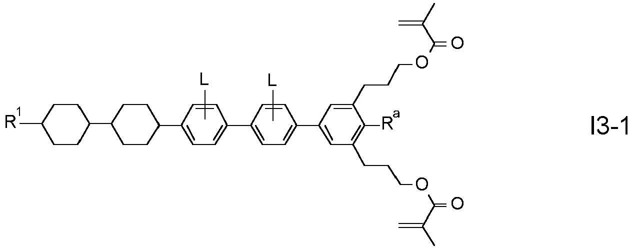 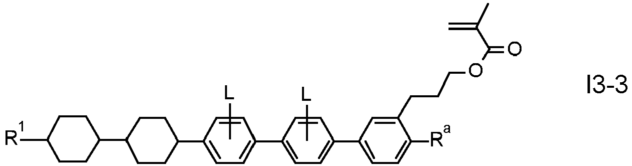 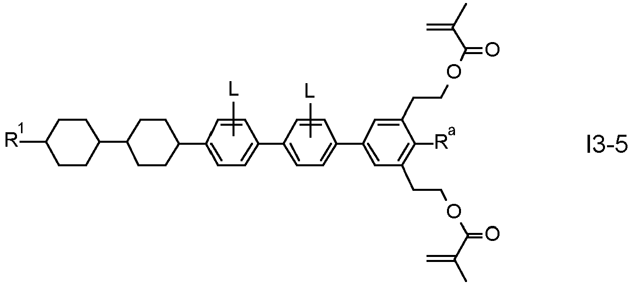 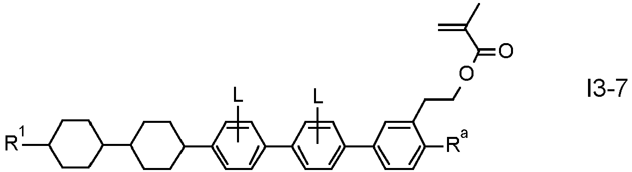 dans lesquelles R¹, L et R^{a} sont définis, dans chaque cas de manière indépendante, tel que pour la formule I-A selon la revendication 1.

8. Composés selon une ou plusieurs des revendications 1 à 7, **caractérisés en ce que** le groupe R^{a} représente un groupe qui est sélectionné parmi les sous-formules

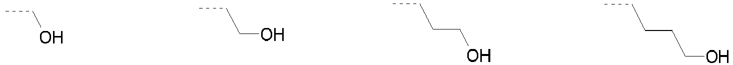

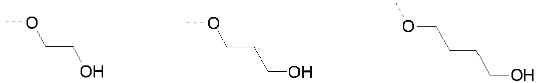

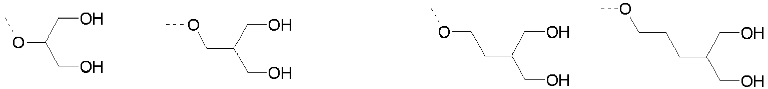

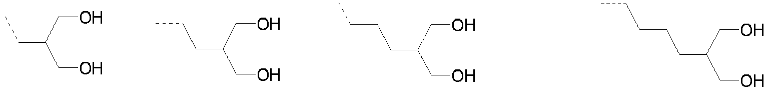

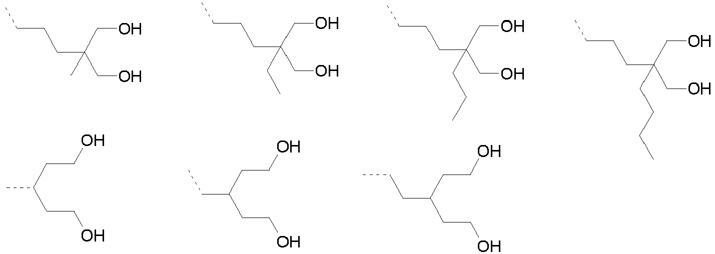

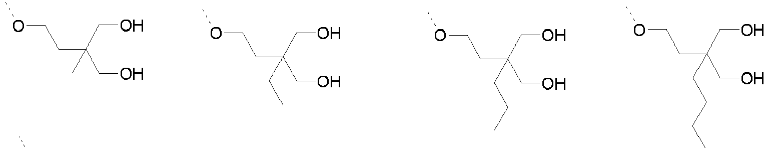

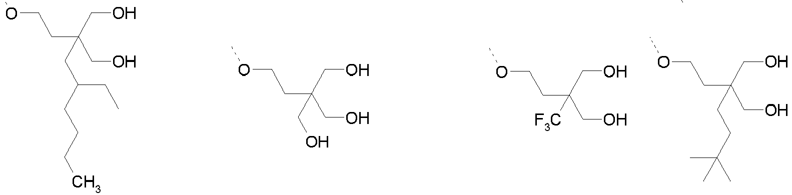

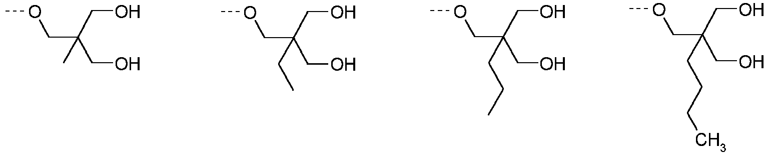

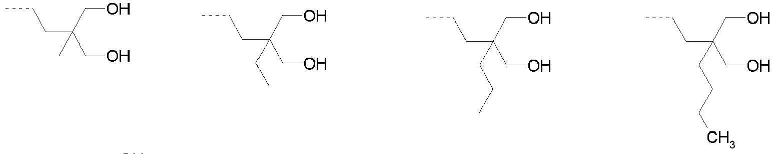

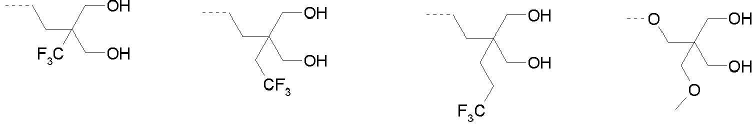

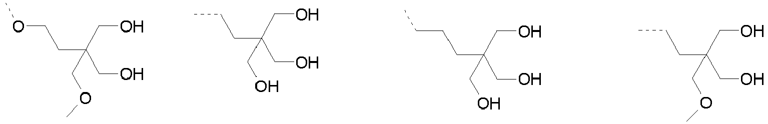

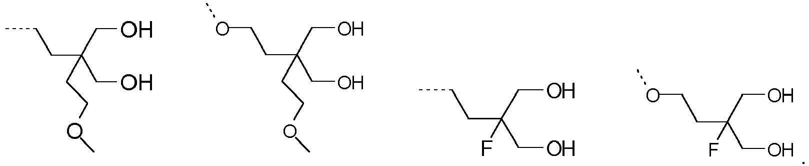

9. Composés selon une ou plusieurs des revendications 1 à 8, **caractérisés en ce que**, pour le composé de la formule I-A, les un ou plusieurs groupes polymérisables P sont sélectionnés parmi acrylate, méthacrylate, éthylacrylate, fluoroacrylate, chloroacrylate, vinyloxy, oxétane et époxyde.

10. Utilisation de composés de la formule I-A selon une ou plusieurs des revendications 1 à 9 en tant qu'additif pour les milieux LC pour réaliser un alignement homéotrope par rapport à une surface qui délimite le milieu LC, où les composés sont en option polymérisés après la réalisation de l'alignement homéotrope.

11. Milieu LC comprenant un composant cristallin liquide non polymérisable et de poids moléculaire faible et un ou plusieurs composé(s) de la formule I-A selon l'une des revendications 1 à 10, ou, si possible, un (co)polymère qui comprend le/les composé(s) de la formule I-A selon une forme polymérisée.

12. Milieu LC selon la revendication 11, **caractérisé en ce qu'**il comprend un composant polymérisable ou polymérisé component, dans lequel le composant polymérisable ou polymérisé comprend un composé de la formule I-A qui contient un ou deux groupe(s) polymérisable(s) P ou plus, dans lequel le composant polymérisé peut être obtenu par polymérisation du composant polymérisable.

13. Milieu LC selon la revendication 11 ou 12, **caractérisé en ce qu'**il comprend un ou plusieurs composé(s) polymérisable(s) de la formule M ou un (co)polymère qui comprend des composés de la formule M selon une forme polymérisée :
P¹-Sp¹-A²-(Z¹-A¹)ₙ-Sp²-P² M
dans laquelle les radicaux individuels présentent la signification qui suit :
P¹, P² représentent dans chaque cas, de manière indépendante, un groupe polymérisable,
Sp¹, Sp² représentent dans chaque cas, de manière indépendante, un groupe d'espaceur,
A¹, A² représentent dans chaque cas, de manière indépendante l'un de l'autre, un radical qui est sélectionné parmi les groupes qui suivent :
a) le groupe qui est constitué par trans-1,4-cyclohexylène, 1,4-cyclohexénylène et 4,4'-bicyclohexylène, où, en outre, un ou plusieurs groupe(s) CH₂ non adjacents peut/peuvent être remplacé(s) par -O- et/ou par -S- et où , en outre, un ou plusieurs atome(s) de H peut/peuvent être remplacé(s) par un groupe L, ou qui est sélectionné parmi 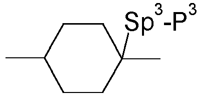
b) le groupe qui est constitué par 1,4-phénylène et 1,3-phénylène, où, en outre, un ou deux groupe(s) CH peut/peuvent être remplacé(s) par N et où, en outre, un ou plusieurs atome(s) de H peut/peuvent être remplacé(s) par un groupe L ou-Sp³-P,
c) le groupe qui est constitué par tétrahydropyran-2,5-diyle, 1,3-dioxane-2,5-diyle, tétrahydrofuran-2,5-diyle, cyclobutane-1,3-diyle, pipéridine-1,4-diyle, thiophène-2,5-diyle et sélénophène-2,5-diyle, dont chacun peut également être mono- ou polysubstitué par un groupe L,
d) le groupe qui est constitué par des radicaux polycycliques saturés, partiellement non saturés ou complètement non saturés, et en option substitués, qui comportent de 5 à 20 atomes de C cycliques, dont un ou plusieurs peut/peuvent, en outre, être remplacé(s) par des hétéroatomes,
P³ représente un groupe polymérisable,
Sp³ représente un groupe d'espaceur,
n représente 0, 1, 2 ou 3,
Z¹ représente dans chaque cas, de manière indépendante les uns des autres, -CO-O-, -O-CO-, -CH₂O-, -OCH₂-, -CF₂O-, -OCF₂- ou -(CH₂)ₙ-, où n est 2, 3 ou 4, -O-, -CO-, -C(R^{c}R^{d})-, -CH₂CF₂-, -CF₂CF₂- ou une liaison simple,
L représente pour chaque occurrence, de manière identique ou différente, F, Cl, CN, SCN, SF₅ ou alkyle, alcoxy, alkylcarbonyle, alcoxycarbonyle, alkylcarbonyloxy ou alcoxycarbonyloxy non ramifié ou ramifié, dans chaque cas en option fluoré, qui comporte de 1 à 12 atome(s) de C,
R^{c} et R^{d} représentent dans chaque cas, de manière indépendante l'un de l'autre, H ou alkyle qui comporte de 1 à 6 atome(s) de C, de préférence H, méthyle ou éthyle,
où un ou plusieurs des groupes P¹-Sp¹-, -Sp²-P² et -Sp³-P³ peut/peuvent représenter un radical R^{aa}, étant entendu qu'au moins l'un des groupes P¹-Sp¹-, -Sp²-P² et -Sp³-P³ qui sont présents ne représente pas R^{aa},
R^{aa} représente H, F, Cl, CN ou alkyle non ramifié ou ramifié qui comporte de 1 à 25 atome(s) de C, où, en outre, un ou plusieurs groupe(s) CH₂ non adjacents peut/peuvent dans chaque cas être remplacé(s), de manière indépendante les uns des autres, par C(R⁰)=C(R⁰⁰)-, -C≡C-, -O-, -S-, -CO-, -CO-O-, -O-CO-, -O-CO-O- de telle sorte que des atomes de O et/ou de S ne soient pas liés directement les uns aux autres, et où, en outre, un ou plusieurs atome(s) de H peut/ peuvent être remplacé(s) par F, Cl, CN ou P¹-Sp¹-, où les groupes -OH, -NH₂, -SH, -NHR, -C(O)OH et -CHO ne sont pas présents dans R^{aa}.

14. Milieu LC selon une ou plusieurs des revendications 11 à 13, **caractérisé en ce qu'**il comprend
- 95 % en poids ou plus d'un composant cristallin liquide non polymérisable et de poids moléculaire faible, et
- 5 % en poids ou moins d'un composant polymérisable ou polymérisé.

15. Milieu LC selon une ou plusieurs des revendications 11 à 14, **caractérisé en ce qu'**il comprend un ou plusieurs composé(s) qui est/sont sélectionné(s) parmi le groupe des composés des formules A, B et C : 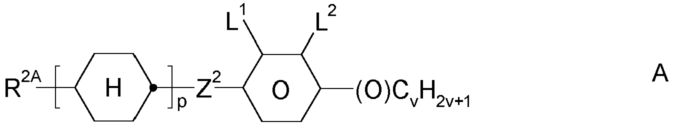 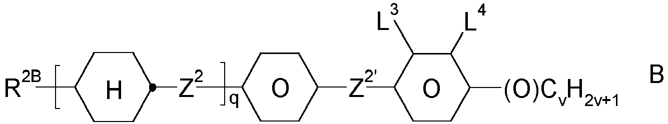 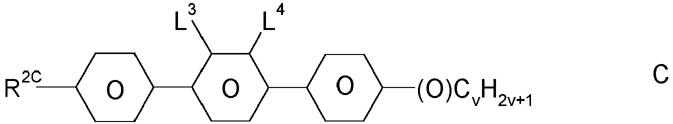 dans lesquelles
R^{2A}, R^{2B} et R^{2C} représentent dans chaque cas, de manière indépendante les uns des autres, H, un radical alkyle qui comporte jusqu'à 15 atomes de C, lequel est non substitué, monosubstitué par CN ou par CF₃ ou au moins monosubstitué par halogène, où, en outre, un ou plusieurs groupe(s) CH₂ dans ces radicaux peut/ peuvent être remplacé(s) par -O-, -S-, 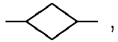 -C≡C-, -CH=CH-, -CF₂O-, -OCF₂-, -OC-O- ou -O-CO- de telle sorte que des atomes de O ne soient pas liés directement les uns aux autres,
L¹⁻⁴ représentent dans chaque cas, de manière indépendante les uns des autres, F, Cl, CF₃ ou CHF₂,
Z² et Z^{2'} représentent dans chaque cas, de manière indépendante l'un de l'autre, une liaison simple, -CH₂CH₂-, -CH=CH-, -CF₂O-, -OCF₂-, -CH₂O-, -OCH₂-, -COO-, -OCO-, -C₂F₄-, -CF=CF- ou -CH=CHCH₂O-,
p représente 1 ou 2, de préférence 1,
q représente 0 ou 1,
(O) représente -O- ou une liaison simple, et
v représente 1 à 6.

16. Affichage LC qui contient une cellule LC qui comporte deux substrats et au moins deux électrodes, dans lequel au moins un substrat est transparent vis-à-vis de la lumière et au moins un substrat comporte une ou deux électrode(s), et qui comporte une couche en un milieu LC selon une ou plusieurs des revendications 11 à 15 qui est localisée entre les substrats,
dans lequel le composé de la formule I-A réalise un alignement homéotrope du milieu LC par rapport aux surfaces de substrat.

17. Affichage LC selon la revendication 16, **caractérisé en ce que** l'un des substrats ou les deux ne comporte(nt) pas de couches d'alignement pour l'alignement homéotrope du milieu LC.

18. Procédé pour la préparation d'un milieu LC, **caractérisé en ce qu'**un ou plusieurs composé(s) de la formule I-A selon l'une des revendications 1 à 9 est/sont mélangé(s) avec un composant cristallin liquide de poids moléculaire faible, et un ou plusieurs autre(s) composé(s) polymérisable(s) et/ou de quelconques additifs souhaités est/sont en option ajouté(s).

19. Procédé pour la fabrication d'un affichage LC qui contient une cellule LC qui comporte deux substrats et au moins deux électrodes, dans lequel au moins un substrat est transparent vis-à-vis de la lumière et au moins un substrat comporte une ou deux électrode(s), comprenant les étapes de procédé qui suivent :
- le remplissage de la cellule avec un milieu LC selon l'une des revendications 11 à 15, dans lequel un alignement homéotrope du milieu LC par rapport aux surfaces de substrat est réalisé, et
- en option, la polymérisation du/des composant(s) polymérisable(s), en option à l'aide de l'application d'une tension sur la cellule ou sous l'effet de l'action d'un champ électrique, selon une ou plusieurs étape(s) de procédé.
